# EUROPEAN PATENT SPECIFICATION

(11) **EP 1 877 073 B1**
(45) Date of publication and mention of the grant of the patent: **25.09.2013**
(21) Application number: 05810711.1
(22) Date of filing: 01.12.2005
(51) Int. Cl.: C07K 14/33

(54) **NON-CYTOTOXIC PROTEIN CONJUGATES**
NICHTZYTOTOXISCHE PROTEINKONJUGATE
CONJUGUÉS PROTÉIQUES NON CYTOTOXIQUES

(30) Priority: 01.12.2004 GB 0426394; 10.03.2005 GB 0504966; 10.03.2005 GB 0504964
(43) Date of publication of application: 16.01.2008
(60) Divisional application: 10157432.5
(73) Proprietor: The Secretary of State for Health, London SW1A 2NS (GB); ALLERGAN, INC., Irvine CA 92612 (US)
(72) Inventor: FOSTER, Keith, Salisbury, Wiltshire SP4 0JG (GB); CHADDOCK, John, Salisbury, Wiltshire SP4 0JG (GB); PENN, Charles, Salisbury, Wiltshire SP4 0JG (GB); AOKI, K., Roger, Irvine, CA 92612 (US); FRANCIS, Joseph, Irvine, CA 92612 (US); STEWARD, Lance, Irvine, CA 92612 (US)
(74) Representative: MacLean, Martin Robert
(86) International application number: PCT/GB2005/004598
(87) International publication number: WO 2006/059105

(56) References cited:
- EP-A- 1 422 240
- WO-A-2004/024909
- WO-A-2005/023309
- US-A- 5 989 545
- MOGIL J S; PASTERNAK G W: 'The molecular and behavioral pharmacology of the orphanin FQ/nociceptin peptide and receptor family' PHARMACOLOGICAL REVIEWS vol. 53, no. 3, 01 September 2001, pages 381 - 415, XP055055773 ISSN: 0031-6997

## Description

This invention relates to a non-cytotoxic protein conjugate, and to the use of said conjugate for treating pain.

Toxins may be generally divided into two groups according to the type of effect that they have on a target cell. In more detail, the first group of toxins kill their natural target cells, and are therefore known as cytotoxic toxin molecules. This group of toxins is exemplified *inter alia* by plant toxins such as ricin, and abrin, and by bacterial toxins such as diphtheria toxin, and *Pseudomonas* exotoxin A. Cytotoxic toxins typically kill their target cells by inhibiting the cellular process of protein synthesis.

In contrast, the second group of toxins, which are known as non-cytotoxic toxins, do not (as their name confirms) kill their natural target cells. Non-cytotoxic toxins have attracted much less commercial interest than have their cytotoxic counterparts, and exert their effects on a target cell by inhibiting cellular processes other than protein synthesis. As with their cytotoxic counterparts, non-cytotoxic toxins are produced from a variety of sources such as plants, and bacteria. Bacterial non-cytotoxic toxins are now described in more detail.

Clostridial neurotoxins are proteins that typically have a molecular mass of the order of 150 kDa. They are produced by various species of bacteria, especially of the genus *Clostridium,* most importantly *C. tetani* and several strains of *C. botulinum, C. butyricum* and *C. argentinense.* There are at present eight different classes of the clostridial neurotoxin, namely: tetanus toxin, and botulinum neurotoxin in its serotypes A, B, C₁, D, E, F and G, and they all share similar structures and modes of action.

Clostridial neurotoxins represent a major group of non-cytotoxic toxin molecules, and are synthesised by the host bacterium as single polypeptides that are modified post-translationally by a proteolytic cleavage event to form two polypeptide chains joined together by a disulphide bond. The two chains are termed the heavy chain (H-chain), which has a molecular mass of approximately 100 kDa, and the light chain (L-chain), which has a molecular mass of approximately 50 kDa.

L-chains possess a protease function (zinc-dependent endopeptidase activity) and exhibit high substrate specificity for vesicle and/or plasma membrane associated proteins involved in the exocytic process. L-chains from different clostridial species or serotypes may hydrolyse different but specific peptide bonds in one of three substrate proteins, namely synaptobrevin, syntaxin or SNAP-25. These substrates are important components of the neurosecretory machinery.

Non-cytotoxic toxins are also produced by other bacteria, such as from the genus *Neisseria*, most importantly from the species *N. gonorrhoeae.* For example, *Neisseria* sp. produces the non-cytotoxic toxin IgA protease (see WO99/58571).

It has been well documented in the art that toxin molecules may be re-targeted to a cell that is not the toxin's natural target cell. When so re-targeted, the modified toxin is capable of binding to a desired target cell and, following subsequent translocation into the cytosol, is capable of exerting its effect on the target cell. Said re-targeting is achieved by replacing the natural Targeting Moiety (TM) of the toxin with a different TM. In this regard, the TM is selected so that it will bind to a desired target cell, and allow subsequent passage of the modified toxin into an endosome within the target cell. The modified toxin also comprises a translocation domain to enable entry of the non-cytotoxic protease into the cell cytosol. The translocation domain can be the natural translocation domain of the toxin or it can be a different translocation domain obtained from a microbial protein with translocation activity.

For example, in the context of non-cytotoxic toxin molecules, it has been well documented that a clostridial neurotoxin may be re-targeted by incorporation of a Targeting Moiety (TM), which is not the natural TM of a clostridial neurotoxin. The described chemical conjugation and recombinant methodologies are now regarded as conventional, and reference is made to Hermanson, G.T. (1996), Bioconjugate techniques, Academic Press, and to Wong, S.S. (1991), Chemistry of protein conjugation and cross-linking, CRC Press.

For example, WO94/21300 describes modified clostridial neurotoxin molecules that are capable of regulating Integral Membrane Protein (IMP) density present at the cell surface of the target cell. The modified neurotoxin molecules are thus capable of controlling cell activity (e.g. glucose uptake) of the target cell. WO96/33273 and WO99/17806 describe modified clostridial neurotoxin molecules that target peripheral sensory afferents. The modified neurotoxin molecules are thus capable of demonstrating an analgesic effect. WO00/10598 describes the preparation of modified clostridial neurotoxin molecules that target mucus hypersecreting cells (or neuronal cells controlling said mucus hypersecreting cells), which modified neurotoxins are capable of inhibiting hypersecretion from said cells. WO01/21213 describes modified clostridial neurotoxin molecules that target a wide range of different types of non-neuronal target cells. The modified molecules are thus capable of preventing secretion from the target cells. Additional publications in the technical field of re-targeted toxin molecules include: WO00/62814; WO00/04926; US5,773,586; WO93/15766; WO00/61192; and WO99/58571.

Thus, from the above-described publications, it will be appreciated that the basic concept of re-targeting a non-cytotoxic protease to a desired target cell, by selecting a TM that has a corresponding receptor present on the target cell, has been well documented.

However, different receptors present on a target cell of interest demonstrate different binding affinities for different TMs. This may be a particular problem with pain-sensing cells, which possess a wide range of receptor types having different binding affinities for different TMs. Thus, a re-targeted conjugate comprising a particular TM (that binds to a receptor on a pain-sensing cell) may demonstrate a low binding affinity for a pain-sensing target cell, which is undesirable.

There is therefore a need to develop modified non-cytotoxic conjugates that address one or more of the above problems. Of particular interest is the development of an improved conjugate for use in treating pain.

The present invention seeks to address one or more of the above problems by using as the conjugate's Targeting Moiety (TM) an "agonist" of a receptor that is present on the pain-sensing target cell of interest. In preferred embodiments, the pain-sensing target cell is a nociceptive sensory afferent, more preferably a primary nociceptive sensory afferent. In particularly preferred embodiments, the TM is an agonist of the opioid-like receptor-1 (ORL₁) receptor.

US 5989545 describes clostridial toxin derivatives able to modify peripheral sensory afferent functions.

WO 2004/024909 and WO 98/07864 describe recombinant toxin fragments.

In a first aspect, the present invention provides a non-cytotoxic conjugate as recited in the claims.

The use of an "agonist", which would normally stimulate a biological process, particularly exocytosis (for example, an increase in cellular secretion, or an up-regulation in membrane protein expression), is an exciting development in the technical field of re-targeted toxins. Furthermore, it is particularly surprising that an agonist may be employed in a therapeutic composition to achieve a reduction or inhibition of a biological process that the agonist would normally stimulate.

The agonist-containing conjugates of the present invention represent a distinct subset of toxin conjugates. In more detail, the conjugates of the present invention comprise TMs that have been selected on the basis of specific agonist properties rather than on the simple basis that they have a corresponding receptor on a pain-sensing target cell of interest.

Conventionally, an agonist has been considered any molecule that can either increase or decrease activities within a cell, namely any molecule that simply causes an alteration of cell activity. For example, the conventional meaning of an agonist would include: a chemical substance capable of combining with a receptor on a cell and initiating a reaction or activity, or a drug that induces an active response by activating receptors, whether the response is an increase or decrease in cellular activity.

However, for the purposes of this invention, an agonist is more specifically defined as a molecule that is capable of stimulating the process of exocytic fusion in a pain-sensing target cell, which process is susceptible to inhibition by a protease (or fragment thereof) capable of cleaving a protein of the exocytic fusion apparatus in said target cell.

Accordingly, the particular agonist definition of the present invention would exclude many molecules that would be conventionally considered as agonists. For example, nerve growth factor (NGF) is an agonist in respect of its ability to promote neuronal differentiation via binding to a TrkA receptor. However, NGF is not an agonist when assessed by the above criteria because it is not a principal inducer of exocytic fusion. In addition, the process that NGF stimulates (i.e. cell differentiation) is not susceptible to inhibition by the protease activity of a non-cytotoxic toxin molecule.

In use, an agonist-containing conjugate of the present invention does not deactivate an agonist receptor on a pain-sensing target cell, but rather the protease activity of the conjugate serves to negate the agonist-mediated response.

Furthermore, once delivered to the cytosol of the pain-sensing target cell, the protease component of a conjugate of the present invention inhibits or blocks the action of all subsequent agonists capable of causing the same effect (i.e. increased exocytic fusion) in the same target cell. This is advantageous and means that the conjugates of the present invention have application in situations where multiple agonists may be responsible for causing the sensation of pain. Thus, when designing a conjugate of the present invention, the TM that is selected for delivery need not necessarily be the principal agonist involved in causing the sensation of pain.

Agonist-mediated delivery according to the present invention provides the following significant advantage over previous non-cytotoxic protease-containing therapeutics: use of an agonist may confer preferential binding and/or internalisation properties on the conjugate. This, in turn, may result in more efficient delivery of the protease component to a pain-sensing target cell.

In addition, use of an agonist as a TM is self-limiting with respect to side-effects. In more detail, binding of an agonist to a pain-sensing target cell increases exocytic fusion, which may exacerbate the sensation of pain. However, the exocytic process that is stimulated by agonist binding is subsequently reduced or inhibited by the protease component of the conjugate.

In preferred embodiments of the invention, the nociceptin TM is an agonist of the ORL₁ receptor. The ORL₁ receptor is present on pain-sensing cells in the body.

The ORL₁ receptor is a member of the G-protein-coupled class of receptors, and has a seven transmembrane domain structure. The properties of the ORL₁ receptor are discussed in detail in Mogil & Pasternak (2001), Pharmacological Reviews, Vol. 53, No. 3, pages 381-415.

Throughout this specification, reference to the "ORL₁ receptor" embraces all members of the ORL₁ receptor family. Members of the ORL₁ receptor family typically have a seven transmembrane domain structure, and are coupled to G-proteins of the Gᵢ and Go families. A method for determining the G-protein-stimulating activity of ligands of the ORL₁ receptor is given in Example 17. A method for measuring reduction in cellular cAMP levels following ORL₁ activation is given in Example 16. A further characteristic of members of the ORL₁ receptor family is that they are typically able to bind nociceptin (the natural ligand of ORL₁). As an example, all alternative splice variants of the ORL₁ receptor, are members of the ORL₁ receptor family.

The conjugates of the present invention generally demonstrate a reduced binding affinity (in the region of up to 100-fold) for nociceptive sensory afferent target cells when compared with the corresponding 'free' TM. However, despite this observation, the conjugates of the present invention surprisingly demonstrate good efficacy. This can be attributed to two principal features. First, the non-cytotoxic protease component is catalytic - thus, the therapeutic effect of a few such molecules is rapidly amplified. Secondly, the receptors present on the nociceptive sensory afferents need only act as a gateway for entry of the therapeutic, and need not necessarily be stimulated to a level required in order to achieve a ligand-receptor mediated pharmacological response. Accordingly, the conjugates of the present invention may be administered at a dosage that is much lower that would be employed for other types of analgesic molecules such as NSAIDS, morphine, and gabapentin. The latter molecules are typically administered at high microgram to milligram (even up to hundreds of milligram) quantities, whereas the conjugates of the present invention may be administered at much lower dosages, typically at least 10-fold lower, and more typically at 100-fold lower.

In a particularly preferred embodiment of the invention, the TM of the conjugate is nociceptin - the natural ligand for the ORL₁ receptor. Nociceptin targets the ORL₁ receptor with high affinity.

Examples of other preferred TMs include:

| **Code** | **Sequence** | **Ref.** | **SEQ ID No.** |
|---|---|---|---|
| Nociceptin 1-17 | FGGFTGARKSARKLANQ | [1] | **1,2** |
| Nociceptin 1-11 | FGGFTGARKSA | [1] | **3,4** |
| Nociceptin [Y10]1-11 | FGGFTGARKYA | [1] | **5,6** |
| Nociceptin [Y11]1-11 | FGGFTGARKSY | [1] | **7,8** |
| Nociceptin [Y14]1-17 | FGGFTGARKSARKYANQ | [1] | **9,10** |
| Nociceptin 1-13 | FGGFTGARKSARK | [2] | **11,12** |
| Nociceptin [R14K15] 1-17 (also known as "variant" nociceptin) | FGGFTGARKSARKRKNQ | [3,4] | **13,14** |
| Nociceptin 1-13-NH₂ | FGGFTGARKSARK-NH₂ | [5] | - |
| Nociceptin Phe (*p*-NO₂) 1-17 | (*p*NO₂)FGGFTGARKSARKLANQ | [5] | - |
| Lofentanil | Non-peptide agonists | [5] | - |
| Etorphine | Non-peptide agonists | [5] | - |
| Peptide agonist | Peptide agonists from combinatorial library approach | [6] | - |

| | | | |
|---|---|---|---|
| [1] Mogil & Pasternak, 2001, Pharmacol. Rev., 53, 381-415 [2] Maile et al., 2003, Neurosci. Lett., 350, 190-192 [3] Rizzi et al., 2002, J. Pharmacol. Exp. Therap., 300, 57-63 [4] Okada et al., 2000, Biochem. Biophys. Res. Commun., 278, 493-498 [5] Zaveri, 2003, Life Sci., 73, 663-678. [6] Dooley et al., 1997, J Pharmacol Exp Ther. 283(2), 735-41. | | | |

The TM preferably comprises a maximum of 50 amino acid residues, more preferably a maximum of 40 amino acid residues, particularly preferably a maximum of 30 amino acid residues, and most preferably a maximum of 20 amino acid residues. For example, nociceptin is a 17 amino acid residue peptide.

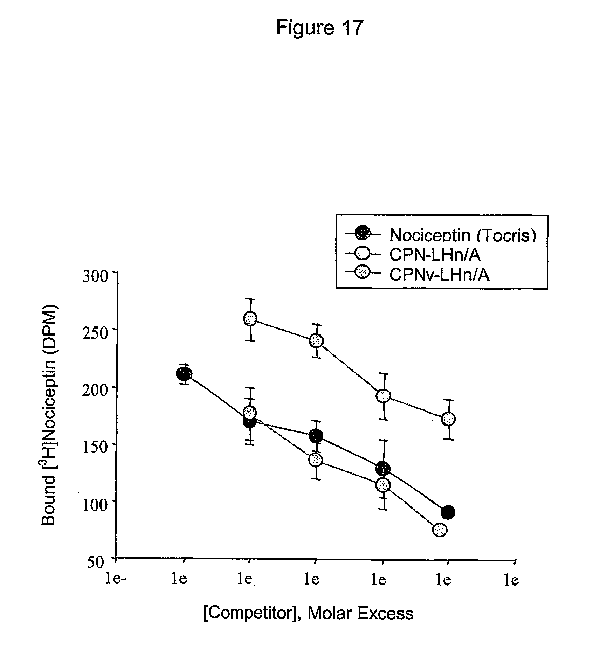

The above-identified "variant" TM demonstrates particularly good binding affinity (when compared with natural nociceptin) for nociceptive sensory afferents. Generally speaking, a TM-containing conjugate will demonstrate an approximate 100-fold reduction in binding ability *vis-à-vis* the TM *per se.* The above-mentioned "variant" TM *per se* demonstrates an approximate 3- to 10-fold increase in binding ability for a nociceptive sensory afferent *vis-à-vis* natural nociceptin. Thus, a "variant" TM-containing fusion might be expected to demonstrate an approximate 10-fold reduction in binding ability for a nociceptive sensory afferent *vis-à-vis* 'free' nociceptin. However, the present inventors have demonstrated that conjugates comprising said "variant" TM demonstrate a binding ability that (most surprisingly) closely mirrors that of 'free' nociceptin - see Figure 17.

In the context of the present invention, the term agonist of the ORL₁ receptor (such as nociceptin, or any one of the peptides listed in the table above) embraces molecules having at least 70%, preferably at least 80%, more preferably at least 90%, and most preferably at least 95% homology with said agonist. The agonist homologues retain the agonist properties of nociceptin at the ORL₁ receptor, which may be tested using the methods provided in Example 10.

The specification describes also fragments, variants, and derivatives of any one of the TMs described above. These fragments, variants, and derivatives substantially retain the properties that are ascribed to said TMs.

The agonist properties of a TM can be confirmed using the methods described in Example 1. These methods are based on previous experiments (see Inoue et al. (1998) Proc. Natl. Acad. Sci., 95, 10949-10953), which confirm that the natural agonist of the ORL₁ receptor, nociceptin, causes the induction of substance P release from nociceptive primary afferent neurons. This is supported by the facts that:
➢ the nociceptin-induced responses are abolished by specific NK1 receptor (the substance P receptor) antagonists; and
➢ pre-treatment of the cells with capsaicin (which depletes substance P from small diameter primary afferent neurons) attenuates the nociceptin-induced responses.

Similarly, Inoue *et al.* confirm that an intraplantar injection of botulinum neurotoxin type A abolishes the nociceptin-induced responses. Since it is known that BoNT inhibits the release of substance P from primary afferent neurons (Welch et al., (2000), Toxicon, 38, 245-258), this confirms the link between nociceptin-ORL₁ interaction and subsequent release of substance P.

Thus, a TM can be said to have agonist activity at the ORL₁ receptor if the TM causes an induction in the release of substance P from a nociceptive sensory afferent neuron (see Example 1).

The specification describes opioids. Within this family of peptides is included enkephalins (met and leu), endomorphins 1 and 2, β-endorphin and dynorphin. Opioid peptides are frequently used in the clinic to modify the activity to nociceptors, and other cells involved in the pain response. As exemplified by the three-step World Health Organisation Analgesic Ladder, opioids have entry points into the pharmacological treatment of chronic cancer and non-cancer pain at all three stages, underlining their importance to the treatment of pain. Reference to opioids embraces fragments, variants and derivatives thereof, which retain the ability to bind to nociceptive sensory afferents. The protease of the present invention embraces all naturally-occurring non-cytotoxic proteases that are capable of cleaving one or more proteins of the exocytic fusion apparatus in eukaryotic cells.

The protease of the present invention is preferably a bacterial protease.

More preferably, the bacterial protease is selected from the genera *Clostridium* or *Neisseria* (e.g. a clostridial L-chain, or a neisserial IgA protease preferably from *N. gonorrhoeae*).

The present invention also embraces modified non-cytotoxic proteases, which include amino acid sequences that do not occur in nature and/or synthetic amino acid residues, so long as the modified proteases still demonstrate the above-mentioned protease activity.

The protease of the present invention preferably demonstrates a serine or metalloprotease activity (e.g. endopeptidase activity). The protease is preferably specific for a SNARE protein (e.g. SNAP-25, synaptobrevin/VAMP, or syntaxin).

Particular mention is made to the protease domains of neurotoxins, for example the protease domains of bacterial neurotoxins. Thus, the present invention embraces the use of neurotoxin domains, which occur in nature, as well as recombinantly prepared versions of said naturally-occurring neurotoxins.

Exemplary neurotoxins are produced by clostridia, and the term clostridial neurotoxin embraces neurotoxins produced by *C. tetani* (TeNT), and by *C. botulinum* (BoNT) serotypes A-G, as well as the closely related BoNT-like neurotoxins produced by C. *baratii* and *C. butyricum.* The above-mentioned abbreviations are used throughout the present specification. For example, the nomenclature BoNT/A denotes the source of neurotoxin as BoNT (serotype A). Corresponding nomenclature applies to other BoNT serotypes.

The term L-chain fragment means a component of the L-chain of a neurotoxin, which fragment demonstrates a metalloprotease activity and is capable of proteolytically cleaving a vesicle and/or plasma membrane associated protein involved in cellular exocytosis.

A Translocation Domain is a molecule that enables translocation of a protease (or fragment thereof) into a pain-sensing target cell such that a functional expression of protease activity occurs within the cytosol of the target cell. Whether any molecule (e.g. a protein or peptide) possesses the requisite translocation function of the present invention may be confirmed by any one of a number of conventional assays.

For example, Shone C. (1987) describes an *in vitro* assay employing liposomes, which are challenged with a test molecule. Presence of the requisite translocation function is confirmed by release from the liposomes of K⁺ and/or labelled NAD, which may be readily monitored (see Shone C. (1987) Eur. J. Biochem; vol. 167(1): pp. 175-180).

A further example is provided by Blaustein R. (1987), which describes a simple *in vitro* assay employing planar phospholipid bilayer membranes. The membranes are challenged with a test molecule and the requisite translocation function is confirmed by an increase in conductance across said membranes (see Blaustein (1987) FEBS Letts; vol. 226, no. 1: pp. 115-120).

Additional methodology to enable assessment of membrane fusion and thus identification of Translocation Domains suitable for use in the present invention are provided by Methods in Enzymology, Vols. 220 and 221, Membrane Fusion Techniques, Parts A and B, Academic Press 1993.

The Translocation Domain is preferably capable of formation of ion-permeable pores in lipid membranes under conditions of low pH. Preferably, it has been found to use only those portions of the protein molecule capable of pore-formation within the endosomal membrane.

The Translocation Domain may be obtained from a microbial protein source, in particular from a bacterial or viral protein source. Hence, in one embodiment, the Translocation Domain is a translocating domain of an enzyme, such as a bacterial toxin or viral protein.

It is well documented that certain domains of bacterial toxin molecules are capable of forming such pores. It is also known that certain translocation domains of virally expressed membrane fusion proteins are capable of forming such pores. Such domains may be employed in the present invention.

The Translocation Domain may be of a clostridial origin, namely the H_{N} domain (or a functional component thereof). H_{N} means a portion or fragment of the H-chain of a clostridial neurotoxin approximately equivalent to the amino-terminal half of the H-chain, or the domain corresponding to that fragment in the intact H-chain. Examples of suitable clostridial Translocation Domains include:

| | |
|---|---|
| Botulinum type A neurotoxin | - amino acid residues (449-871) |
| Botulinum type B neurotoxin | - amino acid residues (441-858) |
| Botulinum type C neurotoxin | - amino acid residues (442-866) |
| Botulinum type D neurotoxin | - amino acid residues (446-862) |
| Botulinum type E neurotoxin | - amino acid residues (423-845) |
| Botulinum type F neurotoxin | - amino acid residues (440-864) |
| Botulinum type G neurotoxin | - amino acid residues (442-863) |
| Tetanus neurotoxin | - amino acid residues (458-879) |

For further details on the genetic basis of toxin production in *Clostridium botulinum* and *C. tetani,* we refer to Henderson et al. (1997) in The Clostridia: Molecular Biology and Pathogenesis, Academic press.

The term H_{N} embraces naturally-occurring neurotoxin H_{N} portions, and modified H_{N} portions having amino acid sequences that do not occur in nature and/or synthetic amino acid residues, so long as the modified H_{N} portions still demonstrate the above-mentioned translocation function.

Alternatively, the Translocation Domain may be of a non-clostridial origin (see table below). Examples of non-clostridial Translocation Domain origins include, but are not restricted to, the translocation domain of diphtheria toxin [O'Keefe et al., Proc. Natl. Acad. Sci. USA (1992) 89, 6202-6206; Silverman et al., J. Biol. Chem. (1993) 269, 22524-22532; and London, E. (1992) Biochem. Biophys. Acta., 1112, pp.25-51*],* the translocation domain of *Pseudomonas* exotoxin type A [Prior et al. Biochemistry (1992) 31, 3555-3559], the translocation domains of anthrax toxin [Blanke et al. Proc. Natl. Acad. Sci. USA (1996) 93, 8437-8442], a variety of fusogenic or hydrophobic peptides of translocating function [Plank et al. J. Biol. Chem. (1994) 269, 12918-12924; and Wagner et al (1992) PNAS, 89, pp. 7934-7938], and amphiphilic peptides [Murata et al (1992) Biochem., 31, pp.1986-1992*].* The Translocation Domain may mirror the Translocation Domain present in a naturally-occurring protein, or may include amino acid variations so long as the variations do not destroy the translocating ability of the Translocation Domain.

Particular examples of viral Translocation Domains suitable for use in the present invention include certain translocating domains of virally expressed membrane fusion proteins. For example, Wagner *et al.* (1992) and Murata *et al.* (1992) describe the translocation (i.e. membrane fusion and vesiculation) function of a number of fusogenic and amphiphilic peptides derived from the N-terminal region of influenza virus haemagglutinin. Other virally expressed membrane fusion proteins known to have the desired translocating activity are a translocating domain of a fusogenic peptide of Semliki Forest Virus (SFV), a translocating-domain of vesicular stomatitis virus (VSV) glycoprotein G, a translocating domain of SER virus F protein and a translocating domain of Foamy virus envelope glycoprotein. Virally encoded "spike proteins" have particular application in the context of the present invention, for example, the E1 protein of SFV and the G protein of VSV.

Use of the Translocation Domains (listed below) includes use of sequence variants thereof. A variant may comprise one or more conservative nucleic acid substitutions and/or nucleic acid deletions or insertions, with the proviso that the variant possesses the requisite translocating function. A variant may also comprise one or more amino acid substitutions and/or amino acid deletions or insertions, so long as the variant possesses the requisite translocating function.

| **Translocation Domain source** | **Amino acid residues** | **References** |
|---|---|---|
| Diphtheria toxin | 194-380 | Silverman et al., 1994, J. Biol. Chem. 269, 22524-22532 |
| | | London E., 1992, Biochem. Biophys. Acta., 1113, 25-51 |
| Domain II of pseudomonas exotoxin | 405-613 | Prior et al., 1992, Biochemistry 31, 3555-3559 |
| | | Kihara & Pastan, 1994, Bioconj Chem. 5, 532-538 |
| Influenza virus haemagglutinin | GLFGAIAGFIENGWE GMIDGWYG, and Variants thereof | Plank et al., 1994, J. Biol. Chem. 269, 12918-12924 |
| | | Wagner et al., 1992, PNAS, 89, 7934-7938 |
| | | Murata et al., 1992, Biochemistry 31, 1986-1992 |
| Semliki Forest virus fusogenic protein | Translocation domain | Kielian et al., 1996, J Cell Biol. 134(4), 863-872 |
| Vesicular Stomatitis virus glycoprotein G | 118-139 | Yao et al., 2003, Virology 310(2), 319-332 |
| SER virus F protein | Translocation domain | Seth et al., 2003, J Virol 77(11) 6520-6527 |
| Foamy virus envelope glycoprotein | Translocation domain | Picard-Maureau et al., 2003, J Virol. 77(8), 4722-4730 |

Once a potential receptor agonist (e.g. an ORL1 agonist) has been identified, one or more of the following optional steps may be carried out:
(A) confirming that the putative agonist molecule or agonist is capable of being combined with a non-cytotoxic protease (or a fragment thereof) and optionally a Translocation Domain to form a conjugate of the present invention; and/or
(B) confirming that said putative agonist molecule or agonist binds to the receptor on the pain-sensing target cell, which receptor is susceptible to receptor-mediated endocytosis; and/or
(C) confirming that said putative agonist molecule or agonist is able to deliver a non-cytotoxic protease (or fragment thereof) into the cytosol of a pain-sensing target cell.

The above steps (A)-(C) may be confirmed by routine tests that would be readily available to a skilled person.

For example, step (A) may be performed by a simple chemical conjugation experiment using conventional conjugation reagents and/or linker molecules, followed by native polyacrylamide gel electrophoresis to confirm that a conjugate of the present invention is formed that has the anticipated molecular weight. The conjugate components are typically linked together (optionally via linker molecules) by covalent bonds.

For example, step (B) may be performed by any one of a range of methodologies for assessment of binding of a ligand. Standard text, for example "Receptor-Ligand Interactions. A Practical Approach. Ed. E. C. Hulme, IRL Press, 1992" are available that describe such approaches in detail. In brief, the agonist or putative agonist molecule is labelled (for example, with 125-iodine) and applied to a cell preparation *in vitro* in the presence of an excess of unlabelled agonist. The purpose of the unlabelled material is to saturate any non-specific binding sites. The agonist is incubated with the cell preparation for sufficient time to achieve equilibrium, and the amount of label bound to the cells assessed by measuring cell associated radioactivity, for example by scintillation or gamma counting.

A further example involves gold-labelling of the agonist (or putative agonist), followed by the use of electron microscopy to monitor the cellular transport progress of the labelled agonist [see the basic methodology described by Rabinowitz S. (1992); J. Cell. Biol. 116(1): pp. 95-112; and that described by van Deurs (1986); J. Cell. Biol. 102: pp. 37-47].

For example, step (C) may be performed by contacting the conjugate prepared in step (A) with a suitable target cell and assessing cleavage of the substrate. This is performed by extraction of the SNARE proteins, followed by Western blotting of SDS-PAGE-separated samples. Cleavage of substrate is indicative of delivery of the protease into the target cell. In this regard, cleavage may be monitored by disappearance of substrate and/or appearance of cleavage product. A particularly useful antibody that selectively binds to the cleaved substrate product is described in WO95/33850.

Preparation of a conjugate according to the present invention is now discussed.

It is known in the art that the H_{C} portion of a neurotoxin molecule can be removed from the other portion of the H-chain, known as H_{N}, such that the H_{N} fragment remains disulphide linked to the L-chain of the neurotoxin providing a fragment known as LH_{N}. Thus, in one embodiment of the present invention the LH_{N} fragment of a neurotoxin is covalently linked, using linkages which may include one or more spacer regions, to a TM.

In another embodiment of the invention, the H_{C} domain of a neurotoxin is mutated, blocked or modified, e.g. by chemical modification, to reduce or preferably incapacitate its ability to bind the neurotoxin to receptors at the neuromuscular junction. This modified neurotoxin is then covalently linked, using linkages which may include one or more spacer regions, to a TM.

In another embodiment of the invention, the H-chain of a neurotoxin, in which the H_{c} domain is mutated, blocked or modified, e.g. by chemical modification, to reduce or preferably incapacitate its native binding ability, is combined with the L-chain of a different neurotoxin, or another protease capable of cleaving a protein of the exocytic fusion apparatus (e.g. IgA protease of *N. gonorrhoeae*). This hybrid, modified neurotoxin is then covalently linked, using linkages which may include one or more spacer regions, to a TM.

In another embodiment of the invention, the H_{N} domain of a neurotoxin is combined with the L-chain of a different neurotoxin, or another protease capable of cleaving a protein of the exocytic fusion apparatus (e.g. IgA protease of *N. gonorrhoeae*). This hybrid is then covalently linked, using linkages which may include one or more spacer regions, to a TM.

In another embodiment of the invention, the protease (for example the L-chain component of a neurotoxin) is covalently linked, using linkages that may include one or more spacer regions, to a TM that can also effect the internalisation of the protease into the cytoplasm of the relevant target cell(s).

In another embodiment of the invention, the protease (for example the L-chain component of a neurotoxin) is covalently linked, using linkages which may include one or more spacer regions, to a translocation domain to effect transport of the protease fragment into the cytosol.

In use, the domains of a conjugate according to the present invention are associated with each other. In one embodiment, two or more of the domains may be joined together either directly (e.g. by a covalent linkage), or via a linker molecule.

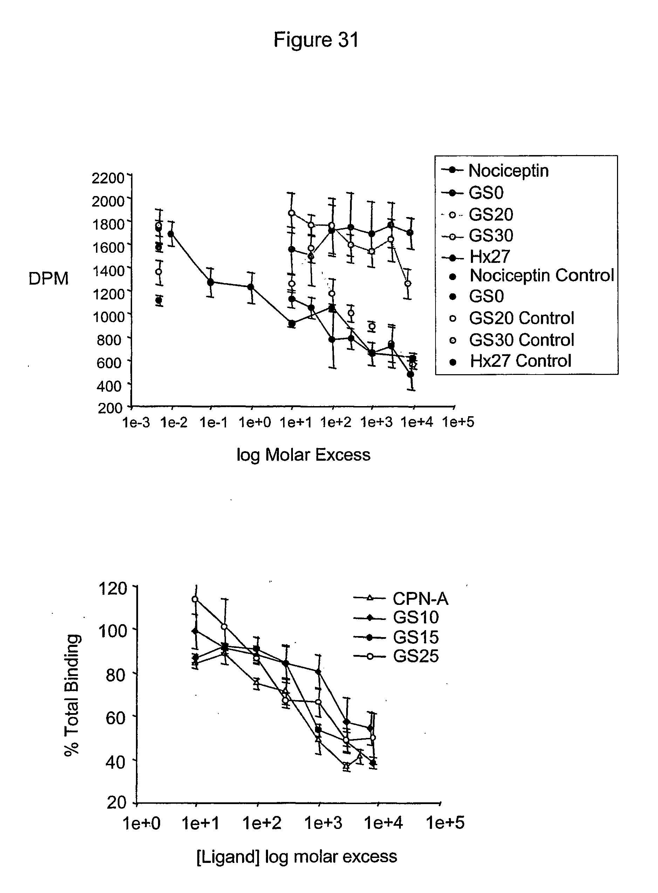

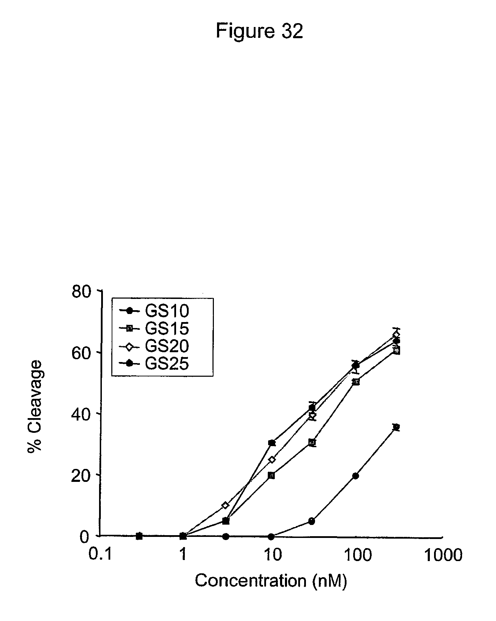

A variety of different linker/ spacer molecules may be employed in any of the fusion proteins of the present invention. Examples of such spacer molecules include those illustrated in Figures 31 and 32. Particular mention here is made to GS15, GS20, GS25, and Hx27 - see Figures 31 and 32.

The present inventors have unexpectedly found that non-cytotoxic protease-TM conjugates (eg. CPNv/A) may demonstrate an improved binding activity for nociceptive sensory afferents when the size of the spacer is selected so that (in use) the TM (preferably the C-terminus thereof) and the translocation domain (preferably the N-terminus thereof) are separated from one another by 40-105 angstroms, preferably by 50-100 angstroms, and more preferably by 50-90 angstroms. In another embodiment, the preferred spacers have an amino acid sequence of 11-29 amino acid residues, preferably 15-27 amino acid residues, and more preferably 20-27 amino acid residues. Suitable spacers may be routinely identified and obtained according to Crasto, C.J. and Feng, J.A. (2000) May, 13(5), pp. 309-312 - see also **http://www.fccc./edu/research/labs/feng/limker.html.**

Conjugation techniques suitable for use in the present invention have been well documented and are routine for a person skilled in the art.

The methodology involved in coupling two protein molecules (A and B) together is simple, and is achieved through the use of a cross-linking agent (also known as a chemical coupling agent). For example, molecules A and B are separately contacted with a cross-linking agent, which chemically-modifies a specific surface group on each of molecules A and B thereby forming derivatised molecules A' and B'. The modified surface group on molecule A' is capable of covalently bonding with the modified surface group on molecule B'. Thus, the coupling reaction is completed by mixing together the two protein molecules A' and B'.

Chemical conjugation is illustrated by reference to the following embodiments, where P = non-cytotoxic protease component, T = translocation component, and TM = targeting moiety.

In one embodiment, a single chain P - T is prepared, which is then conjugated to a TM. In another embodiment, a single chain TM-T (or T-TM) is prepared, which is then conjugated to a P. In a further embodiment, a single chain P - TM (or TM - P) is prepared, which is then conjugated to a T. Another particularly preferred conjugate has the structure P - TM - T (with an optional protease cleavage site between P and TM).

Where the T and P components are prepared as a single chain polypeptide, a protease cleavage site is typically included between said components. Any protease cleavage site may be employed in this regard.

In an alternative embodiment, the three components may be simultaneously or sequentially conjugated together. Thus, the conjugation may be a one- or two-step process, and may include one or more different coupling agents.

Chemical coupling agents and cross-linking agents have been commercially available for many years.

Example 5 of the present invention describes in detail the use of one such coupling agent, namely SPDP, to chemically couple two protein molecules (nociceptin, and the LH_{N} of botulinum neurotoxin). The two molecules are separately contacted with SPDP, and then mixed together to allow covalent conjugation.

The conjugate described in Example 6 confirms that another coupling agent, PDPH/EDAC, or Traut's reagent, may be employed as an alternative coupling agent to SPDP.

SPDP and Traut's reagent are popular and well-documented coupling agents in the technical field of protein conjugation chemistry and are presented here simply as two examples of a well known class of compounds that may be employed to covalently link together the Targeting Moiety component and the clostridial neurotoxin component of the conjugate of the present invention. Other suitable agents include SMPB, SMCC (succinimidyl 4-(N-maleimidomethyl) cyclohexan-1-carboxylate), and LC-SPDP.

In more detail, commercially available members of the well-known coupling agents may be used for conjugation purposes to produce a conjugate of the invention. Details of such agents can be found in the following publications:
Hermanson, G.T. (1996), Bioconjugate techniques, Academic Press;
Wong, S.S. (1991), Chemistry of protein conjugation and cross-linking, CRC Press;
Thorpe et al (1987), Cancer Res, 1987, 47, 5924-31. This paper describes the use of SMBT (sodium S-4-succinimidyloxycarbonyl-alpha-methyl benzyl thiosulfate) and SMPT (4-succinimidyloxycarbonyl-alpha-methyl-alpha(2-pyridyldithio)toluene); and
Peeters et al (1989), J Immunol Methods. 1989, 120, 133-43. This paper describes the use of 4 coupling reagents, MHS (succinimidyl 6-(N-maleimido)-n-hexanoate), SMCC (succinimidyl 4-(N-maleimidomethyl)-cyclohexane-1-carboxylate), MBS (succinimidyl m-maleimidobenzoate), and SPDP.

The conjugates according to the present invention may also be prepared recombinantly, as detailed in Examples 9 to 12.

In one embodiment, the preparation of a recombinant conjugate involves arrangement of the coding sequences of a selected TM, a selected non-cytotoxic protease component, and a translocation component (in any order) in a single genetic construct. These coding sequences may be arranged in-frame so that subsequent transcription and translation is continuous through both coding sequences and results in a fusion protein. All constructs would have a 5' ATG codon to encode an N-terminal methionine, and a C-terminal translational stop codon.

Thus, the recombinant preparation method results in the generation of a single chain polypeptide. In order to activate this polypeptide, a protease cleavage site is present between the non-cytotoxic protease component and the translocation component. Cleavage of this site generates a di-chain polypeptide in which the protease and translocation domains are linked together by way of a covalent bond, preferably a disulphide bond. In this regard, any protease cleavage site may be employed.

In the single polypeptide aspect of the present invention, the TM is preferably either N- or C-terminally located with respect to the fusion protein. In other words, it is preferred that the TM is not located between the P and T components of the single polypeptide fusion protein. In a particularly preferred embodiment, the TM is N-terminally located with respect to the fusion protein.

In one embodiment, an L-chain of a clostridial neurotoxin or another protease capable of cleaving a protein of the exocytic fusion apparatus (e.g. an IgA protease), or a fragment/variant thereof, may be expressed recombinantly as a fusion protein with a TM, which TM can also effect the internalisation of the L-chain component into the cytoplasm of the relevant target cell(s) responsible for secretion. Alternatively, the fusion protein may further comprise a Translocation Domain. The expressed fusion protein may include one or more spacer regions.

By way of example, the following information is required to produce, recombinantly, an agent of the present invention:
(I) DNA sequence data relating to a selected TM;
(II) DNA sequence data relating to the protease component;
(III) DNA sequence data relating to the translocation domain; and
(IV) a protocol to permit construction and expression of the construct comprising (I), (II) and (III).

All of the above basic information (I)-(IV) are either readily available, or are readily determinable by conventional methods. For example, both WO98/07864 and WO99/17806 exemplify recombinant technology suitable for use in the present application.

In addition, methods for the construction and expression of the constructs of the present invention may employ information from the following references and others:
Lorberboum-Galski, H., FitzGerald, D., Chaudhary, V., Adhya, S., Pastan, I. (1988), Cytotoxic activity of an interleukin 2-Pseudomonas exotoxin chimeric protein produced in Escherichia coli. Proc. Natl. Acad. Sci. USA, 85(6):1922-6;
Murphy, J.R. (1988), Diphtheria-related peptide hormone gene fusions: a molecular genetic approach to chimeric toxin development. Cancer Treat. Res.; 37:123-40;
Williams, D.P., Parker, K., Bacha, P., Bishai, W., Borowski, M., Genbauffe, F., Strom, T.B., Murphy, J.R. (1987), Diphtheria toxin receptor binding domain substitution with interleukin-2: genetic construction and properties of a diphtheria toxin-related interleukin-2 fusion protein. Protein Eng;1 (6):493-8;
Arora, N., Williamson, L.C., Leppla, S.H., Halpern, J.L. (1994), Cytotoxic effects of a chimeric protein consisting of tetanus toxin light chain and anthrax toxin lethal factor in non-neuronal cells J. Biol. Chem., 269(42):26165-71;
Brinkmann, U., Reiter, Y., Jung, S.H., Lee, B., Pastan, I. (1993), A recombinant immunotoxin containing a disulphide-stabilized Fv fragment. Proc. Natl. Acad. Sci. USA, 90(16):7538-42; and
O'Hare, M., Brown, A.N., Hussain, K., Gebhardt, A., Watson, G., Roberts, L.M., Vitetta, E.S., Thorpe, P.E., Lord, J.M. (1990), Cytotoxicity of a recombinant ricin-A-chain fusion protein containing a proteolytically-cleavable spacer sequence. FEBS Lett Oct 29;273(1-2):200-4.

Suitable clostridial neurotoxin sequence information relating to L- and LH_{N}-chains may be obtained from, for example, Kurazono, H. (1992) J. Biol. Chem., vol. 267, No. 21, pp.14721-14729; and Popoff, M.R., and Marvaud, J.-C. (1999) The Comprehensive Sourcebook ofBacterial Protein Toxins, 2nd edition (ed. Alouf, J.E., and Freer, J.H.), Academic Press, pp.174-201.

Similarly, suitable TM sequence data are widely available in the art. Alternatively, any necessary sequence data may be obtained by techniques which are well-known to the skilled person.

For example, DNA encoding the TM component may be cloned from a source organism by screening a cDNA library for the correct coding region (for example by using specific oligonucleotides-based on the known sequence information to probe the library), isolating the TM DNA, sequencing this DNA for confirmation purposes, and then placing the isolated DNA in an appropriate expression vector for expression in the chosen host.

As an alternative to isolation of the sequence from a library, the available sequence information may be employed to prepare specific primers for use in PCR, whereby the coding sequence is then amplified directly from the source material and, by suitable use of primers, may be cloned directly into an expression vector.

Another alternative method for isolation of the coding sequence is to use the existing sequence information and synthesise a copy, possibly incorporating alterations, using DNA synthesis technology. For example, DNA sequence data may be generated from existing protein and/or RNA sequence information. Using DNA synthesis technology to do this (and the alternative described above) enables the codon bias of the coding sequence to be modified to be optimal for the chosen expression host. This may give rise to superior expression levels of the fusion protein.

Optimisation of the codon bias for the expression host may be applied to the DNA sequences encoding the TM and clostridial components of the construct. Optimisation of the codon bias is possible by application of the protein sequence into freely available DNA/protein database software, e.g. programs available from Genetics Computer Group, Inc.

Having prepared a conjugate of the invention, it is a matter of routine to confirm that the various domains have retained their specified function.

Protease function after conjugation may be tested by using, for example, any one of the following routine tests:
SNAP-25 (or synaptobrevin, or syntaxin) may be challenged with a conjugate to be tested, and then analysed by SDS-PAGE peptide separation techniques. Subsequent detection of peptides (e.g. by silver staining) having molecular weights corresponding to the cleaved products of SNAP-25 (or other component of the neurosecretory machinery) would confirm the presence of a functional L-chain.

As a further alternative, the conjugate may be tested by assaying for SNAP-25 (or synaptobrevin, or syntaxin) cleavage products via antibody-specific binding (see WO95/33850). In more detail, a specific antibody is employed for detecting cleavage of SNAP-25. Since the antibody recognises cleaved SNAP-25, but not uncleaved SNAP-25, identification of the cleaved product by the antibody confirms the presence of L-chain proteolytic function. By way of exemplification, such a method is described in Examples 2 and 3 of WO96/33273.

Translocation component function after conjugation may be tested using, for example, any one of the following routine tests:
Suitable methods are, for example, described by Shone et al. (1987) Eur. J. Biochem. 167, pp.175-180; and by Blaustein et al. (1987) FEBS 226 (1), pp.115-120.

The Shone *et al.* method employs artificial liposomes loaded with potassium phosphate buffer (pH 7.2) and radiolabelled NAD. Release of K+ and NAD from the liposomes correlates with a positive result for channel forming activity and hence translocation activity. In this regard, K+ release from liposomes may be measured using an electrode and NAD release calculated by measuring the radioactivity in the supernatant (see page 176, column 1, line 33 - column 2, line 17).

The Blaustein *et al.* method employs planar phospholipid bilayer membranes, which are used to test for channel forming activity. In more detail, salt solutions on either side of the membrane are buffered at a different pH - on the cis side, pH 4.7 or 5.5 and on the trans side, pH 7.4. The "conjugate" to be tested is added to the cis side of the membrane and electrical measurements are made under voltage clamp conditions, in order to monitor the flow of current across the membrane (see paragraph 2.2, pages 116-118). The presence of an active translocation function is confirmed by a steady rate of channel turn-on (i.e. a positive result for channel formation) -see paragraph 3, page 118.

Targeting Moiety (TM) function after conjugation may be tested by assaying for the agonist function inherent to the TM. Suitable methods include those described in Example 1.

The ability of the conjugate of the invention to inhibit substance P release from nociceptive afferent cells can be assessed using the methods described in --Example 15.

In Example 15, a nociceptin-LHN/A conjugate according to the first aspect of the invention is assessed for its ability to inhibit the release of substance P from primary nociceptive sensory afferent neurons. As can be seen from Table 1, incubation of the conjugate with cultures of nociceptive afferent neurons results in a significant inhibition of release of substance P (when compared to incubation of the cells with LHN/A alone). The experiment therefore confirms that the conjugate is inhibiting substance P release from these cells.

In use of the present invention, a pain-sensing target cell is selected in which it is desired to reduce or inhibit the process of exocytic fusion, which exocytic process contributes to the symptoms associated with the sensation of pain. For example, the target cell in question may demonstrate an undesirable phenotype (e.g. an undesirable secretion, or the expression of an undesirable concentration of membrane receptor, transporter or membrane channel), which contributes to the symptoms associated with pain. Alternatively, a target cell may be selected in which the process of exocytic fusion contributes to the sensation of pain.

In preferred embodiments of the invention, the target cell is a nociceptive sensory afferent cell, preferably a primary nociceptive afferent cell (e.g. an A-fibre such as an Aδ-fibre or a C-fibre). Thus, the conjugates of the present invention are capable of inhibiting neurotransmitter or neuromodulator (e.g. glutamate, substance P, calcitonin-gene related peptide (CGRP), and/or neuropeptide Y) release from discrete populations of nociceptive sensory afferent neurons. In use, the conjugates reduce or prevent the transmission of sensory afferent signals (e.g. neurotransmitters or neuromodulators) from peripheral to central pain fibres, and therefore have application as therapeutic molecules for the treatment of pain, in particular chronic pain.

It is routine to confirm that a TM binds to a nociceptive sensory afferent. For example, a simple radioactive displacement experiment may be employed in which tissue or cells representative of the nociceptive sensory afferent (for example DRGs) are exposed to labelled (e.g. tritiated) ligand in the presence of an excess of unlabelled ligand. In such an experiment, the relative proportions of non-specific and specific binding may be assessed, thereby allowing confirmation that the ligand binds to the nociceptive sensory afferent target cell. Optionally, the assay may include one or more binding antagonists, and the assay may further comprise observing a loss of ligand binding. Examples of this type of experiment can be found in Hulme, E.C. (1990), Receptor-binding studies, a brief outline, pp 303-311, in Receptor biochemistry, A Practical Approach, Ed. E.C. Hulme, Oxford University Press.

According to a second aspect, the present invention provides a pharmaceutical composition comprising a conjugate according to the first and/or second aspect of the present invention.

The pharmaceutical composition may further comprise a pharmaceutically-acceptable carrier, and/or a suitable diluent and/or excipient, although the exact form of the composition may be tailored to the mode of administration. Administration is preferably to a mammal, more preferably to a human.

The components of the composition may, for example, be employed in the form of an aerosol or nebulisable solution for inhalation or a sterile solution for parenteral administration, intra-articular administration or intra-cranial administration.

The composition may also be administered by i.v. injection, which includes the use of pump systems. Spinal injection (e.g. epidural or intrathecal) or indwelling pumps may also be used.

The dosage ranges for administration of the components of the present invention are those to produce the desired therapeutic effect. It will be appreciated that the dosage range required depends on the precise nature of the components, the route of administration, the nature of the formulation, the age of the patient, the nature, extent or severity of the patient's condition, contraindications, if any, and the judgement of the attending physician.

Suitable daily dosages (for each component) are in the range 0.0001-1 mg/kg, preferably 0.0001-0.5 mg/kg, more preferably 0.002-0.5 mg/kg, and particularly preferably 0.004-0.5 mg/kg. The unit dosage can vary from less that 1 microgram to 30 mg, but typically will be in the region of 0.01 to 1 mg per dose, which may be administered daily or preferably less frequently, such as weekly or six monthly.

A particularly preferred dosing regimen is based on 2.5 ng of fusion protein (e.g. CPNv/A) as the 1X dose. In this regard, preferred dosages are in the range 1X-100X (i.e. 2.5-250 ng). This dosage range is significantly lower (i.e. at least 10-fold, typically 100-fold lower) than would be employed with other types of analgesic molecules such as NSAIDS, morphine, and gabapentin. Moreover, the above-mentioned difference is considerably magnified when the same comparison is made on a molar basis - this is because the fusion proteins of the present invention have a considerably greater Mw than do conventional 'small' molecule therapeutics.

Wide variations in the required dosage, however, are to be expected depending on the precise nature of the components, and the differing efficiencies of various routes of administration. For example, oral administration would be expected to require higher dosages than administration by intravenous injection.

Variations in these dosage levels can be adjusted using standard empirical routines for optimisation, as is well understood in the art.

Compositions suitable for injection may be in the form of solutions, suspensions or emulsions, or dry powders which are dissolved or suspended in a suitable vehicle prior to use.

Fluid unit dosage forms are typically prepared utilising a pyrogen-free sterile vehicle.

The active ingredients, depending on the vehicle and concentration used, can be either dissolved or suspended in the vehicle.

Solutions may be used for all forms of parenteral administration, and are particularly used for intravenous injection. In preparing solutions the components can be dissolved in the vehicle, the solution being made isotonic if necessary by addition of sodium chloride and sterilised by filtration through a sterile filter using aseptic techniques before filling into suitable sterile vials or ampoules and sealing. Alternatively, if solution stability is adequate, the solution in its sealed containers may be sterilised by autoclaving.

Advantageously additives such as buffering, solubilising, stabilising, preservative or bactericidal, suspending or emulsifying agents and/or local anaesthetic agents may be dissolved in the vehicle.

Dry powders which are dissolved or suspended in a suitable vehicle prior to use may be prepared by filling pre-sterilised drug substance and other ingredients into a sterile container using aseptic technique in a sterile area.

Alternatively the components of the composition may be dissolved in an aqueous vehicle, the solution is sterilized by filtration and distributed into suitable containers using aseptic technique in a sterile area. The product is then freeze-dried and the containers are sealed aseptically.

Parenteral suspensions, suitable for intramuscular, subcutaneous or intradermal injection, are prepared in substantially the same manner, except that the sterile components are suspended in the sterile vehicle, instead of being dissolved and sterilisation cannot be accomplished by filtration. The components may be isolated in a sterile state or alternatively it may be sterilised after isolation, e.g. by gamma irradiation.

Advantageously, a suspending agent for example polyvinylpyrrolidone is included in the composition(s) to facilitate uniform distribution of the components.

Compositions suitable for administration via the respiratory tract include aerosols, nebulisable solutions or microfine powders for insufflation. In the latter case, particle size of less than 50 microns, especially less than 10 microns, is preferred. Such compositions may be made up in a conventional manner and employed in conjunction with conventional administration devices.

The compositions described in this invention can be used in vivo, either directly or as a pharmaceutically acceptable salt, for the treatment of conditions involving exocytosis (for example secretion, or the delivery of proteins such as receptors, transporters, and membrane channels to the plasma membrane of a cell).

According to a third aspect, the present invention provides a DNA construct that encodes a conjugate according to the first or second aspects of the invention.

By expressing the construct in a host cell, conjugates of the invention may be prepared.

According to a fourth aspect, the present invention provides for the use of a conjugate, composition or construct according to the first to fourth aspects of the invention, for the manufacture of a medicament for treating pain, preferably chronic pain.

### Definitions Section

Exocytic fusion is a process by which intracellular molecules are transported from the cytosol of a pain-sensing target cell to the plasma (i.e. cell) membrane thereof. Thereafter, the intracellular molecules may become displayed on the outer surface of the plasma membrane, or may be secreted into the extracellular environment.

In a healthy individual, the rate of exocytic fusion is carefully regulated and allows control of the transport of molecules between the cytosol and the plasma membrane of a pain-sensing cell. For example, regulation of the exocytic cycle allows control of the density of receptors, transporters, or membrane channels present at the cell's surface, and/or allows control of the secretion rate of intracellular components (e.g. neurotransmitters) from the cytosol of the cell.

However, in an unhealthy individual, the regulation of exocytic fusion may be modified. For example, exocytic fusion may cause affected pain-sensing cells to enter a state of hypersecretion. Alternatively, exocytic fusion may result in the display of an increased concentration of receptors, transporters, or membrane channels present on the surface of the pain-sensing, which may expose the cell to undesirable external stimuli. Thus, the process of exocytic fusion may contribute to the progression and/or severity of pain, and therefore provides a target for therapeutic intervention.

It should also be appreciated that otherwise normal rates of cellular exocytic fusion may contribute to the progression and severity of pain in compromised patients. Thus, by targeting exocytic fusion in accordance with the present invention, it is also possible to provide therapy in such patients

Targeting Moiety (TM) means any chemical structure associated with a conjugate that functionally interacts with a receptor, e.g. an ORL₁ receptor, to cause a physical association between the conjugate and the surface of a pain-sensing target cell. The term TM embraces any molecule (i.e. a naturally occurring molecule, or a chemically/physically modified variant thereof) that is capable of binding to a receptor on the target cell, which receptor is capable of internalisation (e.g. endosome formation) also referred to as receptor-mediated endocytosis. The TM may possess an endosomal membrane translocation domain, in which case separate TM and Translocation Domain components need not be present in an agent of the present invention.

The term "fragment" means a peptide having at least thirty-five, preferably at least twenty-five, more preferably at least fifteen, and most preferably at least ten amino acid residues of the TM in question. In one embodiment, the first amino acid residue of the fragment is the N-terminal amino acid residue of the TM from which the fragment has been derived.

An example of a "variant" is a peptide or peptide fragment of a TM that contains one or more analogues of an amino acid (e.g. an unnatural amino acid), or a substituted linkage.

A "derivative" comprises the TM in question, and a further peptide sequence. The further peptide sequence should preferably not interfere with the basic folding and thus conformational structure of the TM. Two or more peptides (or fragments, or variants) may be joined together to form a derivative. Alternatively, a peptide (or fragment, or variant) may be joined to an unrelated molecule (e.g. a second, unrelated peptide). Derivatives may be chemically synthesized, but will be typically prepared by recombinant nucleic acid methods. Additional components such as lipid, and/or polysaccharide, and/or polyketide components may be included.

The term non-cytotoxic means that the protease molecule in question does not kill the pain-sensing target cell to which it has been re-targeted.

The "protease cleavage site" of the present invention allows cleavage (preferably controlled cleavage) of the conjugate at a position between the non-cytotoxic protease component and the TM component. In one embodiment, the conjugate may include more than one proteolytic cleavage site. However, where two or more such sites exist, they are different, thereby substantially preventing the occurrence of multiple cleavage events in the presence of a single protease. In another embodiment, it is preferred that the conjugate has a single protease cleavage site. The protease cleavage sequence(s) may be introduced (and/or any inherent cleavage sequence removed) at the DNA level by conventional means, such as by site-directed mutagenesis. Screening to confirm the presence of cleavage sequences may be performed manually or with the assistance of computer software (e.g. the MapDraw program by DNASTAR, Inc.).

Whilst any protease cleavage site may be employed, the following are preferred:

| | |
|---|---|
| Enterokinase | (DDDDK↓) |
| Factor Xa | (IEGR↓ / IDGR↓) |
| TEV(Tobacco Etch virus) | (ENLYFQ↓G) |
| Thrombin | (LVPR↓GS) |
| PreScission | (LEVLFQ↓GP). |

Also embraced by the term protease cleavage site is an intein, which is a self-cleaving sequence. The self-splicing reaction is controllable, for example by varying the concentration of reducing agent present.

The present invention is now described by reference to the following Examples and Figures, without intended limitation thereto.

### Figures

- Figure 1: Expression and purification of *rec*LH_{N}/B fusion protein
- Figure 2: Expression and purification of LH_{N}/C fusion protein
- Figure 3: Expression and purification of N[1-17]-LH_{N}/A fusion protein
- Figure 4: Purification of a LC/A-nociceptin-H_{N}/A fusion protein
- Figure 5: Purification of a nociceptin-LC/A-H_{N}/A fusion protein
- Figure 6: Purification of a LC/C-nociceptin-H_{N}/C fusion protein
- Figure 7: Purification of a LC/A-met enkephalin-H_{N}/A fusion protein
- Figure 8: Comparison of binding efficacy of a LC/A-nociceptin-H_{N}/A fusion protein and a nociceptin-LC/A-H_{N}/A fusion protein
- Figure 9: *In vitro* catalytic activity of a LC/A-nociceptin-H_{N}/A fusion protein
- Figure 10: Purification of a LC/A-nociceptin variant-H_{N}/A fusion protein
- Figure 11: Comparison of binding efficacy of a LC/A-nociceptin-H_{N}/A fusion protein and a LC/A-nociceptin variant-H_{N}/A fusion protein
- Figure 12: Expressed / purified LC/A-nociceptin-H_{N}/A fusion protein family with variable spacer length product(s)
- Figure 13: Inhibition of SP release and cleavage of SNAP-25 by CPN-A
- Figure 14: Inhibition of SP release and cleavage of SNAP-25 over extended time periods after exposure of DRG to CPN-A
- Figure 15: Cleavage of SNAP-25 by CPNv-A
- Figure 16: Cleavage of SNAP-25 over extended time periods after exposure of DRG to CPNv-A
- Figure 17: CPNv-A fusion-mediated displacement of [3H]-nociceptin binding
- Figure 18: Expressed / purified CPNv(Ek)-A product
- Figure 19: Cleavage of SNAP-25 by CPNv(Ek)-A
- Figure 20: Expressed / purified CPNv-C product
- Figure 21: Cleavage of syntaxin by CPNv-C
- Figure 22: CPN-A efficacy in the Acute Capsaicin-Induced Mechanical Allodynia model
- Figure 23: CPN-A efficacy in the Streptozotocin (STZ)-Induced Peripheral Diabetic Neuropathy (Neuropathic Pain) model
- Figure 24: CPNv-A efficacy in the Acute Capsaicin-Induced Mechanical Allodynia model
- Figure 25: Expressed / purified LC/A-CPLE-H_{N}/A product
- Figure 26: Expressed / purified LC/A-CPBE-H_{N}/A product
- Figure 27: Expressed / purified CPOP-A product
- Figure 28: Expressed / purified CPOPv-A product
- Figure 29: *In vitro* SNAP-25 cleavage in a DRG cell model
- Figure 30: Expressed / purified CPNv-A-FXa-HT (removable his-tag)
- Figure 31: *In vitro* efficacy of LC/A-nociceptin-H_{N}/A fusion proteins with variable spacer length, as assessed by ligand competition assay
- Figure 32: *In vitro* efficacy of LC/A-nociceptin-H_{N}/A fusion proteins with variable spacer length, as assessed by *in vitro* SNAP-25 cleavage

The Figures are now described in more detail.

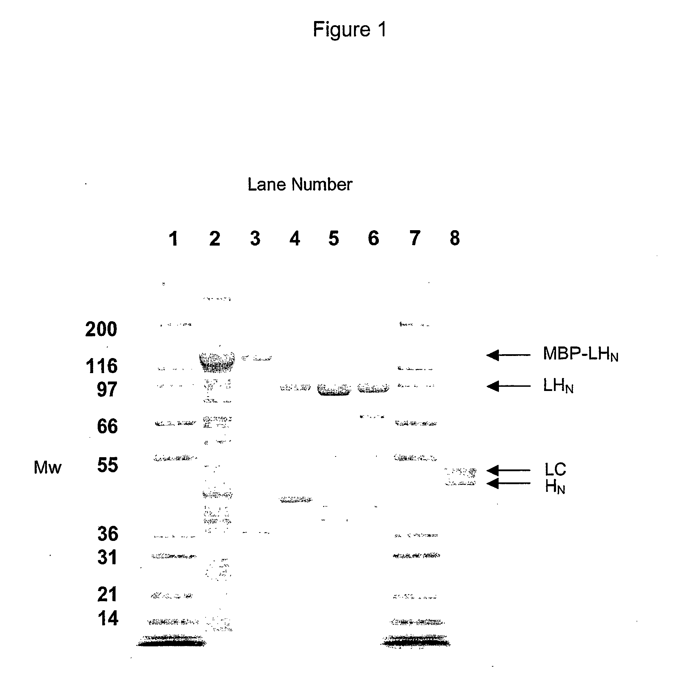

### Figure 1 - Expression and purification of recLH_{N}/B fusion protein

SDS-PAGE analysis of expression and purification of *rec*LH_{N}/B from *E. coli.* In Figure1, *rec*LH_{N}/B is purified from cell paste using a three column strategy as described in Example 3. Protein samples are separated by SDS-PAGE and visualised by staining with simplyblue safestain coomassie reagent. Crude, soluble MBP-LH_{N}/B fusion protein contained within the clarified extract (lane 2) is loaded onto Q-Sepharose FF anion-exchange resin. Lane 3 represents recombinant MBP-LH_{N}/B fusion eluted from column at 150-200 mM salt. This sample is treated with factor Xa protease to remove MBP affinity tag (lane 4), and cleaved mixture diluted to lower salt concentration prior to loading onto a Q-Sepharose FF anion-exchange column. Material eluted between 120-170 mM salt was rich in LH_{N}/B (lane 5). Protein in lanes 6 and 8 represents LH_{N}/B harvested after treatment with enterokinase and final purification using Benzamidine Sepharose, under non-reducing and reducing conditions respectively. Lanes 1 and 7 represent molecular mass markers [Mark 12 (Invitrogen)].

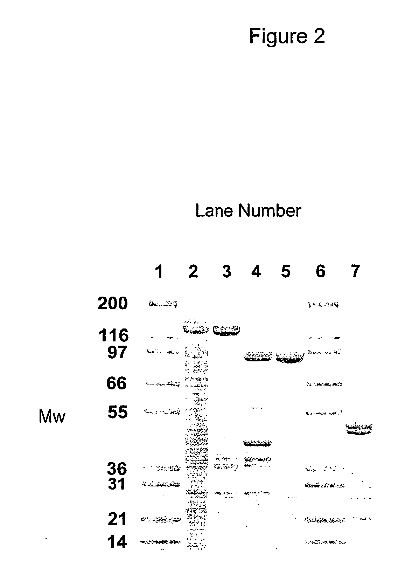

### Figure 2 - Expression and purification of LH_{N}/C fusion protein

SDS-PAGE analysis of expression and purification of LH_{N}/C from *E. coli.* In Figure 2, *rec*LH_{N}/C is purified from *E. coli* cell paste using a two-step strategy described in Example 4. Protein samples are separated by SDS-PAGE and visualised by staining with coomassie blue. Clarified Crude cell lysate (lane 2) is loaded onto Q-Sepharose FF anion-exchange resin. Fusion protein, MBP-LH_{N}/C is eluted with 0.1 M NaCl (lane 3). Eluted material incubated at 22°C for 16 h with factor Xa protease (New England Biolabs) to cleave fusion tag MBP and nick *rec*LH_{N}/C at the linker site. The protein of interest is further purified from cleaved fusion products (lane 4) using Q-Sepharose FF. Lanes 5 and 7 show purified *rec*LH_{N}/C under non-reducing conditions and reduced with 10 mM DTT respectively, to illustrate disulphide bonding at the linker region between LC and H_{N} domains after nicking with factor Xa. Lanes 1 and 6 represent molecular mass markers (shown in KDa); Mark 12 (Invitrogen).

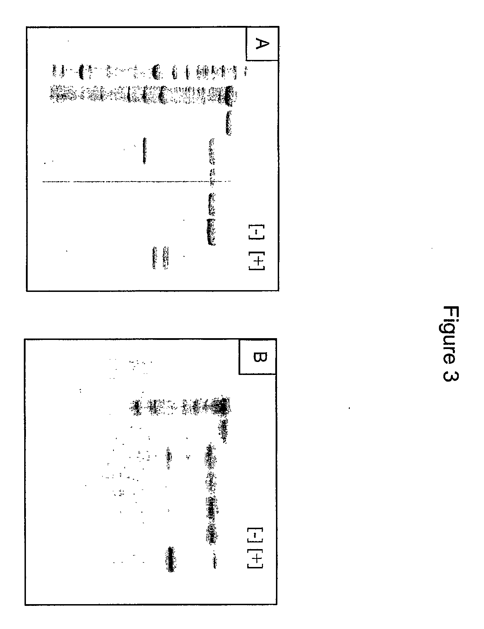

### Figure 3 - Expression and purification of N[1-17]-LH_{N}/A fusion protein

SDS-PAGE analysis of expression and purification of N[1-17]-LH_{N}/A from *E. coli.* In Figure 3, N[1-17]-LH_{N}/A is purified from *E. coli* BL21 cell paste using the methodology outlined in Example 9. Briefly, the soluble products obtained following cell disruption were applied to a nickel-charged affinity capture column. Bound proteins were eluted with 100 mM imidazole, treated with Factor Xa to activate the fusion protein and remove the maltose-binding protein (MBP) tag, then re-applied to a second nickel-charged affinity capture column. Samples from the purification procedure were assessed by SDS-PAGE (Panel A) and Western blotting (Panel B). Anti-nociceptin antisera (obtained from Abcam) were used as the primary antibody for Western blotting. The final purified material in the absence and presence of reducing agent is identified in the lanes marked [-] and [+] respectively.

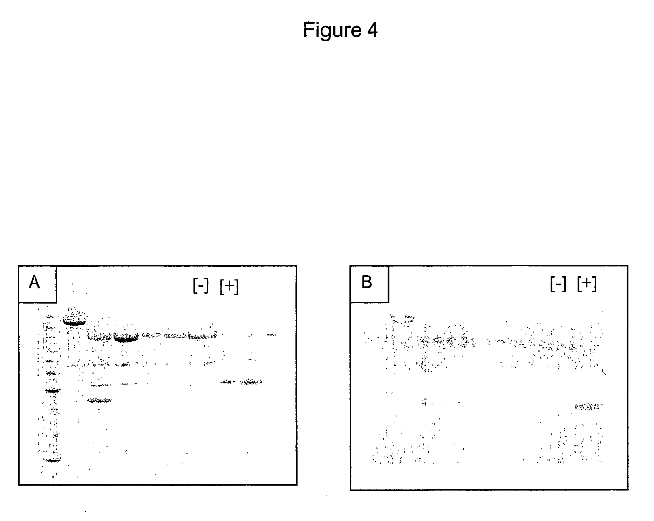

### Figure 4 - Purification of a LC/A-nociceptin-H_{N}/A fusion protein

Using the methodology outlined in Example 26, a LC/A-nociceptin-H_{N}/A fusion protein was purified from *E. coli* BL21 cells. Briefly, the soluble products obtained following cell disruption were applied to a nickel-charged affinity capture column. Bound proteins were eluted with 100 mM imidazole, treated with Factor Xa to activate the fusion protein and remove the maltose-binding protein (MBP) tag, then re-applied to a second nickel-charged affinity capture column. Samples from the purification procedure were assessed by SDS-PAGE (Panel A) and Western blotting (Panel B). Anti-nociceptin antisera (obtained from Abcam) were used as the primary antibody for Western blotting. The final purified material in the absence and presence of reducing agent is identified in the lanes marked [-] and [+] respectively.

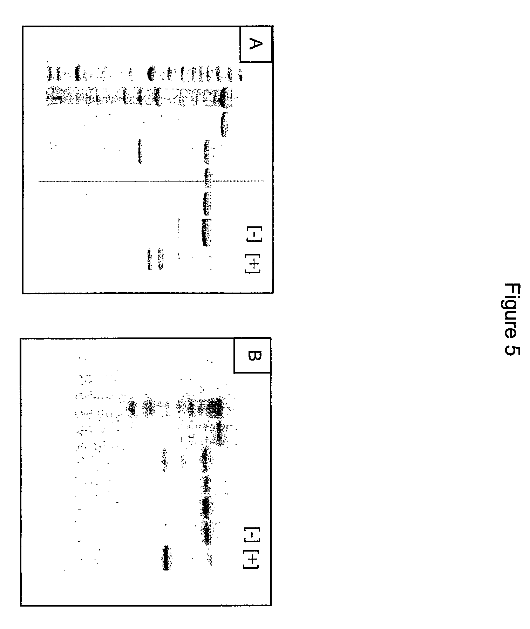

### Figure 5 - Purification of a nociceptin-LC/A-H_{N}/A fusion protein

Using the methodology outlined in Example 26, a nociceptin-LC/A-H_{N}/A fusion protein was purified from *E. coli* BL21 cells. Briefly, the soluble products obtained following cell disruption were applied to a nickel-charged affinity capture column. Bound proteins were eluted with 100 mM imidazole, treated with Factor Xa to activate the fusion protein and remove the maltose-binding protein (MBP) tag, then re-applied to a second nickel-charged affinity capture column. Samples from the purification procedure were assessed by SDS-PAGE (Panel A) and Western blotting (Panel B). Anti-nociceptin antisera (obtained from Abcam) were used as the primary antibody for Western blotting. The final purified material in the absence and presence of reducing agent is identified in the lanes marked [-] and [+] respectively.

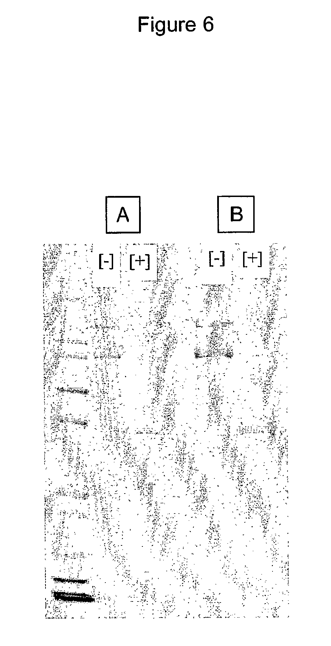

### Figure 6 - Purification of a LC/C-nociceptin-H_{N}/C fusion protein

Using the methodology outlined in Example 26, an LC/C-nociceptin-H_{N}/C fusion protein was purified *from E. coli* BL21 cells. Briefly, the soluble products obtained following cell disruption were applied to a nickel-charged affinity capture column. Bound proteins were eluted with 100 mM imidazole, treated with Factor Xa to activate the fusion protein and remove the maltose-binding protein (MBP) tag, then re-applied to a second nickel-charged affinity capture column. Samples from the purification procedure were assessed by SDS-PAGE (Panel A) and Western blotting (Panel B). Anti-nociceptin antisera (obtained from Abcam) were used as the primary antibody for Western blotting. The final purified material in the absence and presence of reducing agent is identified in the lanes marked [-] and [+] respectively.

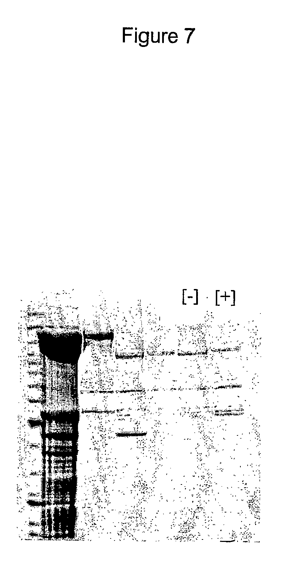

### Figure 7 - Purification of a LC/A-met enkephalin-H_{N}/A fusion protein

Using the methodology outlined in Example 26, an LC/A-met enkephalin-H_{N}/A fusion protein was purified from *E. coli* BL21 cells. Briefly, the soluble products obtained following cell disruption were applied to a nickel-charged affinity capture column. Bound proteins were eluted with 100 mM imidazole, treated with Factor Xa to activate the fusion protein and remove the maltose-binding protein (MBP) tag, then re-applied to a second nickel-charged affinity capture column. Samples from the purification procedure were assessed by SDS-PAGE. The final purified material in the absence and presence of reducing agent is identified in the lanes marked [-] and [+] respectively.

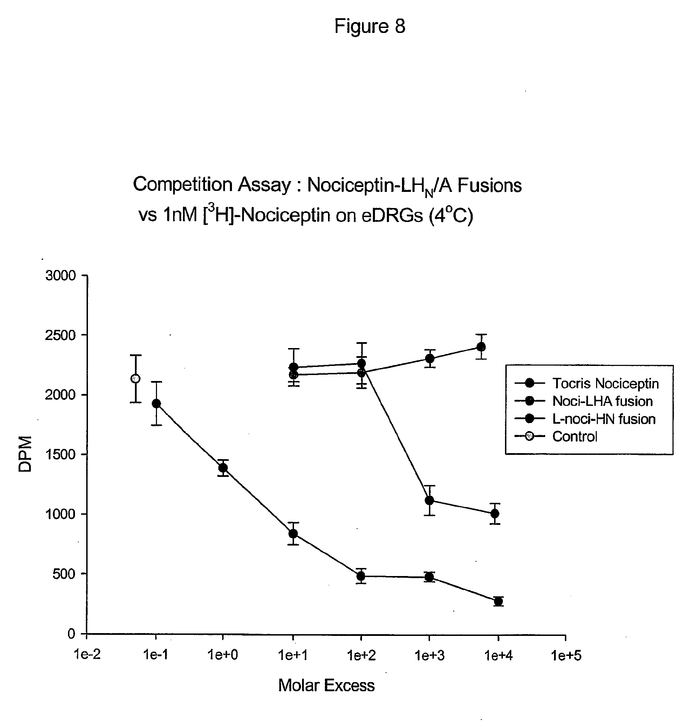

### Figure 8 - Comparison of binding efficacy of a LC/A-nociceptin-H_{N}/A fusion protein and a nociceptin-LC/A-H_{N}/A fusion protein

The ability of nociceptin fusions to bind to the ORL₁ receptor was assessed using a simple competition-based assay. Primary cultures of dorsal root ganglia (DRG) were exposed to varying concentrations of test material in the presence of 1 nM [3H]-nociceptin. The reduction in specific binding of the radiolabelled ligand was assessed by scintillation counting, and plotted in comparison to the efficacy of unlabelled ligand (Tocris nociceptin). It is clear that the LC/A-nociceptin-H_{N}/A fusion is far superior to the nociceptin-LC/A-H_{N}/A fusion at interacting with the ORL₁ receptor.

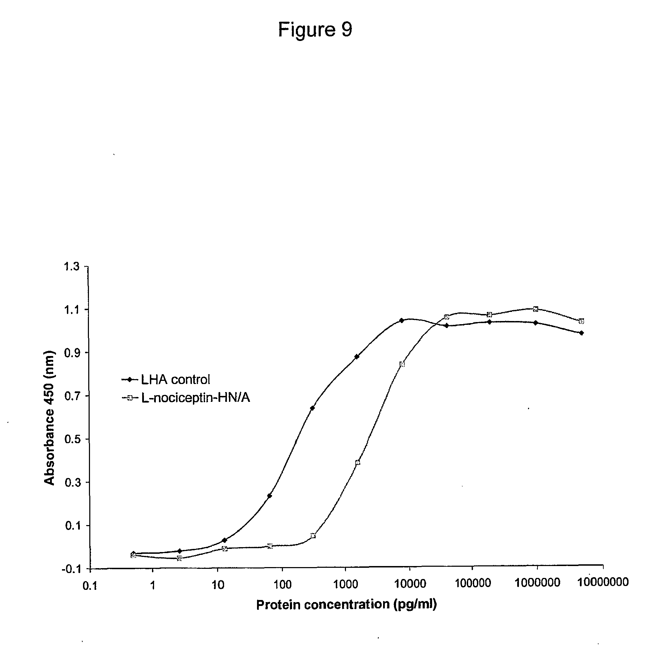

### Figure 9 - In vitro catalytic activity of a LC/A-nociceptin-H_{N}/A fusion protein

The *in vitro* endopeptidase activity of the purified LC/A-nociceptin-H_{N}/A fusion protein was determined essentially as described in Chaddock et al 2002, Prot. Express Purif. 25, 219-228. Briefly, SNAP-25 peptide immobilised to an ELISA plate was exposed to varying concentrations of fusion protein for 1 hour at 37°C. Following a series of washes, the amount of cleaved SNAP-25 peptide was quantified by reactivity with a specific antisera.

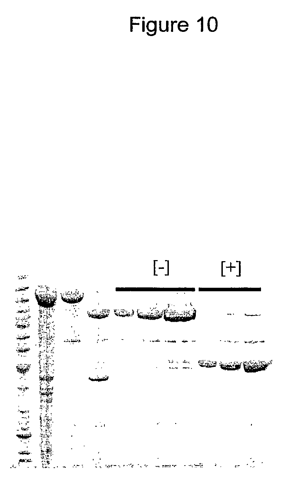

### Figure 10 - Purification of a LC/A-nociceptin variant-H_{N}/A fusion protein

Using the methodology outlined in Example 26, an LC/A-nociceptin variant-H_{N}/A fusion protein was purified from *E. coli* BL21 cells. Briefly, the soluble products obtained following cell disruption were applied to a nickel-charged affinity capture column. Bound proteins were eluted with 100 mM imidazole, treated with Factor Xa to activate the fusion protein and remove the maltose-binding protein (MBP) tag, then re-applied to a second nickel-charged affinity capture column. Samples from the purification procedure were assessed by SDS-PAGE. The final purified material in the absence and presence of reducing agent is identified in the lanes marked [-] and [+] respectively.

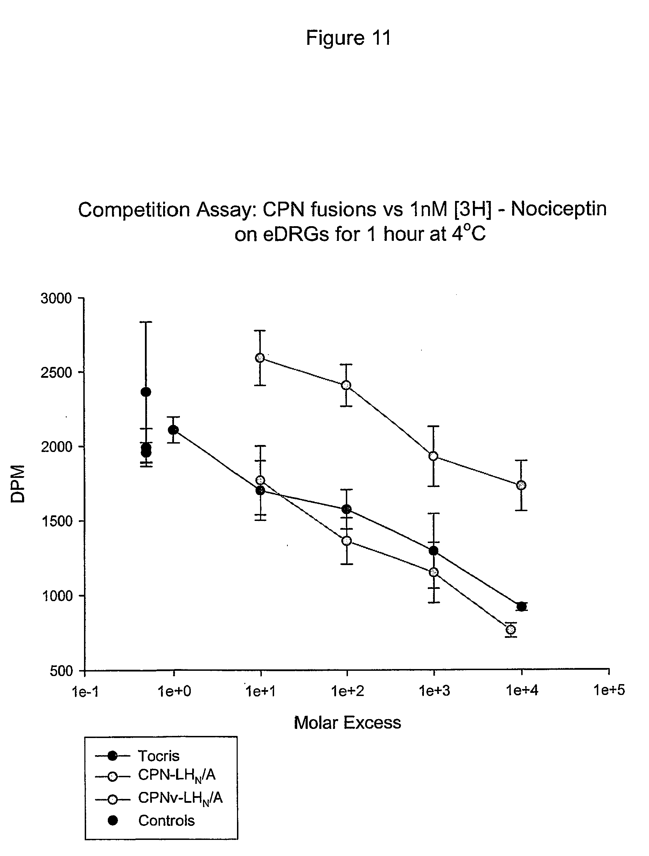

### Figure 11 - Comparison of binding efficacy of a LC/A-nociceptin-H_{N}/A fusion protein and a LC/A-nociceptin variant-H_{N}/A fusion protein

The ability of nociceptin fusions to bind to the ORL₁ receptor was assessed using a simple competition-based assay. Primary cultures of dorsal root ganglia (DRG) were exposed to varying concentrations of test material in the presence of 1 nM [3H]-nociceptin. The reduction in specific binding of the radiolabelled ligand was assessed by scintillation counting, and plotted in comparison to the efficacy of unlabelled ligand (Tocris nociceptin). It is clear that the LC/A-nociceptin variant-H_{N}/A fusion (CPNv-LHA) is superior to the LC/A-nociceptin variant-H_{N}/A fusion (CPN-LHA) at interacting with the ORL₁ receptor.

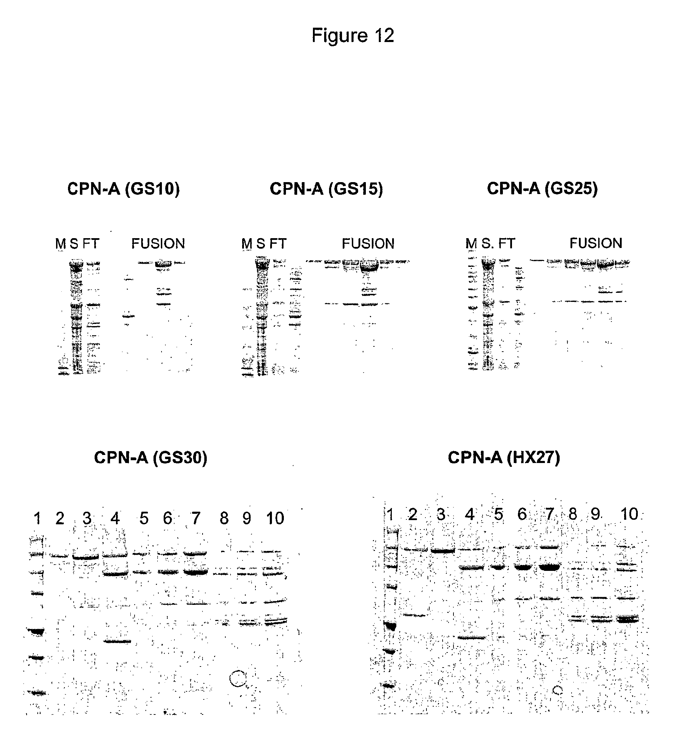

### Figure 12 - Expressed / purified LC/A-nociceptin-H_{N}/A fusion protein family with variable spacer length product(s)

Using the methodology outlined in Example 26, variants of the LC/A-CPN-H_{N}/A fusion consisting of GS10, GS30 and HX27 are purified from *E. coli* cell paste. Samples from the purification of LC/A-CPN(GS10)-H_{N}/A, LCIA-CPN(GS15)-H_{N}/A, LC/A-CPN(GS25)-H_{N}/A, LC/A-CPN(GS30)-H_{N}/A and LC/A-CPN(HX27)-H_{N}/A were assessed by SDS-PAGE prior to staining with Coomassie Blue. The electrophoresis profile indicates purification of a disulphide-bonded di-chain species of the expected molecular mass of CPBE-A. Top panel: M = benchmark molecular mass markers; S = total *E. coli* protein soluble fraction; FT = proteins that did not bind to the Ni²⁺-charged Sepharose column; FUSION = fusion protein eluted by the addition of imidazole. Bottom panel: Lane 1 = benchmark molecular mass markers; Lane 2 = total *E. coli* protein soluble fraction; Lane 3 = purified material following initial capture on Ni²⁺-charged Sepharose; Lane 4 = Factor Xa treated material prior to final capture on Ni²⁺-charged Sepharose; Lane 5 = purified final material post activation with Factor Xa (5 µl); Lane 6 = purified final material post activation with Factor Xa (10 µl); Lane 7 = purified final material post activation with Factor Xa (20 µl); Lane 8 = purified final material post activation with Factor Xa + DTT (5 µl); Lane 9 = purified final material post activation with Factor Xa + DTT (10 µl); Lane 10 = purified final material post activation with Factor Xa + DTT (20 µl).

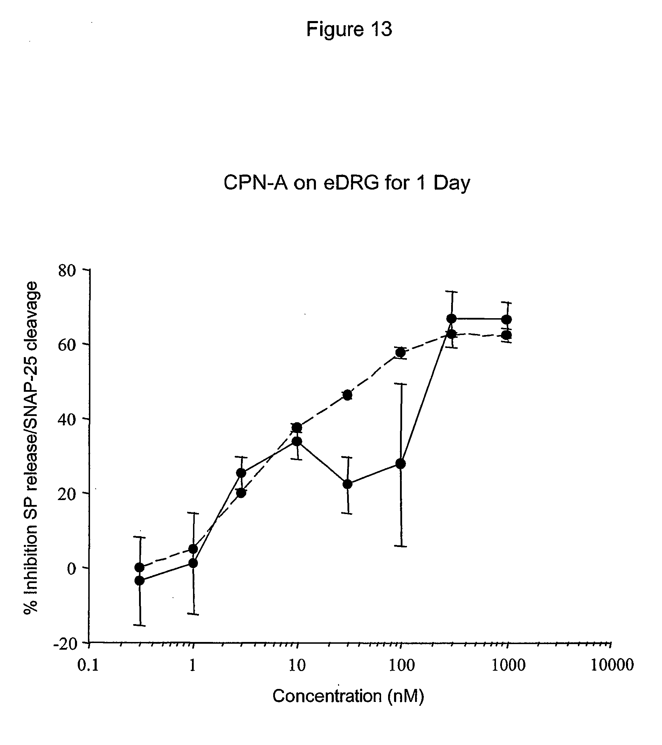

### Figure 13 - Inhibition of SP release and cleavage of SNAP-25 by CPN-A

Briefly, primary cultures of dorsal root ganglia (DRG) were exposed to varying concentrations of CPN-A for 24 hours. Cellular proteins were separated by SDS-PAGE, Western blotted, and probed with anti-SNAP-25 to facilitate an assessment of SNAP-25 cleavage. The percentage of cleaved SNAP-25 was calculated by densitometric analysis and plotted against fusion concentration (dashed line). Material was also recovered for an analysis of substance P content using a specific EIA kit. Inhibition of substance P release is illustrated by the solid line. The fusion concentration required to achieve 50% maximal SNAP-25 cleavage is estimated to be 6.30±2.48 nM.

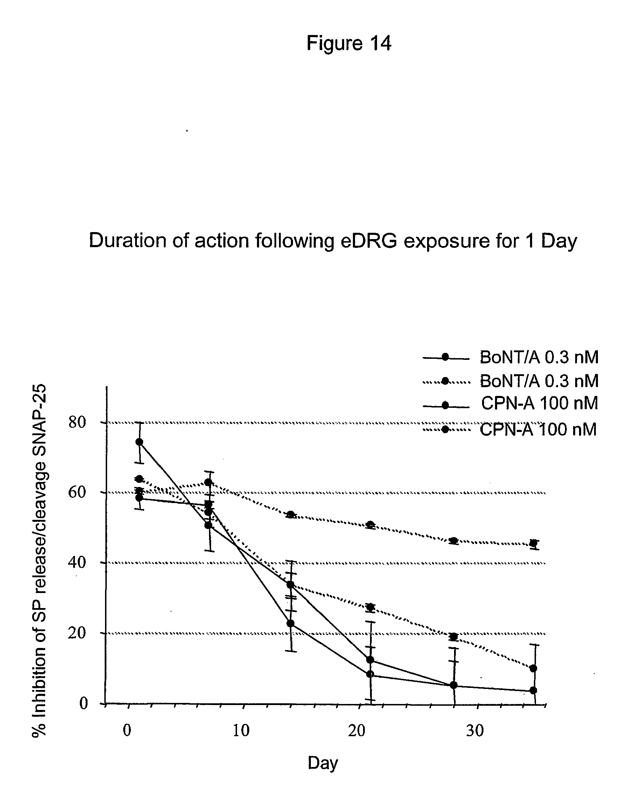

### Figure 14 - Inhibition of SP release and cleavage of SNAP-25 over extended time periods after exposure of DRG to CPN-A

Primary cultures of dorsal root ganglia (DRG) were exposed to varying concentrations of CPN-A for 24 hours. Botulinum neurotoxin (BoNT/A) was used as a control. After this initial exposure, extracellular material was removed by washing, and the cells incubated at 37°C for varying periods of time. At specific time points, cellular proteins were separated by SDS-PAGE, Western blotted, and probed with anti-SNAP-25 to facilitate an assessment of SNAP-25 cleavage. The percentage of cleaved SNAP-25 was calculated by densitometric analysis and illustrated by the dotted lines. Material was also recovered for an analysis of substance P content using a specific EIA kit. Inhibition of substance P release is illustrated by the solid lines.

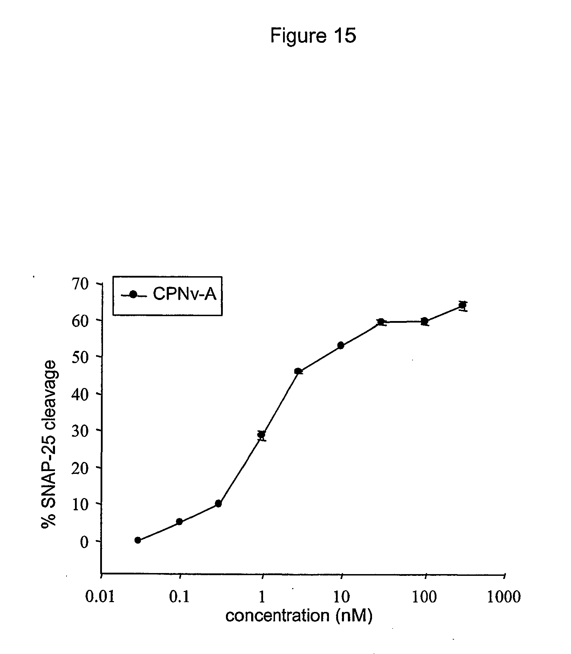

### Figure 15 - Cleavage of SNAP-25 by CPNv-A

Primary cultures of dorsal root ganglia (DRG) were exposed to varying concentrations of CPNv-A for 24 hours. Cellular proteins were separated by SDS-PAGE, Western blotted, and probed with anti-SNAP-25 to facilitate an assessment of SNAP-25 cleavage. The percentage of cleaved SNAP-25 was calculated by densitometric analysis. The fusion concentration required to achieve 50% maximal SNAP-25 cleavage is estimated to be 1.38±0.36 nM.

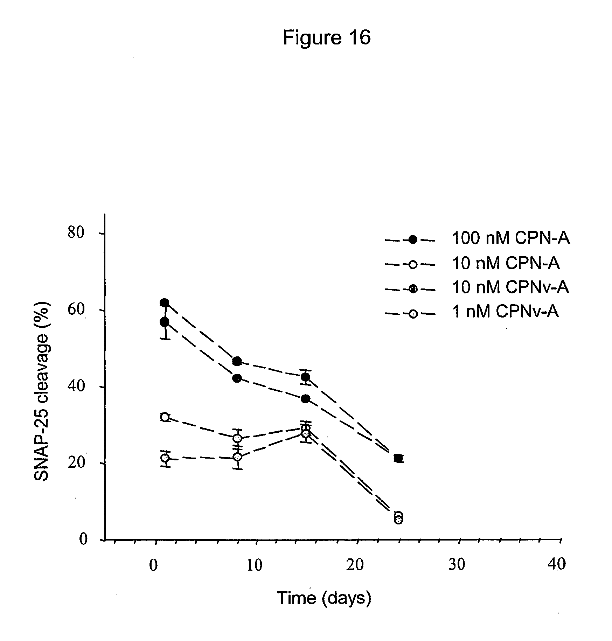

### Figure 16 - Cleavage of SNAP-25 over extended time periods after exposure of DRG to CPNv-A

Primary cultures of dorsal root ganglia (DRG) were exposed to varying concentrations of CPNv-A for 24 hours. CPN-A was used as a control. After this initial exposure, extracellular material was removed by washing, and the cells incubated at 37°C for varying periods of time. At specific time points, cellular proteins were separated by SDS-PAGE, Western blotted, and probed with anti-SNAP-25 to facilitate an assessment of SNAP-25 cleavage. The percentage of cleaved SNAP-25 was calculated by densitometric analysis.

### Figure 17 - CPNv-A fusion-mediated displacement of [3H]-nociceptin binding

The ability of nociceptin fusions to bind to the ORL₁ receptor was assessed using a simple competition-based assay. Primary cultures of dorsal root ganglia (DRG) were exposed to varying concentrations of test material in the presence of 1 nM [3H]-nociceptin. The reduction in specific binding of the radiolabelled ligand was assessed by scintillation counting, and plotted in comparison to the efficacy of unlabelled ligand (Tocris nociceptin). It is clear that the LC/A-nociceptin variant-H_{N}/A fusion (labelled as CPNv-LHnA) is superior to the LC/A-nociceptin-H_{N}/A fusion (labelled as CPN-LHnA) at interacting with the ORL₁ receptor.

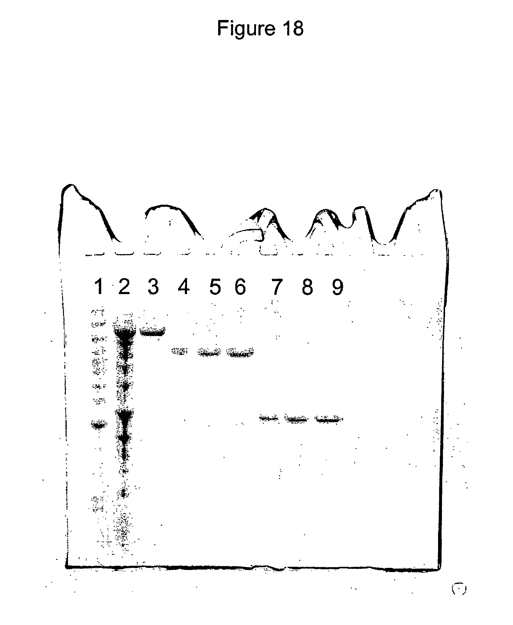

### Figure 18 - Expressed/purified CPNv(Ek)-A product

Proteins were subjected to SDS-PAGE prior to staining with Coomassie Blue. The electrophoresis profile indicates purification of a disulphide-bonded di-chain species of the expected molecular mass of CPNv(Ek)-A. Lane 1 = benchmark molecular mass markers; Lane 2 = total *E. coli* protein soluble fraction; Lane 3 = purified material following initial capture on Ni²⁺-charged Sepharose; Lane 4 = purified final material post activation with enterokinase (5 µl); Lane 5 = purified final material post activation with enterokinase (10 µl); Lane 6 = purified final material post activation with enterokinase (20 µl); Lane 7 = purified final material post activation with enterokinase + DTT (5 µl); Lane 8 = purified final material post activation with enterokinase + DTT (10 µl); Lane 9 = purified final material post activation with enterokinase + DTT (20 µl).

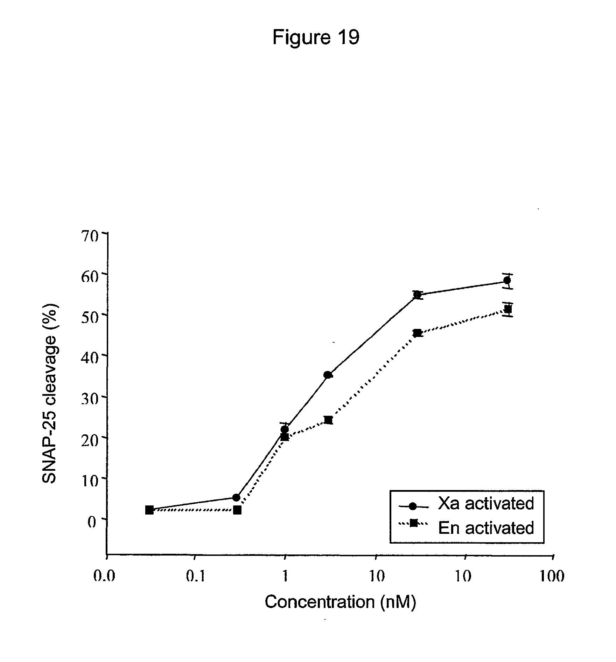

### Figure 19 - Cleavage of SNAP-25 by CPNv(Ek)-A

Primary cultures of dorsal root ganglia (DRG) were exposed to varying concentrations of CPNv(Ek)-A for 24 hours. Cellular proteins were separated by SDS-PAGE, Western blotted, and probed with anti-SNAP-25 to facilitate an assessment of SNAP-25 cleavage. The percentage of cleaved SNAP-25 was calculated by densitometric analysis. CPNv-A as prepared in Example 26 was used for comparison purposes. The percentage cleavage of SNAP-25 by CPNv(Ek)-A (labelled as En activated) and CPNv-A (labelled as Xa activated) are illustrated.

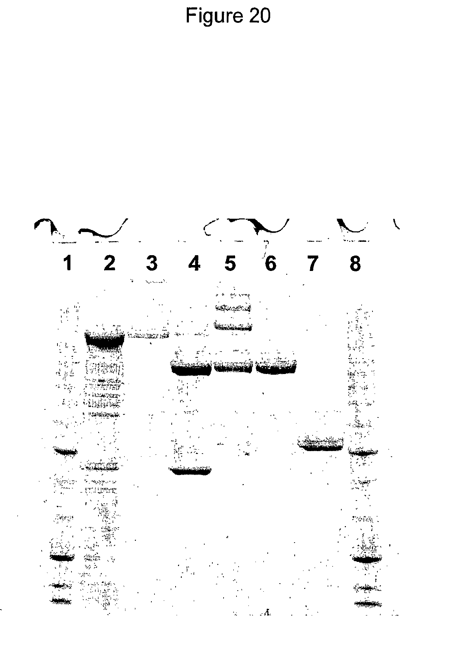

### Figure 20 - Expressed / purified CPNv-C product

Proteins were subjected to SDS-PAGE prior to staining with Coomassie Blue. The electrophoresis profile indicates purification of a disulphide-bonded di-chain species of the expected molecular mass of CPNv-C. Lane 1 = benchmark molecular mass markers; Lane 2 = total *E. coli* protein soluble fraction; Lane 3 = purified material following initial capture on Ni²⁺-charged Sepharose; Lane 4 = Factor Xa treated material prior to final capture on Ni²⁺-charged Sepharose; Lane 5 = purified material following second capture on Ni²⁺-charged Sepharose; Lane 6 = final purified material; Lane 7 = final purified material + DTT; Lane 8 = benchmark molecular mass markers.

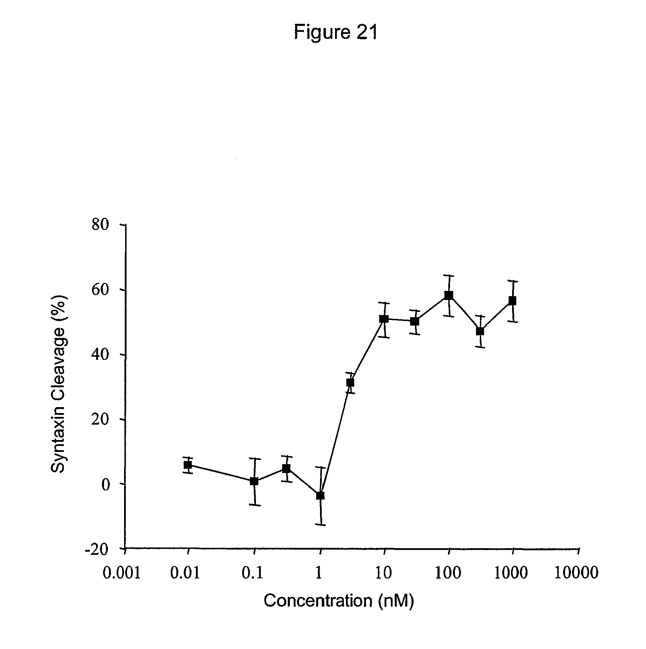

### Figure 21 - Cleavage of syntaxin by CPNv-C

Primary cultures of dorsal root ganglia (DRG) were exposed to varying concentrations of CPNv-C for 24 hours. Cellular proteins were separated by SDS-PAGE, Western blotted, and probed with anti-syntaxin to facilitate an assessment of syntaxin cleavage. The percentage of cleaved syntaxin was calculated by densitometric analysis. The fusion concentration required to achieve 50% maximal syntaxin cleavage is estimated to be 3.13±1.96 nM.

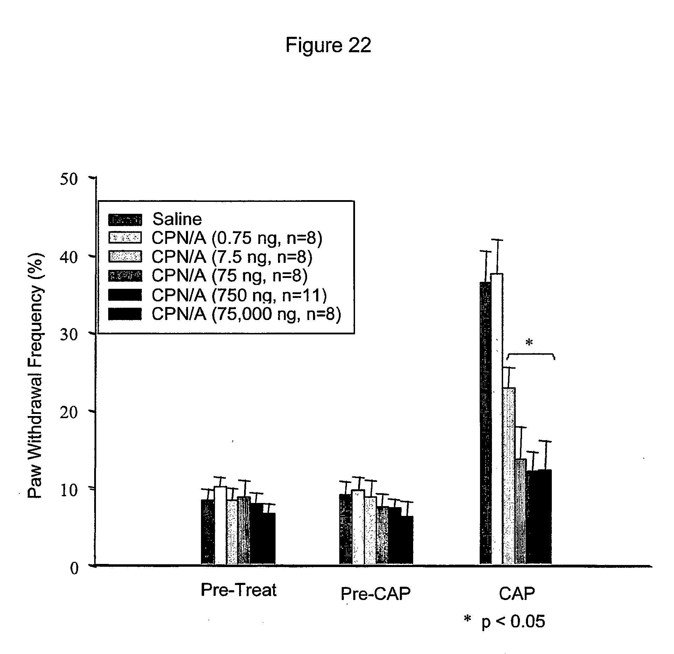

### Figure 22 - CPN-A efficacy in the Acute Capsaicin-Induced Mechanical Allodynia model

The ability of an LC/A-nociceptin-H_{N}/A fusion (CPN/A) to inhibit capsaicin-induced mechanical allodynia was evaluated following subcutaneous intraplantar injection in the rat hind paw. Test animals were evaluated for paw withdrawal frequency (PWF%) in response to a 10 g Von Frey filament stimulus series (10 stimuli x 3 trials) prior to recruitment into the study (Pre-Treat); after subcutaneous intraplantar treatment with CPN/A but before capsaicin (Pre-CAP); and following capsaicin challenge post-injection of CPN/A (average of responses at 15' and 30'; CAP). Capsaicin challenge was achieved by injection of 10 µL of a 0.3% solution. Sample dilutions were prepared in 0.5% BSA/saline.

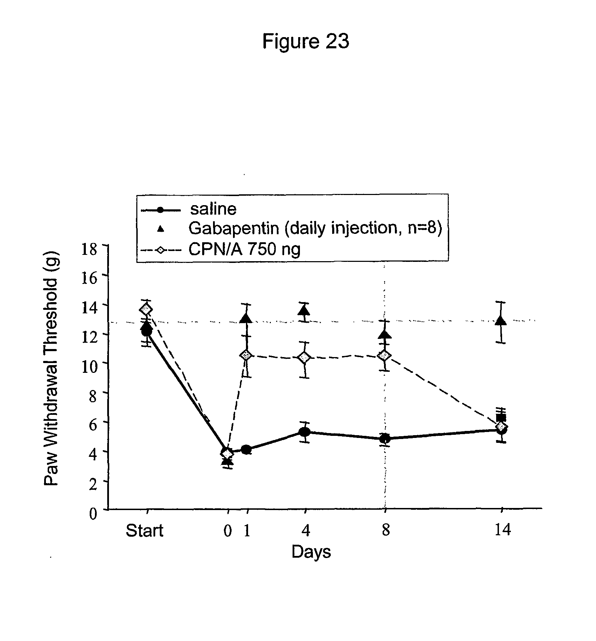

### Figure 23 - CPN-A efficacy in the Streptozotocin (STZ)-Induced Peripheral Diabetic Neuropathy (Neuropathic Pain) model

Male Sprague-Dawley rats (250-300 g) are treated with 65 mg/kg STZ in citrate buffer (I.V.) and blood glucose and lipid are measured weekly to define the readiness of the model. Paw Withdrawal Threshold (PWT) is measured in response to a Von Frey filament stimulus series over a period of time. Allodynia is said to be established when the PWT on two consecutive test dates (separated by 1 week) measures below 6 g on the scale. At this point, rats are randomized to either a saline group (negative efficacy control), gabapentin group (positive efficacy control) or a test group (CPN/A). Test materials (20-25 µl) are injected subcutaneously as a single injection (except gabapentin) and the PWT is measured at 1 day posttreatment and periodically thereafter over a 2 week period. Gabapentin (30 mg/kg i.p. @ 3 ml/kg injection volume) is injected daily, 2 hours prior to the start of PWT testing.

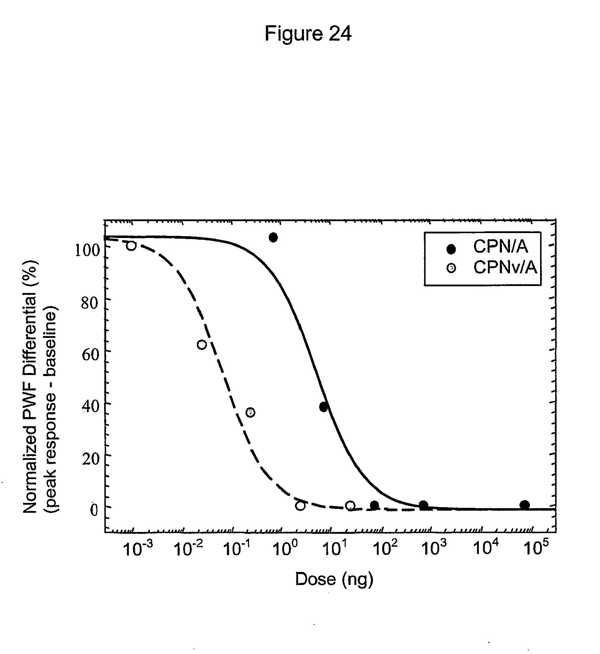

### Figure 24 - CPNv-A efficacy in the Acute Capsaicin-Induced Mechanical Allodynia model

The ability of an LC/A-nociceptin variant-H_{N}/A fusion (CPNv/A) to inhibit capsaicin-induced mechanical allodynia was evaluated following subcutaneous intraplantar injection in the rat hind paw. Test animals were evaluated for paw withdrawal frequency (PWF%) in response to a 10 g Von Frey filament stimulus series (10 stimuli x 3 trials) prior to recruitment into the study (Pre-Treat), after subcutaneous intraplantar treatment with CPNv/A but before capsaicin (Pre-CAP), and following capsaicin challenge post-injection of CPNv/A (average of responses at 15' and 30'; CAP). Capsaicin challenge was achieved by injection of 10 µL of a 0.3% solution. Sample dilutions were prepared in 0.5% BSA/saline. These data are expressed as a normalized paw withdrawal frequency differential, in which the difference between the peak response (post-capsaicin) and the baseline response (pre-capsaicin) is expressed as a percentage. With this analysis, it can be seen that CPNv/A is more potent than CPN/A since a lower dose of CPNv/A is required to achieve similar analgesic effect to that seen with CPN/A.

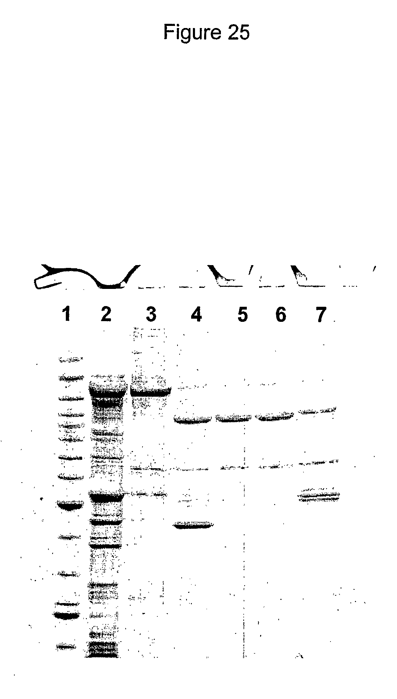

### Figure 25 - Expressed / purified LC/A-CPLE-H_{N}/A product

Proteins were subjected to SDS-PAGE prior to staining with Coomassie Blue. The electrophoresis profile indicates purification of a disulphide-bonded di-chain species of the expected molecular mass of CPLE-A. Lane 1 = benchmark molecular mass markers; Lane 2 = total *E. coli* protein soluble fraction; Lane 3 = purified material following initial capture on Ni²⁺-charged Sepharose; Lane 4 = Factor Xa treated material prior to final capture on Ni²⁺-charged Sepharose; Lane 5 = purified material following second capture on Ni²⁺-charged Sepharose; Lane 6 = final purified material; Lane 7 = final purified material + DTT.

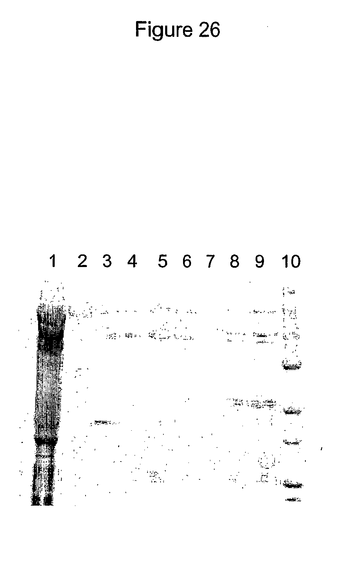

### Figure 26 - Expressed / purified LC/A-CPBE-H_{N}/A product

Proteins were subjected to SDS-PAGE prior to staining with Coomassie Blue. The electrophoresis profile indicates purification of a disulphide-bonded di-chain species of the expected molecular mass of CPBE-A. Lane 1 = total *E. coli* protein soluble fraction; Lane 2 = purified material following initial capture on Ni²⁺-charged Sepharose; Lane 3 = Factor Xa treated material prior to final capture on Ni²⁺-charged Sepharose; Lane 4 = purified final material post activation with Factor Xa (5 µl); Lane 5 = purified final material post activation with Factor Xa (10 µl); Lane 6 = purified final material post activation with Factor Xa (20 µl); Lane 7 = purified final material post activation with Factor Xa + DTT (5 µl); Lane 8 = purified final material post activation with Factor Xa + DTT (10 µl); Lane 9 = purified final material post activation with Factor Xa + DTT (20 µl); Lane 10 = benchmark molecular mass markers.

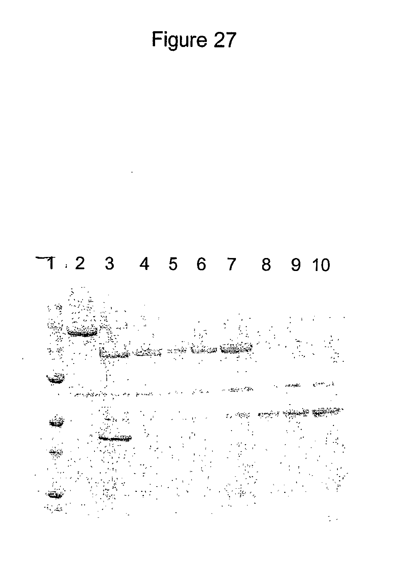

### Figure 27 - Expressed / purified CPOP-A product

Proteins were subjected to SDS-PAGE prior to staining with Coomassie Blue. The electrophoresis profile indicates purification of a disulphide-bonded di-chain species of the expected molecular mass of CPOP-A. Lane 1 = benchmark molecular mass markers; Lane 2 = purified material following initial capture on Ni²⁺-charged Sepharose; Lane 3 = Factor Xa treated material prior to final capture on Ni²⁺-charged Sepharose; Lane 4 = purified material following second capture on Ni²⁺-charged Sepharose; Lane 5 = purified final material post activation with Factor Xa (5 µl); Lane 6 = purified final material post activation with Factor Xa (10 µl); Lane 7 = purified final material post-activation with Factor Xa (20 µl); Lane 8 = purified final material post activation with Factor Xa + DTT (5 µl); Lane 9 = purified final material post activation with Factor Xa + DTT (10 µl); Lane 10 = purified final material post activation with Factor Xa + DTT (20 µl).

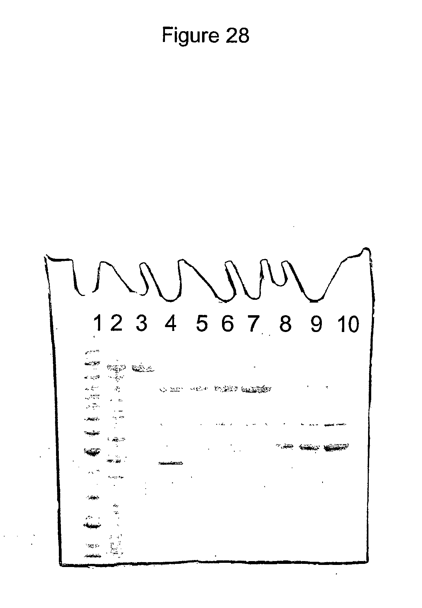

### Figure 28 - Expressed / purified CPOPv-A product

Proteins were subjected to SDS-PAGE prior to staining with Coomassie Blue. The electrophoresis profile indicates purification of a disulphide-bonded di-chain species of the expected molecular mass of CPOPv-A. Lane 1 = benchmark molecular mass markers; Lane 2 = total *E. coli* protein soluble fraction; Lane 3 = purified material following initial capture on Ni²⁺-charged Sepharose; Lane 4 = Factor Xa treated material prior to final capture on Ni²⁺-charged Sepharose; Lane 5 = purified final material post activation with Factor Xa (5 µl); Lane 6 = purified final material post activation with Factor Xa (10 µl); Lane 7 = purified final material post activation with Factor Xa (20 µl); Lane 8 = purified final material post activation with Factor Xa + DTT (5 µl); Lane 9 = purified final material post activation with Factor Xa + DTT (10 µl); Lane 10 = purified final material post activation with Factor Xa + DTT (20 µl).

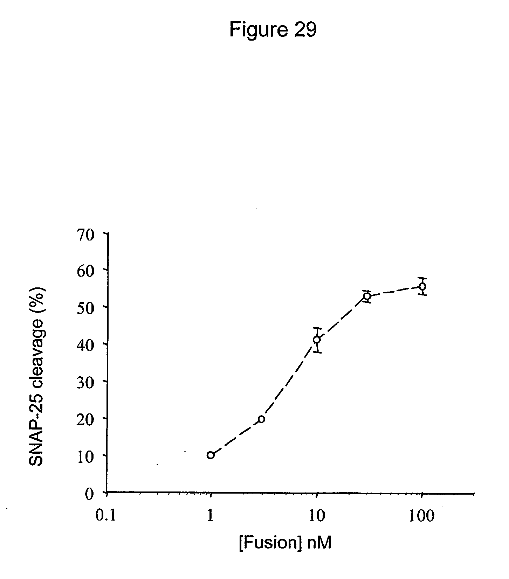

### Figure 29 - In vitro SNAP-25 cleavage in a DRG cell model

Primary cultures of dorsal root ganglia (DRG) were exposed to varying concentrations of CPOPv-A for 24 hours. Cellular proteins were separated by SDS-PAGE, Western blotted, and probed with anti-SNAP-25 to facilitate an assessment of SNAP-25 cleavage. The percentage of cleaved SNAP-25 was calculated by densitometric analysis.

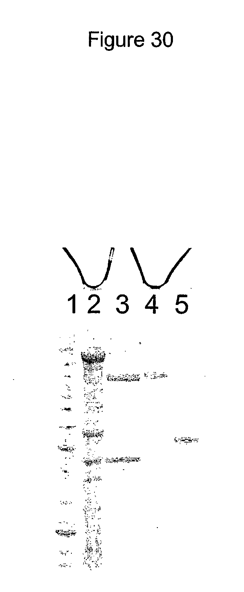

### Figure 30 - Expressed / purified CPNv-A-FXa-HT (removable his-tag)

Proteins were subjected to SDS-PAGE prior to staining with Coomassie Blue. The electrophoresis profile indicates purification of a disulphide-bonded di-chain species of the expected molecular mass of CPNv-A-FXa-HT. Lane 1 = benchmark molecular mass markers; Lane 2 = total *E*. *coli* protein soluble fraction; Lane 3 = Factor Xa treated material prior to final capture on Ni²+-charged Sepharose; Lane 4 = purified final material post activation with Factor Xa; Lane 5 = purified final material post activation with Factor Xa + DTT.

### Figure 31 - In vitro efficacy of LC/A-nociceptin-H_{N}/A fusion proteins with variable spacer length, as assessed by ligand competition assay

The ability of LC/A-nociceptin-H_{N}/A fusions of variable spacer length to bind to the ORL₁ receptor was assessed using a simple competition-based assay. Primary cultures of dorsal root ganglia (DRG) were exposed to varying concentrations of test material in the presence of 1 nM [3H]-nociceptin. The reduction in specific binding of the radiolabelled ligand was assessed by scintillation counting, and plotted in comparison to the efficacy of unlabelled ligand (Tocris nociceptin). The upper panel illustrates the displacement characteristics of the GS0, GS20, GS30 and Hx27 spacers, whilst the lower panel illustrates the displacement achieved by the GS10, GS15 and GS25 spaced fusion proteins. It is concluded that the GS0 and GS30 spacers are ineffective, and the GS10 is poorly effective, at displacing nociceptin from the ORL1 receptor.

### Figure 32 - In vitro efficacy of LC/A-nociceptin-H_{N}/A fusion proteins with variable spacer length, as assessed by in vitro SNAP-25 cleavage

Primary cultures of dorsal root ganglia (DRG) were exposed to varying concentrations of CPN-A (of variable spacer length) for 24 hours. Cellular proteins were separated by SDS-PAGE, Western blotted, and probed with anti-SNAP-25 to facilitate an assessment of SNAP-25 cleavage. The percentage of cleaved SNAP-25 was calculated by densitometric analysis. The poorly effective binding characteristics of the GS10 spaced fusion protein (see Figure 28) are reflected in the higher concentrations of fusion required to achieve cleavage of intracellular SNAP-25. GS0 and GS30 spaced fusion proteins were completely ineffective (date not shown). GS15, 20 and 25 spaced fusion proteins were similarly effective.

### SEQ ID Nos

| | |
|---|---|
| SEQ ID1 | DNA sequence of N[1-17] |
| SEQ ID2 | Protein Sequence of N[1-17] |
| SEQ ID3 | DNA sequence of N[1-11] |
| SEQ ID4 | Protein sequence of N[1-11] |
| SEQ ID5 | DNA sequence of N[[Y10]1-11] |
| SEQ ID6 | Protein sequence of N[[Y10]1-11] |
| SEQ ID7 | DNA sequence of N[[Y11]1-11] |
| SEQ ID8 | Protein sequence of N[[Y11]1-11] |
| SEQ ID9 | DNA sequence of N[[Y14]1-17] |
| SEQ ID10 | Protein sequence of N[[Y14]1-17] |
| SEQ ID11 | DNA sequence of N[1-13] |
| SEQ ID12 | Protein sequence of N[1-13] |
| SEQ ID13 | DNA sequence of Nv (also known as N[[R14K15]1-17]) |
| SEQ ID14 | Protein sequence of Nv (also known as N[[R14K15]1-17]) |
| SEQ ID15 | DNA sequence of N[1-17]-LH_{N}/A fusion protein |
| SEQ ID16 | Protein sequence of N[1-17]-LH_{N}/A fusion protein |
| SEQ ID17 | DNA sequence of N[[Y11]1-11]-LHN/A fusion protein |
| SEQ ID18 | Protein sequence of N[[Y11]1-11]-LHN/A fusion protein |
| SEQ ID19 | DNA sequence of N[1-13]-LHN/A fusion protein |
| SEQ ID20 | Protein sequence of N[1-13]-LHN/A fusion protein |
| SEQ ID21 | DNA sequence of LHN/A-N[1-17] fusion protein |
| SEQ ID22 | Protein sequence of LHN/A-N[1-17] fusion protein |
| SEQ ID23 | DNA sequence of LHN/C-N[1-11] fusion protein |
| SEQ ID24 | Protein sequence of LHN/C-N[1-11] fusion protein |
| SEQ ID25 | DNA sequence of N[[Y14]1-17]-LHN/C fusion protein |
| SEQ ID26 | Protein sequence of N[[Y14]1-17]-LHN/C fusion protein |
| SEQ ID27 | DNA sequence of the LC/A |
| SEQ ID28 | DNA sequence of the H_{N}/A |
| SEQ ID29 | DNA sequence of the LC/B |
| SEQ ID30 | DNA sequence of the H_{N}/B |
| SEQ ID31 | DNA sequence of the LC/C |
| SEQ ID32 | DNA sequence of the H_{N}/C |
| SEQ ID33 | DNA sequence of the CPN-A linker |
| SEQ ID34 | DNA sequence of the A linker |
| SEQ ID35 | DNA sequence of the N-terminal presentation nociceptin insert |
| SEQ ID36 | DNA sequence of the CPN-C linker |
| SEQ ID37 | DNA sequence of the CPBE-A linker |
| SEQ ID38 | DNA sequence of the CPNvar-A linker |
| SEQ ID39 | DNA sequence of the LC/A-CPN-H_{N}/A fusion |
| SEQ ID40 | Protein sequence of the LC/A-CPN-H_{N}/A fusion |
| SEQ ID41 | DNA sequence of the N-LC/A-H_{N}/A fusion |
| SEQ ID42 | Protein sequence of the N-LC/A-H_{N}/A fusion |
| SEQ ID43 | DNA sequence of the LC/C-CPN-H_{N}/C fusion |
| SEQ ID44 | Protein sequence of the LC/C-CPN-H_{N}/C fusion |
| SEQ ID45 | DNA sequence of the LC/C-CPN-H_{N}/C (A-linker) fusion |
| SEQ ID46 | Protein sequence of the LC/C-CPN-H_{N}/C (A-linker) fusion |
| SEQ ID47 | DNA sequence of the LC/A-CPME-H_{N}/A fusion |
| SEQ ID48 | Protein sequence of the LC/A-CPME-H_{N}/A fusion |
| SEQ ID49 | DNA sequence of the LC/A-CPBE-H_{N}/A fusion |
| SEQ ID50 | Protein sequence of the LC/A-CPBE-H_{N}/A fusion |
| SEQ ID51 | DNA sequence of the LC/A-CPNv-H_{N}/A fusion |
| SEQ ID52 | Protein sequence of the LC/A-CPNv-H_{N}/A fusion |
| SEQ ID53 | DNA sequence of the LC/A-CPN[1-11]-HN/A fusion |
| SEQ ID54 | Protein sequence of the LC/A-CPN[1-11]-HN/A fusion |
| SEQ ID55 | DNA sequence of the LC/A-CPN[[Y10]1-11]-HN/A fusion |
| SEQ ID56 | Protein sequence of the LC/A-CPN[[Y10]1-11]-HN/A fusion |
| SEQ ID57 | DNA sequence of the LC/A-CPN[[Y11]1-11]-HN/A fusion |
| SEQ ID58 | Protein sequence of the LC/A-CPN[[Y11]1-11]-HN/A fusion |
| SEQ ID59 | DNA sequence of the LC/A-CPN[[Y14]1-17]-HN/A fusion |
| SEQ ID60 | Protein sequence of the LC/A-CPN[[Y14]1-17]-HN/A fusion |
| SEQ ID61 | DNA sequence of the LC/A-CPN[1-13]-HN/A fusion |
| SEQ ID62 | Protein sequence of the LC/A- CPN[1-13]-HN/A fusion |
| SEQ ID63 | DNA sequence of the nociceptin-spacer-LC/A-H_{N}/A fusion |
| SEQ ID64 | Protein sequence of the nociceptin-spacer-LC/A-H_{N}/A fusion |
| SEQ ID65 | DNA sequence of the CPN-A GS10 linker |
| SEQ ID66 | DNA sequence of the CPN-A GS15 linker |
| SEQ ID67 | DNA sequence of the CPN-A GS25 linker |
| SEQ ID68 | DNA sequence of the CPN-A GS30 linker |
| SEQ ID69 | DNA sequence of the CPN-A HX27 linker |
| SEQ ID70 | DNA sequence of the LC/A-CPN(GS15)-H_{N}/A fusion |
| SEQ ID71 | Protein sequence of the LC/A-CPN(GS15)-H_{N}/A fusion |
| SEQ ID72 | DNA sequence of the LC/A-CPN(GS25)-H_{N}/A fusion |
| SEQ ID73 | Protein sequence of the LC/A-CPN(GS25)-H_{N}/A fusion |
| SEQ ID74 | DNA sequence of the CPNvar-A Enterokinase activatable linker |
| SEQ ID75 | DNA sequence of the LC/A-CPNv(Ek)-H_{N}/A fusion |
| SEQ ID76 | Protein sequence of the LC/A-CPNv(Ek)-H_{N}/A fusion |
| SEQ ID77 | DNA sequence of the CPNvar-A linker |
| SEQ ID78 | DNA sequence of the LC/C-CPNv-H_{N}/C fusion (act. A) |
| SEQ ID79 | Protein sequence of the LC/C-CPNv-H_{N}/C fusion (act. A) |
| SEQ ID80 | DNA sequence of the LC/A-CPLE-H_{N}/A fusion |
| SEQ ID81 | Protein sequence of the LC/A-CPLE-H_{N}/A fusion |
| SEQ ID82 | DNA sequence of the LC/A-CPOP-H_{N}/A fusion |
| SEQ ID83 | Protein sequence of the LC/A-CPOP-H_{N}/A fusion |
| SEQ ID84 | DNA sequence of the LC/A-CPOPv-H_{N}/A fusion |
| SEQ ID85 | Protein sequence of the LC/A-CPOPv-H_{N}/A fusion |
| SEQ ID86 | DNA sequence of the IgA protease |
| SEQ ID87 | DNA sequence of the IgA-CPNv-H_{N}/A fusion |
| SEQ ID88 | Protein sequence of the IgA-CPNv-H_{N}/A fusion |
| SEQ ID89 | DNA sequence of the FXa-HT |
| SEQ ID90 | DNA sequence of the CPNv-A-FXa-HT |
| SEQ ID91 | Protein sequence of the CPNv-A-FXa-HT fusion |
| SEQ ID92 | DNA sequence of the DT translocation domain |
| SEQ ID93 | DNA sequence of the CPLE-DT-A |
| SEQ ID94 | Protein sequence of the CPLE-DT-A fusion |
| SEQ ID95 | DNA sequence of the TeNT LC |
| SEQ ID96 | DNA sequence of the CPNv-TENT LC |
| SEQ ID97 | Protein sequence of the CPNV-TeNT LC fusion |
| SEQ ID98 | DNA sequence of the CPNvar-C linker |
| SEQ ID99 | DNA sequence of the LC/C-CPNv-H_{N}/C fusion (act. C) |
| SEQ ID100 | Protein sequence of the LC/C-CPNv-H_{N}/C fusion (act. C) |

### Examples

### Example 1 - Confirmation of TM Agonist Activity by measuring release of substance P from neuronal cell cultures

### Materials

Substance P EIA is obtained from R&D Systems, UK.

### Methods

Primary neuronal cultures of eDRG are established as described previously (Duggan *et al.,* 2002). Substance P release from the cultures is assessed by EIA, essentially as deseribed previously (Duggan *et al.,* 2002): The TM of interest is added to the neuronal cultures (established for at least 2 weeks prior to treatment); control cultures are performed in parallel by addition of vehicle in place of TM. Stimulated (100 mM KCl) and basal release, together with total cell lysate content, of substance P are obtained for both control and TM treated cultures. Substance P immunoreactivity is measured using Substance P Enzyme Immunoassay Kits (Cayman Chemical Company, USA or R&D Systems, UK) according to manufacturers' instructions.

The amount of Substance P released by the neuronal cells in the presence of the TM of interest is compared to the release obtained in the presence and absence of 100 mM KCl. Stimulation of Substance P release by the TM of interest above the basal release, establishes that the TM of interest is an "agonist ligand" as defined in this specification. If desired the stimulation of Substance P release by the TM of interest can be compared to a standard Substance P release-curve produced using the natural ORL-1 receptor ligand, nociceptin (Tocris).

### Example 2 - Expression and purification of catalytically active LH_{N}/A

### Materials

Synthetic DNA obtained from Sigma Genosys.
Restriction enzymes obtained from New England Biolabs.

### Methods

The expression and purification of catalytically active LH_{N}/A was carried out essentially as described in Sutton et al., (2005), Prot. Express. Purif., 40, pp 31-41.

Briefly, DNA encoding the light chain plus 423 amino acids from the N-terminal of the heavy chain of BoNT/A was synthesised by Sigma-Genosys to produce a synthetic LH_{N}/A gene with an *E. coli* codon bias. The linker region between the light chain and H_{N} domain was engineered to contain a Factor Xa cleavage site by splice-overlap extension PCR. Two PCR products were generated using primer pairs consisting of a long, mutagenic primer and a shorter, non-mutagenic primer:
(5'-tccaaaactaaatctctgATAGAAGGTAGAaacaaagcgctgaacgac) with (5'-CTTGATGTACTCTGTGAACGTGCTC); and
(5'-gtcgttcagcgctttgttTCTACCTTCTATcagagatttagttttgga) with (5'-ATGGAGTTCGTTAACAAACAGTTC).

The products from these two reactions were used as templates for the splice-overlap extension PCR. A further PCR reaction was set up to add *Bam*HI and *Sal*I sites at either end of the activatable recLH_{N}/A gene and these sites were used for insertion into an Invitrogen gateway entry vector. The entry vector was then used, along with a gateway recombination site adapted pMAL c2x, in a LR clonase reaction to form pMAL c2x recLH_{N}/A. The pMAL c2x recLH_{N}/A was modified to incorporate a 6'HIS tag at the N-terminus of the MBP. This was achieved by the insertion of annealed oligonucleotides encoding the HIS tag into the *Nde*I site of pMAL.

The expression vector expressing LH_{N}/A was transformed into *E*. *coli* HMS174 or AD494(DE3) (Novagen). Cultures were grown in Terrific broth complex medium supplemented with ZnCl₂ (1 µM), ampicillin (100 µg/ml), 0.2% (w/v) glucose. Parameters for expression of all the constructs were initially determined in shake flask cultures before transferring into 8 L fermentor systems. Starter cultures were grown for 16 hours at 37°C, 220 rpm and used to inoculate 1 L in which growth was continued at 37°C, 250 rpm. At an OD600 nm of 0.6 the temperature was reduced to 25°C for 30 minutes before induction with 1 mM IPTG. Induction was continued for 4 hours before the cells were harvested and stored at -70°C.

Typically 16 g of cell paste was suspended in 160 ml PBS and lysed by sonication (MSE Soniprep 150). The resulting lysate was clarified by centrifugation prior loading onto a 25 ml amylose column and eluted with 10 mM maltose in PBS. The eluant contained approx. 50% pure fusion protein and was treated with Factor Xa (1 unit Factor Xa /100 µg fusion protein; 20 hours; 26°C) to remove the HISMBP and cleave the LC-HN junction to activate the protein. After incubation the sample was filtered (0.45 mm) and diluted two fold with water to give a 0.5 x PBS buffer composition. The cleaved, filtered and diluted recLH_{N}/A was processed through a Q Sepharose FF column (10 ml) and eluted with a step gradient of 80 mM NaCl containing HISMBP and 120 mM NaCl containing approx. 75% pure recLH_{N}/A. The addition of His tag to MBP overcame previous co-elution problems with LH_{N}/A and MBP. As a final polishing step to ensure complete removal of the HISMBP, the 120 mM NaCl elution from the Q Sepharose column was passed through a Nickel charged 5 ml HisTrap column (Amersham). The flow through from the HisTrap column contained approx. 95% pure recLH_{N}/A (see the Figures in Sutton et al., (2005), Prot. Express. Purif., 40, pp 31-41 for an illustration of the purification scheme for LHN/A).

### Example 3 - Expression and purification of catalytically active recombinant LH_{N}/B

The methodology described below will purify catalytically active LH_{N}/B protease from *E. coli* transformed with the appropriate plasmid encoding the LH_{N}/B polypeptide. It should be noted that various sequences of suitable LH_{N}/B polypeptides have been described in PCT/GB97/02273, granted US 6 461617 and US patent application 10/241596, incorporated herein by reference.

### Methods

The coding region for LH_{N}/B is inserted in-frame to the 3' of the gene encoding maltose binding protein (MBP) in the expression vector pMAL (New England Biolabs) to create pMAL- c2x-LH_{N}/B. In this construct, the expressed MBP and LH_{N}/B polypeptides are separated by a Factor Xa cleavage site, and the LC and H_{N} domains are separated by a peptide that is susceptible to cleavage with enterokinase. The expression clone is termed pMAL-c2X-synLH_{N}/B. pMAL-c2X-synLH_{N}/B is transformed into *E. coli* HMS174 and cultured in Terrific broth complex medium in 8 L fermentor systems. Pre-induction bacterial growth is maintained at 37°C to an OD600 nm of 5.0, at which stage expression of recMBP-LH_{N}/B is induced by addition of IPTG to 0.5 mM and a reduction in temperature to 30°C. After four hours at 30°C the bacteria are harvested by centrifugation and the resulting paste stored at -70°C.

The cell paste is resuspended in 20 mM Hepes pH 7.2, 125 mM NaCl, 1 µM ZnCl₂ and cell disruption achieved using an APV-Gaulin lab model 1000 homogeniser or a MSE Soniprep 150 sonicator. The resulting suspension is clarified by centrifugation prior to purification.

Following cell disruption, the MBP-fusion protein is captured either on an amylose affinity resin in 20 mM Hepes pH 7.2, 125 mM NaCl, 1 µM ZnCl₂, or on a Q-Sepharose FF anion-exchange resin in 50 mM Hepes pH 7.2, 1 µM ZnCl₂ with no salt. A single peak is eluted from the amylose resin in the same buffer plus 10 mM maltose and from the Q-Sepharose in 150-200 mM salt. Cleavage of the MBP-LH_{N}/B junction is completed in an 18 hours incubation step at 22°C with Factor Xa (NEB) at 1 U/50 µg fusion protein. A substrate (MBP-LH_{N}/B) concentration of at least 4 mg/ml is desirable for efficient cleavage to take place.

The cleaved protein is diluted with 20 mM Hepes to a buffer composition of 20 mM Hepes, 25 mM NaCl, 1 µM ZnCl₂, pH 7.2 and processed through a Q Sepharose column to separate the MBP from LH_{N}/B. The LH_{N}/B is eluted from the Q-Sepharose column with 120-170 mM salt. The linker between the light chain and H_{N} domain is then nicked by incubation with enterokinase at 1 U/100 µg of LH_{N}/B at 22°C for 16 hours. Finally, the enterokinase is separated from the nicked LH_{N}/B and other contaminating proteins on a Benzamidine Sepharose column, the enzyme preferentially binding to the resin over an incubation of 30 minutes at 4°C. Purified LH_{N}/B is stored at -20°C until required. See Figure 1 for an illustration of the purification scheme for recLH_{N}/B.

### Example 4 - Expression and purification of catalytically active recombinant LH_{N}/C

The coding region for LH_{N}/C is inserted in-frame to the 3' of the gene encoding maltose binding protein (MBP) in the expression vector pMAL (New England Biolabs) to create pMAL- c2x-LH_{N}/C. In this construct the expressed MBP and LH_{N}/C polypeptides are separated by a Factor Xa cleavage site.

pMAL-c2x-LH_{N}/C is transformed into *E*. *coli* AD494 (DE3, IRL) and cultured in Terrific broth complex medium in 8 L fermentor systems. Pre-induction bacterial growth are maintained at 30°C to an OD600 nm of 8.0, at which stage expression of *rec*MBP-c2x-LH_{N}/C is induced by addition of IPTG to 0.5 mM and a reduction in temperature of culture to 25°C. After 4 hours at 25°C the bacteria are harvested by centrifugation and the resulting paste stored at -70°C.

The cell paste is resuspended in 50 mM Hepes pH 7.2, 1 µM ZnCl₂ at 1:6 (w/v) and cell disruption is achieved using an APV-Gaulin lab model 1000 homogeniser or a MSE Soniprep 150 sonicator. The resulting suspension is clarified by centrifugation prior to purification.

Following cell disruption and clarification, the MBP-fusion protein is separated on a Q-Sepharose Fast Flow anion-exchange resin in 50 mM Hepes pH 7.2, 1 µM ZnCl₂ and eluted with the same buffer plus 100 mM NaCl. A double point cleavage is performed at the MBP-LH_{N}/C junction and the H_{N}-LC linker in a single incubation step with Factor Xa. The reaction is completed in a 16-hour incubation step at 22°C with Factor Xa (NEB) at 1 U/100 ìg fusion protein. The cleaved protein is diluted with 20 mM Hepes to a buffer composition of 20 mM Hepes, 25 mM NaCl, pH 7.2 and processed through a second Q-Sepharose column to separate the MBP from LH_{N}/C. Activated (disulphide-bonded cleaved linker) LH_{N}/C is eluted from the Q-Sepharose column by a salt gradient (20 mM Hepes, 500 mM NaCl, 1 µM ZnCl₂, pH 7.2) in 120-170 mM salt. See Figure 2 for an illustration of the purification of LH_{N}/C.

### Example 5 - Production of a chemical conjugate of nociceptin and LH_{N}/A

### Materials

C-terminally extended nociceptin peptide obtained from Sigma Genosys.
Conjugation chemicals obtained from Pierce.

### Methods

In order to couple the nociceptin peptide via a C-terminal Cys, the peptide was first synthesised (by standard procedures, commercially obtainable) to include a Cys as the final C-terminal amino acid.

This peptide was then used as the second component in a sulphydryl based coupling reaction as described below (see also previous publications WO 99/17806 and WO 96/33273 and Duggan et al., (2002), J. Biol. Chem. 277, 24846-34852 and Chaddock et al., (2000), Infect lmmun., 68, 2587-2593).

### Sulphydryl based coupling reaction

Briefly, approximately two reactive leaving groups were introduced into LH_{N}/A (5 mg/ml in phosphate-buffered saline) by reaction with *N*-succinimidyl-3-(2-pyridyldithio)propionate (SPDP).

Derivatised material was isolated from excess SPDP by size exclusion chromatography. Reconstituted cysteine-tagged nociceptin ligand was mixed with the derivatised LH_{N}/A in a 4:1 molar ratio, and incubated at room temperature for 1 hour with gentle agitation in order to create a chemical conjugate through a reducible covalent disulphide bond. Initial fractionation of the conjugate mixture to remove unconjugated peptide was performed by size exclusion chromatography (Superose-12, or Superdex G-200 depending on scale of conjugation).

### Example 6 - Production of a chemical conjugate of nociceptin and LH_{N}/B

### Materials

C-terminally extended nociceptin peptide obtained from Sigma Genosys.
Conjugation chemicals obtained from Pierce.

### Methods

Lyophilised nociceptin was dissolved by the addition of water and dialysed into MES buffer (0.1 M MES, 0.1 M NaCl, pH 5.0). To this solution (at a concentration of about 0.3 mg/ml) was added PDPH (100 mg/ml in DMF) to a final concentration of 1 mg/ml. After mixing, solid EDAC was added to produce a final concentration of about 0.2 mg/ml. The reaction was allowed to proceed for at least 30 minutes at room temperature. Excess PDPH was then removed by desalting over a PD-10 column (Pharmacia) previously equilibrated with MES buffer.

An amount of LH_{N}/B equivalent to half the weight of nociceptin used dissolved in triethanolamine buffer (0.02 M triethanolamine/HCl, 0.1 M sodium chloride, pH 7.8) at a concentration of about 1 mg/ml, was reacted with Traut's reagent (100 mM stock solution in 1 M triethanolamine/HCl, pH 8.0) at a final concentration of 2 mM. After 1 hour, the LH_{N}/B was desalted into PBSE (phosphate buffered saline with 1 mM EDTA) using a PD-10 column (Pharmacia). The protein peak from the column eluate was concentrated using a Microcon 50 (Amicon) to a concentration of about 2 mg/ml.

The derivatised nociceptin was subjected to a final concentration step resulting in a reduction in volume to less than 10% of the starting volume and then mixed with the derivatised LH_{N}/B overnight at room temperature. The products of the reaction were analysed by polyacrylamide gel electrophoresis in the presence of sodium dodecylsulphate (SDS-PAGE).

The conjugate resulting from the above reaction was partially purified by size exclusion chromatography over Bio-Gel P-100 (BioRad). The elution profile was followed by measuring the optical density at 280 nm and SDS-PAGE analysis of the fractions. This allowed the separation of conjugate from free nociceptin and byproducts of the reaction.

### Example 7 - Production of a chemical conjugate of nociceptin 1-11 and LH_{N}/B

### Materials

C-terminally extended nociceptin 1-11 peptide obtained from Sigma Genosys.
Conjugation chemicals obtained from Pierce.

### Methods

In order to couple the nociceptin 1-11 peptide via a C-terminal Cys, the peptide was first synthesised (by standard procedures, commercially obtainable) to include a Cys as the final C-terminal amino-acid.

This peptide was then used as the second component in a sulphydryl based coupling reaction as described in Example 5.

### Example 8 - Production of a chemical conjugate of nociceptin N[[Y14]1-17] and LH_{N}/C

### Materials

C-terminally extended nociceptin N[[Y14]1-17] peptide obtained from Sigma Genosys.
Conjugation chemicals obtained from Pierce.

### Methods

In order to couple the peptide via a C-terminal Cys, the peptide was first synthesised (by standard procedures, commercially obtainable) to include a Cys as the final C-terminal amino acid.

This peptide was then used as the second component in a sulphydryl based coupling reaction as described in Example 5.

### Example 9 - Recombinant production of a single polypeptide fusion of nociceptin-LH_{N}/A (SEQ ID15 and SEQ ID16)

The DNA sequence for the nociceptin-LH_{N}/A was designed by back translation of the LC/A, H_{N}/A, and nociceptin amino acid sequences. The complete ORF containing the nociceptin-LC/A-activation loop-H_{N}/A sequence was assembled within standard DNA sequence manipulation software (EditSeq). The activation loop between the LC/A cysteine and the H_{N}/A cysteine (CVRGIITSKTKSLDKGYNKALNDLC) was modified to incorporate a Factor Xa protease recognition site.

Restriction sites appropriate to facilitate cloning into the required expression vector (for example BamHI/SalI) were incorporated at the 5' and 3' ends respectively of the sequence maintaining the correct reading frame. The DNA sequence was screened (using software such as MapDraw, DNASTAR Inc.) for restriction enzyme cleavage sequences incorporated during the back translation. Any cleavage sequences that were found to be common to those required by the cloning system were removed manually from the proposed coding sequence ensuring common *E. coli* codon usage was maintained. *E. coli* codon usage was assessed by reference to software programs such as Graphical Codon Usage Analyser (Geneart), and the %GC content and codon usage ratio assessed by reference to published codon usage tables (for example GenBank Release 143, 13 September 2004).

This optimised DNA sequence containing the nociceptin-LC/A-activation loop-H_{N}/A open reading frame (ORF) was then commercially synthesized and provided in the pCR 4 vector.

The DNA encoding the nociceptin-LH_{N}/A fusion was isolated from pCR 4 and transferred into pMAL vector backbone to facilitate protein expression. The resultant pMAL NO-LHN/A vector was transformed into competent *E*. *coli* BL21 and correct transformants selected. A single colony of pMAL NO-LH_{N}/A was grown in Terrific broth complex medium supplemented with ZnCl₂ (1 mM), ampicillin (100 µg/ml), 0.2% (w/v) glucose. Expression of the insert was induced by the addition of IPTG (0.1 mM) and the culture maintained at 16°C for 16 hours. After this period of expression the bacteria were isolated by centrifugation and the cell pellet stored at -20°C until use.

10 g of *E. coli* BL21 cell paste was defrosted in a falcon tube containing 25 ml 50 mM HEPES, pH 7.2,200 mM NaCl. The thawed cell paste was made up to 80 ml with 50 mM HEPES, pH 7.2, 200 mM NaCl and sonicated on ice 30 seconds on, 30 seconds off for 10 cycles at a power of 22 microns ensuring the sample remained cool. The lysed cells were centrifuged at 18 000 rpm, 4°C for 30 minutes. The supernatant was loaded onto a 0.1 M NiSO₄ charged chelating column (20-30 ml column is sufficient) and equilibrated with 50 mM HEPES, pH 7.2, 200 mM NaCl.

Using a step gradient of 10 and 40 mM imidazol, the non-specific bound protein was washed away and the fusion protein eluted with 100 mM imidazol. The eluted fusion protein was dialysed against 5 L of 50 mM HEPES, pH 7.2, 200 mM NaCl at 4°C overnight and the OD of the dialysed fusion protein measured. 1 unit of Factor Xa was added per 100 µg fusion protein and incubated at 25°C static overnight. The cleavage mixture was loaded onto a 0.1 M NiSO₄ charged Chelating column (20-30 ml column is sufficient) and equilibrated with 50 mM HEPES, pH 7.2, 200 mM NaCl.

Using a step gradient of 10 and 40 mM imidazol, the non-specific bound protein was washed away and the fusion protein eluted with 100 mM imidazol. The eluted fusion protein was dialysed against 5 L of 50 mM HEPES, pH 7.2, 200 mM NaCl at 4°C overnight and the fusion concentrated to about 2 mg/ml, aliquoted and stored at -20°C.

Figure 3 shows the SDS-PAGE analysis of expression and purification of N[1-17]-LH_{N}/A.

### Example 10 - Recombinant production of a single polypeptide fusion of (nociceptin 1-11)-LH_{N}/B

The DNA sequence for the (nociceptin 1-11)-LH_{N}/B was designed by back translation of the LC/B, H_{N}/B, and nociceptin 1-11 amino acid sequences. The complete ORF containing the (nociceptin1-11)-LC/B-activation loop-H_{N}/B sequence was assembled within standard DNA sequence manipulation software (EditSeq). The activation loop between the LC/B cysteine and the H_{N}/B cysteine was modified to incorporate a Factor Xa protease recognition site.

The recombinant fusion protein was then produced essentially as described in Example 9.

### Example 11 - Recombinant production of a single polypeptide fusion of (nociceptin N[[Y14]1-17]) -LH_{N}/C (SEQ ID25 and SEQ ID26)

The DNA sequence for the nociceptin N[[Y14]1-17] was designed by back translation of the LC/C, H_{N}/C, and nociceptin N[[Y14]1-17] amino acid sequences. The complete ORF containing the (nociceptin N[[Y14]1-17])-LC/C-activation loop-H_{N}/C sequence was assembled within standard DNA sequence manipulation software (EditSeq). The activation loop between the LC/C cysteine and the H_{N}/C cysteine was modified to incorporate a Factor Xa protease recognition site.

The recombinant fusion protein was then produced essentially as described in Example 9.

### Example 12 - Recombinant production of a single polypeptide fusion of LH_{N}/C-(nociceptin 1-11) (SEQ ID23 and SEQ ID24)

The DNA sequence for the LH_{N}/C-(nociceptin 1-11) was designed by back translation of the LC/C, H_{N}/C and nociceptin 1-11 amino acid sequences. The complete ORF (SEQ ID23) containing the LC/C-activation loop-H_{N}/C-flexible spacer-(nociceptin 1-11) was assembled within standard DNA sequence manipulation software (EditSeq).

The recombinant fusion protein (SEQ ID24) was then produced essentially as described in Example 9.

### Example 13 - Production of a conjugate for delivery of DNA encoding LC/C into a cell

The construction of a nociceptin-H_{N}-[LC/C] conjugate is described below, where [LC/C] represents the polylysine condensed DNA encoding the light chain of botulinum neurotoxin type C.

### Materials

SPDP is from Pierce Chemical Co.
Additional reagents are obtained from Sigma Ltd.

### Methods

Using a plasmid containing the gene encoding LC/C under the control of a CMV (immediate early) promoter, condensation of DNA was achieved using SPDP-derivatised polylysine to a ratio of 2 DNA to 1 polylysine. Conjugates were then prepared by mixing condensed DNA (0.4 mg/ml) with H_{N}-nociceptin (100 µg/ml) for 16 h at 25°C. The SPDP-derivatised polylysine and the free -SH group present on the H_{N} domain combine to facilitate covalent attachment of the DNA and protein.

### Example 14 - Production of a conjugate for delivery of DNA encoding LC/B into a cell

The construction of a (nociceptin 1-11)-H_{N}-[LC/B] conjugate is described below, where [LC/B] represents the polylysine condensed DNA encoding the light chain of botulinum neurotoxin type B.

### Materials

SPDP is from Pierce Chemical Co.
Additional reagents are obtained from Sigma Ltd.

### Methods

Using a plasmid containing the gene encoding LC/B under the control of a CMV (immediate early) promoter, condensation of DNA was achieved using SPDP-derivatised polylysine to a ratio of 2 DNA to 1 polylysine. Conjugates were then prepared by mixing condensed DNA (0.4 mg/ml) with H_{N}-(nociceptin 1-11) (100 µg/ml) for 16 h at 25°C. The SPDP-derivatised polylysine and the free -SH group present on the H_{N} domain combine to facilitate covalent attachment of the DNA and protein.

### Example 15 - Assessment of the activity of nociceptin-LH_{N}/A in substance P releasing neuronal cells

Using methodology described in Duggan et al., (2002, J. Biol. Chem., 277, 34846-34852), the activity of nociceptin-LH_{N}/A in substance P releasing neuronal cells was assessed.

Nociceptin-LH_{N}/A fusion protein was applied to 2-week old dorsal root ganglia neuronal cultures, and incubated at 37°C for 16 hours. Following the incubation, the media was removed and the ability of the cells to undergo stimulated release of substance P (SP) was assessed.

The release of SP from the neuronal cells incubated with the nociceptin-LH_{N}/A fusion protein was assayed in comparison to (i) LH_{N}/A-only treated cells and (ii) cells treated with media alone. This allowed the % inhibition of substance P from the eDRG to be calculated. The ability of the nociceptin-LH_{N}/A fusion protein to inhibit SP release (relative to cells treated with media alone) was reported in Table 1. The data represent the mean of 3 determinations:

**Table 1**

| **Test Material (µM)** | **nociceptin-LH_{N}/A fusion protein** | **LH_{N}/A-only** |
|---|---|---|
| | **% Inhibition** | **% Inhibition** |
| **1.0** | 47.3 | 25.6 |
| **0.1** | 13.8 | -11.5 |

### Example 16 - Confirmation of ORL₁ receptor activation by measuring forskolin-stimulated cAMP production

Confirmation that a given TM is acting via the ORL₁ receptor is provided by the following test, in which the TMs ability to inhibit forskolin-stimulated cAMP production is assessed.

### Materials

### [³H]adenine and [¹⁴C]cAMP are obtained from GE Healthcare

### Methods

The test is conducted essentially as described previously by Meunier et al. [Isolation and structure of the endogenous agonist of opioid receptor-like ORL1 receptor. Nature 377: 532-535, 1995] in intact transfected-CHO cells plated on 24-well plastic plates.

To the cells is added [3H]adenine (1.0 µCi) in 0.4 ml of culture medium. The cells remain at 37°C for 2 h to allow the adenine to incorporate into the intracellular ATP pool. After 2 h, the cells are washed once with incubation buffer containing: 130 mM NaCl, 4.8 mM KCI, 1.2 mM KH₂PO₄, 1.3 mM CaCl₂, 1.2 mM MgSO₄, 10 mM glucose, 1 mg/ml bovine serum albumin and 25 mM HEPES, pH 7.4, and replaced with buffer containing forskolin (10 µM) and isobutylmethylxanthine (50 µM) with or without the TM of interest. After 10 min., the medium is aspirated and replaced with 0.5 ml, 0.2 M HCl. Approximately 1000 cpm of [¹⁴C]cAMP is added to each well and used as an internal standard. The contents of the wells are then transferred to columns of 0.65 g dry alumina powder. The columns are eluted with 4 ml of 5 mM HCl, 0.5 ml of 0.1 M ammonium acetate, then two additional millilitres of ammonium acetate. The final eluate is collected into scintillation vials and counted for ¹⁴C and tritium. Amounts collected are corrected for recovery of [¹⁴C]cAMP. TMs that are agonists at the ORL₁ receptor cause a reduction in the level of cAMP produced in response to forskolin.

### Example 17 - Confirmation of ORL₁ receptor activation using a GTPyS binding functional assay

Confirmation that a given TM is acting via the ORL₁ receptor is also provided by the following test, a GTPyS binding functional assay.

### Materials

[³⁵S]GTPyS is obtained from GE Healthcare
Wheatgerm agglutinin-coated (SPA) beads are obtained from GE Healthcare

### Methods

This assay is carried out essentially as described by Traynor and Nahorski [Modulation by µ-opioid agonists of guanosine-5 -O-(3-[35S]thio)triphosphate binding to membranes from human neuroblastoma SH-SY5Y cells. Mol. Pharmacol. 47: 848-854, 1995].

Cells are scraped from tissue culture dishes into 20 mM HEPES, 1 mM ethylenediaminetetraacetic acid, then centrifuged at 500 × *g* for 10 min. Cells are resuspended in this buffer and homogenized with a Polytron Homogenizer.

The homogenate is centrifuged at 27,000 × *g* for 15 min., and the pellet resuspended in buffer A, containing: 20 mM HEPES, 10 mM MgCl₂, 100 mM NaCl, pH 7.4. The suspension is recentrifuged at 20,000 × *g* and suspended once more in buffer A. For the binding assay, membranes (8-15 µg protein) are incubated with [³⁵S]GTP S (50 pM), GDP (10 µM), with and without the TM of interest, in a total volume of 1.0 ml, for 60 min. at 25°C. Samples are filtered over glass fibre filters and counted as described for the binding assays.

### Example 18 - Preparation of a LC/A and H_{N}/A backbone clones

The following procedure creates the LC and H_{N} fragments for use as the component backbone for multidomain fusion expression. This example is based on preparation of a serotype A based clone (SEQ ID27 and SEQ ID28), though the procedures and methods are equally applicable to the other serotypes [illustrated by the sequence listing for serotype B (SEQ ID29 and SEQ ID30) and serotype C (SEQ ID31 and SEQ ID32)].

### Preparation of cloning and expression vectors

pCR 4 (Invitrogen) is the chosen standard cloning vector, selected due to the lack of restriction sequences within the vector and adjacent sequencing primer sites for easy construct confirmation. The expression vector is based on the pMAL (NEB) expression vector, which has the desired restriction sequences within the multiple cloning site in the correct orientation for construct insertion (*Bam*HI-*Sal*I-*Pst*I-*Hind*III). A fragment of the expression vector has been removed to create a non-mobilisable plasmid and a variety of different fusion tags have been inserted to increase purification options.

### Preparation of protease (e.g. LC/A) insert

The LC/A (SEQ ID27) is created by one of two ways:
The DNA sequence is designed by back translation of the LC/A amino acid sequence [obtained from freely available database sources such as GenBank (accession number P10845) or Swissprot (accession locus BXA1_CLOBO) using one of a variety of reverse translation software tools (for example EditSeq best *E*. *coli* reverse translation (DNASTAR Inc.), or Backtranslation tool v2.0 (Entelechon)]. *Bam*HI/*Sal*I recognition sequences are incorporated at the 5' and 3' ends respectively of the sequence, maintaining the correct reading frame. The DNA sequence is screened (using software such as MapDraw, DNASTAR Inc.) for restriction enzyme cleavage sequences incorporated during the back translation. Any cleavage sequences that are found to be common to those required by the cloning system are removed manually from the proposed coding sequence ensuring common *E. coli* codon usage is maintained. *E. coli* codon usage is assessed by reference to software programs such as Graphical Codon Usage Analyser (Geneart), and the %GC content and codon usage ratio assessed by reference to published codon usage tables (for example GenBank Release 143, 13 September 2004). This optimised DNA sequence containing the LC/A open reading frame (ORF) is then commercially synthesized (for example by Entelechon, Geneart or Sigma-Genosys) and is provided in the pCR 4 vector.

The alternative method is to use PCR amplification from an existing DNA sequence with *Bam*HI and *Sal*I restriction enzyme sequences incorporated into the 5' and 3' PCR primers respectively. Complementary oligonucleotide primers are chemically synthesised by a supplier (for example MWG or Sigma-Genosys), so that each pair has the ability to hybridize to the opposite strands (3' ends pointing "towards" each other) flanking the stretch of *Clostridium* target DNA, one oligonucleotide for each of the two DNA strands. To generate a PCR product the pair of short oligonucleotide primers specific for the *Clostridium* DNA sequence are mixed with the *Clostridium* DNA template and other reaction components and placed in a machine (the 'PCR machine') that can change the incubation temperature of the reaction tube automatically, cycling between approximately 94°C (for denaturation), 55°C (for oligonucleotide annealing), and 72°C (for synthesis). Other reagents required for amplification of a PCR product include a DNA polymerase (such as *Taq* or *Pfu* polymerase), each of the four nucleotide dNTP building blocks of DNA in equimolar amounts (50-200 µM) and a buffer appropriate for the enzyme optimised for Mg²⁺ concentration (0.5-5 mM).

The amplification product is cloned into pCR 4 using either, TOPO TA cloning for *Taq* PCR products or Zero Blunt TOPO cloning for *Pfu* PCR products (both kits commercially available from Invitrogen). The resultant clone is checked by sequencing. Any additional restriction sequences which are not compatible with the cloning system are then removed using site directed mutagenesis [for example, using Quickchange (Stratagene Inc.)].

### Preparation of translocation (e.g. H_{N}) insert

The H_{N}/A (SEQ ID28) is created by one of two ways:
The DNA sequence is designed by back translation of the H_{N}/A amino acid sequence [obtained from freely available database sources such as GenBank (accession number P10845) or Swissprot (accession locus BXA1_CLOBO)] using one of a variety of reverse translation software tools [for example EditSeq best *E*. *coli* reverse translation (DNASTAR Inc.), or Backtranslation tool v2.0 (Entelechon)]. A Pstl restriction sequence added to the N-terminus and *Xba*I-stop codon-*Hind*III to the C-terminus ensuring the correct reading frame is maintained. The DNA sequence is screened (using software such as MapDraw, DNASTAR Inc.) for restriction enzyme cleavage sequences incorporated during the back translation. Any sequences that are found to be common to those required by the cloning system are removed manually from the proposed coding sequence ensuring common *E*. *coli* codon usage is maintained. *E. coli* codon usage is assessed by reference to software programs such as Graphical Codon Usage Analyser (Geneart), and the %GC content and codon usage ratio assessed by reference to published codon usage tables (for example GenBank Release 143, 13 September 2004). This optimised DNA sequence is then commercially synthesized (for example by Entelechon, Geneart or Sigma-Genosys) and is provided in the pCR 4 vector.

The alternative method is to use PCR amplification from an existing DNA sequence with *Pst*I and *Xba*I-stop codon-*Hin*dIII restriction enzyme sequences incorporated into the 5' and 3' PCR primers respectively. The PCR amplification is performed as described above. The PCR product is inserted into pCR 4 vector and checked by sequencing. Any additional restriction sequences which are not compatible with the cloning system are then removed using site directed mutagenesis [for example using Quickchange (Stratagene Inc.)].

### Example 19 - Preparation of a LC/A-nociceptin-H_{N}/A fusion protein (nociceptin is N-terminal of the H_{N}-chain)

### Preparation of linker-nociceptin-spacer insert

The LC-H_{N} linker can be designed from first principle, using the existing sequence information for the linker as the template. For example, the serotype A linker (in this case defined as the inter-domain polypeptide region that exists between the cysteines of the disulphide bridge between LC and H_{N}) is 23 amino acids long and has the sequence VRGIITSKTKSLDKGYNKALNDL. Within this sequence, it is understood that proteolytic activation in nature leads to an H_{N} domain that has an N-terminus of the sequence ALNDL. This sequence information is freely available from available database sources such as GenBank (accession number P10845) or Swissprot (accession locus BXA1_CLOBO). Into this linker a Factor Xa site, nociceptin and spacer are incorporated; and using one of a variety of reverse translation software tools [for example EditSeq best *E. coli* reverse translation (DNASTAR Inc.), or Backtranslation tool v2.0 (Entelechon)], the DNA sequence encoding the linker-ligand-spacer region is determined. Restriction sites are then incorporated into the DNA sequence and can be arranged as *Bam*HI-*Sal*I-linker-protease site-nociceptin-*Nhe*I-spacer-*Spe*I-*Pst*I-*Xba*I-stop codon-*Hin*dIII (SEQ ID33). It is important to ensure the correct reading frame is maintained for the spacer, nociceptin and restriction sequences and that the *Xba*I sequence is not preceded by the bases, TC, which would result on DAM methylation. The DNA sequence is screened for restriction sequence incorporation, and any additional sequences are removed manually from the remaining sequence ensuring common *E. coli* codon usage is maintained. *E. coli* codon usage is assessed by reference to software programs such as Graphical Codon Usage Analyser (Geneart), and the %GC content and codon usage ratio assessed by reference to published codon usage tables (for example, GenBank Release 143, 13 September 2004). This optimised DNA sequence is then commercially synthesized (for example by Entelechon, Geneart or Sigma-Genosys) and is provided in the pCR 4 vector.

### Preparation of the LC/A-nociceptin-H_{N}/A fusion

In order to create the LC-linker-nociceptin-spacer-H_{N} construct (SEQ ID39), the pCR 4 vector encoding the linker (SEQ ID33) is cleaved with *Bam*HI + *Sal*I restriction enzymes. This cleaved vector then serves as the recipient vector for insertion and ligation of the LC/A DNA (SEQ ID27) cleaved with *Bam*HI + *Sal*I. The resulting plasmid DNA is then cleaved with *Pst*I + *Xba*I restriction enzymes and serves as the recipient vector for the insertion and ligation of the H_{N}/A DNA (SEQ ID28) cleaved with *Pst*I + *Xba*I*.* The final construct contains the LC-linker-nociceptin-spacer-H_{N} ORF (SEQ ID39) for transfer into expression vectors for expression to result in a fusion protein of the sequence illustrated in SEQ ID40.

### Example 20 - Preparation of a nociceptin-LC/A-H_{N}/A fusion protein (nociceptin is N-terminal of the LC-chain)

The LC/A-H_{N}/A backbone is constructed as described in Example 19 using the synthesised A serotype linker with the addition of a Factor Xa site for activation, arranged as *Bam*HI-*Sal*I-linker-protease site-linker-*Pst*I-*Xba*I-stop codon-*Hin*dIII (SEQ ID34). The LC/A-H_{N}/A backbone and the synthesised N-terminal presentation nociceptin insert (SEQ ID35) are cleaved with *Bam*HI + *Hind*III restriction enzymes, gel purified and ligated together to create a nociceptin-spacer-LC-linker-H_{N}. The ORF (SEQ ID41) is then cut out using restriction enzymes *Ava*I + *Xba*I for transfer into expression vectors for expression to result in a fusion protein of the sequence illustrated in SEQ ID42.

### Example 21 - Preparation of a LC/C-nociceptin-H_{N}/C fusion protein

Following the methods used in Examples 1 and 2, the LC/C (SEQ ID31) and H_{N}/C (SEQ ID32) are created and inserted into the C serotype linker arranged as *Bam*HI-*Sal*I-linker-protease site-nociceptin-*Nhe*I-spacer-*Spe*I-*Pst*I-*Xba*I-stop codon-*Hin*dIII (SEQ ID36). The final construct contains the LC-linker-nociceptin-spacer-H_{N} ORF (SEQ ID43) for expression as a protein of the sequence illustrated in SEQ ID44.

### Example 22 - Preparation of a LC/C-nociceptin-H_{N}/C fusion protein with a serotype A activation sequence

Following the methods used in Examples 1 and 2, the LC/C (SEQ ID31) and H_{N}/C (SEQ ID32) are created and inserted into the A serotype linker arranged as *Bam*HI-*Sal*I-linker-protease site-nociceptin-*Nhe*I-spacer-*Spe*I-*Pst*I-*Xba*I-stop codon-*Hin*dIII (SEQ ID33). The final construct contains the LC-linker-nociceptin-spacer-H_{N} ORF (SEQ ID45) for expression as a protein of the sequence illustrated in SEQ ID46.

### Example 23 - Preparation of a LC/A-met enkephalin-H_{N}/A fusion protein

Due to the small, five-amino acid, size of the met-enkephalin ligand the LC/A-met enkephalin-H_{N}/A fusion is created by site directed mutagenesis [for example using Quickchange (Stratagene Inc.)] using the LC/A-nociceptin-H_{N}/A fusion (SEQ ID39) as a template. Oligonucleotides are designed encoding the YGGFM met-enkephalin peptide, ensuring standard *E.coli* codon usage is maintained and no additional restriction sites are incorporated, flanked by sequences complimentary to the linker region of the LC/A-nociceptin-H_{N}/A fusion (SEQ ID39) either side on the nociceptin section. The SDM product is checked by sequencing and the final construct containing the LC-linker-met enkephalin-spacer-H_{N} ORF (SEQ ID47) for expression as a protein of the sequence illustrated in SEQ ID48.

### Example 24 - Preparation of a LC/A-β endorphin-H_{N}/A fusion protein

Following the methods used in Examples 1 and 2, the LC/A (SEQ ID27) and H_{N}/A (SEQ ID28) are created and inserted into the A serotype β endorphin linker arranged as *Bam*HI-*Sal*I-linker-protease site-β endorphin-*Nhe*I-spacer-*Spe*I-*Pst*I-*Xba*I-stop codon-*Hin*dIII (SEQ ID37). The final construct contains the LC-linker-β endorphin-spacer-H_{N} ORF (SEQ ID49) for expression as a protein of the sequence illustrated in SEQ ID50.

### -Example 25 - Preparation of a LC/A-nociceptin variant-H_{N}/A fusion protein

Following the methods used in Examples 1 and 2, the LC/A (SEQ ID27) and H_{N}/A (SEQ ID28) are created and inserted into the A serotype nociceptin variant linker arranged as *Bam*HI-*Sal*I-linker-protease site-nociceptin variant-*Nhe*I-spacer-*Spe*I-*Pst*I-*Xba*I-stop codon-*Hin*dIII (SEQ ID38). The final construct contains the LC-linker-nociceptin variant-spacer-H_{N} ORF (SEQ ID51) for expression as a protein of the sequence illustrated in SEQ ID52.

### Example 26 - Purification method for LC/A-nociceptin-H_{N}/A fusion protein

Defrost falcon tube containing 25 ml 50 mM HEPES pH-7.2, 200 mM NaCl and approximately 10 g of *E*. *coli* BL21 cell paste. Make the thawed cell paste up to 80 ml with 50 mM HEPES pH 7.2, 200 mM NaCl and sonicate on ice 30 seconds on, 30 seconds off for 10 cycles at a power of 22 microns ensuring the sample remains cool. Spin the lysed cells at 18 000 rpm, 4°C for 30 minutes. Load the supernatant onto a 0.1 M NiSO₄ charged Chelating column (20-30 ml column is sufficient) equilibrated with 50 mM HEPES pH 7.2, 200 mM NaCl. Using a step gradient of 10 and 40 mM imidazol, wash away the non-specific bound protein and elute the fusion protein with 100 mM imidazol. Dialyse the eluted fusion protein against 5 L of 50 mM HEPES pH 7.2, 200 mM NaCl at 4°C overnight and measure the OD of the dialysed fusion protein. Add 1 unit of factor Xa per 100 µg fusion protein and Incubate at 25°C static overnight. Load onto a 0.1 M NiSO₄ charged Chelating column (20-30 ml column is sufficient) equilibrated with 50 mM HEPES pH 7.2, 200 mM NaCl. Wash column to baseline with 50 mM HEPES pH 7.2, 200 mM NaCl. Using a step gradient of 10 and 40 mM imidazol, wash away the non-specific bound protein and elute the fusion protein with 100 mM imidazol. Dialyse the eluted fusion protein against 5 L of 50 mM HEPES pH 7.2, 200 mM NaCl at 4°C overnight and concentrate the fusion to about 2 mg/ml, aliquot sample and freeze at -20°C. Test purified protein using OD, BCA, purity analysis and SNAP-25 assessments.

### Example 27 - Preparation of a LC/A-nociceptin-H_{N}/A fusion protein (nociceptin is N-terminal of the H_{N}-chain)

The linker-nociceptin-spacer insert is prepared as described in Example 19.

### Preparation of the LC/A-nociceptin-H_{N}/A fusion

In order to create the LC-linker-nociceptin-spacer-H_{N} construct (SEQ ID39), the pCR 4 vector encoding the linker (SEQ ID33) is cleaved with *Bam*HI + *Sal*I restriction enzymes. This cleaved vector then serves as the recipient for insertion and ligation of the LC/A DNA (SEQ ID27) also cleaved with *Bam*HI + *Sal*I. The resulting plasmid DNA is then cleaved with *Bam*HI + *Hind*III restriction enzymes and the LC/A-linker fragment inserted into a similarly cleaved vector containing a unique multiple cloning site for *Bam*HI, *Sal*I, *Pst*I, and *Hind*III such as the pMAL vector (NEB). The H_{N}/A DNA (SEQ ID28) is then cleaved with *Pst*I + *Hind*III restriction enzymes and inserted into the similarly cleaved pMAL-LC/A-linker construct. The final construct contains the LC-linker-nociceptin-spacer-H_{N} ORF (SEQ ID39) for expression as a protein of the sequence illustrated in SEQ ID40.

### Example 28 - Preparation of a nociceptin-LC/A-H_{N}/A fusion protein (nociceptin is N-terminal of the LC-chain)

In order to create the nociceptin-spacer-LC/A-H_{N}/A construct, an A serotype linker with the addition of a Factor Xa site for activation, arranged as *Bam*HI-*Sal*I-linker-protease site-linker-*Pst*I-*Xba*Istop codon-*Hin*dIII (SEQ ID34) is synthesised as described in Example 27. The pCR 4 vector encoding the linker is cleaved with *Bam*HI+ *Sal*Irestriction enzymes. This cleaved vector then serves as the recipient for insertion and ligation of the LC/A DNA (SEQ ID27) also cleaved with *Bam*HI+ *Sal*I. The resulting plasmid DNA is then cleaved with *Bam*HI + *Hind*III restriction enzymes and the LC/A-linker fragment inserted into a similarly cleaved vector containing the synthesised N-terminal presentation nociceptin insert (SEQ ID35). This construct is then cleaved with *Ava*I + *Hind*III and inserted into an expression vector such as the pMAL plasmid (NEB). The H_{N}/A DNA (SEQ ID28) is then cleaved with *Pst*I + *Hind*III restriction enzymes and inserted into the similarly cleaved pMAL-nociceptin-LC/A-linker construct. The final construct contains the nociceptin-spacer-LC/A-H_{N}/A ORF (SEQ ID63) for expression as a protein of the sequence illustrated in SEQ ID64.

### Example 29 - Preparation and purification of an LC/A-nociceptin-H_{N}/A fusion protein family with variable spacer length

Using the same strategy as employed in Example 19, a range of DNA linkers were prepared that encoded nociceptin and variable spacer content. Using one of a variety of reverse translation software tools [for example EditSeq best *E. coli* reverse translation (DNASTAR Inc.), or Backtranslation tool v2.0 (Entelechon)], the DNA sequence encoding the linker-ligand-spacer region is determined. Restriction sites are then incorporated into the DNA sequence and can be arranged as *Bam*HI-SalI-linker-protease site-nociceptin-*Nhe*I-spacer-*Spe*I-*Pst*I-*Xba*I-stop codon-*Hin*dIII (SEQ ID65 to SEQ ID69). It is important to ensure the correct reading frame is maintained for the spacer, nociceptin and restriction sequences and that the *Xba*I sequence is not preceded by the bases, TC which would result on DAM methylation. The DNA sequence is screened for restriction sequence incorporation and any additional sequences are removed manually from the remaining sequence ensuring common *E. coli* codon usage is maintained. *E. coli* codon usage is assessed by reference to software programs such as Graphical Codon Usage Analyser (Geneart), and the %GC content and codon usage ratio assessed by reference to published codon usage tables (for example GenBank Release 143, 13 September 2004). This optimised DNA sequence is then commercially synthesized (for example by Entelechon, Geneart or Sigma-Genosys) and is provided in the pCR 4 vector.

The spacers that were created included:

**Table 2**

| **Code** | **Protein sequence of the linker** | **SEQ ID of the linker DNA** |
|---|---|---|
| GS10 | ALAGGGGSALVLQ | 53 |
| GS15 | ALAGGGGSGGGGSALVLQ | 54 |
| GS25 | ALAGGGGSGGGGSGGGGSGGGGSALVLQ | 55 |
| GS30 | ALAGGGGSGGGGSGGGGSGGGGSGGGGSALVLQ | 56 |
| HX27 | ALAAEAAAKEAAAKEAAAKAGGGGSALVLQ | 57 |

By way of example, in order to create the LC/A-CPN(GS15)-H_{N}/A fusion construct (SEQ ID70), the pCR 4 vector encoding the linker (SEQ ID66) is cleaved with *Bam*HI + *Sal*I restriction enzymes. This cleaved vector then serves as the recipient vector for insertion and ligation of the LC/A DNA (SEQ ID27) also cleaved with *Bam*HI + "*Sal*I. The resulting plasmid DNA is then cleaved with *Bam*HI + *Hind*III restriction enzymes and the LC/A-linker fragment inserted into a similarly cleaved vector containing a unique multiple cloning site for *Bam*HI, *Sal*I, *Pst*I, and *Hind*III such as the pMAL vector (NEB). The H_{N}/A DNA (SEQ ID28) is then cleaved with *Pst*I + *Hind*III restriction enzymes and inserted into the similarly cleaved pMAL-LC/A-linker construct. The final construct contains the LC/A-CPN(GS15)-H_{N}/A ORF (SEQ ID70) for expression as a protein of the sequence illustrated in SEQ ID71.

As a further example, to create the LC/A-CPN(GS25)-H_{N}/A fusion construct (SEQ ID72), the pCR 4 vector encoding the linker (SEQ ID67) is cleaved with *Bam*HI + *Sal*I restriction enzymes. This cleaved vector then serves as the recipient vector for insertion and ligation of the LC/A DNA (SEQ ID27) cleaved with *Bam*HI + *Sal*I. The resulting plasmid DNA is then cleaved with *Bam*HI + *Hind*III restriction enzymes and the LC/A-linkerfragment inserted into a similarly cleaved vector containing a unique multiple cloning site for *Bam*HI, *Sal*I, *Pst*I, and *Hind*III such as the pMAL vector (NEB). The H_{N}/A DNA (SEQ ID28) is then cleaved with *Pstl + Hind*III restriction enzymes and inserted into the similarly cleaved pMAL-LC/A-linker construct. The final construct contains the LC/A-CPN(GS25)-H_{N}/A ORF (SEQ ID72) for expression as a protein of the sequence illustrated in SEQ ID73.

Variants of the LC/A-CPN-H_{N}/A fusion consisting of GS10, GS30 and HX27 are similarly created. Using the purification methodology described in Example 26, fusion protein is purified from *E*. *coli* cell paste. Figure 12 illustrates the purified product obtained in the case of LC/A-CPN(GS10)-H_{N}/A, LC/A-CPN(GS15)-H_{N}/A, LC/A-CPN(GS25)-H_{N}/A, LC/A-CPN(GS30)-H_{N}/A and LC/A-CPN(HX27)-H_{N}/A.

### Example 30 - Assessment of in vitro efficacy of an LC/A-nociceptin-H_{N}/A fusion

Fusion protein prepared according to Examples 2 and 9 was assessed in the eDRG neuronal cell model.

Assays for the inhibition of substance P release and cleavage of SNAP-25 have been previously reported (Duggan et al., 2002, J. Biol. Chem., 277, 34846-34852). Briefly, dorsal root ganglia neurons are harvested from 15-day-old fetal Sprague-Dawley rats and dissociated cells plated onto 24-well plates coated with Matrigel at a density of 1 x 10⁶ cells/well. One day post-plating the cells are treated with 10 µM cytosine β-D-arabinofuranoside for 48 h. Cells are maintained in Dulbecco's minimal essential medium supplemented with 5% heat-inactivated fetal bovine serum, 5 mM L-glutamine, 0.6% D-glucose, 2% B27 supplement, and 100 ng/ml 2.5S mouse nerve growth factor. Cultures are maintained for 2 weeks at 37°C in 95% air/5% CO₂ before addition of test materials.

Release of substance P from eDRG is assessed by enzyme-linked immunosorbent assay. Briefly, eDRG cells are washed twice with low potassium-balanced salt solution (BSS: 5 mM KCl, 137 mM NaCl, 1.2 mM MgCl₂, 5 mM glucose, 0.44 mM KH₂PO₄, 20 mM HEPES, pH 7.4, 2 mM CaCl₂). Basal samples are obtained by incubating each well for 5 min. with 1 ml of low potassium BSS. After removal of this buffer, the cells are stimulated to release by incubation with 1 ml of high potassium buffer (BSS as above with modification to include 100 mM KCI isotonically balanced with NaCl) for 5 min. All samples are removed to tubes on ice prior to assay of substance P. Total cell lysates are prepared by addition of 250 µl of 2 M acetic acid/0.1% trifluoroacetic acid to lyse the cells, centrifugal evaporation, and resuspension in 500 µl of assay buffer. Diluted samples are assessed for substance P content. Substance P immunoreactivity is measured using Substance P Enzyme Immunoassay Kits (Cayman Chemical Company or R&D Systems) according to manufacturers' instructions. Substance P is expressed in pg/ml relative to a standard substance P curve run in parallel.

SDS-PAGE and Western blot analysis were performed using standard protocols (Novex). SNAP-25 proteins were resolved on a 12% Tris/glycine polyacrylamide gel (Novex) and subsequently transferred to nitrocellulose membrane. The membranes were probed with a monoclonal antibody (SMI-81) that recognises cleaved and intact SNAP-25. Specific binding was visualised using peroxidase-conjugated secondary antibodies and a chemiluminescent detection system. Cleavage of SNAP-25 was quantified by scanning densitometry (Molecular Dynamics Personal SI, ImageQuant data analysis software). Percent SNAP-25 cleavage was calculated according to the formula: (Cleaved SNAP-25/(Cleaved+Intact SNAP-25))x100.

Following exposure of eDRG neurons to an LC/A-nociceptin-H_{N}/A fusion (termed CPN-A), both inhibition of substance P release and cleavage of SNAP-25 are observed (Figure 13). After 24 h exposure to the fusion, 50% of maximal SNAP-25 cleavage is achieved by a fusion concentration of 6.3±2.5 nM.

The effect of the fusion is also assessed at defined time points following a 16 h exposure of eDRG to CPN-A. Figure 14 illustrates the prolonged duration of action of the CPN-A fusion protein, with measurable activity still being observed at 28 days post exposure.

### Example 31 - Assessment of in vitro efficacy of an LC/A-nociceptin variant-H_{N}/A fusion

Fusion protein prepared according to Examples 8 and 9 was assessed in the eDRG neuronal cell mode using the method described in Example 30.

Following exposure of eDRG neurons to an LC/A-nociceptin variant-H_{N}/A fusion (termed CPNv-A), both inhibition of substance P release and cleavage of SNAP-25 are observed. After 24 h exposure to the fusion, 50% of maximal SNAP-25 cleavage is achieved by a fusion concentration of 1.4±0.4 nM (Figure 15).

The effect of the fusion is also assessed at defined time points following a 16 h exposure of eDRG to CPN-A. Figure 16 illustrates the prolonged duration of action of the CPN-A fusion protein, with measurable activity still being observed at 24 days post exposure.

The binding capability of the CPNv-A fusion protein is also assessed in comparison to the CPN-A fusion. Figure 17 illustrates the results of a competition experiment to determine binding efficacy at the ORL-1 receptor. CPNv-A is demonstrated to displace [3H]-nociceptin, thereby confirming that access to the receptor is possible with the ligand in the central presentation format.

### Example 32 - Preparation of an LC/A-nociceptin variant-H_{N}/A fusion protein that is activated by treatment with Enterokinase

Following the methods used in Examples 1 and 2, the LC/A (SEQ ID27) and H_{N}/A (SEQ ID28) are created and inserted into the A serotype nociceptin variant linker arranged as *Bam*HI-*Sal*I-linker-enterokinase protease site-nociceptin variant-*Nhe*I-spacer-*Spe*I-*Pst*I-*Xba*I-stop codon-*Hin*dIII (SEQ ID74). The final construct contains the LC-linker-nociceptin variant-spacer-H_{N} ORF sequences (SEQ ID75) for expression as a protein of the sequence illustrated in SEQ ID76. The fusion protein is termed CPNv(Ek)-A. Figure 18 illustrates the purification of CPNv(Ek)-A from *E*. *coli* following the methods used in Example 26 but using Enterokinase for activation at 0.00064 µg per 100 µg of fusion protein.

### Example33 - Assessment of in vitro efficacy of an LC/A-nociceptin variant-H_{N}/A fusion that has been activated by treatment with enterokinase

The CPNv(Ek)-A prepared in Example 32 is obtained in a purified form and applied to the eDRG cell model to assess cleavage of SNAP-25 (using methodology from Example 30). Figure 19 illustrates the cleavage of SNAP-25 following 24 h exposure of eDRG to CPNv(Ek)-A. The efficiency of cleavage is observed to be similar to that achieved with the Factor Xa-cleaved material, as recorded in Example 31.

### Example 34 - Preparation of an LC/C-nociceptin variant-H_{N}/C fusion protein with a Factor Xa activation linker derived from serotype A

Following the methods used in Example 21, the LC/C (SEQ ID31) and H_{N}/C (SEQ ID32) are created and inserted into the A serotype nociceptin variant linker arranged as *Bam*HI-*Sal*I-linker-nociceptin variant-*Nhe*I-spacer-*Spe*I-*Pst*I-X*ba*I-stop codon-*Hin*dIII (SEQ ID77). The final construct contains the LC-linker-nociceptin variant-spacer-H_{N} ORF sequences (SEQ ID78) for expression as a protein of the sequence illustrated in SEQ ID79. The fusion protein is termed CPNv-C (act. A). Figure 20 illustrates the purification of CPNv-C (act. A) from *E. coli* following the methods used in Example 26.

### Example 35 - Assessment of in vitro efficacy of an LC/C-nociceptin variant-H_{N}/C fusion protein

Following the methods used in Example 26, the CPNv-C (act. A) prepared in Example 34 is obtained in a purified form and applied to the eDRG cell model to assess cleavage of SNAP-25 (using methodology from Example 30). After 24 h exposure to the fusion, 50% of maximal syntaxin cleavage is achieved by a fusion concentration of 3.1±2.0 nM. Figure 21 illustrates the cleavage of syntaxin following 24 h exposure of eDRG to CPNv-C (act. A).

### Example 36 - Assessment of in vivo efficacy of an LC/A-nociceptin-HN/A fusion

The ability of an LC/A-nociceptin- H_{N}/A fusion (CPN/A) to inhibit acute capsaicin-induced mechanical allodynia is evaluated following subcutaneous intraplantar injection in the rat hind paw. Test animals are evaluated for paw withdrawal frequency (PWF%) in response to a 10 g Von Frey filament stimulus series (10 stimuli x 3 trials) prior to recruitment into the study, after subcutaneous treatment with CPN/A but before capsaicin, and following capsaicin challenge post-injection of CPN/A (average of responses at 15' and 30'). Capsaicin challenge is achieved by injection of 10 µL of a 0.3% solution. Sample dilutions are prepared in 0.5% BSA/saline. Figure 22 illustrates the reversal of mechanical allodynia that is achieved by pre-treatment of the animals with a range of concentrations of LC/A-nociceptin-HN/A fusion.

The ability of an LC/A-nociceptin-HN/A fusion (CPN/A) to inhibit streptozotocin (STZ)-induced mechanical (tactile) allodynia in rats is evaluated. STZ-induced mechanical allodynia in rats is achieved by injection of streptozotocin (i.p. or i.v.) which yields destruction of pancreatic β-cells leading to loss of insulin production, with concomitant metabolic stress (hyperglycemia and hyperlipidemia). As such, STZ induces Type I diabetes. In addition, STZ treatment leads to progressive development of neuropathy, which serves as a model of chronic pain with hyperalgesia and allodynia that may reflect signs observed in diabetic humans (peripheral diabetic neuropathy).

Male Sprague-Dawley rats (250-300 g) are treated with 65 mg/kg STZ in citrate buffer (I.V.) and blood glucose and lipid are measured weekly to define the readiness of the model. Paw Withdrawal Threshold (PWT) is measured in response to a Von Frey filament stimulus series over a period of time. Allodynia is said to be established when the PWT on two consecutive test dates (separated by 1 week) measures below 6 g on the scale. At this point, rats are randomized to either a saline group (negative efficacy control), gabapentin group (positive efficacy control) or a test group (CPN/A). Test materials (20-25 µl) are injected subcutaneously as a single injection (except gabapentin) and the PWT is measured at 1 day-posttreatment and periodically thereafter over a 2-week period. Gabapentin (30 mg/kg i.p. @ 3 ml/kg injection volume) is injected daily, 2 hours prior to the start of PWT testing. Figure 23 illustrates the reversal of allodynia achieved by pre-treatment of the animals with 750 ng of CPN/A. Data were obtained over a 2-week period after a single injection of CPN/A

### Example 37 - Assessment of in vivo efficacy of an LC/A-nociceptin variant-H_{N}/A fusion

The ability of an LC/A-nociceptin variant-H_{N}/A fusion (CPNv/A) to inhibit capsaicin-induced mechanical allodynia is evaluated following subcutaneous intraplantar injection in the rat hind paw. Test animals are evaluated for paw withdrawal frequency (PWF%) in response to a 10 g Von Frey filament stimulus series (10 stimuli x 3 trials) prior to recruitment into the study (Pre-Treat); after subcutaneous intraplantar treatment with CPNv/A but before capsaicin (Pre-CAP); and following capsaicin challenge post-injection of CPNv/A (average of responses at 15' and 30'; CAP). Capsaicin challenge is achieved by injection of 10 µL of a 0.3% solution. Sample dilutions are prepared in 0.5% BSA/saline.

Figure 24 illustrates the reversal of allodynia that is achieved by pre-treatment of the animals with a range of concentrations of LC/A-nociceptin variant-H_{N}/A fusion in comparison to the reversal achieved with the addition of LC/A-nociceptin-H_{N}/A fusion. These data are expressed as a normalized paw withdrawal frequency differential, in which the difference between the peak response (post-capsaicin) and the baseline response (pre-capsaicin) is expressed as a percentage. With this analysis, it can be seen that CPNv/A is more potent than CPN/A since a lower dose of CPNv/A is required to achieve similar analgesic effect to that seen with CPN/A.

### Example 38 - Preparation of an LC/A-leu enkephalin-H_{N}/A fusion protein

Due to the small, five-amino acid, size of the leu-enkephalin ligand the LC/A-leu enkephalin-H_{N}/A fusion is created by site directed mutagenesis [for example using Quickchange (Stratagene Inc.)] using the LC/A-nociceptin-H_{N}/A fusion (SEQ ID39) as a template. Oligonucleotides are designed encoding the YGGFL leu-enkephalin peptide, ensuring standard *E. coli* codon usage is maintained and no additional restriction sites are incorporated, flanked by sequences complimentary to the linker region of the LC/A-nociceptin-H_{N}/A fusion (SEQ ID39) either side on the nociceptin section. The SDM product is checked by sequencing and the final construct containing the LC-linker-leu enkephalin-spacer-H_{N} ORF (SEQ ID80) for expression as a protein of the sequence illustrated in SEQ ID81. The fusion protein is termed CPLE-A. Figure 25 illustrates the purification of CPLE-A from *E. coli* following the methods used in Example 26.

### Example 39 - Expression and purification of an LC/A-beta-endorphin-H_{N}/A fusion protein

Following the methods used in Example 26, and with the LC/A-beta-endorphin-H_{N}/A fusion protein (termed CPBE-A) created in Example 24, the CPBE-A is purified from *E. coli*. Figure 26 illustrates the purified protein as analysed by SDS-PAGE.

### Example 40 - Preparation of an LC/A-nociceptin mutant-H_{N}/A fusion protein

Due to the single amino acid modification necessary to mutate the nociceptin sequence at position 1 from a Phe to a Tyr, the LC/A-nociceptin mutant-H_{N}/A fusion is created by site directed mutagenesis [for example using Quickchange (Stratagene Inc.)] using the LC/A-nociceptin-H_{N}/A fusion (SEQ ID39) as a template. Oligonucleotides are designed encoding tyrosine at position 1 of the nociceptin sequence, ensuring standard *E. coli* codon usage is maintained and no additional restriction sites are incorporated, flanked by sequences complimentary to the linker region of the LC/A-nociceptin-H_{N}/A fusion (SEQ ID39) either side on the nociceptin section. The SDM product is checked by sequencing and the final construct containing the LC/A-nociceptin mutant-spacer-H_{N}/A fusion ORF (SEQ ID82) for expression as a protein of the sequence illustrated in SEQ ID83. The fusion protein is termed CPOP-A. Figure 27 illustrates the purification of CPOP-A from *E*. *coli* following the methods used in Example 26.

### Example 41 - Preparation and assessment of an LC/A-nociceptin variant mutant-H_{N}/A fusion protein

Due to the single amino acid modification necessary to mutate the nociceptin sequence at position 1 from a Phe to a Tyr, the LC/A-nociceptin variant mutant-H_{N}/A fusion is created by site directed mutagenesis [for example using Quickchange (Stratagene Inc.)] using the LC/A-nociceptin variant-H_{N}/A fusion (SEQ ID51) as a template. Oligonucleotides are designed encoding tyrosine at position 1 of the nociceptin sequence, ensuring standard *E. coli*-codon usage is maintained and no additional restriction sites are incorporated, flanked by sequences complimentary to the linker region of the LC/A-nociceptin variant-H_{N}/A fusion (SEQ ID51) either side on the nociceptin section. The SDM product is checked by sequencing and the final construct containing the LC/A-nociceptin mutant-spacer-H_{N}/A fusion ORF (SEQ ID84) for expression as a protein of the sequence illustrated in SEQ ID85. The fusion protein is termed CPOPv-A. Figure 28 illustrates the purification of CPOPv-A from *E. coli* following the methods used in Example 26.

Using methodology described in Example 30, CPOPv-A is assessed for its ability to cleave SNAP-25 in the eDRG cell model. Figure 29 illustrates that CPOPv-A is able to cleave SNAP-25 in the eDRG model, achieving cleavage of 50% of the maximal SNAP-25 after exposure of the cells to approximately 5.9 nM fusion for 24 h.

### Example 42 - Preparation of an IgA protease-nociceptin variant-H_{N}/A fusion protein

The IgA protease amino acid sequence was obtained from freely available database sources such as GenBank (accession number P09790). Information regarding the structure of the *N. Gonorrhoeae* IgA protease gene is available in the literature (Pohlner et al., Gene structure and extracellular secretion of Neisseria gonorrhoeae IgA protease, Nature, 1987, 325(6103), 458-62). Using Backtranslation tool v2.0 (Entelechon), the DNA sequence encoding the IgA protease modified for *E*. *coli* expression was determined. A *Bam*HI recognition sequence was incorporated at the 5' end and a codon encoding a cysteine amino acid and *Sal*I recognition sequence were incorporated at the 3' end of the IgA DNA. The DNA sequence was screened using MapDraw, (DNASTAR Inc.) for restriction enzyme cleavage sequences incorporated during the back translation. Any cleavage sequences that are found to be common to those required for cloning were removed manually from the proposed coding sequence ensuring common *E. coli* codon usage is maintained. *E*. *coli* codon usage was assessed Graphical Codon Usage Analyser (Geneart), and the %GC content and codon usage ratio assessed by reference to published codon usage tables. This optimised DNA sequence (SEQ ID86) containing the IgA open reading frame (ORF) is then commercially synthesized.

The IgA (SEQ ID86) is inserted into the LC-linker-nociceptin variant-spacer-H_{N} ORF (SEQ ID51) using *Bam*HI and *Sal*I restriction enzymes to replace the LC with the IgA protease DNA. The final construct contains the IgA-linker-nociceptin variant-spacer-H_{N} ORF (SEQ ID87) for expression as a protein of the sequence illustrated in SEQ ID88.

### Example 43 - Preparation and assessment of a nociceptin targeted endopeptidase fusion protein with a removable histidine purification tag.

DNA was prepared that encoded a Factor Xa removable his-tag (his6), although it is clear that alternative proteases site such as Enterokinase and alternative purification tags such as longer histidine tags are also possible. Using one of a variety of reverse translation software tools [for example EditSeq best *E. col*i reverse translation (DNASTAR Inc.), or Backtranslation tool v2.0 (Entelechon)], the DNA sequence encoding the Factor Xa removable his-tag region is determined. Restriction sites are then incorporated into the DNA sequence and can be arranged as *Nhe*I-linker-*Spe*I-*Pst*I-*H_{N}*/*A*-*Xba*I-*LEIEGRSGHHHHHHStop codon-Hind*III (SEQ ID89). The DNA sequence is screened for restriction sequence incorporated and any additional sequences are-removed manually from the remaining sequence ensuring common *E*. *coli* codon usage is maintained. *E. coli* codon usage is assessed by reference to software programs such as Graphical Codon Usage Analyser (Geneart), and the %GC content and codon usage ratio assessed by reference to published codon usage tables (for example GenBank Release 143, 13 September 2004). This optimised DNA sequence is then commercially synthesized (for example by Entelechon, Geneart or Sigma-Genosys) and is provided in the pCR 4 vector. In order to create CPNv-A-FXa-HT (SEQ ID90, removable his-tag construct) the pCR 4 vector encoding the removable his-tag is cleaved with *Nhel* and *Hind*III. The *Nhe*I - *Hind*III fragment is then inserted into the LC/A-CPNv-H_{N}/A vector (SEQ ID51) that has also been cleaved by *Nhe*I and *Hind*III. The final construct contains the LC/A-linker-nociceptin variant-spacer-H_{N}-FXa-Histag-*Hind*III ORF sequences (SEQ ID90) for expression as a protein of the sequence illustrated in SEQ ID91. Figure 30 illustrates the purification of CPNv-A-FXa-HT from *E*. *coli* following the methods used in Example 26.

### Example 44 - Preparation of a leu-enkephalin targeted endopeptidase fusion protein containing a translocation domain derived from diphtheria toxin

The DNA sequence is designed by back translation of the amino acid sequence of the translocation domain of the diphtheria toxin (obtained from freely available database sources such as GenBank (accession number 1XDTT) using one of a variety of reverse translation software tools [for example EditSeq best *E*. *coli* reverse translation (DNASTAR Inc.), or Backtranslation tool v2.0 (Entelechon)]. Restriction sites are then incorporated into the DNA sequence and can be arranged as *Nhe*I-Linker-*Spe*I-*Pst*I- diphtheria translocation domain-*Xba*I-stop codon-*Hind*III (SEQ ID92). *Pst*I/*Xba*I recognition sequences are incorporated at the 5' and 3' ends of the translocation domain respectively of the sequence maintaining the correct reading frame. The DNA sequence is screened (using software such as MapDraw, DNASTAR Inc.) for restriction enzyme cleavage sequences incorporated during the back translation. Any cleavage sequences that are found to be common to those required by the cloning system are removed manually from the proposed coding sequence ensuring common *E*. *coli* codon usage is maintained. *E. coli* codon usage is assessed by reference to software programs such as Graphical Codon Usage Analyser (Geneart), and the %GC content and codon usage ratio assessed by reference to published codon usage tables (for example GenBank Release 143, 13 September 2004). This optimised DNA sequence containing the diphtheria translocation domain is then commercially synthesized as *Nhe*I-Linker-*Spe*I-*Pst*I-diphtheria translocation domain-*Xba*I-stop codon-*Hind*III I (for example by Entelechon, Geneart or Sigma-Genosys) and is provided in the pCR 4 vector (Invitrogen). The pCR 4 vector encoding the diphtheria translocation domain is cleaved with *Nhe*I and *Xb*aI. The *Nhe*I - *Xba*I fragment is then inserted into the LC/A-CPLE-H_{N}/A vector (SEQ ID80) that has also been cleaved by *Nhe*I and *Xba*I. The final construct contains the LC/A-leu-enkephalin-spacer-diphtheria translocation domain ORF sequences (SEQ ID93) for expression as a protein of the sequence illustrated in SEQ ID94.

### Example 45 - Preparation of a nociceptin variant targeted endopeptidase fusion protein containing a LC domain derived from tetanus toxin.

The DNA sequence is designed by back translation of the tetanus toxin LC amino acid sequence (obtained from freely available database sources such as GenBank (accession number X04436) using one of a variety of reverse translation software tools [for example EditSeq best *E*. *coli* reverse translation (DNASTAR Inc.), or Backtranslation tool v2.0 (Entelechon)]. *Bam*HI/*Sal*I recognition sequences are incorporated at the 5' and 3' ends respectively of the sequence maintaining the correct reading frame (SEQ ID95). The DNA sequence is screened (using software such as MapDraw, DNASTAR Inc.) for restriction enzyme cleavage sequences incorporated during the back translation. Any cleavage sequences that are found to be common to those required by the cloning system are removed manually from the proposed coding sequence ensuring common *E. coli* codon usage is maintained. *E. coli* codon usage is assessed by reference to software programs such as Graphical Codon Usage Analyser (Geneart), and the %GC content and codon usage ratio assessed by reference to published codon usage tables (for example GenBank Release 143, 13 September 2004). This optimised DNA sequence containing the tetanus toxin LC open reading frame (ORF) is then commercially synthesized (for example by Entelechon, Geneart or Sigma-Genosys) and is provided in the pCR 4 vector (invitrogen). The pCR 4 vector encoding the TeNT LC is cleaved with *Bam*HI and *Sal*I. The *Bam*HI - *Sal*I fragment is then inserted into the LC/A-CPNv-H_{N}/A vector (SEQ ID51) that has also been cleaved by *Bam*HI and *Sal*I. The final construct contains the TeNT LC-linker-nociceptin variant-spacer-H_{N} ORF sequences (SEQ ID96) for expression as a protein of the sequence illustrated in SEQ ID97.

### Example 46 - Preparation of an LC/C-nociceptin variant-H_{N}/C fusion protein with a native serotype C linker that is susceptible to Factor Xa cleavage

Following the methods used in Example 21, the LC/C (SEQ ID31) and H_{N}/C (SEQ ID32) are created and inserted into the C serotype nociceptin variant linker arranged as *Bam*HI-*Sal*I-linker-nociceptin variant-*Nhe*I-spacer-*Spe*I-*Pst*I-*Xba*I-stop codon-*Hin*dIII (SEQ ID98). The final construct contains the LC-linker-nociceptin variant-spacer-H_{N} ORF sequences (SEQ ID99) for expression as a protein of the sequence illustrated in SEQ ID100. The fusion protein is termed CPNv-C (act. C).

## Claims

1. A non-cytotoxic protein conjugate for inhibition or reduction of exocytic fusion in a nociceptive sensory afferent cell, comprising:
(i) a nociceptin Targeting Moiety (TM),
wherein said TM is an agonist of a receptor present on said nociceptive sensory afferent cell, and wherein said receptor undergoes endocytosis to be incorporated into an endosome within the nociceptive sensory afferent cell;
(ii) a non-cytotoxic protease or a fragment thereof,
wherein the protease or protease fragment is capable of cleaving a protein of the exocytic fusion apparatus of said nociceptive sensory afferent cell; and
(iii) a Translocation Domain,
wherein the Translocation Domain translocates the protease or protease fragment from within the endosome, across the endosomal membrane, and into the cytosol of the nociceptive sensory afferent cell,
wherein the nociceptin TM, the Translocation Domain and the non-cytotoxic protease or fragment thereof are covalently linked.

2. The non-cytotoxic conjugate of Claim 1, wherein the receptor is an ORL₁ receptor.

3. The non-cytotoxic conjugate of Claim 1 or 2, wherein the TM has at least 70% or at least 80% homology to SEQ ID NO. 2 or a fragment thereof.

4. The non-cytotoxic conjugate of Claim 1 or 2, wherein the TM has at least 90% homology to SEQ ID NO. 2 or a fragment thereof; or wherein the TM has at least 95% homology to SEQ ID NO. 2 or a fragment thereof.

5. The non-cytotoxic conjugate of Claim 1 or Claim 2, wherein the TM is SEQ ID NO. 2 or a fragment thereof.

6. The non-cytotoxic conjugate of Claim 1 or Claim 2, wherein the TM is a nociceptin selected from the group consisting of SEQ ID NOs. 4, 6, 8, 10, 12, 14.

7. The non-cytotoxic conjugate of Claim 1 or Claim 2, wherein the TM has at least 70% or at least 80% or at least 90% or at least 95% homologoy to SEQ ID NO. 14 or a fragment thereof.

8. The non-cytotoxic conjugate of any preceding claim, wherein the non-cytotoxic protease is a bacterial protein, or a fragment thereof, capable of cleaving a protein of the exocytic fusion apparatus of the nociceptive sensory afferent cell.

9. The non-cytotoxic conjugate of Claim 8, wherein the non-cytotoxic protease is selected from a clostridial neurotoxin, or an IgA protease.

10. The non-cytotoxic conjugate of any preceding claim, wherein the Translocation Domain is derived from a clostridial source;
preferably wherein the Translocation Doman is a botulinum H_{N} domain.

11. The non-cytotoxic conjugate of any preceding claim, wherein the nociceptive sensory afferent cell is a primary nociceptive sensory afferent cell.

12. The non-cytotoxic conjugate according to Claim 1, wherein said conjugate comprises an amino acid sequence selected from the group consisting of SEQ ID NOs. 14, 16, 18, 20, 22, 24, and 26.

13. A pharmaceutical composition, comprising a conjugate according to any of Claims 1 to 12 and a pharmaceutically acceptable carrier.

14. A DNA construct encoding the conjugate of any of Claims 1 to 12.

15. A DNA construct according to Claim 14, wherein the construct comprises a DNA sequence selected from SEQ ID NOs. 13, 15, 17, 19, 21, 23, and 25.

16. A method of preparing the conjugate of any of Claims 1 to 12, comprising expressing the DNA construct of Claim 15 in a host cell.

17. Use of a conjugate according to any of Claims 1-12 or a composition according to Claim 13, for the manufacture of a medicament for treating pain.

18. A conjugate according to any of Claims 1-12 or a composition according to Claim 13, for use in treating pain.

## Patentansprüche

1. Nicht-zytotoxisches Protein-Konjugat zur Hemmung oder Verringerung der Exozytose-Fusion in einer nozizeptiven sensorischen afferenten Zelle, das Folgendes umfasst:
(i) eine Nociceptin-Zielrichtungseinheit (Targeting Moiety - TM),
wobei die TM ein Agonist eines Rezeptors ist, der auf der nocioeptiven sensorischen afferenten Zelle vorhanden ist, und wobei der Rezeptor eine Endocytose durchläuft, um in ein Endosom in der nozizeptiven sensorischen afferenten Zelle eingebracht zu werden;
(ii) eine nicht-zytotoxische Protease oder ein Fragment davon,
wobei die Protease oder das Proteasefragment ein Protein der exozytischen Fusionsvorrichtung der nozizeptiven sensorischen afferenten Zelle spalten kann und
(iii)eine Translokationsdomäne,
wobei die Translokationsdomäne die Protease oder das Proteasefragment aus dem Endosom durch die endosomale Membran in das Zytosol der nozizeptiven sensorischen afferenten Zelle transloziert,
wobei die Nociceptin-TM, die Translokationsdomäne und die nicht-zytotoxische Protease oder das Fragment davon kovalent verknüpft sind.

2. Nicht-zytotoxisches Konjugat nach Anspruch 1, wobei der Rezeptor ein ORL₁-Rezeptor ist.

3. Nicht-zytotoxisches Konjugat nach Anspruch 1 oder 2, wobei die TM mindestens eine 70%ige oder mindestens eine 80%ige Homologie zu SEQ ID Nr. 2 oder einem Fragment davon aufweist.

4. Nicht-zytotoxisches Konjugat nach Anspruch 1 oder 2, wobei die TM mindestens eine 90%ige Homologie zu SEQ ID Nr. 2 oder einem Fragment davon aufweist, oder wobei die TM mindestens eine 95%ige Homologie zu SEQ ID Nr. 2 oder einem Fragment davon aufweist.

5. Nicht-zytotoxisches Konjugat nach Anspruch 1 oder Anspruch 2, wobei die TM die SEQ ID Nr. 2 oder ein Fragment davon ist.

6. Nicht-zytotoxisches Konjugat nach Anspruch 1 oder Anspruch 2, wobei die TM ein Nociceptin ist, das aus der Gruppe ausgewählt wird, die aus SEQ ID Nrn. 4, 6, 8, 10, 12, 14 besteht.

7. Nicht-zytotoxisches Konjugat nach Anspruch 1 oder Anspruch 2, wobei die TM mindestens eine 70%ige oder mindestens eine 80%ige oder mindestens eine 90%ige oder mindestens eine 95%ige Homologie zu SEQ ID Nr. 14 oder einem Fragment davon aufweist.

8. Nicht-zytotoxisches Konjugat nach einem der vorhergehenden Ansprüche, wobei die nicht-zytotoxische Protease ein bakterielles Protein oder ein Fragment davon ist, das ein Protein der exozytischen Fusionsvorrichtung der nozizeptiven sensorischen afferenten Zelle spalten kann.

9. Nicht-zytotoxisches Konjugat nach Anspruch 8, wobei die nicht-zytotoxische Protease aus einem Clostridien-Neurotoxin oder einer IgA-Protease ausgewählt wird.

10. Nicht-zytotoxisches Konjugat nach einem der vorhergehenden Ansprüche, wobei die Translokationsdomäne von einer Clostridien-Quelle abgeleitet ist;
wobei die Translokationsdomäne vorzugsweise eine Botulinum-H_{N}-Domäne ist.

11. Nicht-zytotoxisches Konjugat nach einem der vorhergehenden Ansprüche, wobei die nozizeptive sensorische afferente Zelle eine primäre nozizeptive sensorische afferente Zelle ist.

12. Nicht-zytotoxisches Konjugat nach Anspruch 1, wobei das Konjugat eine Aminosäuresequenz umfasst, die aus der Gruppe ausgewählt wird, die aus SEQ ID Nrn. 14, 16, 18, 20, 22, 24 und 26 besteht.

13. Pharmazeutische Zusammensetzung, die ein Konjugat nach einem der Ansprüche 1 bis 12 und einen pharmazeutisch akzeptablen Träger umfasst.

14. DNA-Konstrukt, das das Konjugat nach einem der Ansprüche 1 bis 12 codiert.

15. DNA-Konstrukt nach Anspruch 14, wobei das Konstrukt eine DNA-Sequenz umfasst, die aus SEQ ID Nrn. 13, 15, 17, 19, 21, 23 und 25 ausgewählt ist.

16. Verfahren zur Herstellung des Konjugats nach einem der Ansprüche 1 bis 12, das das Exprimieren des DNA - Konstrukts nach Anspruch 15 in einer Wirtszelle umfasst.

17. Verwendung eines Konjugats nach einem der Ansprüche 1 - 12 oder einer Zusammensetzung nach Anspruch 13 zur Herstellung eines Medikaments zur Schmerzbehandlung.

18. Konjugat nach einem der Ansprüche 1 - 12 oder eine Zusammensetzung nach Anspruch 13 zur Verwendung bei der Schmerzbehandlung.

## Revendications

1. Conjugué protéique non cytotoxique pour l'inhibition ou la réduction de la fusion exocytique dans une cellule afférente sensorielle nociceptive, comprenant :
(i) une fraction de ciblage (TM) nociceptine,
ladite TM étant un agoniste d'un récepteur présent sur ladite cellule afférente sensorielle nociceptive, et ledit récepteur subissant une endocytose pour son incorporation dans un endosome à l'intérieur de la cellule afférente sensorielle nociceptive ;
(ii) une protéase non cytotoxique ou un fragment de celle-ci,
la protéase ou le fragment de protéase étant capable de cliver une protéine de l'appareil de fusion exocytique de ladite cellule afférente sensorielle nociceptive ; et
(iii)un domaine de translocation,
le domaine de translocation assurant la translocation de la protéase ou du fragment de protéase à partir de l'intérieur de l'endosome, provoquant son passage à travers la membrane endosomale et dans le cytosol de la cellule afférente sensorielle nociceptive,
la TM nociceptine, le domaine de translocation et la protéase non cytotoxique ou le fragment de celle-ci étant liés par des liaisons covalentes.

2. Conjugué non cytotoxique selon la revendication 1, dans lequel le récepteur est un récepteur ORL₁.

3. Conjugué non cytotoxique selon la revendication 1 ou 2, dans lequel la TM a au moins 70 % ou au moins 80 % d'homologie avec la SÉQ n° 2 ou un fragment de celle-ci.

4. Conjugué non cytotoxique selon la revendication 1 ou 2, dans lequel la TM a au moins 90 % d'homologie avec la SÉQ n° 2 ou un fragment de celle-ci ; ou dans lequel la TM a au moins 95 % d'homologie avec la SÉQ n° 2 ou un fragment de celle-ci.

5. Conjugué non cytotoxique selon la revendication 1 ou la revendication 2, dans lequel la TM est la SÉQ n° 2 ou un fragment de celle-ci.

6. Conjugué non cytotoxique selon la revendication 1 ou la revendication 2, dans lequel la TM est une nociceptine sélectionnée dans le groupe constitué des SÉQ n^{os} 4, 6, 8, 10, 12, 14.

7. Conjugué non cytotoxique selon la revendication 1 ou la revendication 2, dans lequel la TM a au moins 70 % ou au moins 80 % ou au moins 90 % ou au moins 95 % d'homologie avec la SÉQ n° 14 ou un fragment de celle-ci.

8. Conjugué non cytotoxique selon l'une quelconque des revendications précédentes, dans lequel la protéase non cytotoxique est une protéine bactérienne, ou un fragment de celle-ci, capable de cliver une protéine de l'appareil de fusion exocytique de la cellule afférente sensorielle nociceptive.

9. Conjugué non cytotoxique selon la revendication 8, dans lequel la protéase non cytotoxique est sélectionnée parmi une neurotoxine clostridienne, ou une protéase IgA.

10. Conjugué non cytotoxique selon l'une quelconque des revendications précédentes, dans lequel le domaine de translocation est dérivé d'une source clostridienne ;
préférablement dans lequel le domaine de translocation est un domaine H_{N} de botulinum.

11. Conjugué non cytotoxique selon l'une quelconque des revendications précédentes, dans lequel la cellule afférente sensorielle nociceptive est une cellule afférente sensorielle nociceptive primaire.

12. Conjugué non cytotoxique selon la revendication 1, ledit conjugué comprenant une séquence d'acides aminés sélectionnée dans le groupe constitué des SÉQ n^{os} 14, 16, 18, 20, 22, 24, et 26.

13. Composition pharmaceutique, comprenant un conjugué selon l'une quelconque des revendications 1 à 12 et un support pharmaceutiquement acceptable.

14. Segment d'ADN codant pour le conjugué selon l'une quelconque des revendications 1 à 12.

15. Segment d'ADN selon la revendication 14, le segment comprenant une séquence d'ADN sélectionnée parmi les SÉQ n^{os} 13, 15, 17, 19, 21, 23, et 25.

16. Procédé de préparation du conjugué selon l'une quelconque des revendications 1 à 12, comprenant l'expression du segment d'ADN selon la revendication 15 dans une cellule hôte.

17. Utilisation d'un conjugué selon l'une quelconque des revendications 1 à 12 ou d'une composition selon la revendication 13 pour la fabrication d'un médicament destiné au traitement de la douleur.

18. Conjugué selon l'une quelconque des revendications 1 à 12 ou composition selon la revendication 13, destiné(e) à une utilisation dans le traitement de la douleur.


### SEQUENCE LISTING

<110> Health Protection Agency Allergan, Inc. Chaddock, John Foster, Keith Penn, Charles Aoki, K Roger Francis, Joseph Steward, Lance
<120> Re-targeted toxin conjugates
<130> P27211WO/MRM
<150> GB0426394.3
   <151> 2004-12-01
<160> 100
<170> PatentIn version 3.3
<210> 1
   <211> 51
   <212> DNA
   <213> Artificial Sequence
<220>
   <223> Synthetic
<400> 1
   tttggcggtt tcacgggcgc acgcaaatca gcgcgtaaat tagctaacca g 51
<210> 2
   <211> 17
   <212> PRT
   <213> Artificial Sequence
<220>
   <223> Synthetic
<400> 2
   Phe Gly Gly Phe Thr Gly Ala Arg Lys Ser Ala Arg Lys Leu Ala Asn
   1 5 10 15
   Gln
<210> 3
   <211> 33
   <212> DNA
   <213> Artificial Sequence
<220>
   <223> Synthetic
<400> 3
   tttggcggtt tcacgggcgc acgcaaatca gcg 33
<210> 4
   <211> 11
   <212> PRT
   <213> Artificial Sequence
<220>
   <223> Synthetic
<400> 4
   Phe Gly Gly Phe Thr Gly Ala Arg Lys Ser Ala
   1 5 10
<210> 5
   <211> 33
   <212> DNA
   <213> Artificial Sequence
<220>
   <223> Synthetic
<400> 5
   tttggcggtt tcacgggcgc acgcaaatat gcg- 33
<210> 6
   <211> 11
   <212> PRT
   <213> Artificial Sequence
<220>
   <223> Synthetic
<400> 6
   Phe Gly Gly Phe Thr Gly Ala Arg Lys Tyr Ala
   1               5                   10
<210> 7
   <211> 33
   <212> DNA
   <213> Artificial Sequence
<220>
   <223> Synthetic
<400> 7
   tttggcggtt tcacgggcgc acgcaaatca tat 33
<210> 8
   <211> 11
   <212> PRT
   <213> Artificial Sequence
<220>
   <223> Synthetic
<400> 8
   Phe Gly Gly Phe Thr Gly Ala Arg Lys Ser Tyr
   1               5                   10
<210> 9
   <211> 51
   <212> DNA
   <213> Artificial Sequence
<220>
   <223> Synthetic
<400> 9
   tttggcggtt tcacgggcgc acgcaaatca gcgcgtaaat atgctaacca g 51
<210> 10
   <211> 17
   <212> PRT
   <213> Artificial Sequence
<220>
   <223> Synthetic
<400> 10
   Phe Gly Gly Phe Thr Gly Ala Arg Lys Ser Ala Arg Lys Tyr Ala Asn
   1 5 10 15
   Gln
<210> 11
   <211> 39
   <212> DNA
   <213> Artificial Sequence
<220>
   <223> Synthetic
<400> 11
   tttggcggtt tcacgggcgc acgcaaatca gcgcgtaaa 39
<210> 12
   <211> 13
   <212> PRT
   <213> Artificial Sequence
<220>
   <223> Synthetic
<400> 12
   Phe Gly Gly Phe Thr Gly Ala Arg Lys Ser Ala Arg Lys
   1 5 10
<210> 13
   <211> 51
   <212> DNA
   <213> Artificial Sequence
<220>
   <223> Synthetic
<400> 13
   tttggcggtt tcacgggcgc acgcaaatca gcgcgtaaac gcaaaaacca g 51
<210> 14
   <211> 17
   <212> PRT
   <213> Artificial Sequence
<220>
   <223> Synthetic
<400> 14
   Phe Gly Gly Phe Thr Gly Ala Arg Lys Ser Ala Arg Lys Arg Lys Asn
   1               5                   10                  15
   Gln
<210> 15
   <211> 2736
   <212> DNA
   <213> Artificial Sequence
<220>
   <223> Synthetic
<400> 15
   ctcgggattg agggtcgttt tggcggtttc acgggcgcac gcaaatcagc gcgtaaatta     60
   gctaaccaga ctagtggcgg tgggggtagt ggcggtggcg gttcgggcgg gggtgggagc    120
   cctaggggat ccatggagtt cgttaacaaa cagttcaact ataaagaccc agttaacggt    180
   gttgacattg cttacatcaa aatcccgaac gctggccaga tgcagccggt aaaggcattc    240
   aaaatccaca acaaaatctg ggttatcccg gaacgtgata cctttactaa cccggaagaa    300
   ggtgacctga acccgccacc ggaagcgaaa caggtgccgg tatcttacta tgactccacc    360
   tacctgtcta ccgataacga aaaggacaac tacctgaaag gtgttactaa actgttcgag    420
   cgtatttact ccaccgacct gggccgtatg ctgctgacta gcatcgttcg cggtatcccg    480
   ttctggggcg gttctaccat cgataccgaa ctgaaagtaa tcgacactaa ctgcatcaac    540
   gttattcagc cggacggttc ctatcgttcc gaagaactga acctggtgat catcggcccg    600
   tctgctgata tcatccagtt cgagtgtaag agctttggtc acgaagttct gaacctcacc 660
   cgtaacggct acggttccac tcagtacatc cgtttctctc cggacttcac cttcggtttt 720
   gaagaatccc tggaagtaga cacgaaccca ctgctgggcg ctggtaaatt cgcaactgat 780
   cctgcggtta ccctggctca cgaactgatt catgcaggcc accgcctgta cggtatcgcc 840
   atcaatccga accgtgtctt caaagttaac accaacgcgt attacgagat gtccggtctg 900
   gaagttagct tcgaagaact gcgtactttt ggcggtcacg acgctaaatt catcgactct 960
   ctgcaagaaa acgagttccg tctgtactac tataacaagt tcaaagatat cgcatccacc 1020
   ctgaacaaag cgaaatccat cgtgggtacc actgcttctc tccagtacat gaagaacgtt 1080
   tttaaagaaa aatacctgct cagcgaagac acctccggca aattctctgt agacaagttg 1140
   aaattcgata aactttacaa aatgctgact gaaatttaca ccgaagacaa cttcgttaag 1200
   ttctttaaag ttctgaaccg caaaacctat ctgaacttcg acaaggcagt attcaaaatc 1260
   aacatcgtgc cgaaagttaa ctacactatc tacgatggtt tcaacctgcg taacaccaac 1320
   ctggctgcta attttaacgg ccagaacacg gaaatcaaca acatgaactt cacaaaactg 1380
   aaaaacttca ctggtctgtt cgagttttac aagctgctgt gcgtcgacgg catcattacc 1440
   tccaaaacta aatctctgat agaaggtaga aacaaagcgc tgaacgacct ctgtatcaag 1500
   gttaacaact gggatttatt cttcagcccg agtgaagaca acttcaccaa cgacctgaac 1560
   aaaggtgaag aaatcacctc agatactaac atcgaagcag ccgaagaaaa catctcgctg 1620
   gacctgatcc agcagtacta cctgaccttt aatttcgaca acgagccgga aaacatttct 1680
   atcgaaaacc tgagctctga tatcatcggc cagctggaac tgatgccgaa catcgaacgt 1740
   ttcccaaacg gtaaaaagta cgagctggac aaatatacca tgttccacta cctgcgcgcg 1800
   caggaatttg aacacggcaa atcccgtatc gcactgacta actccgttaa cgaagctctg 1860
   ctcaacccgt cccgtgtata caccttcttc tctagcgact acgtgaaaaa ggtcaacaaa 1920
   gcgactgaag ctgcaatgtt cttgggttgg gttgaacagc ttgtttatga ttttaccgac 1980
   gagacgtccg aagtatctac taccgacaaa attgcggata tcactatcat catcccgtac 2040
   atcggtccgg ctctgaacat tggcaacatg ctgtacaaag acgacttcgt tggcgcactg 2100
   atcttctccg gtgcggtgat cctgctggag ttcatcccgg aaatcgccat cccggtactg 2160
   ggcacctttg ctctggtttc ttacattgca aacaaggttc tgactgtaca aaccatcgac 2220
   aacgcgctga gcaaacgtaa cgaaaaatgg gatgaagttt acaaatatat cgtgaccaac 2280
   tggctggcta aggttaatac tcagatcgac ctcatccgca aaaaaatgaa agaagcactg 2340
   gaaaaccagg cggaagctac caaggcaatc attaactacc agtacaacca gtacaccgag 2400
   gaagaaaaaa acaacatcaa cttcaacatc gacgatctgt cctctaaact gaacgaatcc 2460
   atcaacaaag ctatgatcaa catcaacaag ttcctgaacc agtgctctgt aagctatctg 2520
   atgaactcca tgatcccgta cggtgttaaa cgtctggagg acttcgatgc gtctctgaaa 2580
   gacgccctgc tgaaatacat ttacgacaac cgtggcactc tgatcggtca ggttgatcgt 2640
   ctgaaggaca aagtgaacaa taccttatcg accgacatcc cttttcagct cagtaaatat 2700
   gtcgataacc aacgcctttt gtccactcta gactag 2736
<210> 16
   <211> 911
   <212> PRT
   <213> Artificial Sequence
<220>
   <223> Synthetic
<400> 16
   Leu Gly Ile Glu Gly Arg Phe Gly Gly Phe Thr Gly Ala Arg Lys Ser
   1               5                   10                  15
   Ala Arg Lys Leu Ala Asn Gln Thr Ser Gly Gly Gly Gly Ser Gly Gly
               20                  25                  30
   Gly Gly Ser Gly Gly Gly Gly Ser Pro Arg Gly Ser Met Glu Phe Val
           35                  40                  45
   Asn Lys Gln Phe Asn Tyr Lys Asp Pro Val Asn Gly Val Asp Ile Ala
       50                  55                  60
   Tyr Ile Lys Ile Pro Asn Ala Gly Gln Met Gln Pro Val Lys Ala Phe
   65                  70                  75                  80
   Lys Ile His Asn Lys Ile Trp Val Ile Pro Glu Arg Asp Thr Phe Thr
                   85                  90                  95
   Asn Pro Glu Glu Gly Asp Leu Asn Pro Pro Pro Glu Ala Lys Gln Val
               100                 105                 110
   Pro Val Ser Tyr Tyr Asp Ser Thr Tyr Leu Ser Thr Asp Asn Glu Lys
           115                 120                 125
   Asp Asn Tyr Leu Lys Gly Val Thr Lys Leu Phe Glu Arg Ile Tyr Ser
       130                 135                 140
   Thr Asp Leu Gly Arg Met Leu Leu Thr Ser Ile Val Arg Gly Ile Pro
   145                 150                 155                 160
   Phe Trp Gly Gly Ser Thr Ile Asp Thr Glu Leu Lys Val Ile Asp Thr
   165 170 175
   Asn Cys Ile Asn Val Ile Gln Pro Asp Gly Ser Tyr Arg Ser Glu Glu
   180 185 190
   Leu Asn Leu Val Ile Ile Gly Pro Ser Ala Asp Ile Ile Gln Phe Glu
   195 200 205
   Cys Lys Ser Phe Gly His Glu Val Leu Asn Leu Thr Arg Asn Gly Tyr
   210 215 220
   Gly Ser Thr Gln Tyr Ile Arg Phe Ser Pro Asp Phe Thr Phe Gly Phe
   225 230 235 240
   Glu Glu Ser Leu Glu Val Asp Thr Asn Pro Leu Leu Gly Ala Gly Lys
   245 250 255
   Phe Ala Thr Asp Pro Ala Val Thr Leu Ala His Glu Leu Ile His Ala
   260 265 270
   Gly His Arg Leu Tyr Gly Ile Ala Ile Asn Pro Asn Arg Val Phe Lys
   275 280 285
   Val Asn Thr Asn Ala Tyr Tyr Glu Met Ser Gly Leu Glu Val Ser Phe
   290 295 300
   Glu Glu Leu Arg Thr Phe Gly Gly His Asp Ala Lys Phe Ile Asp Ser
   305 310 315 320
   Leu Gln Glu Asn Glu Phe Arg Leu Tyr Tyr Tyr Asn Lys Phe Lys Asp
   325 330 335
   Ile Ala Ser Thr Leu Asn Lys Ala Lys Ser Ile Val Gly Thr Thr Ala
   340 345 350
   Ser Leu Gln Tyr Met Lys Asn Val Phe Lys Glu Lys Tyr Leu Leu Ser
   355 360 365
   Glu Asp Thr Ser Gly Lys Phe Ser Val Asp Lys Leu Lys Phe Asp Lys
   370 375 380
   Leu Tyr Lys Met Leu Thr Glu Ile Tyr Thr Glu Asp Asn Phe Val Lys
   385 390 395 400
   Phe Phe Lys Val Leu Asn Arg Lys Thr Tyr Leu Asn Phe Asp Lys Ala
   405 410 415
   Val Phe Lys Ile Asn Ile Val Pro Lys Val Asn Tyr Thr Ile Tyr Asp
   420 425 430
   Gly Phe Asn Leu Arg Asn Thr Asn Leu Ala Ala Asn Phe Asn Gly Gln
   435 440 445
   Asn Thr Glu Ile Asn Asn Met Asn Phe Thr Lys Leu Lys Asn Phe Thr
   450 455 460
   Gly Leu Phe Glu Phe Tyr Lys Leu Leu Cys Val Asp Gly Ile Ile Thr
   465 470 475 480
   Ser Lys Thr Lys Ser Leu Ile Glu Gly Arg Asn Lys Ala Leu Asn Asp
   485 490 495
   Leu Cys Ile Lys Val Asn Asn Trp Asp Leu Phe Phe Ser Pro Ser Glu
   500 505 510
   Asp Asn Phe Thr Asn Asp Leu Asn Lys Gly Glu Glu Ile Thr Ser Asp
   515 520 525
   Thr Asn Ile Glu Ala Ala Glu Glu Asn Ile Ser Leu Asp Leu Ile Gln
   530 535 540
   Gln Tyr Tyr Leu Thr Phe Asn Phe Asp Asn Glu Pro Glu Asn Ile Ser
   545 550 555 560
   Ile Glu Asn Leu Ser Ser Asp Ile Ile Gly Gln Leu Glu Leu Met Pro
   565 570 575
   Asn Ile Glu Arg Phe Pro Asn Gly Lys Lys Tyr Glu Leu Asp Lys Tyr
   580 585 590
   Thr Met Phe His Tyr Leu Arg Ala Gln Glu Phe Glu His Gly Lys Ser
   595 600 605
   Arg Ile Ala Leu Thr Asn Ser Val Asn Glu Ala Leu Leu Asn Pro Ser
   610 615 620
   Arg Val Tyr Thr Phe Phe Ser Ser Asp Tyr Val Lys Lys Val Asn Lys
   625 630 635 640
   Ala Thr Glu Ala Ala Met Phe Leu Gly Trp Val Glu Gln Leu Val Tyr
   645 650 655
   Asp Phe Thr Asp Glu Thr Ser Glu Val Ser Thr Thr Asp Lys Ile Ala
   660 665 670
   Asp Ile Thr Ile Ile Ile Pro Tyr Ile Gly Pro Ala Leu Asn Ile Gly
   675 680 685
   Asn Met Leu Tyr Lys Asp Asp Phe Val Gly Ala Leu Ile Phe Ser Gly
   690 695 700
   Ala Val Ile Leu Leu Glu Phe Ile Pro Glu Ile Ala Ile Pro Val Leu
   705 710 715 720
   Gly Thr Phe Ala Leu Val Ser Tyr Ile Ala Asn Lys Val Leu Thr Val
   725 730 735
   Gln Thr Ile Asp Asn Ala Leu Ser Lys Arg Asn Glu Lys Trp Asp Glu
   740 745 750
   Val Tyr Lys Tyr Ile Val Thr Asn Trp Leu Ala Lys Val Asn Thr Gln
   755 760 765
   Ile Asp Leu Ile Arg Lys Lys Met Lys Glu Ala Leu Glu Asn Gln Ala
   770 775 780
   Glu Ala Thr Lys Ala Ile Ile Asn Tyr Gln Tyr Asn Gln Tyr Thr Glu
   785 790 795 800
   Glu Glu Lys Asn Asn Ile Asn Phe Asn Ile Asp Asp Leu Ser Ser Lys
   805 810 815
   Leu Asn Glu Ser Ile Asn Lys Ala Met Ile Asn Ile Asn Lys Phe Leu
   820 825 830
   Asn Gln Cys Ser Val Ser Tyr Leu Met Asn Ser Met Ile Pro Tyr Gly
   835 840 845
   Val Lys Arg Leu Glu Asp Phe Asp Ala Ser Leu Lys Asp Ala Leu Leu
   850 855 860
   Lys Tyr Ile Tyr Asp Asn Arg Gly Thr Leu Ile Gly Gln Val Asp Arg
   865 870 875 880
   Leu Lys Asp Lys Val Asn Asn Thr Leu Ser Thr Asp Ile Pro Phe Gln
   885 890 895
   Leu Ser Lys Tyr Val Asp Asn Gln Arg Leu Leu Ser Thr Leu Asp
   900 905 910
<210> 17
   <211> 2700
   <212> DNA
   <213> Artificial Sequence
<220>
   <223> Synthetic
<400> 17
   tttggcggtt tcacgggcgc acgcaaatca tatactagtg gcggtggggg tagtggcggt    60
   ggcggttcgg gcgggggtgg gagccctagg ggatccatgg agttcgttaa caaacagttc   120
   aactataaag acccagttaa cggtgttgac attgcttaca tcaaaatccc gaacgctggc   180
   cagatgcagc cggtaaaggc attcaaaatc cacaacaaaa tctgggttat cccggaacgt   240
   gataccttta ctaacccgga agaaggtgac ctgaacccgc caccggaagc gaaacaggtg   300
   ccggtatctt actatgactc cacctacctg tctaccgata acgaaaagga caactacctg   360
   aaaggtgtta ctaaactgtt cgagcgtatt tactccaccg acctgggccg tatgctgctg   420
   actagcatcg ttcgcggtat cccgttctgg ggcggttcta ccatcgatac cgaactgaaa   480
   gtaatcgaca ctaactgcat caacgttatt cagccggacg gttcctatcg ttccgaagaa   540
   ctgaacctgg tgatcatcgg cccgtctgct gatatcatcc agttcgagtg taagagcttt   600
   ggtcacgaag ttctgaacct cacccgtaac ggctacggtt ccactcagta catccgtttc   660
   tctccggact tcaccttcgg ttttgaagaa tccctggaag tagacacgaa cccactgctg   720
   ggcgctggta aattcgcaac tgatcctgcg gttaccctgg ctcacgaact gattcatgca   780
   ggccaccgcc tgtacggtat cgccatcaat ccgaaccgtg tcttcaaagt taacaccaac   840
   gcgtattacg agatgtccgg tctggaagtt agcttcgaag aactgcgtac ttttggcggt   900
   cacgacgcta aattcatcga ctctctgcaa gaaaacgagt tccgtctgta ctactataac   960
   aagttcaaag atatcgcatc caccctgaac aaagcgaaat ccatcgtggg taccactgct  1020
   tctctccagt acatgaagaa cgtttttaaa gaaaaatacc tgctcagcga agacacctcc  1080
   ggcaaattct ctgtagacaa gttgaaattc gataaacttt acaaaatgct gactgaaatt  1140
   tacaccgaag acaacttcgt taagttcttt aaagttctga accgcaaaac ctatctgaac  1200
   ttcgacaagg cagtattcaa aatcaacatc gtgccgaaag ttaactacac tatctacgat  1260
   ggtttcaacc tgcgtaacac caacctggct gctaatttta acggccagaa cacggaaatc  1320
   aacaacatga acttcacaaa actgaaaaac ttcactggtc tgttcgagtt ttacaagctg  1380
   ctgtgcgtcg acggcatcat tacctccaaa actaaatctc tgatagaagg tagaaacaaa   1440
   gcgctgaacg acctctgtat caaggttaac aactgggatt tattcttcag cccgagtgaa   1500
   gacaacttca ccaacgacct gaacaaaggt gaagaaatca cctcagatac taacatcgaa   1560
   gcagccgaag aaaacatctc gctggacctg atccagcagt actacctgac ctttaatttc   1620
   gacaacgagc cggaaaacat ttctatcgaa aacctgagct ctgatatcat cggccagctg   1680
   gaactgatgc cgaacatcga acgtttccca aacggtaaaa agtacgagct ggacaaatat   1740
   accatgttcc actacctgcg cgcgcaggaa tttgaacacg gcaaatcccg tatcgcactg   1800
   actaactccg ttaacgaagc tctgctcaac ccgtcccgtg tatacacctt cttctctagc   1860
   gactacgtga aaaaggtcaa caaagcgact gaagctgcaa tgttcttggg ttgggttgaa   1920
   cagcttgttt atgattttac cgacgagacg tccgaagtat ctactaccga caaaattgcg   1980
   gatatcacta tcatcatccc gtacatcggt ccggctctga acattggcaa catgctgtac   2040
   aaagacgact tcgttggcgc actgatcttc tccggtgcgg tgatcctgct ggagttcatc   2100
   ccggaaatcg ccatcccggt actgggcacc tttgctctgg tttcttacat tgcaaacaag   2160
   gttctgactg tacaaaccat cgacaacgcg ctgagcaaac gtaacgaaaa atgggatgaa   2220
   gtttacaaat atatcgtgac caactggctg gctaaggtta atactcagat cgacctcatc   2280
   cgcaaaaaaa tgaaagaagc actggaaaac caggcggaag ctaccaaggc aatcattaac   2340
   taccagtaca accagtacac cgaggaagaa aaaaacaaca tcaacttcaa catcgacgat   2400
   ctgtcctcta aactgaacga atccatcaac aaagctatga tcaacatcaa caagttcctg   2460
   aaccagtgct ctgtaagcta tctgatgaac tccatgatcc cgtacggtgt taaacgtctg   2520
   gaggacttcg atgcgtctct gaaagacgcc ctgctgaaat acatttacga caaccgtggc   2580
   actctgatcg gtcaggttga tcgtctgaag gacaaagtga acaatacctt atcgaccgac   2640
   atcccttttc agctcagtaa atatgtcgat aaccaacgcc ttttgtccac tctagactag   2700
<210> 18
   <211> 899
   <212> PRT
   <213> Artificial Sequence
<220>
   <223> Synthetic
<400> 18
   Phe Gly Gly Phe Thr Gly Ala Arg Lys Ser Tyr Thr Ser Gly Gly Gly
   1               5                   10                  15
   Gly Ser Gly Gly Gly Gly Ser Gly Gly Gly Gly Ser Pro Arg Gly Ser
   20 25 30
   Met Glu Phe Val Asn Lys Gln Phe Asn Tyr Lys Asp Pro Val Asn Gly
   35 40 45
   Val Asp Ile Ala Tyr Ile Lys Ile Pro Asn Ala Gly Gln Met Gln Pro
   50 55 60
   Val Lys Ala Phe Lys Ile His Asn Lys Ile Trp Val Ile Pro Glu Arg
   65 70 75 80
   Asp Thr Phe Thr Asn Pro Glu Glu Gly Asp Leu Asn Pro Pro Pro Glu
   85 90 95
   Ala Lys Gln Val Pro Val Ser Tyr Tyr Asp Ser Thr Tyr Leu Ser Thr
   100 105 110
   Asp Asn Glu Lys Asp Asn Tyr Leu Lys Gly Val Thr Lys Leu Phe Glu
   115 120 125
   Arg Ile Tyr Ser Thr Asp Leu Gly Arg Met Leu Leu Thr Ser Ile Val
   130 135 140
   Arg Gly Ile Pro Phe Trp Gly Gly Ser Thr Ile Asp Thr Glu Leu Lys
   145 150 155 160
   Val Ile Asp Thr Asn Cys Ile Asn Val Ile Gln Pro Asp Gly Ser Tyr
   165 170 175
   Arg Ser Glu Glu Leu Asn Leu Val Ile Ile Gly Pro Ser Ala Asp Ile
   180 185 190
   Ile Gln Phe Glu Cys Lys Ser Phe Gly His Glu Val Leu Asn Leu Thr
   195 200 205
   Arg Asn Gly Tyr Gly Ser Thr Gln Tyr Ile Arg Phe Ser Pro Asp Phe
   210 215 220
   Thr Phe Gly Phe Glu Glu Ser Leu Glu Val Asp Thr Asn Pro Leu Leu
   225 230 235 240
   Gly Ala Gly Lys Phe Ala Thr Asp Pro Ala Val Thr Leu Ala His Glu
   245 250 255
   Leu Ile His Ala Gly His Arg Leu Tyr Gly Ile Ala Ile Asn Pro Asn
   260 265 270
   Arg Val Phe Lys Val Asn Thr Asn Ala Tyr Tyr Glu Met Ser Gly Leu
           275                 280                 285
   Glu Val Ser Phe Glu Glu Leu Arg Thr Phe Gly Gly His Asp Ala Lys
       290                 295                 300
   Phe Ile Asp Ser Leu Gln Glu Asn Glu Phe Arg Leu Tyr Tyr Tyr Asn
   305                 310                 315                 320
   Lys Phe Lys Asp Ile Ala Ser Thr Leu Asn Lys Ala Lys Ser Ile Val
                   325                 330                 335
   Gly Thr Thr Ala Ser Leu Gln Tyr Met Lys Asn Val Phe Lys Glu Lys
               340                 345                 350
   Tyr Leu Leu Ser Glu Asp Thr Ser Gly Lys Phe Ser Val Asp Lys Leu
           355                 360                 365
   Lys Phe Asp Lys Leu Tyr Lys Met Leu Thr Glu Ile Tyr Thr Glu Asp
       370                 375                 380
   Asn Phe Val Lys Phe Phe Lys Val Leu Asn Arg Lys Thr Tyr Leu Asn
   385                 390                 395                 400
   Phe Asp Lys Ala Val Phe Lys Ile Asn Ile Val Pro Lys Val Asn Tyr
                   405                 410                 415
   Thr Ile Tyr Asp Gly Phe Asn Leu Arg Asn Thr Asn Leu Ala Ala Asn
               420                 425                 430
   Phe Asn Gly Gln Asn Thr Glu Ile Asn Asn Met Asn Phe Thr Lys Leu
           435                 440                 445
   Lys Asn Phe Thr Gly Leu Phe Glu Phe Tyr Lys Leu Leu Cys Val Asp
       450                 455                 460
   Gly Ile Ile Thr Ser Lys Thr Lys Ser Leu Ile Glu Gly Arg Asn Lys
   465                 470                 475                 480
   Ala Leu Asn Asp Leu Cys Ile Lys Val Asn Asn Trp Asp Leu Phe Phe
                   485                 490                 495
   Ser Pro Ser Glu Asp Asn Phe Thr Asn Asp Leu Asn Lys Gly Glu Glu
               500                 505                 510
   Ile Thr Ser Asp Thr Asn Ile Glu Ala Ala Glu Glu Asn Ile Ser Leu
   515 520 525
   Asp Leu Ile Gln Gln Tyr Tyr Leu Thr Phe Asn Phe Asp Asn Glu Pro
   530 535 540
   Glu Asn Ile Ser Ile Glu Asn Leu Ser Ser Asp Ile Ile Gly Gln Leu
   545 550 555 560
   Glu Leu Met Pro Asn Ile Glu Arg Phe Pro Asn Gly Lys Lys Tyr Glu
   565 570 575
   Leu Asp Lys Tyr Thr Met Phe His Tyr Leu Arg Ala Gln Glu Phe Glu
   580 585 590
   His Gly Lys Ser Arg Ile Ala Leu Thr Asn Ser Val Asn Glu Ala Leu
   595 600 605
   Leu Asn Pro Ser Arg Val Tyr Thr Phe Phe Ser Ser Asp Tyr Val Lys
   610 615 620
   Lys Val Asn Lys Ala Thr Glu Ala Ala Met Phe Leu Gly Trp Val Glu
   625 630 635 640
   Gln Leu Val Tyr Asp Phe Thr Asp Glu Thr Ser Glu Val Ser Thr Thr
   645 650 655
   Asp Lys Ile Ala Asp Ile Thr Ile Ile Ile Pro Tyr Ile Gly Pro Ala
   660 665 670
   Leu Asn Ile Gly Asn Met Leu Tyr Lys Asp Asp Phe Val Gly Ala Leu
   675 680 685
   Ile Phe Ser Gly Ala Val Ile Leu Leu Glu Phe Ile Pro Glu Ile Ala
   690 695 700
   Ile Pro Val Leu Gly Thr Phe Ala Leu Val Ser Tyr Ile Ala Asn Lys
   705 710 715 720
   Val Leu Thr Val Gln Thr Ile Asp Asn Ala Leu Ser Lys Arg Asn Glu
   725 730 735
   Lys Trp Asp Glu Val Tyr Lys Tyr Ile Val Thr Asn Trp Leu Ala Lys
   740 745 750
   Val Asn Thr Gln Ile Asp Leu Ile Arg Lys Lys Met Lys Glu Ala Leu
           755                 760                 765
   Glu Asn Gln Ala Glu Ala Thr Lys Ala Ile Ile Asn Tyr Gln Tyr Asn
       770                 775                 780
   Gln Tyr Thr Glu Glu Glu Lys Asn Asn Ile Asn Phe Asn Ile Asp Asp
   785                 790                 795                 800
   Leu Ser Ser Lys Leu Asn Glu Ser Ile Asn Lys Ala Met Ile Asn Ile
                   805                 810                 815
   Asn Lys Phe Leu Asn Gln Cys Ser Val Ser Tyr Leu Met Asn Ser Met
               820                 825                 830
   Ile Pro Tyr Gly Val Lys Arg Leu Glu Asp Phe Asp Ala Ser Leu Lys
           835                 840                 845
   Asp Ala Leu Leu Lys Tyr Ile Tyr Asp Asn Arg Gly Thr Leu Ile Gly
       850                 855                 860
   Gln Val Asp Arg Leu Lys Asp Lys Val Asn Asn Thr Leu Ser Thr Asp
   865                 870                 875                 880
   Ile Pro Phe Gln Leu Ser Lys Tyr Val Asp Asn Gln Arg Leu Leu Ser
                   885                 890                 895
   Thr Leu Asp
<210> 19
   <211> 2706
   <212> DNA
   <213> Artificial Sequence
<220>
   <223> Synthetic
<400> 19
   tttggcggtt tcacgggcgc acgcaaatca gcgcgtaaaa ctagtggcgg tgggggtagt     60
   ggcggtggcg gttcgggcgg gggtgggagc cctaggggat ccatggagtt cgttaacaaa    120
   cagttcaact ataaagaccc agttaacggt gttgacattg cttacatcaa aatcccgaac    180
   gctggccaga tgcagccggt aaaggcattc aaaatccaca acaaaatctg ggttatcccg    240
   gaacgtgata cctttactaa cccggaagaa ggtgacctga acccgccacc ggaagcgaaa    300
   caggtgccgg tatcttacta tgactccacc tacctgtcta ccgataacga aaaggacaac    360
   tacctgaaag gtgttactaa actgttcgag cgtatttact ccaccgacct gggccgtatg    420
   ctgctgacta gcatcgttcg cggtatcccg ttctggggcg gttctaccat cgataccgaa    480
   ctgaaagtaa tcgacactaa ctgcatcaac gttattcagc cggacggttc ctatcgttcc    540
   gaagaactga acctggtgat catcggcccg tctgctgata tcatccagtt cgagtgtaag    600
   agctttggtc acgaagttct gaacctcacc cgtaacggct acggttccac tcagtacatc    660
   cgtttctctc cggacttcac cttcggtttt gaagaatccc tggaagtaga cacgaaccca    720
   ctgctgggcg ctggtaaatt cgcaactgat cctgcggtta ccctggctca cgaactgatt    780
   catgcaggcc accgcctgta cggtatcgcc atcaatccga accgtgtctt caaagttaac    840
   accaacgcgt attacgagat gtccggtctg gaagttagct tcgaagaact gcgtactttt    900
   ggcggtcacg acgctaaatt catcgactct ctgcaagaaa acgagttccg tctgtactac    960
   tataacaagt tcaaagatat cgcatccacc ctgaacaaag cgaaatccat cgtgggtacc   1020
   actgcttctc tccagtacat gaagaacgtt tttaaagaaa aatacctgct cagcgaagac   1080
   acctccggca aattctctgt agacaagttg aaattcgata aactttacaa aatgctgact   1140
   gaaatttaca ccgaagacaa cttcgttaag ttctttaaag ttctgaaccg caaaacctat   1200
   ctgaacttcg acaaggcagt attcaaaatc aacatcgtgc cgaaagttaa ctacactatc   1260
   tacgatggtt tcaacctgcg taacaccaac ctggctgcta attttaacgg ccagaacacg   1320
   gaaatcaaca acatgaactt cacaaaactg aaaaacttca ctggtctgtt cgagttttac   1380
   aagctgctgt gcgtcgacgg catcattacc tccaaaacta aatctctgat agaaggtaga   1440
   aacaaagcgc tgaacgacct ctgtatcaag gttaacaact gggatttatt cttcagcccg   1500
   agtgaagaca acttcaccaa cgacctgaac aaaggtgaag aaatcacctc agatactaac   1560
   atcgaagcag ccgaagaaaa catctcgctg gacctgatcc agcagtacta cctgaccttt   1620
   aatttcgaca acgagccgga aaacatttct atcgaaaacc tgagctctga tatcatcggc   1680
   cagctggaac tgatgccgaa catcgaacgt ttcccaaacg gtaaaaagta cgagctggac   1740
   aaatatacca tgttccacta cctgcgcgcg caggaatttg aacacggcaa atcccgtatc   1800
   gcactgacta actccgttaa cgaagctctg ctcaacccgt cccgtgtata caccttcttc   1860
   tctagcgact acgtgaaaaa ggtcaacaaa gcgactgaag ctgcaatgtt cttgggttgg   1920
   gttgaacagc ttgtttatga ttttaccgac gagacgtccg aagtatctac taccgacaaa   1980
   attgcggata tcactatcat catcccgtac atcggtccgg ctctgaacat tggcaacatg   2040
   ctgtacaaag acgacttcgt tggcgcactg atcttctccg gtgcggtgat cctgctggag   2100
   ttcatcccgg aaatcgccat cccggtactg ggcacctttg ctctggtttc ttacattgca   2160
   aacaaggttc tgactgtaca aaccatcgac aacgcgctga gcaaacgtaa cgaaaaatgg   2220
   gatgaagttt acaaatatat cgtgaccaac tggctggcta aggttaatac tcagatcgac 2280
   ctcatccgca aaaaaatgaa agaagcactg gaaaaccagg cggaagctac caaggcaatc 2340
   attaactacc agtacaacca gtacaccgag gaagaaaaaa acaacatcaa cttcaacatc 2400
   gacgatctgt cctctaaact gaacgaatcc atcaacaaag ctatgatcaa catcaacaag 2460
   ttcctgaacc agtgctctgt aagctatctg atgaactcca tgatcccgta cggtgttaaa 2520
   cgtctggagg acttcgatgc gtctctgaaa gacgccctgc tgaaatacat ttacgacaac 2580
   cgtggcactc tgatcggtca ggttgatcgt ctgaaggaca aagtgaacaa taccttatcg 2640
   accgacatcc cttttcagct cagtaaatat gtcgataacc aacgcctttt gtccactcta 2700
   gactag 2706
<210> 20
   <211> 901
   <212> PRT
   <213> Artificial Sequence
<220>
   <223> Synthetic
<400> 20
   Phe Gly Gly Phe Thr Gly Ala Arg Lys Ser Ala Arg Lys Thr Ser Gly
   1 5 10 15
   Gly Gly Gly Ser Gly Gly Gly Gly Ser Gly Gly Gly Gly Ser Pro Arg
   20 25 30
   Gly Ser Met Glu Phe Val Asn Lys Gln Phe Asn Tyr Lys Asp Pro Val
   35 40 45
   Asn Gly Val Asp Ile Ala Tyr Ile Lys Ile Pro Asn Ala Gly Gln Met
   50 55 60
   Gln Pro Val Lys Ala Phe Lys Ile His Asn Lys Ile Trp Val Ile Pro
   65 70 75 80
   Glu Arg Asp Thr Phe Thr Asn Pro Glu Glu Gly Asp Leu Asn Pro Pro
   85 90 95
   Pro Glu Ala Lys Gln Val Pro Val Ser Tyr Tyr Asp Ser Thr Tyr Leu
   100 105 110
   Ser Thr Asp Asn Glu Lys Asp Asn Tyr Leu Lys Gly Val Thr Lys Leu
   115 120 125
   Phe Glu Arg Ile Tyr Ser Thr Asp Leu Gly Arg Met Leu Leu Thr Ser
       130                 135                 140
   Ile Val Arg Gly Ile Pro Phe Trp Gly Gly Ser Thr Ile Asp Thr Glu
   145                 150                 155                 160
   Leu Lys Val Ile Asp Thr Asn Cys Ile Asn Val Ile Gln Pro Asp Gly
                   165                 170                 175
   Ser Tyr Arg Ser Glu Glu Leu Asn Leu Val Ile Ile Gly Pro Ser Ala
               180                 185                 190
   Asp Ile Ile Gln Phe Glu Cys Lys Ser Phe Gly His Glu Val Leu Asn
           195                 200                 205
   Leu Thr Arg Asn Gly Tyr Gly Ser Thr Gln Tyr Ile Arg Phe Ser Pro
       210                 215                 220
   Asp Phe Thr Phe Gly Phe Glu Glu Ser Leu Glu Val Asp Thr Asn Pro
   225                 230                 235                 240
   Leu Leu Gly Ala Gly Lys Phe Ala Thr Asp Pro Ala Val Thr Leu Ala
                   245                 250                 255
   His Glu Leu Ile His Ala Gly His Arg Leu Tyr Gly Ile Ala Ile Asn
               260                 265                 270
   Pro Asn Arg Val Phe Lys Val Asn Thr Asn Ala Tyr Tyr Glu Met Ser
           275                 280                 285
   Gly Leu Glu Val Ser Phe Glu Glu Leu Arg Thr Phe Gly Gly His Asp
       290                 295                 300
   Ala Lys Phe Ile Asp Ser Leu Gln Glu Asn Glu Phe Arg Leu Tyr Tyr
   305                 310                 315                 320
   Tyr Asn Lys Phe Lys Asp Ile Ala Ser Thr Leu Asn Lys Ala Lys Ser
                   325                 330                 335
   Ile Val Gly Thr Thr Ala Ser Leu Gln Tyr Met Lys Asn Val Phe Lys
               340                 345                 350
   Glu Lys Tyr Leu Leu Ser Glu Asp Thr Ser Gly Lys Phe Ser Val Asp
           355                 360                 365
   Lys Leu Lys Phe Asp Lys Leu Tyr Lys Met Leu Thr Glu Ile Tyr Thr
   370 375 380
   Glu Asp Asn Phe Val Lys Phe Phe Lys Val Leu Asn Arg Lys Thr Tyr
   385 390 395 400
   Leu Asn Phe Asp Lys Ala Val Phe Lys Ile Asn Ile Val Pro Lys Val
   405 410 415
   Asn Tyr Thr Ile Tyr Asp Gly Phe Asn Leu Arg Asn Thr Asn Leu Ala
   420 425 430
   Ala Asn Phe Asn Gly Gln Asn Thr Glu Ile Asn Asn Met Asn Phe Thr
   435 440 445
   Lys Leu Lys Asn Phe Thr Gly Leu Phe Glu Phe Tyr Lys Leu Leu Cys
   450 455 460
   Val Asp Gly Ile Ile Thr Ser Lys Thr Lys Ser Leu Ile Glu Gly Arg
   465 470 475 480
   Asn Lys Ala Leu Asn Asp Leu Cys Ile Lys Val Asn Asn Trp Asp Leu
   485 490 495
   Phe Phe Ser Pro Ser Glu Asp Asn Phe Thr Asn Asp Leu Asn Lys Gly
   500 505 510
   Glu Glu Ile Thr Ser Asp Thr Asn Ile Glu Ala Ala Glu Glu Asn Ile
   515 520 525
   Ser Leu Asp Leu Ile Gln Gln Tyr Tyr Leu Thr Phe Asn Phe Asp Asn
   530 535 540
   Glu Pro Glu Asn Ile Ser Ile Glu Asn Leu Ser Ser Asp Ile Ile Gly
   545 550 555 560
   Gln Leu Glu Leu Met Pro Asn Ile Glu Arg Phe Pro Asn Gly Lys Lys
   565 570 575
   Tyr Glu Leu Asp Lys Tyr Thr Met Phe His Tyr Leu Arg Ala Gln Glu
   580 585 590
   Phe Glu His Gly Lys Ser Arg Ile Ala Leu Thr Asn Ser Val Asn Glu
   595 600 605
   Ala Leu Leu Asn Pro Ser Arg Val Tyr Thr Phe Phe Ser Ser Asp Tyr
   610 615 620
   Val Lys Lys Val Asn Lys Ala Thr Glu Ala Ala Met Phe Leu Gly Trp
   625 630 635 640
   Val Glu Gln Leu Val Tyr Asp Phe Thr Asp Glu Thr Ser Glu Val Ser
   645 650 655
   Thr Thr Asp Lys Ile Ala Asp Ile Thr Ile Ile Ile Pro Tyr Ile Gly
   660 665 670
   Pro Ala Leu Asn Ile Gly Asn Met Leu Tyr Lys Asp Asp Phe Val Gly
   675 680 685
   Ala Leu Ile Phe Ser Gly Ala Val Ile Leu Leu Glu Phe Ile Pro Glu
   690 695 700
   Ile Ala Ile Pro Val Leu Gly Thr Phe Ala Leu Val Ser Tyr Ile Ala
   705 710 715 720
   Asn Lys Val Leu Thr Val Gln Thr Ile Asp Asn Ala Leu Ser Lys Arg
   725 730 735
   Asn Glu Lys Trp Asp Glu Val Tyr Lys Tyr Ile Val Thr Asn Trp Leu
   740 745 750
   Ala Lys Val Asn Thr Gln Ile Asp Leu Ile Arg Lys Lys Met Lys Glu
   755 760 765
   Ala Leu Glu Asn Gln Ala Glu Ala Thr Lys Ala Ile Ile Asn Tyr Gln
   770 775 780
   Tyr Asn Gln Tyr Thr Glu Glu Glu Lys Asn Asn Ile Asn Phe Asn Ile
   785 790 795 800
   Asp Asp Leu Ser Ser Lys Leu Asn Glu Ser Ile Asn Lys Ala Met Ile
   805 810 815
   Asn Ile Asn Lys Phe Leu Asn Gln Cys Ser Val Ser Tyr Leu Met Asn
   820 825 830
   Ser Met Ile Pro Tyr Gly Val Lys Arg Leu Glu Asp Phe Asp Ala Ser
   835 840 845
   Leu Lys Asp Ala Leu Leu Lys Tyr Ile Tyr Asp Asn Arg Gly Thr Leu
   850 855 860
   Ile Gly Gln Val Asp Arg Leu Lys Asp Lys Val Asn Asn Thr Leu Ser
   865                 870                 875                 880
   Thr Asp Ile Pro Phe Gln Leu Ser Lys Tyr Val Asp Asn Gln Arg Leu
                   885                 890                 895
   Leu Ser Thr Leu Asp
               900
<210> 21
   <211> 2691
   <212> DNA
   <213> Artificial Sequence
<220>
   <223> Synthetic
<400> 21
   atggagttcg ttaacaaaca gttcaactat aaagacccag ttaacggtgt tgacattgct     60
   tacatcaaaa tcccgaacgc tggccagatg cagccggtaa aggcattcaa aatccacaac    120
   aaaatctggg ttatcccgga acgtgatacc tttactaacc cggaagaagg tgacctgaac    180
   ccgccaccgg aagcgaaaca ggtgccggta tcttactatg actccaccta cctgtctacc    240
   gataacgaaa aggacaacta cctgaaaggt gttactaaac tgttcgagcg tatttactcc    300
   accgacctgg gccgtatgct gctgactagc atcgttcgcg gtatcccgtt ctggggcggt    360
   tctaccatcg ataccgaact gaaagtaatc gacactaact gcatcaacgt tattcagccg    420
   gacggttcct atcgttccga agaactgaac ctggtgatca tcggcccgtc tgctgatatc    480
   atccagttcg agtgtaagag ctttggtcac gaagttctga acctcacccg taacggctac    540
   ggttccactc agtacatccg tttctctccg gacttcacct tcggttttga agaatccctg    600
   gaagtagaca cgaacccact gctgggcgct ggtaaattcg caactgatcc tgcggttacc    660
   ctggctcacg aactgattca tgcaggccac cgcctgtacg gtatcgccat caatccgaac    720
   cgtgtcttca aagttaacac caacgcgtat tacgagatgt ccggtctgga agttagcttc    780
   gaagaactgc gtacttttgg cggtcacgac gctaaattca tcgactctct gcaagaaaac    840
   gagttccgtc tgtactacta taacaagttc aaagatatcg catccaccct gaacaaagcg    900
   aaatccatcg tgggtaccac tgcttctctc cagtacatga agaacgtttt taaagaaaaa    960
   tacctgctca gcgaagacac ctccggcaaa ttctctgtag acaagttgaa attcgataaa   1020
   ctttacaaaa tgctgactga aatttacacc gaagacaact tcgttaagtt ctttaaagtt   1080
   ctgaaccgca aaacctatct gaacttcgac aaggcagtat tcaaaatcaa catcgtgccg   1140
   aaagttaact acactatcta cgatggtttc aacctgcgta acaccaacct ggctgctaat   1200
   tttaacggcc agaacacgga aatcaacaac atgaacttca caaaactgaa aaacttcact   1260
   ggtctgttcg agttttacaa gctgctgtgc gtcgacggca tcattacctc caaaactaaa   1320
   tctctgatag aaggtagaaa caaagcgctg aacgacctct gtatcaaggt taacaactgg   1380
   gatttattct tcagcccgag tgaagacaac ttcaccaacg acctgaacaa aggtgaagaa   1440
   atcacctcag atactaacat cgaagcagcc gaagaaaaca tctcgctgga cctgatccag   1500
   cagtactacc tgacctttaa tttcgacaac gagccggaaa acatttctat cgaaaacctg   1560
   agctctgata tcatcggcca gctggaactg atgccgaaca tcgaacgttt cccaaacggt   1620
   aaaaagtacg agctggacaa atataccatg ttccactacc tgcgcgcgca ggaatttgaa   1680
   cacggcaaat cccgtatcgc actgactaac tccgttaacg aagctctgct caacccgtcc   1740
   cgtgtataca ccttcttctc tagcgactac gtgaaaaagg tcaacaaagc gactgaagct   1800
   gcaatgttct tgggttgggt tgaacagctt gtttatgatt ttaccgacga gacgtccgaa   1860
   gtatctacta ccgacaaaat tgcggatatc actatcatca tcccgtacat cggtccggct   1920
   ctgaacattg gcaacatgct gtacaaagac gacttcgttg gcgcactgat cttctccggt   1980
   gcggtgatcc tgctggagtt catcccggaa atcgccatcc cggtactggg cacctttgct   2040
   ctggtttctt acattgcaaa caaggttctg actgtacaaa ccatcgacaa cgcgctgagc   2100
   aaacgtaacg aaaaatggga tgaagtttac aaatatatcg tgaccaactg gctggctaag   2160
   gttaatactc agatcgacct catccgcaaa aaaatgaaag aagcactgga aaaccaggcg   2220
   gaagctacca aggcaatcat taactaccag tacaaccagt acaccgagga agaaaaaaac   2280
   aacatcaact tcaacatcga cgatctgtcc tctaaactga acgaatccat caacaaagct   2340
   atgatcaaca tcaacaagtt cctgaaccag tgctctgtaa gctatctgat gaactccatg   2400
   atcccgtacg gtgttaaacg tctggaggac ttcgatgcgt ctctgaaaga cgccctgctg   2460
   aaatacattt acgacaaccg tggcactctg atcggtcagg ttgatcgtct gaaggacaaa   2520
   gtgaacaata ccttatcgac cgacatccct tttcagctca gtaaatatgt cgataaccaa   2580
   cgccttttgt ccactggcgg tgggggtagt ggcggtggcg gttcgggcgg gggtgggagc   2640
   tttggcggtt tcacgggcgc acgcaaatca gcgcgtaaat tagctaacca g            2691
<210> 22
   <211> 897
   <212> PRT
   <213> Artificial Sequence
<220>
   <223> Synthetic
<400> 22
   Met Glu Phe Val Asn Lys Gln Phe Asn Tyr Lys Asp Pro Val Asn Gly
   1 5 10 15
   Val Asp Ile Ala Tyr Ile Lys Ile Pro Asn Ala Gly Gln Met Gln Pro
   20 25 30
   Val Lys Ala Phe Lys Ile His Asn Lys Ile Trp Val Ile Pro Glu Arg
   35 40 45
   Asp Thr Phe Thr Asn Pro Glu Glu Gly Asp Leu Asn Pro Pro Pro Glu
   50 55 60
   Ala Lys Gln Val Pro Val Ser Tyr Tyr Asp Ser Thr Tyr Leu Ser Thr
   65 70 75 80
   Asp Asn Glu Lys Asp Asn Tyr Leu Lys Gly Val Thr Lys Leu Phe Glu
   85 90 95
   Arg Ile Tyr Ser Thr Asp Leu Gly Arg Met Leu Leu Thr Ser Ile Val
   100 105 110
   Arg Gly Ile Pro Phe Trp Gly Gly Ser Thr Ile Asp Thr Glu Leu Lys
   115 120 125
   Val Ile Asp Thr Asn Cys Ile Asn Val Ile Gln Pro Asp Gly Ser Tyr
   130 135 140
   Arg Ser Glu Glu Leu Asn Leu Val Ile Ile Gly Pro Ser Ala Asp Ile
   145 150 155 160
   Ile Gln Phe Glu Cys Lys Ser Phe Gly His Glu Val Leu Asn Leu Thr
   165 170 175
   Arg Asn Gly Tyr Gly Ser Thr Gln Tyr Ile Arg Phe Ser Pro Asp Phe
   180 185 190
   Thr Phe Gly Phe Glu Glu Ser Leu Glu Val Asp Thr Asn Pro Leu Leu
   195 200 205
   Gly Ala Gly Lys Phe Ala Thr Asp Pro Ala Val Thr Leu Ala His Glu
   210 215 220
   Leu Ile His Ala Gly His Arg Leu Tyr Gly Ile Ala Ile Asn Pro Asn
   225 230 235 240
   Arg Val Phe Lys Val Asn Thr Asn Ala Tyr Tyr Glu Met Ser Gly Leu
                   245                 250                 255
   Glu Val Ser Phe Glu Glu Leu Arg Thr Phe Gly Gly His Asp Ala Lys
               260                 265                 270
   Phe Ile Asp Ser Leu Gln Glu Asn Glu Phe Arg Leu Tyr Tyr Tyr Asn
           275                 280                 285
   Lys Phe Lys Asp Ile Ala Ser Thr Leu Asn Lys Ala Lys Ser Ile Val
       290                 295                 300
   Gly Thr Thr Ala Ser Leu Gln Tyr Met Lys Asn Val Phe Lys Glu Lys
   305                 310                 315                 320
   Tyr Leu Leu Ser Glu Asp Thr Ser Gly Lys Phe Ser Val Asp Lys Leu
                   325                 330                 335
   Lys Phe Asp Lys Leu Tyr Lys Met Leu Thr Glu Ile Tyr Thr Glu Asp
               340                 345                 350
   Asn Phe Val Lys Phe Phe Lys Val Leu Asn Arg Lys Thr Tyr Leu Asn
           355                 360                 365
   Phe Asp Lys Ala Val Phe Lys Ile Asn Ile Val Pro Lys Val Asn Tyr
       370                 375                 380
   Thr Ile Tyr Asp Gly Phe Asn Leu Arg Asn Thr Asn Leu Ala Ala Asn
   385                 390                 395                 400
   Phe Asn Gly Gln Asn Thr Glu Ile Asn Asn Met Asn Phe Thr Lys Leu
                   405                 410                 415
   Lys Asn Phe Thr Gly Leu Phe Glu Phe Tyr Lys Leu Leu Cys Val Asp
               420                 425                 430
   Gly Ile Ile Thr Ser Lys Thr Lys Ser Leu Ile Glu Gly Arg Asn Lys
           435                 440                 445
   Ala Leu Asn Asp Leu Cys Ile Lys Val Asn Asn Trp Asp Leu Phe Phe
       450                 455                 460
   Ser Pro Ser Glu Asp Asn Phe Thr Asn Asp Leu Asn Lys Gly Glu Glu
   465                 470                 475                 480
   Ile Thr Ser Asp Thr Asn Ile Glu Ala Ala Glu Glu Asn Ile Ser Leu
   485 490 495
   Asp Leu Ile Gln Gln Tyr Tyr Leu Thr Phe Asn Phe Asp Asn Glu Pro
   500 505 510
   Glu Asn Ile Ser Ile Glu Asn Leu Ser Ser Asp Ile Ile Gly Gln Leu
   515 520 525
   Glu Leu Met Pro Asn Ile Glu Arg Phe Pro Asn Gly Lys Lys Tyr Glu
   530 535 540
   Leu Asp Lys Tyr Thr Met Phe His Tyr Leu Arg Ala Gln Glu Phe Glu
   545 550 555 560
   His Gly Lys Ser Arg Ile Ala Leu Thr Asn Ser Val Asn Glu Ala Leu
   565 570 575
   Leu Asn Pro Ser Arg Val Tyr Thr Phe Phe Ser Ser Asp Tyr Val Lys
   580 585 590
   Lys Val Asn Lys Ala Thr Glu Ala Ala Met Phe Leu Gly Trp Val Glu
   595 600 605
   Gln Leu Val Tyr Asp Phe Thr Asp Glu Thr Ser Glu Val Ser Thr Thr
   610 615 620
   Asp Lys Ile Ala Asp Ile Thr Ile Ile Ile Pro Tyr Ile Gly Pro Ala
   625 630 635 640
   Leu Asn Ile Gly Asn Met Leu Tyr Lys Asp Asp Phe Val Gly Ala Leu
   645 650 655
   Ile Phe Ser Gly Ala Val Ile Leu Leu Glu Phe Ile Pro Glu Ile Ala
   660 665 670
   Ile Pro Val Leu Gly Thr Phe Ala Leu Val Ser Tyr Ile Ala Asn Lys
   675 680 685
   Val Leu Thr Val Gln Thr Ile Asp Asn Ala Leu Ser Lys Arg Asn Glu
   690 695 700
   Lys Trp Asp Glu Val Tyr Lys Tyr Ile Val Thr Asn Trp Leu Ala Lys
   705 710 715 720
   Val Asn Thr Gln Ile Asp Leu Ile Arg Lys Lys Met Lys Glu Ala Leu
   725 730 735
   Glu Asn Gln Ala Glu Ala Thr Lys Ala Ile Ile Asn Tyr Gln Tyr Asn
   740 745 750
   Gln Tyr Thr Glu Glu Glu Lys Asn Asn Ile Asn Phe Asn Ile Asp Asp
   755 760 765
   Leu Ser Ser Lys Leu Asn Glu Ser Ile Asn Lys Ala Met Ile Asn Ile
   770 775 780
   Asn Lys Phe Leu Asn Gln Cys Ser Val Ser Tyr Leu Met Asn Ser Met
   785 790 795 800
   Ile Pro Tyr Gly Val Lys Arg Leu Glu Asp Phe Asp Ala Ser Leu Lys
   805 810 815
   Asp Ala Leu Leu Lys Tyr Ile Tyr Asp Asn Arg Gly Thr Leu Ile Gly
   820 825 830
   Gln Val Asp Arg Leu Lys Asp Lys Val Asn Asn Thr Leu Ser Thr Asp
   835 840 845
   Ile Pro Phe Gln Leu Ser Lys Tyr Val Asp Asn Gln Arg Leu Leu Ser
   850 855 860
   Thr Gly Gly Gly Gly Ser Gly Gly Gly Gly Ser Gly Gly Gly Gly Ser
   865 870 875 880
   Phe Gly Gly Phe Thr Gly Ala Arg Lys Ser Ala Arg Lys Leu Ala Asn
   885 890 895
   Gln
<210> 23
   <211> 2676
   <212> DNA
   <213> Artificial Sequence
<220>
   <223> Synthetic
<400> 23
   atgccaataa caattaacaa ctttaattat tcagatcctg ttgataataa aaatatttta     60
   tatttagata ctcatttaaa tacactagct aatgagcctg aaaaagcctt tcgcattaca    120
   ggaaatatat gggtaatacc tgatagattt tcaagaaatt ctaatccaaa tttaaataaa    180
   cctcctcgag ttacaagccc taaaagtggt tattatgatc ctaattattt gagtactgat    240
   tctgacaaag atacattttt aaaagaaatt ataaagttat ttaaaagaat taattctaga    300
   gaaataggag aagaattaat atatagactt tcgacagata taccctttcc tgggaataac    360
   aatactccaa ttaatacctt tgattttgat gtagatttta acagtgttga tgttaaaact    420
   agacaaggta acaactgggt taaaactggt agcataaatc ctagtgttat aataactgga    480
   cctagagaaa acattataga tccagaaact tctacgttta aattaactaa caataccttt    540
   gcggcacaag aaggatttgg tgctttatca ataatttcaa tatcacctag atttatgcta    600
   acatatagta atgcaactaa tgatgtagga gagggtagat tttctaagtc tgaattttgc    660
   atggatccaa tactaatttt aatgcatgaa cttaatcatg caatgcataa tttatatgga    720
   atagctatac caaatgatca aacaatttca tctgtaacta gtaatatttt ttattctcaa    780
   tataatgtga aattagagta tgcagaaata tatgcatttg gaggtccaac tatagacctt    840
   attcctaaaa gtgcaaggaa atattttgag gaaaaggcat tggattatta tagatctata    900
   gctaaaagac ttaatagtat aactactgca aatccttcaa gctttaataa atatataggg    960
   gaatataaac agaaacttat tagaaagtat agattcgtag tagaatcttc aggtgaagtt   1020
   acagtaaatc gtaataagtt tgttgagtta tataatgaac ttacacaaat atttacagaa   1080
   tttaactacg ctaaaatata taatgtacaa aataggaaaa tatatctttc aaatgtatat   1140
   actccggtta cggcgaatat attagacgat aatgtttatg atatacaaaa tggatttaat   1200
   atacctaaaa gtaatttaaa tgtactattt atgggtcaaa atttatctcg aaatccagca   1260
   ttaagaaaag tcaatcctga aaatatgctt tatttattta caaaattttg tcataaagca   1320
   atagatggta gatcattata taataaaaca ttagattgta gagagctttt agttaaaaat   1380
   actgacttac cctttatagg tgatattagt gatgttaaaa ctgatatatt tttaagaaaa   1440
   gatattaatg aagaaactga agttatatac tatccggaca atgtttcagt agatcaagtt   1500
   attctcagta agaatacctc agaacatgga caactagatt tattataccc tagtattgac   1560
   agtgagagtg aaatattacc aggggagaat caagtctttt atgataatag aactcaaaat   1620
   gttgattatt tgaattctta ttattaccta gaatctcaaa aactaagtga taatgttgaa   1680
   gattttactt ttacgagatc aattgaggag gctttggata atagtgcaaa agtatatact   1740
   tactttccta cactagctaa taaagtaaat gcgggtgttc aaggtggttt atttttaatg   1800
   tgggcaaatg atgtagttga agattttact acaaatattc taagaaaaga tacattagat   1860
   aaaatatcag atgtatcagc tattattccc tatataggac ccgcattaaa tataagtaat   1920
   tctgtaagaa gaggaaattt tactgaagca tttgcagtta ctggtgtaac tattttatta   1980
   gaagcatttc ctgaatttac aatacctgca cttggtgcat ttgtgattta tagtaaggtt 2040
   caagaaagaa acgagattat taaaactata gataattgtt tagaacaaag gattaagaga 2100
   tggaaagatt catatgaatg gatgatggga acgtggttat ccaggattat tactcaattt 2160
   aataatataa gttatcaaat gtatgattct ttaaattatc aggcaggtgc aatcaaagct 2220
   aaaatagatt tagaatataa aaaatattca ggaagtgata aagaaaatat aaaaagtcaa 2280
   gttgaaaatt taaaaaatag tttagatgta aaaatttcgg aagcaatgaa taatataaat 2340
   aaatttatac gagaatgttc cgtaacatat ttatttaaaa atatgttacc taaagtaatt 2400
   gatgaattaa atgagtttga tcgaaatact aaagcaaaat taattaatct tatagatagt 2460
   cataatatta ttctagttgg tgaagtagat aaattaaaag caaaagtaaa taatagcttt 2520
   caaaatacaa taccctttaa tattttttca tatactaata attctttatt aaaagatata 2580
   attaatgaat atttcaatgg cggtgggggt agtggcggtg gcggttcggg cgggggtggg 2640
   agctttggcg gtttcacggg cgcacgcaaa tcagcg 2676
<210> 24
   <211> 892
   <212> PRT
   <213> Artificial Sequence
<220>
   <223> Synthetic
<400> 24
   Met Pro Ile Thr Ile Asn Asn Phe Asn Tyr Ser Asp Pro Val Asp Asn
   1 5 10 15
   Lys Asn Ile Leu Tyr Leu Asp Thr His Leu Asn Thr Leu Ala Asn Glu
   20 25 30
   Pro Glu Lys Ala Phe Arg Ile Thr Gly Asn Ile Trp Val Ile Pro Asp
   35 40 45
   Arg Phe Ser Arg Asn Ser Asn Pro Asn Leu Asn Lys Pro Pro Arg Val
   50 55 60
   Thr Ser Pro Lys Ser Gly Tyr Tyr Asp Pro Asn Tyr Leu Ser Thr Asp
   65 70 75 80
   Ser Asp Lys Asp Thr Phe Leu Lys Glu Ile Ile Lys Leu Phe Lys Arg
   85 90 95
   Ile Asn Ser Arg Glu Ile Gly Glu Glu Leu Ile Tyr Arg Leu Ser Thr
   100 105 110
   Asp Ile Pro Phe Pro Gly Asn Asn Asn Thr Pro Ile Asn Thr Phe Asp
           115                 120                 125
   Phe Asp Val Asp Phe Asn Ser Val Asp Val Lys Thr Arg Gln Gly Asn
       130                 135                 140
   Asn Trp Val Lys Thr Gly Ser Ile Asn Pro Ser Val Ile Ile Thr Gly
   145                 150                 155                 160
   Pro Arg Glu Asn Ile Ile Asp Pro Glu Thr Ser Thr Phe Lys Leu Thr
                   165                 170                 175
   Asn Asn Thr Phe Ala Ala Gln Glu Gly Phe Gly Ala Leu Ser Ile Ile
               180                 185                 190
   Ser Ile Ser Pro Arg Phe Met Leu Thr Tyr Ser Asn Ala Thr Asn Asp
           195                 200                 205
   Val Gly Glu Gly Arg Phe Ser Lys Ser Glu Phe Cys Met Asp Pro Ile
       210                 215                 220
   Leu Ile Leu Met His Glu Leu Asn His Ala Met His Asn Leu Tyr Gly
   225                 230                 235                 240
   Ile Ala Ile Pro Asn Asp Gln Thr Ile Ser Ser Val Thr Ser Asn Ile
                   245                 250                 255
   Phe Tyr Ser Gln Tyr Asn Val Lys Leu Glu Tyr Ala Glu Ile Tyr Ala
               260                 265                 270
   Phe Gly Gly Pro Thr Ile Asp Leu Ile Pro Lys Ser Ala Arg Lys Tyr
           275                 280                 285
   Phe Glu Glu Lys Ala Leu Asp Tyr Tyr Arg Ser Ile Ala Lys Arg Leu
       290                 295                 300
   Asn Ser Ile Thr Thr Ala Asn Pro Ser Ser Phe Asn Lys Tyr Ile Gly
   305                 310                 315                 320
   Glu Tyr Lys Gln Lys Leu Ile Arg Lys Tyr Arg Phe Val Val Glu Ser
                   325                 330                 335
   Ser Gly Glu Val Thr Val Asn Arg Asn Lys Phe Val Glu Leu Tyr Asn
               340                 345                 350
   Glu Leu Thr Gln Ile Phe Thr Glu Phe Asn Tyr Ala Lys Ile Tyr Asn
           355                 360                 365
   Val Gln Asn Arg Lys Ile Tyr Leu Ser Asn Val Tyr Thr Pro Val Thr
       370                 375                 380
   Ala Asn Ile Leu Asp Asp Asn Val Tyr Asp Ile Gln Asn Gly Phe Asn
   385                 390                 395                 400
   Ile Pro Lys Ser Asn Leu Asn Val Leu Phe Met Gly Gln Asn Leu Ser
                   405                 410                 415
   Arg Asn Pro Ala Leu Arg Lys Val Asn Pro Glu Asn Met Leu Tyr Leu
               420                 425                 430
   Phe Thr Lys Phe Cys His Lys Ala Ile Asp Gly Arg Ser Leu Tyr Asn
           435                 440                 445
   Lys Thr Leu Asp Cys Arg Glu Leu Leu Val Lys Asn Thr Asp Leu Pro
       450                 455                 460
   Phe Ile Gly Asp Ile Ser Asp Val Lys Thr Asp Ile Phe Leu Arg Lys
   465                 470                 475                 480
   Asp Ile Asn Glu Glu Thr Glu Val Ile Tyr Tyr Pro Asp Asn Val Ser
                   485                 490                 495
   Val Asp Gln Val Ile Leu Ser Lys Asn Thr Ser Glu His Gly Gln Leu
               500                 505                 510
   Asp Leu Leu Tyr Pro Ser Ile Asp Ser Glu Ser Glu Ile Leu Pro Gly
           515                 520                 525
   Glu Asn Gln Val Phe Tyr Asp Asn Arg Thr Gln Asn Val Asp Tyr Leu
       530                 535                 540
   Asn Ser Tyr Tyr Tyr Leu Glu Ser Gln Lys Leu Ser Asp Asn Val Glu
   545                 550                 555                 560
   Asp Phe Thr Phe Thr Arg Ser Ile Glu Glu Ala Leu Asp Asn Ser Ala
                   565                 570                 575
   Lys Val Tyr Thr Tyr Phe Pro Thr Leu Ala Asn Lys Val Asn Ala Gly
               580                 585                 590
   Val Gln Gly Gly Leu Phe Leu Met Trp Ala Asn Asp Val Val Glu Asp
           595                 600                 605
   Phe Thr Thr Asn Ile Leu Arg Lys Asp Thr Leu Asp Lys Ile Ser Asp
       610                 615                 620
   Val Ser Ala Ile Ile Pro Tyr Ile Gly Pro Ala Leu Asn Ile Ser Asn
   625                 630                 635                 640
   Ser Val Arg Arg Gly Asn Phe Thr Glu Ala Phe Ala Val Thr Gly Val
                   645                 650                 655
   Thr Ile Leu Leu Glu Ala Phe Pro Glu Phe Thr Ile Pro Ala Leu Gly
               660                 665                 670
   Ala Phe Val Ile Tyr Ser Lys Val Gln Glu Arg Asn Glu Ile Ile Lys
           675                 680                 685
   Thr Ile Asp Asn Cys Leu Glu Gln Arg Ile Lys Arg Trp Lys Asp Ser
       690                 695                 700
   Tyr Glu Trp Met Met Gly Thr Trp Leu Ser Arg Ile Ile Thr Gln Phe
   705                 710                 715                 720
   Asn Asn Ile Ser Tyr Gln Met Tyr Asp Ser Leu Asn Tyr Gln Ala Gly
                   725                 730                 735
   Ala Ile Lys Ala Lys Ile Asp Leu Glu Tyr Lys Lys Tyr Ser Gly Ser
               740                 745                 750
   Asp Lys Glu Asn Ile Lys Ser Gln Val Glu Asn Leu Lys Asn Ser Leu
           755                 760                 765
   Asp Val Lys Ile Ser Glu Ala Met Asn Asn Ile Asn Lys Phe Ile Arg
       770                 775                 780
   Glu Cys Ser Val Thr Tyr Leu Phe Lys Asn Met Leu Pro Lys Val Ile
   785                 790                 795                 800
   Asp Glu Leu Asn Glu Phe Asp Arg Asn Thr Lys Ala Lys Leu Ile Asn
                   805                 810                 815
   Leu Ile Asp Ser His Asn Ile Ile Leu Val Gly Glu Val Asp Lys Leu
               820                 825                 830
   Lys Ala Lys Val Asn Asn Ser Phe Gln Asn Thr Ile Pro Phe Asn Ile
   835 840 845
   Phe Ser Tyr Thr Asn Asn Ser Leu Leu Lys Asp Ile Ile Asn Glu Tyr
   850 855 860
   Phe Asn Gly Gly Gly Gly Ser Gly Gly Gly Gly Ser Gly Gly Gly Gly
   865 870 875 880
   Ser Phe Gly Gly Phe Thr Gly Ala Arg Lys Ser Ala
   885 890
<210> 25
   <211> 2712
   <212> DNA
   <213> Artificial Sequence
<220>
   <223> Synthetic
<400> 25
   tttggcggtt tcacgggcgc acgcaaatca gcgcgtaaat atgctaacca gactagtggc     60
   ggtgggggta gtggcggtgg cggttcgggc gggggtggga gccctagggg atccatgcca    120
   ataacaatta acaactttaa ttattcagat cctgttgata ataaaaatat tttatattta    180
   gatactcatt taaatacact agctaatgag cctgaaaaag cctttcgcat tacaggaaat    240
   atatgggtaa tacctgatag attttcaaga aattctaatc caaatttaaa taaacctcct    300
   cgagttacaa gccctaaaag tggttattat gatcctaatt atttgagtac tgattctgac    360
   aaagatacat ttttaaaaga aattataaag ttatttaaaa gaattaattc tagagaaata    420
   ggagaagaat taatatatag actttcgaca gatataccct ttcctgggaa taacaatact    480
   ccaattaata cctttgattt tgatgtagat tttaacagtg ttgatgttaa aactagacaa    540
   ggtaacaact gggttaaaac tggtagcata aatcctagtg ttataataac tggacctaga    600
   gaaaacatta tagatccaga aacttctacg tttaaattaa ctaacaatac ctttgcggca    660
   caagaaggat ttggtgcttt atcaataatt tcaatatcac ctagatttat gctaacatat    720
   agtaatgcaa ctaatgatgt aggagagggt agattttcta agtctgaatt ttgcatggat    780
   ccaatactaa ttttaatgca tgaacttaat catgcaatgc ataatttata tggaatagct    840
   ataccaaatg atcaaacaat ttcatctgta actagtaata ttttttattc tcaatataat     900
   gtgaaattag agtatgcaga aatatatgca tttggaggtc caactataga ccttattcct    960
   aaaagtgcaa ggaaatattt tgaggaaaag gcattggatt attatagatc tatagctaaa   1020
   agacttaata gtataactac tgcaaatcct tcaagcttta ataaatatat aggggaatat   1080
   aaacagaaac ttattagaaa gtatagattc gtagtagaat cttcaggtga agttacagta   1140
   aatcgtaata agtttgttga gttatataat gaacttacac aaatatttac agaatttaac   1200
   tacgctaaaa tatataatgt acaaaatagg aaaatatatc tttcaaatgt atatactccg   1260
   gttacggcga atatattaga cgataatgtt tatgatatac aaaatggatt taatatacct   1320
   aaaagtaatt taaatgtact atttatgggt caaaatttat ctcgaaatcc agcattaaga   1380
   aaagtcaatc ctgaaaatat gctttattta tttacaaaat tttgtcataa agcaatagat   1440
   ggtagatcat tatataataa aacattagat tgtagagagc ttttagttaa aaatactgac   1500
   ttacccttta taggtgatat tagtgatgtt aaaactgata tatttttaag aaaagatatt   1560
   aatgaagaaa ctgaagttat atactatccg gacaatgttt cagtagatca agttattctc   1620
   agtaagaata cctcagaaca tggacaacta gatttattat accctagtat tgacagtgag   1680
   agtgaaatat taccagggga gaatcaagtc ttttatgata atagaactca aaatgttgat   1740
   tatttgaatt cttattatta cctagaatct caaaaactaa gtgataatgt tgaagatttt   1800
   acttttacga gatcaattga ggaggctttg gataatagtg caaaagtata tacttacttt   1860
   cctacactag ctaataaagt aaatgcgggt gttcaaggtg gtttattttt aatgtgggca   1920
   aatgatgtag ttgaagattt tactacaaat attctaagaa aagatacatt agataaaata   1980
   tcagatgtat cagctattat tccctatata ggacccgcat taaatataag taattctgta   2040
   agaagaggaa attttactga agcatttgca gttactggtg taactatttt attagaagca   2100
   tttcctgaat ttacaatacc tgcacttggt gcatttgtga tttatagtaa ggttcaagaa   2160
   agaaacgaga ttattaaaac tatagataat tgtttagaac aaaggattaa gagatggaaa   2220
   gattcatatg aatggatgat gggaacgtgg ttatccagga ttattactca atttaataat   2280
   ataagttatc aaatgtatga ttctttaaat tatcaggcag gtgcaatcaa agctaaaata   2340
   gatttagaat ataaaaaata ttcaggaagt gataaagaaa atataaaaag tcaagttgaa   2400
   aatttaaaaa atagtttaga tgtaaaaatt tcggaagcaa tgaataatat aaataaattt   2460
   atacgagaat gttccgtaac atatttattt aaaaatatgt tacctaaagt aattgatgaa   2520
   ttaaatgagt ttgatcgaaa tactaaagca aaattaatta atcttataga tagtcataat   2580
   attattctag ttggtgaagt agataaatta aaagcaaaag taaataatag ctttcaaaat   2640
   acaataccct ttaatatttt ttcatatact aataattctt tattaaaaga tataattaat   2700
   gaatatttca at                                                       2712
<210> 26
   <211> 904
   <212> PRT
   <213> Artificial Sequence
<220>
   <223> Synthetic
<400> 26
   Phe Gly Gly Phe Thr Gly Ala Arg Lys Ser Ala Arg Lys Tyr Ala Asn
   1               5                   10                  15
   Gln Thr Ser Gly Gly Gly Gly Ser Gly Gly Gly Gly Ser Gly Gly Gly
               20                  25                  30
   Gly Ser Pro Arg Gly Ser Met Pro Ile Thr Ile Asn Asn Phe Asn Tyr
           35                  40                  45
   Ser Asp Pro Val Asp Asn Lys Asn Ile Leu Tyr Leu Asp Thr His Leu
       50                  55                  60
   Asn Thr Leu Ala Asn Glu Pro Glu Lys Ala Phe Arg Ile Thr Gly Asn
   65                  70                  75                  80
   Ile Trp Val Ile Pro Asp Arg Phe Ser Arg Asn Ser Asn Pro Asn Leu
                   85                  90                  95
   Asn Lys Pro Pro Arg Val Thr Ser Pro Lys Ser Gly Tyr Tyr Asp Pro
               100                 105                 110
   Asn Tyr Leu Ser Thr Asp Ser Asp Lys Asp Thr Phe Leu Lys Glu Ile
           115                 120                 125
   Ile Lys Leu Phe Lys Arg Ile Asn Ser Arg Glu Ile Gly Glu Glu Leu
       130                 135                 140
   Ile Tyr Arg Leu Ser Thr Asp Ile Pro Phe Pro Gly Asn Asn Asn Thr
   145                 150                 155                 160
   Pro Ile Asn Thr Phe Asp Phe Asp Val Asp Phe Asn Ser Val Asp Val
                   165                 170                 175
   Lys Thr Arg Gln Gly Asn Asn Trp Val Lys Thr Gly Ser Ile Asn Pro
               180                 185                 190
   Ser Val Ile Ile Thr Gly Pro Arg Glu Asn Ile Ile Asp Pro Glu Thr
           195                 200                 205
   Ser Thr Phe Lys Leu Thr Asn Asn Thr Phe Ala Ala Gln Glu Gly Phe
       210                 215                 220
   Gly Ala Leu Ser Ile Ile Ser Ile Ser Pro Arg Phe Met Leu Thr Tyr
   225 230 235 240
   Ser Asn Ala Thr Asn Asp Val Gly Glu Gly Arg Phe Ser Lys Ser Glu
   245 250 255
   Phe Cys Met Asp Pro Ile Leu Ile Leu Met His Glu Leu Asn His Ala
   260 265 270
   Met His Asn Leu Tyr Gly Ile Ala Ile Pro Asn Asp Gln Thr Ile Ser
   275 280 285
   Ser Val Thr Ser Asn Ile Phe Tyr Ser Gln Tyr Asn Val Lys Leu Glu
   290 295 300
   Tyr Ala Glu Ile Tyr Ala Phe Gly Gly Pro Thr Ile Asp Leu Ile Pro
   305 310 315 320
   Lys Ser Ala Arg Lys Tyr Phe Glu Glu Lys Ala Leu Asp Tyr Tyr Arg
   325 330 335
   Ser Ile Ala Lys Arg Leu Asn Ser Ile Thr Thr Ala Asn Pro Ser Ser
   340 345 350
   Phe Asn Lys Tyr Ile Gly Glu Tyr Lys Gln Lys Leu Ile Arg Lys Tyr
   355 360 365
   Arg Phe Val Val Glu Ser Ser Gly Glu Val Thr Val Asn Arg Asn Lys
   370 375 380
   Phe Val Glu Leu Tyr Asn Glu Leu Thr Gln Ile Phe Thr Glu Phe Asn
   385 390 395 400
   Tyr Ala Lys Ile Tyr Asn Val Gln Asn Arg Lys Ile Tyr Leu Ser Asn
   405 410 415
   Val Tyr Thr Pro Val Thr Ala Asn Ile Leu Asp Asp Asn Val Tyr Asp
   420 425 430
   Ile Gln Asn Gly Phe Asn Ile Pro Lys Ser Asn Leu Asn Val Leu Phe
   435 440 445
   Met Gly Gln Asn Leu Ser Arg Asn Pro Ala Leu Arg Lys Val Asn Pro
   450 455 460
   Glu Asn Met Leu Tyr Leu Phe Thr Lys Phe Cys His Lys Ala Ile Asp
   465 470 475 480
   Gly Arg Ser Leu Tyr Asn Lys Thr Leu Asp Cys Arg Glu Leu Leu Val
   485 490 495
   Lys Asn Thr Asp Leu Pro Phe Ile Gly Asp Ile Ser Asp Val Lys Thr
   500 505 510
   Asp Ile Phe Leu Arg Lys Asp Ile Asn Glu Glu Thr Glu Val Ile Tyr
   515 520 525
   Tyr Pro Asp Asn Val Ser Val Asp Gln Val Ile Leu Ser Lys Asn Thr
   530 535 540
   Ser Glu His Gly Gln Leu Asp Leu Leu Tyr Pro Ser Ile Asp Ser Glu
   545 550 555 560
   Ser Glu Ile Leu Pro Gly Glu Asn Gln Val Phe Tyr Asp Asn Arg Thr
   565 570 575
   Gln Asn Val Asp Tyr Leu Asn Ser Tyr Tyr Tyr Leu Glu Ser Gln Lys
   580 585 590
   Leu Ser Asp Asn Val Glu Asp Phe Thr Phe Thr Arg Ser Ile Glu Glu
   595 600 605
   Ala Leu Asp Asn Ser Ala Lys Val Tyr Thr Tyr Phe Pro Thr Leu Ala
   610 615 620
   Asn Lys Val Asn Ala Gly Val Gln Gly Gly Leu Phe Leu Met Trp Ala
   625 630 635 640
   Asn Asp Val Val Glu Asp Phe Thr Thr Asn Ile Leu Arg Lys Asp Thr
   645 650 655
   Leu Asp Lys Ile Ser Asp Val Ser Ala Ile Ile Pro Tyr Ile Gly Pro
   660 665 670
   Ala Leu Asn Ile Ser Asn Ser Val Arg Arg Gly Asn Phe Thr Glu Ala
   675 680 685
   Phe Ala Val Thr Gly Val Thr Ile Leu Leu Glu Ala Phe Pro Glu Phe
   690 695 700
   Thr Ile Pro Ala Leu Gly Ala Phe Val Ile Tyr Ser Lys Val Gln Glu
   705 710 715 720
   Arg Asn Glu Ile Ile Lys Thr Ile Asp Asn Cys Leu Glu Gln Arg Ile
                   725                 730                 735
   Lys Arg Trp Lys Asp Ser Tyr Glu Trp Met Met Gly Thr Trp Leu Ser
               740                 745                 750
   Arg Ile Ile Thr Gln Phe Asn Asn Ile Ser Tyr Gln Met Tyr Asp Ser
           755                 760                 765
   Leu Asn Tyr Gln Ala Gly Ala Ile Lys Ala Lys Ile Asp Leu Glu Tyr
       770                 775                 780
   Lys Lys Tyr Ser Gly Ser Asp Lys Glu Asn Ile Lys Ser Gln Val Glu
   785                 790                 795                 800
   Asn Leu Lys Asn Ser Leu Asp Val Lys Ile Ser Glu Ala Met Asn Asn
                   805                 810                 815
   Ile Asn Lys Phe Ile Arg Glu Cys Ser Val Thr Tyr Leu Phe Lys Asn
               820                 825                 830
   Met Leu Pro Lys Val Ile Asp Glu Leu Asn Glu Phe Asp Arg Asn Thr
           835                 840                 845
   Lys Ala Lys Leu Ile Asn Leu Ile Asp Ser His Asn Ile Ile Leu Val
       850                 855                 860
   Gly Glu Val Asp Lys Leu Lys Ala Lys Val Asn Asn Ser Phe Gln Asn
   865                 870                 875                 880
   Thr Ile Pro Phe Asn Ile Phe Ser Tyr Thr Asn Asn Ser Leu Leu Lys
                   885                 890                 895
   Asp Ile Ile Asn Glu Tyr Phe Asn
               900
<210> 27
   <211> 1302
   <212> DNA
   <213> Artificial Sequence
<220>
   <223> Synthetic
<400> 27
   ggatccatgg agttcgttaa caaacagttc aactataaag acccagttaa cggtgttgac      60
   attgcttaca tcaaaatccc gaacgctggc cagatgcagc cggtaaaggc attcaaaatc 120
   cacaacaaaa tctgggttat cccggaacgt gataccttta ctaacccgga agaaggtgac 180
   ctgaacccgc caccggaagc gaaacaggtg ccggtatctt actatgactc cacctacctg 240
   tctaccgata acgaaaagga caactacctg aaaggtgtta ctaaactgtt cgagcgtatt 300
   tactccaccg acctgggccg tatgctgctg actagcatcg ttcgcggtat cccgttctgg 360
   ggcggttcta ccatcgatac cgaactgaaa gtaatcgaca ctaactgcat caacgttatt 420
   cagccggacg gttcctatcg ttccgaagaa ctgaacctgg tgatcatcgg cccgtctgct 480
   gatatcatcc agttcgagtg taagagcttt ggtcacgaag ttctgaacct cacccgtaac 540
   ggctacggtt ccactcagta catccgtttc tctccggact tcaccttcgg ttttgaagaa 600
   tccctggaag tagacacgaa cccactgctg ggcgctggta aattcgcaac tgatcctgcg 660
   gttaccctgg ctcacgaact gattcatgca ggccaccgcc tgtacggtat cgccatcaat 720
   ccgaaccgtg tcttcaaagt taacaccaac gcgtattacg agatgtccgg tctggaagtt 780
   agcttcgaag aactgcgtac ttttggcggt cacgacgcta aattcatcga ctctctgcaa 840
   gaaaacgagt tccgtctgta ctactataac aagttcaaag atatcgcatc caccctgaac 900
   aaagcgaaat ccatcgtggg taccactgct tctctccagt acatgaagaa cgtttttaaa 960
   gaaaaatacc tgctcagcga agacacctcc ggcaaattct ctgtagacaa gttgaaattc 1020
   gataaacttt acaaaatgct gactgaaatt tacaccgaag acaacttcgt taagttcttt 1080
   aaagttctga accgcaaaac ctatctgaac ttcgacaagg cagtattcaa aatcaacatc 1140
   gtgccgaaag ttaactacac tatctacgat ggtttcaacc tgcgtaacac caacctggct 1200
   gctaatttta acggccagaa cacggaaatc aacaacatga acttcacaaa actgaaaaac 1260
   ttcactggtc tgttcgagtt ttacaagctg ctgtgcgtcg ac 1302
<210> 28
   <211> 1257
   <212> DNA
   <213> Artificial Sequence
<220>
   <223> Synthetic
<400> 28
   ctgcagtgta tcaaggttaa caactgggat ttattcttca gcccgagtga agacaacttc     60
   accaacgacc tgaacaaagg tgaagaaatc acctcagata ctaacatcga agcagccgaa    120
   gaaaacatct cgctggacct gatccagcag tactacctga cctttaattt cgacaacgag    180
   ccggaaaaca tttctatcga aaacctgagc tctgatatca tcggccagct ggaactgatg    240
   ccgaacatcg aacgtttccc aaacggtaaa aagtacgagc tggacaaata taccatgttc    300
   cactacctgc gcgcgcagga atttgaacac ggcaaatccc gtatcgcact gactaactcc    360
   gttaacgaag ctctgctcaa cccgtcccgt gtatacacct tcttctctag cgactacgtg    420
   aaaaaggtca acaaagcgac tgaagctgca atgttcttgg gttgggttga acagcttgtt    480
   tatgatttta ccgacgagac gtccgaagta tctactaccg acaaaattgc ggatatcact    540
   atcatcatcc cgtacatcgg tccggctctg aacattggca acatgctgta caaagacgac    600
   ttcgttggcg cactgatctt ctccggtgcg gtgatcctgc tggagttcat cccggaaatc    660
   gccatcccgg tactgggcac ctttgctctg gtttcttaca ttgcaaacaa ggttctgact    720
   gtacaaacca tcgacaacgc gctgagcaaa cgtaacgaaa aatgggatga agtttacaaa    780
   tatatcgtga ccaactggct ggctaaggtt aatactcaga tcgacctcat ccgcaaaaaa    840
   atgaaagaag cactggaaaa ccaggcggaa gctaccaagg caatcattaa ctaccagtac    900
   aaccagtaca ccgaggaaga aaaaaacaac atcaacttca acatcgacga tctgtcctct    960
   aaactgaacg aatccatcaa caaagctatg atcaacatca acaagttcct gaaccagtgc   1020
   tctgtaagct atctgatgaa ctccatgatc ccgtacggtg ttaaacgtct ggaggacttc   1080
   gatgcgtctc tgaaagacgc cctgctgaaa tacatttacg acaaccgtgg cactctgatc   1140
   ggtcaggttg atcgtctgaa ggacaaagtg aacaatacct tatcgaccga catccctttt   1200
   cagctcagta aatatgtcga taaccaacgc cttttgtcca ctctagacta gaagctt       1257
<210> 29
   <211> 1323
   <212> DNA
   <213> Artificial Sequence
<220>
   <223> Synthetic
<400> 29
   ggatccatgc cggttaccat caacaacttc aactacaacg acccgatcga caacaacaac 60
   atcattatga tggaaccgcc gttcgcacgt ggtaccggac gttactacaa ggcttttaag 120
   atcaccgacc gtatctggat catcccggaa cgttacacct tcggttacaa acctgaggac 180
   ttcaacaaga gtagcgggat tttcaatcgt gacgtctgcg agtactatga tccagattat 240
   ctgaatacca acgataagaa gaacatattc cttcagacta tgattaaact cttcaaccgt 300
   atcaaaagca aaccgctcgg tgaaaaactc ctcgaaatga ttatcaacgg tatcccgtac 360
   ctcggtgacc gtcgtgtccc gcttgaagag ttcaacacca acatcgcaag cgtcaccgtc 420
   aacaaactca tcagcaaccc aggtgaagtc gaacgtaaaa aaggtatctt cgcaaacctc 480
   atcatcttcg gtccgggtcc ggtcctcaac gaaaacgaaa ccatcgacat cggtatccag 540
   aaccacttcg caagccgtga aggtttcggt ggtatcatgc agatgaaatt ctgcccggaa 600
   tacgtcagtg tcttcaacaa cgtccaggaa aacaaaggtg caagcatctt caaccgtcgt 660
   ggttacttca gcgacccggc actcatcctc atgcatgaac tcatccacgt cctccacggt 720
   ctctacggta tcaaagttga cgacctcccg atcgtcccga acgagaagaa attcttcatg 780
   cagagcaccg acgcaatcca ggctgaggaa ctctacacct tcggtggcca agacccaagt 840
   atcataaccc cgtccaccga caaaagcatc tacgacaaag tcctccagaa cttcaggggt 900
   atcgtggaca gactcaacaa agtcctcgtc tgcatcagcg acccgaacat caatatcaac 960
   atatacaaga acaagttcaa agacaagtac aaattcgtcg aggacagcga aggcaaatac 1020
   agcatcgacg tagaaagttt cgacaagctc tacaaaagcc tcatgttcgg tttcaccgaa 1080
   accaacatcg ccgagaacta caagatcaag acaagggcaa gttacttcag cgacagcctc 1140
   ccgcctgtca aaatcaagaa cctcttagac aacgagattt acacaattga agagggcttc 1200
   aacatcagtg acaaagacat ggagaaggaa tacagaggtc agaacaaggc tatcaacaaa 1260
   caggcatacg aggagatcag caaagaacac ctcgcagtct acaagatcca gatgtgcgtc 1320
   gac 1323
<210> 30
   <211> 1260
   <212> DNA
   <213> Artificial Sequence
<220>
   <223> Synthetic
<400> 30
   ctgcagtgca tcgacgttga caacgaagac ctgttcttca tcgctgacaa aaacagcttc 60
   agtgacgacc tgagcaaaaa cgaacgtatc gaatacaaca cccagagcaa ctacatcgaa 120
   aacgacttcc cgatcaacga actgatcctg gacaccgacc tgataagtaa aatcgaactg 180
   ccgagcgaaa acaccgaaag tctgaccgac ttcaacgttg acgttccggt ttacgaaaaa 240
   cagccggcta tcaagaaaat cttcaccgac gaaaacacca tcttccagta cctgtacagc 300
   cagaccttcc cgctggacat ccgtgacatc agtctgacca gcagtttcga cgacgctctg 360
   ctgttcagca acaaagttta cagtttcttc agcatggact acatcaaaac cgctaacaaa 420
   gttgttgaag cagggctgtt cgctggttgg gttaaacaga tcgttaacga cttcgttatc 480
   gaagctaaca aaagcaacac tatggacaaa atcgctgaca tcagtctgat cgttccgtac 540
   atcggtctgg ctctgaacgt tggtaacgaa accgctaaag gtaactttga aaacgctttc 600
   gagatcgctg gtgcaagcat cctgctggag ttcatcccgg aactgctgat cccggttgtt 660
   ggtgctttcc tgctggaaag ttacatcgac aacaaaaaca agatcatcaa aaccatcgac 720
   aacgctctga ccaaacgtaa cgaaaaatgg agtgatatgt acggtctgat cgttgctcag 780
   tggctgagca ccgtcaacac ccagttctac accatcaaag aaggtatgta caaagctctg 840
   aactaccagg ctcaggctct ggaagagatc atcaaatacc gttacaacat ctacagtgag 900
   aaggaaaaga gtaacatcaa catcgacttc aacgacatca acagcaaact gaacgaaggt 960
   atcaaccagg ctatcgacaa catcaacaac ttcatcaacg gttgcagtgt tagctacctg 1020
   atgaagaaga tgatcccgct ggctgttgaa aaactgctgg acttcgacaa caccctgaaa 1080
   aagaacctgc tgaactacat cgacgaaaac aagctgtacc tgatcggtag tgctgaatac 1140
   gaaaaaagta aagtgaacaa atacctgaag accatcatgc cgttcgacct gagtatctac 1200
   accaacgaca ccatcctgat cgaaatgttc aacaaataca actctctaga ctagaagctt 1260
<210> 31
   <211> 1329
   <212> DNA
   <213> Artificial Sequence
<220>
   <223> Synthetic
<400> 31
   ggatccgaat tcatgccgat caccatcaac aacttcaact acagcgatcc ggtggataac      60
   aaaaacatcc tgtacctgga tacccatctg aataccctgg cgaacgaacc ggaaaaagcg     120
   tttcgtatca ccggcaacat ttgggttatt ccggatcgtt ttagccgtaa cagcaacccg     180
   aatctgaata aaccgccgcg tgttaccagc ccgaaaagcg gttattacga tccgaactat     240
   ctgagcaccg atagcgataa agataccttc ctgaaagaaa tcatcaaact gttcaaacgc     300
   atcaacagcc gtgaaattgg cgaagaactg atctatcgcc tgagcaccga tattccgttt     360
   ccgggcaaca acaacacccc gatcaacacc tttgatttcg atgtggattt caacagcgtt     420
   gatgttaaaa cccgccaggg taacaattgg gtgaaaaccg gcagcattaa cccgagcgtg     480
   attattaccg gtccgcgcga aaacattatt gatccggaaa ccagcacctt taaactgacc     540
   aacaacacct ttgcggcgca ggaaggtttt ggcgcgctga gcattattag cattagcccg     600
   cgctttatgc tgacctatag caacgcgacc aacgatgttg gtgaaggccg tttcagcaaa     660
   agcgaatttt gcatggaccc gatcctgatc ctgatgcatg aactgaacca tgcgatgcat     720
   aacctgtatg gcatcgcgat tccgaacgat cagaccatta gcagcgtgac cagcaacatc     780
   ttttacagcc agtacaacgt gaaactggaa tatgcggaaa tctatgcgtt tggcggtccg     840
   accattgatc tgattccgaa aagcgcgcgc aaatacttcg aagaaaaagc gctggattac     900
   tatcgcagca ttgcgaaacg tctgaacagc attaccaccg cgaatccgag cagcttcaac     960
   aaatatatcg gcgaatataa acagaaactg atccgcaaat atcgctttgt ggtggaaagc    1020
   agcggcgaag ttaccgttaa ccgcaataaa ttcgtggaac tgtacaacga actgacccag    1080
   atcttcaccg aatttaacta tgcgaaaatc tataacgtgc agaaccgtaa aatctacctg    1140
   agcaacgtgt ataccccggt gaccgcgaat attctggatg ataacgtgta cgatatccag    1200
   aacggcttta acatcccgaa aagcaacctg aacgttctgt ttatgggcca gaacctgagc    1260
   cgtaatccgg cgctgcgtaa agtgaacccg gaaaacatgc tgtacctgtt caccaaattt    1320
   tgcgtcgac                                                            1329
<210> 32
   <211> 1263
   <212> DNA
   <213> Artificial Sequence
<220>
   <223> Synthetic
<400> 32
   ctgcagtgtc gtgaactgct ggtgaaaaac accgatctgc cgtttattgg cgatatcagc    60
   gatgtgaaaa ccgatatctt cctgcgcaaa gatatcaacg aagaaaccga agtgatctac   120
   tacccggata acgtgagcgt tgatcaggtg atcctgagca aaaacaccag cgaacatggt   180
   cagctggatc tgctgtatcc gagcattgat agcgaaagcg aaattctgcc gggcgaaaac   240
   caggtgtttt acgataaccg tacccagaac gtggattacc tgaacagcta ttactacctg   300
   gaaagccaga aactgagcga taacgtggaa gattttacct ttacccgcag cattgaagaa   360
   gcgctggata acagcgcgaa agtttacacc tattttccga ccctggcgaa caaagttaat   420
   gcgggtgttc agggcggtct gtttctgatg tgggcgaacg atgtggtgga agatttcacc   480
   accaacatcc tgcgtaaaga taccctggat aaaatcagcg atgttagcgc gattattccg   540
   tatattggtc cggcgctgaa cattagcaat agcgtgcgtc gtggcaattt taccgaagcg   600
   tttgcggtta ccggtgtgac cattctgctg gaagcgtttc cggaatttac cattccggcg   660
   ctgggtgcgt ttgtgatcta tagcaaagtg caggaacgca acgaaatcat caaaaccatc   720
   gataactgcc tggaacagcg tattaaacgc tggaaagata gctatgaatg gatgatgggc   780
   acctggctga gccgtattat cacccagttc aacaacatca gctaccagat gtacgatagc   840
   ctgaactatc aggcgggtgc gattaaagcg aaaatcgatc tggaatacaa aaaatacagc   900
   ggcagcgata aagaaaacat caaaagccag gttgaaaacc tgaaaaacag cctggatgtg   960
   aaaattagcg aagcgatgaa taacatcaac aaattcatcc gcgaatgcag cgtgacctac  1020
   ctgttcaaaa acatgctgcc gaaagtgatc gatgaactga acgaatttga tcgcaacacc  1080
   aaagcgaaac tgatcaacct gatcgatagc cacaacatta ttctggtggg cgaagtggat  1140
   aaactgaaag cgaaagttaa caacagcttc cagaacacca tcccgtttaa catcttcagc  1200
   tataccaaca acagcctgct gaaagatatc atcaacgaat acttcaatct agactagaag  1260
   ctt                                                                1263
<210> 33
   <211> 207
   <212> DNA
   <213> Artificial Sequence
<220>
   <223> Synthetic
<400> 33
   ggatccacgc acgtcgacgg catcattacc tccaaaacta aatctctgat cgaaggtcgt 60
   tttggcggtt tcacgggcgc acgcaaatca gcgcgtaaat tagctaacca ggcgctagcg 120
   ggcggtggcg gtagcggcgg tggcggtagc ggcggtggcg gtagcgcact agtgctgcag 180
   acgcacggtc tagaatgata aaagctt 207
<210> 34
   <211> 108
   <212> DNA
   <213> Artificial Sequence
<220>
   <223> Synthetic
<400> 34
   ggatccacgc acgtcgacgg catcattacc tccaaaacta aatctctgat agaaggtaga 60
   aacaaagcgc tgaacctgca gacgcacggt ctagaatgat aaaagctt 108
<210> 35
   <211> 186
   <212> DNA
   <213> Artificial Sequence
<220>
   <223> Synthetic
<400> 35
   catatgaata acctcgggat tgagggtcgt tttggcggtt tcacgggcgc acgcaaatca 60
   gcgcgtaaat tagctaacca gactagtggc ggtgggggta gtggcggtgg cggttcgggc 120
   gggggtggga gccctagggg atccgtcgac ctgcagggtc tagaagcgct agcgtgataa 180
   aagctt 186
<210> 36
   <211> 180
   <212> DNA
   <213> Artificial Sequence
<220>
   <223> Synthetic
<400> 36
   ggatccacgc acgtcgacgc gattgatggt cgttttggcg gtttcacggg cgcacgcaaa 60
   tcagcgcgta aattagctaa ccaggcgcta gcgggcggtg gcggtagcgg cggtggcggt 120
   agcggcggtg gcggtagcgc actagtgctg cagacgcacg gtctagaatg ataaaagctt 180
<210> 37
   <211> 249
   <212> DNA
   <213> Artificial Sequence
<220>
   <223> Synthetic
<400> 37
   ggatccacgc acgtcgacgg catcattacc tccaaaacta aatctctgat cgaaggtcgt 60
   tacggtggtt tcatgacctc tgaaaaatct cagaccccgc tggttaccct gttcaaaaac 120
   gctatcatca aaaacgctta caaaaaaggt gaagcgctag cgggtggtgg tggttctggt 180
   ggtggtggtt ctggtggtgg tggttctgca ctagtgctgc agacgcacgg tctagaatga 240
   taaaagctt 249
<210> 38
   <211> 207
   <212> DNA
   <213> Artificial Sequence
<220>
   <223> Synthetic
<400> 38
   ggatccacgc acgtcgacgg catcattacc tccaaaacta aatctctgat cgaaggtcgt 60
   tttggcggtt tcacgggcgc acgcaaatca gcgcgtaaac gtaagaacca ggcgctagcg 120
   ggcggtggcg gtagcggcgg tggcggtagc ggcggtggcg gtagcgcact agtgctgcag 180
   acgcacggtc tagaatgata aaagctt 207
<210> 39
   <211> 2709
   <212> DNA
   <213> Artificial Sequence
<220>
   <223> Synthetic
<400> 39
   ggatccatgg agttcgttaa caaacagttc aactataaag acccagttaa cggtgttgac 60
   attgcttaca tcaaaatccc gaacgctggc cagatgcagc cggtaaaggc attcaaaatc     120
   cacaacaaaa tctgggttat cccggaacgt gataccttta ctaacccgga agaaggtgac     180
   ctgaacccgc caccggaagc gaaacaggtg ccggtatctt actatgactc cacctacctg     240
   tctaccgata acgaaaagga caactacctg aaaggtgtta ctaaactgtt cgagcgtatt     300
   tactccaccg acctgggccg tatgctgctg actagcatcg ttcgcggtat cccgttctgg     360
   ggcggttcta ccatcgatac cgaactgaaa gtaatcgaca ctaactgcat caacgttatt     420
   cagccggacg gttcctatcg ttccgaagaa ctgaacctgg tgatcatcgg cccgtctgct     480
   gatatcatcc agttcgagtg taagagcttt ggtcacgaag ttctgaacct cacccgtaac     540
   ggctacggtt ccactcagta catccgtttc tctccggact tcaccttcgg ttttgaagaa     600
   tccctggaag tagacacgaa cccactgctg ggcgctggta aattcgcaac tgatcctgcg     660
   gttaccctgg ctcacgaact gattcatgca ggccaccgcc tgtacggtat cgccatcaat     720
   ccgaaccgtg tcttcaaagt taacaccaac gcgtattacg agatgtccgg tctggaagtt     780
   agcttcgaag aactgcgtac ttttggcggt cacgacgcta aattcatcga ctctctgcaa     840
   gaaaacgagt tccgtctgta ctactataac aagttcaaag atatcgcatc caccctgaac     900
   aaagcgaaat ccatcgtggg taccactgct tctctccagt acatgaagaa cgtttttaaa     960
   gaaaaatacc tgctcagcga agacacctcc ggcaaattct ctgtagacaa gttgaaattc    1020
   gataaacttt acaaaatgct gactgaaatt tacaccgaag acaacttcgt taagttcttt    1080
   aaagttctga accgcaaaac ctatctgaac ttcgacaagg cagtattcaa aatcaacatc    1140
   gtgccgaaag ttaactacac tatctacgat ggtttcaacc tgcgtaacac caacctggct    1200
   gctaatttta acggccagaa cacggaaatc aacaacatga acttcacaaa actgaaaaac    1260
   ttcactggtc tgttcgagtt ttacaagctg ctgtgcgtcg acggcatcat tacctccaaa    1320
   actaaatctc tgatagaagg tagatttggc ggtttcacgg gcgcacgcaa atcagcgcgt    1380
   aaattagcta accaggcgct agcgggcggt ggcggtagcg gcggtggcgg tagcggcggt    1440
   ggcggtagcg cactagtgct gcagtgtatc aaggttaaca actgggattt attcttcagc    1500
   ccgagtgaag acaacttcac caacgacctg aacaaaggtg aagaaatcac ctcagatact    1560
   aacatcgaag cagccgaaga aaacatctcg ctggacctga tccagcagta ctacctgacc    1620
   tttaatttcg acaacgagcc ggaaaacatt tctatcgaaa acctgagctc tgatatcatc    1680
   ggccagctgg aactgatgcc gaacatcgaa cgtttcccaa acggtaaaaa gtacgagctg    1740
   gacaaatata ccatgttcca ctacctgcgc gcgcaggaat ttgaacacgg caaatcccgt    1800
   atcgcactga ctaactccgt taacgaagct ctgctcaacc cgtcccgtgt atacaccttc    1860
   ttctctagcg actacgtgaa aaaggtcaac aaagcgactg aagctgcaat gttcttgggt 1920
   tgggttgaac agcttgttta tgattttacc gacgagacgt ccgaagtatc tactaccgac 1980
   aaaattgcgg atatcactat catcatcccg tacatcggtc cggctctgaa cattggcaac 2040
   atgctgtaca aagacgactt cgttggcgca ctgatcttct ccggtgcggt gatcctgctg 2100
   gagttcatcc cggaaatcgc catcccggta ctgggcacct ttgctctggt ttcttacatt 2160
   gcaaacaagg ttctgactgt acaaaccatc gacaacgcgc tgagcaaacg taacgaaaaa 2220
   tgggatgaag tttacaaata tatcgtgacc aactggctgg ctaaggttaa tactcagatc 2280
   gacctcatcc gcaaaaaaat gaaagaagca ctggaaaacc aggcggaagc taccaaggca 2340
   atcattaact accagtacaa ccagtacacc gaggaagaaa aaaacaacat caacttcaac 2400
   atcgacgatc tgtcctctaa actgaacgaa tccatcaaca aagctatgat caacatcaac 2460
   aagttcctga accagtgctc tgtaagctat ctgatgaact ccatgatccc gtacggtgtt 2520
   aaacgtctgg aggacttcga tgcgtctctg aaagacgccc tgctgaaata catttacgac 2580
   aaccgtggca ctctgatcgg tcaggttgat cgtctgaagg acaaagtgaa caatacctta 2640
   tcgaccgaca tcccttttca gctcagtaaa tatgtcgata accaacgcct tttgtccact   2700
   ctagactag                                                           2709
<210> 40
   <211> 902
   <212> PRT
   <213> Artificial Sequence
<220>
   <223> Synthetic
<400> 40
   Gly Ser Met Glu Phe Val Asn Lys Gln Phe Asn Tyr Lys Asp Pro Val
   1               5                   10                  15
   Asn Gly Val Asp Ile Ala Tyr Ile Lys Ile Pro Asn Ala Gly Gln Met
               20                  25                  30
   Gln Pro Val Lys Ala Phe Lys Ile His Asn Lys Ile Trp Val Ile Pro
           35                  40                  45
   Glu Arg Asp Thr Phe Thr Asn Pro Glu Glu Gly Asp Leu Asn Pro Pro
       50                  55                  60
   Pro Glu Ala Lys Gln Val Pro Val Ser Tyr Tyr Asp Ser Thr Tyr Leu
   65                  70                  75                  80
   Ser Thr Asp Asn Glu Lys Asp Asn Tyr Leu Lys Gly Val Thr Lys Leu
   85 90 95
   Phe Glu Arg Ile Tyr Ser Thr Asp Leu Gly Arg Met Leu Leu Thr Ser
   100 105 110
   Ile Val Arg Gly Ile Pro Phe Trp Gly Gly Ser Thr Ile Asp Thr Glu
   115 120 125
   Leu Lys Val Ile Asp Thr Asn Cys Ile Asn Val Ile Gln Pro Asp Gly
   130 135 140
   Ser Tyr Arg Ser Glu Glu Leu Asn Leu Val Ile Ile Gly Pro Ser Ala
   145 150 155 160
   Asp Ile Ile Gln Phe Glu Cys Lys Ser Phe Gly His Glu Val Leu Asn
   165 170 175
   Leu Thr Arg Asn Gly Tyr Gly Ser Thr Gln Tyr Ile Arg Phe Ser Pro
   180 185 190
   Asp Phe Thr Phe Gly Phe Glu Glu Ser Leu Glu Val Asp Thr Asn Pro
   195 200 205
   Leu Leu Gly Ala Gly Lys Phe Ala Thr Asp Pro Ala Val Thr Leu Ala
   210 215 220
   His Glu Leu Ile His Ala Gly His Arg Leu Tyr Gly Ile Ala Ile Asn
   225 230 235 240
   Pro Asn Arg Val Phe Lys Val Asn Thr Asn Ala Tyr Tyr Glu Met Ser
   245 250 255
   Gly Leu Glu Val Ser Phe Glu Glu Leu Arg Thr Phe Gly Gly His Asp
   260 265 270
   Ala Lys Phe Ile Asp Ser Leu Gln Glu Asn Glu Phe Arg Leu Tyr Tyr
   275 280 285
   Tyr Asn Lys Phe Lys Asp Ile Ala Ser Thr Leu Asn Lys Ala Lys Ser
   290 295 300
   Ile Val Gly Thr Thr Ala Ser Leu Gln Tyr Met Lys Asn Val Phe Lys
   305 310 315 320
   Glu Lys Tyr Leu Leu Ser Glu Asp Thr Ser Gly Lys Phe Ser Val Asp
   325 330 335
   Lys Leu Lys Phe Asp Lys Leu Tyr Lys Met Leu Thr Glu Ile Tyr Thr
   340 345 350
   Glu Asp Asn Phe Val Lys Phe Phe Lys Val Leu Asn Arg Lys Thr Tyr
   355 360 365
   Leu Asn Phe Asp Lys Ala Val Phe Lys Ile Asn Ile Val Pro Lys Val
   370 375 380
   Asn Tyr Thr Ile Tyr Asp Gly Phe Asn Leu Arg Asn Thr Asn Leu Ala
   385 390 395 400
   Ala Asn Phe Asn Gly Gln Asn Thr Glu Ile Asn Asn Met Asn Phe Thr
   405 410 415
   Lys Leu Lys Asn Phe Thr Gly Leu Phe Glu Phe Tyr Lys Leu Leu Cys
   420 425 430
   Val Asp Gly Ile Ile Thr Ser Lys Thr Lys Ser Leu Ile Glu Gly Arg
   435 440 445
   Phe Gly Gly Phe Thr Gly Ala Arg Lys Ser Ala Arg Lys Leu Ala Asn
   450 455 460
   Gln Ala Leu Ala Gly Gly Gly Gly Ser Gly Gly Gly Gly Ser Gly Gly
   465 470 475 480
   Gly Gly Ser Ala Leu Val Leu Gln Cys Ile Lys Val Asn Asn Trp Asp
   485 490 495
   Leu Phe Phe Ser Pro Ser Glu Asp Asn Phe Thr Asn Asp Leu Asn Lys
   500 505 510
   Gly Glu Glu Ile Thr Ser Asp Thr Asn Ile Glu Ala Ala Glu Glu Asn
   515 520 525
   Ile Ser Leu Asp Leu Ile Gln Gln Tyr Tyr Leu Thr Phe Asn Phe Asp
   530 535 540
   Asn Glu Pro Glu Asn Ile Ser Ile Glu Asn Leu Ser Ser Asp Ile Ile
   545 550 555 560
   Gly Gln Leu Glu Leu Met Pro Asn Ile Glu Arg Phe Pro Asn Gly Lys
   565 570 575
   Lys Tyr Glu Leu Asp Lys Tyr Thr Met Phe His Tyr Leu Arg Ala Gln
   580 585 590
   Glu Phe Glu His Gly Lys Ser Arg Ile Ala Leu Thr Asn Ser Val Asn
   595 600 605
   Glu Ala Leu Leu Asn Pro Ser Arg Val Tyr Thr Phe Phe Ser Ser Asp
   610 615 620
   Tyr Val Lys Lys Val Asn Lys Ala Thr Glu Ala Ala Met Phe Leu Gly
   625 630 635 640
   Trp Val Glu Gln Leu Val Tyr Asp Phe Thr Asp Glu Thr Ser Glu Val
   645 650 655
   Ser Thr Thr Asp Lys Ile Ala Asp Ile Thr Ile Ile Ile Pro Tyr Ile
   660 665 670
   Gly Pro Ala Leu Asn Ile Gly Asn Met Leu Tyr Lys Asp Asp Phe Val
   675 680 685
   Gly Ala Leu Ile Phe Ser Gly Ala Val Ile Leu Leu Glu Phe Ile Pro
   690 695 700
   Glu Ile Ala Ile Pro Val Leu Gly Thr Phe Ala Leu Val Ser Tyr Ile
   705 710 715 720
   Ala Asn Lys Val Leu Thr Val Gln Thr Ile Asp Asn Ala Leu Ser Lys
   725 730 735
   Arg Asn Glu Lys Trp Asp Glu Val Tyr Lys Tyr Ile Val Thr Asn Trp
   740 745 750
   Leu Ala Lys Val Asn Thr Gln Ile Asp Leu Ile Arg Lys Lys Met Lys
   755 760 765
   Glu Ala Leu Glu Asn Gln Ala Glu Ala Thr Lys Ala Ile Ile Asn Tyr
   770 775 780
   Gln Tyr Asn Gln Tyr Thr Glu Glu Glu Lys Asn Asn Ile Asn Phe Asn
   785 790 795 800
   Ile Asp Asp Leu Ser Ser Lys Leu Asn Glu Ser Ile Asn Lys Ala Met
   805 810 815
   Ile Asn Ile Asn Lys Phe Leu Asn Gln Cys Ser Val Ser Tyr Leu Met
   820 825 830
   Asn Ser Met Ile Pro Tyr Gly Val Lys Arg Leu Glu Asp Phe Asp Ala
   835 840 845
   Ser Leu Lys Asp Ala Leu Leu Lys Tyr Ile Tyr Asp Asn Arg Gly Thr
   850 855 860
   Leu Ile Gly Gln Val Asp Arg Leu Lys Asp Lys Val Asn Asn Thr Leu
   865 870 875 880
   Ser Thr Asp Ile Pro Phe Gln Leu Ser Lys Tyr Val Asp Asn Gln Arg
   885 890 895
   Leu Leu Ser Thr Leu Asp
   900
<210> 41
   <211> 2736
   <212> DNA
   <213> Artificial Sequence
<220>
   <223> Synthetic
<400> 41
   ctcgggattg agggtcgttt tggcggtttc acgggcgcac gcaaatcagc gcgtaaatta     60
   gctaaccaga ctagtggcgg tgggggtagt ggcggtggcg gttcgggcgg gggtgggagc    120
   cctaggggat ccatggagtt cgttaacaaa cagttcaact ataaagaccc agttaacggt    180
   gttgacattg cttacatcaa aatcccgaac gctggccaga tgcagccggt aaaggcattc    240
   aaaatccaca acaaaatctg ggttatcccg gaacgtgata cctttactaa cccggaagaa    300
   ggtgacctga acccgccacc ggaagcgaaa caggtgccgg tatcttacta tgactccacc    360
   tacctgtcta ccgataacga aaaggacaac tacctgaaag gtgttactaa actgttcgag    420
   cgtatttact ccaccgacct gggccgtatg ctgctgacta gcatcgttcg cggtatcccg    480
   ttctggggcg gttctaccat cgataccgaa ctgaaagtaa tcgacactaa ctgcatcaac    540
   gttattcagc cggacggttc ctatcgttcc gaagaactga acctggtgat catcggcccg    600
   tctgctgata tcatccagtt cgagtgtaag agctttggtc acgaagttct gaacctcacc    660
   cgtaacggct acggttccac tcagtacatc cgtttctctc cggacttcac cttcggtttt    720
   gaagaatccc tggaagtaga cacgaaccca ctgctgggcg ctggtaaatt cgcaactgat    780
   cctgcggtta ccctggctca cgaactgatt catgcaggcc accgcctgta cggtatcgcc    840
   atcaatccga accgtgtctt caaagttaac accaacgcgt attacgagat gtccggtctg 900
   gaagttagct tcgaagaact gcgtactttt ggcggtcacg acgctaaatt catcgactct 960
   ctgcaagaaa acgagttccg tctgtactac tataacaagt tcaaagatat cgcatccacc 1020
   ctgaacaaag cgaaatccat cgtgggtacc actgcttctc tccagtacat gaagaacgtt 1080
   tttaaagaaa aatacctgct cagcgaagac acctccggca aattctctgt agacaagttg 1140
   aaattcgata aactttacaa aatgctgact gaaatttaca ccgaagacaa cttcgttaag 1200
   ttctttaaag ttctgaaccg caaaacctat ctgaacttcg acaaggcagt attcaaaatc 1260
   aacatcgtgc cgaaagttaa ctacactatc tacgatggtt tcaacctgcg taacaccaac 1320
   ctggctgcta attttaacgg ccagaacacg gaaatcaaca acatgaactt cacaaaactg 1380
   aaaaacttca ctggtctgtt cgagttttac aagctgctgt gcgtcgacgg catcattacc 1440
   tccaaaacta aatctctgat agaaggtaga aacaaagcgc tgaacgacct ctgtatcaag 1500
   gttaacaact gggatttatt cttcagcccg agtgaagaca acttcaccaa cgacctgaac 1560
   aaaggtgaag aaatcacctc agatactaac atcgaagcag ccgaagaaaa catctcgctg 1620
   gacctgatcc agcagtacta cctgaccttt aatttcgaca acgagccgga aaacatttct 1680
   atcgaaaacc tgagctctga tatcatcggc cagctggaac tgatgccgaa catcgaacgt 1740
   ttcccaaacg gtaaaaagta cgagctggac aaatatacca tgttccacta cctgcgcgcg 1800
   caggaatttg aacacggcaa atcccgtatc gcactgacta actccgttaa cgaagctctg 1860
   ctcaacccgt cccgtgtata caccttcttc tctagcgact acgtgaaaaa ggtcaacaaa 1920
   gcgactgaag ctgcaatgtt cttgggttgg gttgaacagc ttgtttatga ttttaccgac 1980
   gagacgtccg aagtatctac taccgacaaa attgcggata tcactatcat catcccgtac 2040
   atcggtccgg ctctgaacat tggcaacatg ctgtacaaag acgacttcgt tggcgcactg 2100
   atcttctccg gtgcggtgat cctgctggag ttcatcccgg aaatcgccat cccggtactg 2160
   ggcacctttg ctctggtttc ttacattgca aacaaggttc tgactgtaca aaccatcgac 2220
   aacgcgctga gcaaacgtaa cgaaaaatgg gatgaagttt acaaatatat cgtgaccaac 2280
   tggctggcta aggttaatac tcagatcgac ctcatccgca aaaaaatgaa agaagcactg 2340
   gaaaaccagg cggaagctac caaggcaatc attaactacc agtacaacca gtacaccgag 2400
   gaagaaaaaa acaacatcaa cttcaacatc gacgatctgt cctctaaact gaacgaatcc 2460
   atcaacaaag ctatgatcaa catcaacaag ttcctgaacc agtgctctgt aagctatctg 2520
   atgaactcca tgatcccgta cggtgttaaa cgtctggagg acttcgatgc gtctctgaaa 2580
   gacgccctgc tgaaatacat ttacgacaac cgtggcactc tgatcggtca ggttgatcgt 2640
   ctgaaggaca aagtgaacaa taccttatcg accgacatcc cttttcagct cagtaaatat 2700
   gtcgataacc aacgcctttt gtccactcta gactag 2736
<210> 42
   <211> 911
   <212> PRT
   <213> Artificial Sequence
<220>
   <223> Synthetic
<400> 42
   Leu Gly Ile Glu Gly Arg Phe Gly Gly Phe Thr Gly Ala Arg Lys Ser
   1 5 10 15
   Ala Arg Lys Leu Ala Asn Gln Thr Ser Gly Gly Gly Gly Ser Gly Gly
   20 25 30
   Gly Gly Ser Gly Gly Gly Gly Ser Pro Arg Gly Ser Met Glu Phe Val
   35 40 45
   Asn Lys Gln Phe Asn Tyr Lys Asp Pro Val Asn Gly Val Asp Ile Ala
   50 55 60
   Tyr Ile Lys Ile Pro Asn Ala Gly Gln Met Gln Pro Val Lys Ala Phe
   65 70 75 80
   Lys Ile His Asn Lys Ile Trp Val Ile Pro Glu Arg Asp Thr Phe Thr
   85 90 95
   Asn Pro Glu Glu Gly Asp Leu Asn Pro Pro Pro Glu Ala Lys Gln Val
   100 105 110
   Pro Val Ser Tyr Tyr Asp Ser Thr Tyr Leu Ser Thr Asp Asn Glu Lys
   115 120 125
   Asp Asn Tyr Leu Lys Gly Val Thr Lys Leu Phe Glu Arg Ile Tyr Ser
   130 135 140
   Thr Asp Leu Gly Arg Met Leu Leu Thr Ser Ile Val Arg Gly Ile Pro
   145 150 155 160
   Phe Trp Gly Gly Ser Thr Ile Asp Thr Glu Leu Lys Val Ile Asp Thr
   165 170 175
   Asn Cys Ile Asn Val Ile Gln Pro Asp Gly Ser Tyr Arg Ser Glu Glu
   180 185 190
   Leu Asn Leu Val Ile Ile Gly Pro Ser Ala Asp Ile Ile Gln Phe Glu
   195 200 205
   Cys Lys Ser Phe Gly His Glu Val Leu Asn Leu Thr Arg Asn Gly Tyr
   210 215 220
   Gly Ser Thr Gln Tyr Ile Arg Phe Ser Pro Asp Phe Thr Phe Gly Phe
   225 230 235 240
   Glu Glu Ser Leu Glu Val Asp Thr Asn Pro Leu Leu Gly Ala Gly Lys
   245 250 255
   Phe Ala Thr Asp Pro Ala Val Thr Leu Ala His Glu Leu Ile His Ala
   260 265 270
   Gly His Arg Leu Tyr Gly Ile Ala Ile Asn Pro Asn Arg Val Phe Lys
   275 280 285
   Val Asn Thr Asn Ala Tyr Tyr Glu Met Ser Gly Leu Glu Val Ser Phe
   290 295 300
   Glu Glu Leu Arg Thr Phe Gly Gly His Asp Ala Lys Phe Ile Asp Ser
   305 310 315 320
   Leu Gln Glu Asn Glu Phe Arg Leu Tyr Tyr Tyr Asn Lys Phe Lys Asp
   325 330 335
   Ile Ala Ser Thr Leu Asn Lys Ala Lys Ser Ile Val Gly Thr Thr Ala
   340 345 350
   Ser Leu Gln Tyr Met Lys Asn Val Phe Lys Glu Lys Tyr Leu Leu Ser
   355 360 365
   Glu Asp Thr Ser Gly Lys Phe Ser Val Asp Lys Leu Lys Phe Asp Lys
   370 375 380
   Leu Tyr Lys Met Leu Thr Glu Ile Tyr Thr Glu Asp Asn Phe Val Lys
   385 390 395 400
   Phe Phe Lys Val Leu Asn Arg Lys Thr Tyr Leu Asn Phe Asp Lys Ala
   405 410 415
   Val Phe Lys Ile Asn Ile Val Pro Lys Val Asn Tyr Thr Ile Tyr Asp
   420 425 430
   Gly Phe Asn Leu Arg Asn Thr Asn Leu Ala Ala Asn Phe Asn Gly Gln
           435                 440                 445
   Asn Thr Glu Ile Asn Asn Met Asn Phe Thr Lys Leu Lys Asn Phe Thr
       450                 455                 460
   Gly Leu Phe Glu Phe Tyr Lys Leu Leu Cys Val Asp Gly Ile Ile Thr
   465                 470                 475                 480
   Ser Lys Thr Lys Ser Leu Ile Glu Gly Arg Asn Lys Ala Leu Asn Asp
                   485                 490                 495
   Leu Cys Ile Lys Val Asn Asn Trp Asp Leu Phe Phe Ser Pro Ser Glu
               500                 505                 510
   Asp Asn Phe Thr Asn Asp Leu Asn Lys Gly Glu Glu Ile Thr Ser Asp
           515                 520                 525
   Thr Asn Ile Glu Ala Ala Glu Glu Asn Ile Ser Leu Asp Leu Ile Gln
       530                 535                 540
   Gln Tyr Tyr Leu Thr Phe Asn Phe Asp Asn Glu Pro Glu Asn Ile Ser
   545                 550                 555                 560
   Ile Glu Asn Leu Ser Ser Asp Ile Ile Gly Gln Leu Glu Leu Met Pro
                   565                 570                 575
   Asn Ile Glu Arg Phe Pro Asn Gly Lys Lys Tyr Glu Leu Asp Lys Tyr
               580                 585                 590
   Thr Met Phe His Tyr Leu Arg Ala Gln Glu Phe Glu His Gly Lys Ser
           595                 600                 605
   Arg Ile Ala Leu Thr Asn Ser Val Asn Glu Ala Leu Leu Asn Pro Ser
       610                 615                 620
   Arg Val Tyr Thr Phe Phe Ser Ser Asp Tyr Val Lys Lys Val Asn Lys
   625                 630                 635                 640
   Ala Thr Glu Ala Ala Met Phe Leu Gly Trp Val Glu Gln Leu Val Tyr
                   645                 650                 655
   Asp Phe Thr Asp Glu Thr Ser Glu Val Ser Thr Thr Asp Lys Ile Ala
               660                 665                 670
   Asp Ile Thr Ile Ile Ile Pro Tyr Ile Gly Pro Ala Leu Asn Ile Gly
           675                 680                 685
   Asn Met Leu Tyr Lys Asp Asp Phe Val Gly Ala Leu Ile Phe Ser Gly
       690                 695                 700
   Ala Val Ile Leu Leu Glu Phe Ile Pro Glu Ile Ala Ile Pro Val Leu
   705                 710                 715                 720
   Gly Thr Phe Ala Leu Val Ser Tyr Ile Ala Asn Lys Val Leu Thr Val
                   725                 730                 735
   Gln Thr Ile Asp Asn Ala Leu Ser Lys Arg Asn Glu Lys Trp Asp Glu
               740                 745                 750
   Val Tyr Lys Tyr Ile Val Thr Asn Trp Leu Ala Lys Val Asn Thr Gln
           755                 760                 765
   Ile Asp Leu Ile Arg Lys Lys Met Lys Glu Ala Leu Glu Asn Gln Ala
       770                 775                 780
   Glu Ala Thr Lys Ala Ile Ile Asn Tyr Gln Tyr Asn Gln Tyr Thr Glu
   785                 790                 795                 800
   Glu Glu Lys Asn Asn Ile Asn Phe Asn Ile Asp Asp Leu Ser Ser Lys
                   805                 810                 815
   Leu Asn Glu Ser Ile Asn Lys Ala Met Ile Asn Ile Asn Lys Phe Leu
               820                 825                 830
   Asn Gln Cys Ser Val Ser Tyr Leu Met Asn Ser Met Ile Pro Tyr Gly
           835                 840                 845
   Val Lys Arg Leu Glu Asp Phe Asp Ala Ser Leu Lys Asp Ala Leu Leu
       850                 855                 860
   Lys Tyr Ile Tyr Asp Asn Arg Gly Thr Leu Ile Gly Gln Val Asp Arg
   865                 870                 875                 880
   Leu Lys Asp Lys Val Asn Asn Thr Leu Ser Thr Asp Ile Pro Phe Gln
                   885                 890                 895
   Leu Ser Lys Tyr Val Asp Asn Gln Arg Leu Leu Ser Thr Leu Asp
               900                 905                 910
<210> 43
   <211> 2715
   <212> DNA
   <213> Artificial Sequence
<220>
   <223> Synthetic
<400> 43
   ggatccgaat tcatgccgat caccatcaac aacttcaact acagcgatcc ggtggataac 60
   aaaaacatcc tgtacctgga tacccatctg aataccctgg cgaacgaacc ggaaaaagcg 120
   tttcgtatca ccggcaacat ttgggttatt ccggatcgtt ttagccgtaa cagcaacccg 180
   aatctgaata aaccgccgcg tgttaccagc ccgaaaagcg gttattacga tccgaactat 240
   ctgagcaccg atagcgataa agataccttc ctgaaagaaa tcatcaaact gttcaaacgc 300
   atcaacagcc gtgaaattgg cgaagaactg atctatcgcc tgagcaccga tattccgttt 360
   ccgggcaaca acaacacccc gatcaacacc tttgatttcg atgtggattt caacagcgtt 420
   gatgttaaaa cccgccaggg taacaattgg gtgaaaaccg gcagcattaa cccgagcgtg 480
   attattaccg gtccgcgcga aaacattatt gatccggaaa ccagcacctt taaactgacc 540
   aacaacacct ttgcggcgca ggaaggtttt ggcgcgctga gcattattag cattagcccg 600
   cgctttatgc tgacctatag caacgcgacc aacgatgttg gtgaaggccg tttcagcaaa 660
   agcgaatttt gcatggaccc gatcctgatc ctgatgcatg aactgaacca tgcgatgcat 720
   aacctgtatg gcatcgcgat tccgaacgat cagaccatta gcagcgtgac cagcaacatc 780
   ttttacagcc agtacaacgt gaaactggaa tatgcggaaa tctatgcgtt tggcggtccg 840
   accattgatc tgattccgaa aagcgcgcgc aaatacttcg aagaaaaagc gctggattac 900
   tatcgcagca ttgcgaaacg tctgaacagc attaccaccg cgaatccgag cagcttcaac 960
   aaatatatcg gcgaatataa acagaaactg atccgcaaat atcgctttgt ggtggaaagc 1020
   agcggcgaag ttaccgttaa ccgcaataaa ttcgtggaac tgtacaacga actgacccag 1080
   atcttcaccg aatttaacta tgcgaaaatc tataacgtgc agaaccgtaa aatctacctg 1140
   agcaacgtgt ataccccggt gaccgcgaat attctggatg ataacgtgta cgatatccag 1200
   aacggcttta acatcccgaa aagcaacctg aacgttctgt ttatgggcca gaacctgagc 1260
   cgtaatccgg cgctgcgtaa agtgaacccg gaaaacatgc tgtacctgtt caccaaattt 1320
   tgcgtcgacg cgatagatgg tagatttggc ggtttcacgg gcgcacgcaa atcagcgcgt 1380
   aaattagcta accaggcgct agcgggcggt ggcggtagcg gcggtggcgg tagcggcggt 1440
   ggcggtagcg cactagtgct gcagtgtcgt gaactgctgg tgaaaaacac cgatctgccg 1500
   tttattggcg atatcagcga tgtgaaaacc gatatcttcc tgcgcaaaga tatcaacgaa 1560
   gaaaccgaag tgatctacta cccggataac gtgagcgttg atcaggtgat cctgagcaaa 1620
   aacaccagcg aacatggtca gctggatctg ctgtatccga gcattgatag cgaaagcgaa 1680
   attctgccgg gcgaaaacca ggtgttttac gataaccgta cccagaacgt ggattacctg 1740
   aacagctatt actacctgga aagccagaaa ctgagcgata acgtggaaga ttttaccttt 1800
   acccgcagca ttgaagaagc gctggataac agcgcgaaag tttacaccta ttttccgacc 1860
   ctggcgaaca aagttaatgc gggtgttcag ggcggtctgt ttctgatgtg ggcgaacgat 1920
   gtggtggaag atttcaccac caacatcctg cgtaaagata ccctggataa aatcagcgat 1980
   gttagcgcga ttattccgta tattggtccg gcgctgaaca ttagcaatag cgtgcgtcgt 2040
   ggcaatttta ccgaagcgtt tgcggttacc ggtgtgacca ttctgctgga agcgtttccg 2100
   gaatttacca ttccggcgct gggtgcgttt gtgatctata gcaaagtgca ggaacgcaac 2160
   gaaatcatca aaaccatcga taactgcctg gaacagcgta ttaaacgctg gaaagatagc 2220
   tatgaatgga tgatgggcac ctggctgagc cgtattatca cccagttcaa caacatcagc 2280
   taccagatgt acgatagcct gaactatcag gcgggtgcga ttaaagcgaa aatcgatctg 2340
   gaatacaaaa aatacagcgg cagcgataaa gaaaacatca aaagccaggt tgaaaacctg 2400
   aaaaacagcc tggatgtgaa aattagcgaa gcgatgaata acatcaacaa attcatccgc 2460
   gaatgcagcg tgacctacct gttcaaaaac atgctgccga aagtgatcga tgaactgaac 2520
   gaatttgatc gcaacaccaa agcgaaactg atcaacctga tcgatagcca caacattatt 2580
   ctggtgggcg aagtggataa actgaaagcg aaagttaaca acagcttcca gaacaccatc 2640
   ccgtttaaca tcttcagcta taccaacaac agcctgctga aagatatcat caacgaatac 2700
   ttcaatctag actag 2715
<210> 44
   <211> 904
   <212> PRT
   <213> Artificial Sequence
<220>
   <223> Synthetic
<400> 44
   Gly Ser Glu Phe Met Pro Ile Thr Ile Asn Asn Phe Asn Tyr Ser Asp
   1 5 10 15
   Pro Val Asp Asn Lys Asn Ile Leu Tyr Leu Asp Thr His Leu Asn Thr
   20 25 30
   Leu Ala Asn Glu Pro Glu Lys Ala Phe Arg Ile Thr Gly Asn Ile Trp
   35 40 45
   Val Ile Pro Asp Arg Phe Ser Arg Asn Ser Asn Pro Asn Leu Asn Lys
       50                  55                  60
   Pro Pro Arg Val Thr Ser Pro Lys Ser Gly Tyr Tyr Asp Pro Asn Tyr
   65                  70                  75                  80
   Leu Ser Thr Asp Ser Asp Lys Asp Thr Phe Leu Lys Glu Ile Ile Lys
                   85                  90                  95
   Leu Phe Lys Arg Ile Asn Ser Arg Glu Ile Gly Glu Glu Leu Ile Tyr
               100                 105                 110
   Arg Leu Ser Thr Asp Ile Pro Phe Pro Gly Asn Asn Asn Thr Pro Ile
           115                 120                 125
   Asn Thr Phe Asp Phe Asp Val Asp Phe Asn Ser Val Asp Val Lys Thr
       130                 135                 140
   Arg Gln Gly Asn Asn Trp Val Lys Thr Gly Ser Ile Asn Pro Ser Val
   145                 150                 155                 160
   Ile Ile Thr Gly Pro Arg Glu Asn Ile Ile Asp Pro Glu Thr Ser Thr
                   165                 170                 175
   Phe Lys Leu Thr Asn Asn Thr Phe Ala Ala Gln Glu Gly Phe Gly Ala
               180                 185                 190
   Leu Ser Ile Ile Ser Ile Ser Pro Arg Phe Met Leu Thr Tyr Ser Asn
           195                 200                 205
   Ala Thr Asn Asp Val Gly Glu Gly Arg Phe Ser Lys Ser Glu Phe Cys
       210                 215                 220
   Met Asp Pro Ile Leu Ile Leu Met His Glu Leu Asn His Ala Met His
   225                 230                 235                 240
   Asn Leu Tyr Gly Ile Ala Ile Pro Asn Asp Gln Thr Ile Ser Ser Val
                   245                 250                 255
   Thr Ser Asn Ile Phe Tyr Ser Gln Tyr Asn Val Lys Leu Glu Tyr Ala
               260                 265                 270
   Glu Ile Tyr Ala Phe Gly Gly Pro Thr Ile Asp Leu Ile Pro Lys Ser
           275                 280                 285
   Ala Arg Lys Tyr Phe Glu Glu Lys Ala Leu Asp Tyr Tyr Arg Ser Ile
   290 295 300
   Ala Lys Arg Leu Asn Ser Ile Thr Thr Ala Asn Pro Ser Ser Phe Asn
   305 310 315 320
   Lys Tyr Ile Gly Glu Tyr Lys Gln Lys Leu Ile Arg Lys Tyr Arg Phe
   325 330 335
   Val Val Glu Ser Ser Gly Glu Val Thr Val Asn Arg Asn Lys Phe Val
   340 345 350
   Glu Leu Tyr Asn Glu Leu Thr Gln Ile Phe Thr Glu Phe Asn Tyr Ala
   355 360 365
   Lys Ile Tyr Asn Val Gln Asn Arg Lys Ile Tyr Leu Ser Asn Val Tyr
   370 375 380
   Thr Pro Val Thr Ala Asn Ile Leu Asp Asp Asn Val Tyr Asp Ile Gln
   385 390 395 400
   Asn Gly Phe Asn Ile Pro Lys Ser Asn Leu Asn Val Leu Phe Met Gly
   405 410 415
   Gln Asn Leu Ser Arg Asn Pro Ala Leu Arg Lys Val Asn Pro Glu Asn
   420 425 430
   Met Leu Tyr Leu Phe Thr Lys Phe Cys Val Asp Ala Ile Asp Gly Arg
   435 440 445
   Phe Gly Gly Phe Thr Gly Ala Arg Lys Ser Ala Arg Lys Leu Ala Asn
   450 455 460
   Gln Ala Leu Ala Gly Gly Gly Gly Ser Gly Gly Gly Gly Ser Gly Gly
   465 470 475 480
   Gly Gly Ser Ala Leu Val Leu Gln Cys Arg Glu Leu Leu Val Lys Asn
   485 490 495
   Thr Asp Leu Pro Phe Ile Gly Asp Ile Ser Asp Val Lys Thr Asp Ile
   500 505 510
   Phe Leu Arg Lys Asp Ile Asn Glu Glu Thr Glu Val Ile Tyr Tyr Pro
   515 520 525
   Asp Asn Val Ser Val Asp Gln Val Ile Leu Ser Lys Asn Thr Ser Glu
   530 535 540
   His Gly Gln Leu Asp Leu Leu Tyr Pro Ser Ile Asp Ser Glu Ser Glu
   545 550 555 560
   Ile Leu Pro Gly Glu Asn Gln Val Phe Tyr Asp Asn Arg Thr Gln Asn
   565 570 575
   Val Asp Tyr Leu Asn Ser Tyr Tyr Tyr Leu Glu Ser Gln Lys Leu Ser
   580 585 590
   Asp Asn Val Glu Asp Phe Thr Phe Thr Arg Ser Ile Glu Glu Ala Leu
   595 600 605
   Asp Asn Ser Ala Lys Val Tyr Thr Tyr Phe Pro Thr Leu Ala Asn Lys
   610 615 620
   Val Asn Ala Gly Val Gln Gly Gly Leu Phe Leu Met Trp Ala Asn Asp
   625 630 635 640
   Val Val Glu Asp Phe Thr Thr Asn Ile Leu Arg Lys Asp Thr Leu Asp
   645 650 655
   Lys Ile Ser Asp Val Ser Ala Ile Ile Pro Tyr Ile Gly Pro Ala Leu
   660 665 670
   Asn Ile Ser Asn Ser Val Arg Arg Gly Asn Phe Thr Glu Ala Phe Ala
   675 680 685
   Val Thr Gly Val Thr Ile Leu Leu Glu Ala Phe Pro Glu Phe Thr Ile
   690 695 700
   Pro Ala Leu Gly Ala Phe Val Ile Tyr Ser Lys Val Gln Glu Arg Asn
   705 710 715 720
   Glu Ile Ile Lys Thr Ile Asp Asn Cys Leu Glu Gln Arg Ile Lys Arg
   725 730 735
   Trp Lys Asp Ser Tyr Glu Trp Met Met Gly Thr Trp Leu Ser Arg Ile
   740 745 750
   Ile Thr Gln Phe Asn Asn Ile Ser Tyr Gln Met Tyr Asp Ser Leu Asn
   755 760 765
   Tyr Gln Ala Gly Ala Ile Lys Ala Lys Ile Asp Leu Glu Tyr Lys Lys
   770 775 780
   Tyr Ser Gly Ser Asp Lys Glu Asn Ile Lys Ser Gln Val Glu Asn Leu
   785 790 795 800
   Lys Asn Ser Leu Asp Val Lys Ile Ser Glu Ala Met Asn Asn Ile Asn
   805 810 815
   Lys Phe Ile Arg Glu Cys Ser Val Thr Tyr Leu Phe Lys Asn Met Leu
   820 825 830
   Pro Lys Val Ile Asp Glu Leu Asn Glu Phe Asp Arg Asn Thr Lys Ala
   835 840 845
   Lys Leu Ile Asn Leu Ile Asp Ser His Asn Ile Ile Leu Val Gly Glu
   850 855 860
   Val Asp Lys Leu Lys Ala Lys Val Asn Asn Ser Phe Gln Asn Thr Ile
   865 870 875 880
   Pro Phe Asn Ile Phe Ser Tyr Thr Asn Asn Ser Leu Leu Lys Asp Ile
   885 890 895
   Ile Asn Glu Tyr Phe Asn Leu Asp
   900
<210> 45
   <211> 2742
   <212> DNA
   <213> Artificial Sequence
<220>
   <223> Synthetic
<400> 45
   ggatccgaat tcatgccgat caccatcaac aacttcaact acagcgatcc ggtggataac 60
   aaaaacatcc tgtacctgga tacccatctg aataccctgg cgaacgaacc ggaaaaagcg 120
   tttcgtatca ccggcaacat ttgggttatt ccggatcgtt ttagccgtaa cagcaacccg 180
   aatctgaata aaccgccgcg tgttaccagc ccgaaaagcg gttattacga tccgaactat 240
   ctgagcaccg atagcgataa agataccttc ctgaaagaaa tcatcaaact gttcaaacgc 300
   atcaacagcc gtgaaattgg cgaagaactg atctatcgcc tgagcaccga tattccgttt 360
   ccgggcaaca acaacacccc gatcaacacc tttgatttcg atgtggattt caacagcgtt 420
   gatgttaaaa cccgccaggg taacaattgg gtgaaaaccg gcagcattaa cccgagcgtg 480
   attattaccg gtccgcgcga aaacattatt gatccggaaa ccagcacctt taaactgacc 540
   aacaacacct ttgcggcgca ggaaggtttt ggcgcgctga gcattattag cattagcccg 600
   cgctttatgc tgacctatag caacgcgacc aacgatgttg gtgaaggccg tttcagcaaa 660
   agcgaatttt gcatggaccc gatcctgatc ctgatgcatg aactgaacca tgcgatgcat 720
   aacctgtatg gcatcgcgat tccgaacgat cagaccatta gcagcgtgac cagcaacatc 780
   ttttacagcc agtacaacgt gaaactggaa tatgcggaaa tctatgcgtt tggcggtccg 840
   accattgatc tgattccgaa aagcgcgcgc aaatacttcg aagaaaaagc gctggattac 900
   tatcgcagca ttgcgaaacg tctgaacagc attaccaccg cgaatccgag cagcttcaac 960
   aaatatatcg gcgaatataa acagaaactg atccgcaaat atcgctttgt ggtggaaagc 1020
   agcggcgaag ttaccgttaa ccgcaataaa ttcgtggaac tgtacaacga actgacccag 1080
   atcttcaccg aatttaacta tgcgaaaatc tataacgtgc agaaccgtaa aatctacctg 1140
   agcaacgtgt ataccccggt gaccgcgaat attctggatg ataacgtgta cgatatccag 1200
   aacggcttta acatcccgaa aagcaacctg aacgttctgt ttatgggcca gaacctgagc 1260
   cgtaatccgg cgctgcgtaa agtgaacccg gaaaacatgc tgtacctgtt caccaaattt 1320
   tgcgtcgacg gcatcattac ctccaaaact aaatctctga tagaaggtag atttggcggt 1380
   ttcacgggcg cacgcaaatc agcgcgtaaa ttagctaacc aggcgctagc gggcggtggc 1440
   ggtagcggcg gtggcggtag cggcggtggc ggtagcgcac tagtgctgca gtgtcgtgaa 1500
   ctgctggtga aaaacaccga tctgccgttt attggcgata tcagcgatgt gaaaaccgat 1560
   atcttcctgc gcaaagatat caacgaagaa accgaagtga tctactaccc ggataacgtg 1620
   agcgttgatc aggtgatcct gagcaaaaac accagcgaac atggtcagct ggatctgctg 1680
   tatccgagca ttgatagcga aagcgaaatt ctgccgggcg aaaaccaggt gttttacgat 1740
   aaccgtaccc agaacgtgga ttacctgaac agctattact acctggaaag ccagaaactg 1800
   agcgataacg tggaagattt tacctttacc cgcagcattg aagaagcgct ggataacagc 1860
   gcgaaagttt acacctattt tccgaccctg gcgaacaaag ttaatgcggg tgttcagggc 1920
   ggtctgtttc tgatgtgggc gaacgatgtg gtggaagatt tcaccaccaa catcctgcgt 1980
   aaagataccc tggataaaat cagcgatgtt agcgcgatta ttccgtatat tggtccggcg 2040
   ctgaacatta gcaatagcgt gcgtcgtggc aattttaccg aagcgtttgc ggttaccggt 2100
   gtgaccattc tgctggaagc gtttccggaa tttaccattc cggcgctggg tgcgtttgtg 2160
   atctatagca aagtgcagga acgcaacgaa atcatcaaaa ccatcgataa ctgcctggaa 2220
   cagcgtatta aacgctggaa agatagctat gaatggatga tgggcacctg gctgagccgt 2280
   attatcaccc agttcaacaa catcagctac cagatgtacg atagcctgaa ctatcaggcg 2340
   ggtgcgatta aagcgaaaat cgatctggaa tacaaaaaat acagcggcag cgataaagaa 2400
   aacatcaaaa gccaggttga aaacctgaaa aacagcctgg atgtgaaaat tagcgaagcg   2460
   atgaataaca tcaacaaatt catccgcgaa tgcagcgtga cctacctgtt caaaaacatg   2520
   ctgccgaaag tgatcgatga actgaacgaa tttgatcgca acaccaaagc gaaactgatc   2580
   aacctgatcg atagccacaa cattattctg gtgggcgaag tggataaact gaaagcgaaa   2640
   gttaacaaca gcttccagaa caccatcccg tttaacatct tcagctatac caacaacagc   2700
   ctgctgaaag atatcatcaa cgaatacttc aatctagact ag                      2742
<210> 46
   <211> 913
   <212> PRT
   <213> Artificial Sequence
<220>
   <223> Synthetic
<400> 46
   Gly Ser Glu Phe Met Pro Ile Thr Ile Asn Asn Phe Asn Tyr Ser Asp
   1               5                   10                  15
   Pro Val Asp Asn Lys Asn Ile Leu Tyr Leu Asp Thr His Leu Asn Thr
               20                  25                  30
   Leu Ala Asn Glu Pro Glu Lys Ala Phe Arg Ile Thr Gly Asn Ile Trp
           35                  40                  45
   Val Ile Pro Asp Arg Phe Ser Arg Asn Ser Asn Pro Asn Leu Asn Lys
       50                  55                  60
   Pro Pro Arg Val Thr Ser Pro Lys Ser Gly Tyr Tyr Asp Pro Asn Tyr
   65                  70                  75                  80
   Leu Ser Thr Asp Ser Asp Lys Asp Thr Phe Leu Lys Glu Ile Ile Lys
                   85                  90                  95
   Leu Phe Lys Arg Ile Asn Ser Arg Glu Ile Gly Glu Glu Leu Ile Tyr
               100                 105                 110
   Arg Leu Ser Thr Asp Ile Pro Phe Pro Gly Asn Asn Asn Thr Pro Ile
           115                 120                 125
   Asn Thr Phe Asp Phe Asp Val Asp Phe Asn Ser Val Asp Val Lys Thr
       130                 135                 140
   Arg Gln Gly Asn Asn Trp Val Lys Thr Gly Ser Ile Asn Pro Ser Val
   145 150 155 160
   Ile Ile Thr Gly Pro Arg Glu Asn Ile Ile Asp Pro Glu Thr Ser Thr
   165 170 175
   Phe Lys Leu Thr Asn Asn Thr Phe Ala Ala Gln Glu Gly Phe Gly Ala
   180 185 190
   Leu Ser Ile Ile Ser Ile Ser Pro Arg Phe Met Leu Thr Tyr Ser Asn
   195 200 205
   Ala Thr Asn Asp Val Gly Glu Gly Arg Phe Ser Lys Ser Glu Phe Cys
   210 215 220
   Met Asp Pro Ile Leu Ile Leu Met His Glu Leu Asn His Ala Met His
   225 230 235 240
   Asn Leu Tyr Gly Ile Ala Ile Pro Asn Asp Gln Thr Ile Ser Ser Val
   245 250 255
   Thr Ser Asn Ile Phe Tyr Ser Gln Tyr Asn Val Lys Leu Glu Tyr Ala
   260 265 270
   Glu Ile Tyr Ala Phe Gly Gly Pro Thr Ile Asp Leu Ile Pro Lys Ser
   275 280 285
   Ala Arg Lys Tyr Phe Glu Glu Lys Ala Leu Asp Tyr Tyr Arg Ser Ile
   290 295 300
   Ala Lys Arg Leu Asn Ser Ile Thr Thr Ala Asn Pro Ser Ser Phe Asn
   305 310 315 320
   Lys Tyr Ile Gly Glu Tyr Lys Gln Lys Leu Ile Arg Lys Tyr Arg Phe
   325 330 335
   Val Val Glu Ser Ser Gly Glu Val Thr Val Asn Arg Asn Lys Phe Val
   340 345 350
   Glu Leu Tyr Asn Glu Leu Thr Gln Ile Phe Thr Glu Phe Asn Tyr Ala
   355 360 365
   Lys Ile Tyr Asn Val Gln Asn Arg Lys Ile Tyr Leu Ser Asn Val Tyr
   370 375 380
   Thr Pro Val Thr Ala Asn Ile Leu Asp Asp Asn Val Tyr Asp Ile Gln
   385 390 395 400
   Asn Gly Phe Asn Ile Pro Lys Ser Asn Leu Asn Val Leu Phe Met Gly
   405 410 415
   Gln Asn Leu Ser Arg Asn Pro Ala Leu Arg Lys Val Asn Pro Glu Asn
   420 425 430
   Met Leu Tyr Leu Phe Thr Lys Phe Cys Val Asp Gly Ile Ile Thr Ser
   435 440 445
   Lys Thr Lys Ser Leu Ile Glu Gly Arg Phe Gly Gly Phe Thr Gly Ala
   450 455 460
   Arg Lys Ser Ala Arg Lys Leu Ala Asn Gln Ala Leu Ala Gly Gly Gly
   465 470 475 480
   Gly Ser Gly Gly Gly Gly Ser Gly Gly Gly Gly Ser Ala Leu Val Leu
   485 490 495
   Gln Cys Arg Glu Leu Leu Val Lys Asn Thr Asp Leu Pro Phe Ile Gly
   500 505 510
   Asp Ile Ser Asp Val Lys Thr Asp Ile Phe Leu Arg Lys Asp Ile Asn
   515 520 525
   Glu Glu Thr Glu Val Ile Tyr Tyr Pro Asp Asn Val Ser Val Asp Gln
   530 535 540
   Val Ile Leu Ser Lys Asn Thr Ser Glu His Gly Gln Leu Asp Leu Leu
   545 550 555 560
   Tyr Pro Ser Ile Asp Ser Glu Ser Glu Ile Leu Pro Gly Glu Asn Gln
   565 570 575
   Val Phe Tyr Asp Asn Arg Thr Gln Asn Val Asp Tyr Leu Asn Ser Tyr
   580 585 590
   Tyr Tyr Leu Glu Ser Gln Lys Leu Ser Asp Asn Val Glu Asp Phe Thr
   595 600 605
   Phe Thr Arg Ser Ile Glu Glu Ala Leu Asp Asn Ser Ala Lys Val Tyr
   610 615 620
   Thr Tyr Phe Pro Thr Leu Ala Asn Lys Val Asn Ala Gly Val Gln Gly
   625 630 635 640
   Gly Leu Phe Leu Met Trp Ala Asn Asp Val Val Glu Asp Phe Thr Thr
   645 650 655
   Asn Ile Leu Arg Lys Asp Thr Leu Asp Lys Ile Ser Asp Val Ser Ala
   660 665 670
   Ile Ile Pro Tyr Ile Gly Pro Ala Leu Asn Ile Ser Asn Ser Val Arg
   675 680 685
   Arg Gly Asn Phe Thr Glu Ala Phe Ala Val Thr Gly Val Thr Ile Leu
   690 695 700
   Leu Glu Ala Phe Pro Glu Phe Thr Ile Pro Ala Leu Gly Ala Phe Val
   705 710 715 720
   Ile Tyr Ser Lys Val Gln Glu Arg Asn Glu Ile Ile Lys Thr Ile Asp
   725 730 735
   Asn Cys Leu Glu Gln Arg Ile Lys Arg Trp Lys Asp Ser Tyr Glu Trp
   740 745 750
   Met Met Gly Thr Trp Leu Ser Arg Ile Ile Thr Gln Phe Asn Asn Ile
   755 760 765
   Ser Tyr Gln Met Tyr Asp Ser Leu Asn Tyr Gln Ala Gly Ala Ile Lys
   770 775 780
   Ala Lys Ile Asp Leu Glu Tyr Lys Lys Tyr Ser Gly Ser Asp Lys Glu
   785 790 795 800
   Asn Ile Lys Ser Gln Val Glu Asn Leu Lys Asn Ser Leu Asp Val Lys
   805 810 815
   Ile Ser Glu Ala Met Asn Asn Ile Asn Lys Phe Ile Arg Glu Cys Ser
   820 825 830
   Val Thr Tyr Leu Phe Lys Asn Met Leu Pro Lys Val Ile Asp Glu Leu
   835 840 845
   Asn Glu Phe Asp Arg Asn Thr Lys Ala Lys Leu Ile Asn Leu Ile Asp
   850 855 860
   Ser His Asn Ile Ile Leu Val Gly Glu Val Asp Lys Leu Lys Ala Lys
   865 870 875 880
   Val Asn Asn Ser Phe Gln Asn Thr Ile Pro Phe Asn Ile Phe Ser Tyr
                   885                 890                 895
   Thr Asn Asn Ser Leu Leu Lys Asp Ile Ile Asn Glu Tyr Phe Asn Leu
               900                 905                 910
   Asp
<210> 47
   <211> 2673
   <212> DNA
   <213> Artificial Sequence
<220>
   <223> Synthetic
<400> 47
   ggatccatgg agttcgttaa caaacagttc aactataaag acccagttaa cggtgttgac     60
   attgcttaca tcaaaatccc gaacgctggc cagatgcagc cggtaaaggc attcaaaatc    120
   cacaacaaaa tctgggttat cccggaacgt gataccttta ctaacccgga agaaggtgac     180
   ctgaacccgc caccggaagc gaaacaggtg ccggtatctt actatgactc cacctacctg    240
   tctaccgata acgaaaagga caactacctg aaaggtgtta ctaaactgtt cgagcgtatt    300
   tactccaccg acctgggccg tatgctgctg actagcatcg ttcgcggtat cccgttctgg    360
   ggcggttcta ccatcgatac cgaactgaaa gtaatcgaca ctaactgcat caacgttatt    420
   cagccggacg gttcctatcg ttccgaagaa ctgaacctgg tgatcatcgg cccgtctgct    480
   gatatcatcc agttcgagtg taagagcttt ggtcacgaag ttctgaacct cacccgtaac    540
   ggctacggtt ccactcagta catccgtttc tctccggact tcaccttcgg ttttgaagaa    600
   tccctggaag tagacacgaa cccactgctg ggcgctggta aattcgcaac tgatcctgcg    660
   gttaccctgg ctcacgaact gattcatgca ggccaccgcc tgtacggtat cgccatcaat    720
   ccgaaccgtg tcttcaaagt taacaccaac gcgtattacg agatgtccgg tctggaagtt    780
   agcttcgaag aactgcgtac ttttggcggt cacgacgcta aattcatcga ctctctgcaa    840
   gaaaacgagt tccgtctgta ctactataac aagttcaaag atatcgcatc caccctgaac    900
   aaagcgaaat ccatcgtggg taccactgct tctctccagt acatgaagaa cgtttttaaa    960
   gaaaaatacc tgctcagcga agacacctcc ggcaaattct ctgtagacaa gttgaaattc   1020
   gataaacttt acaaaatgct gactgaaatt tacaccgaag acaacttcgt taagttcttt   1080
   aaagttctga accgcaaaac ctatctgaac ttcgacaagg cagtattcaa aatcaacatc   1140
   gtgccgaaag ttaactacac tatctacgat ggtttcaacc tgcgtaacac caacctggct   1200
   gctaatttta acggccagaa cacggaaatc aacaacatga acttcacaaa actgaaaaac   1260
   ttcactggtc tgttcgagtt ttacaagctg ctgtgcgtcg acggcatcat tacctccaaa   1320
   actaaatctc tgatagaagg tagatacggt ggtttcatgg cgctagcggg cggtggcggt   1380
   agcggcggtg gcggtagcgg cggtggcggt agcgcactag tgctgcagtg tatcaaggtt   1440
   aacaactggg atttattctt cagcccgagt gaagacaact tcaccaacga cctgaacaaa   1500
   ggtgaagaaa tcacctcaga tactaacatc gaagcagccg aagaaaacat ctcgctggac   1560
   ctgatccagc agtactacct gacctttaat ttcgacaacg agccggaaaa catttctatc   1620
   gaaaacctga gctctgatat catcggccag ctggaactga tgccgaacat cgaacgtttc   1680
   ccaaacggta aaaagtacga gctggacaaa tataccatgt tccactacct gcgcgcgcag   1740
   gaatttgaac acggcaaatc ccgtatcgca ctgactaact ccgttaacga agctctgctc   1800
   aacccgtccc gtgtatacac cttcttctct agcgactacg tgaaaaaggt caacaaagcg   1860
   actgaagctg caatgttctt gggttgggtt gaacagcttg tttatgattt taccgacgag   1920
   acgtccgaag tatctactac cgacaaaatt gcggatatca ctatcatcat cccgtacatc   1980
   ggtccggctc tgaacattgg caacatgctg tacaaagacg acttcgttgg cgcactgatc   2040
   ttctccggtg cggtgatcct gctggagttc atcccggaaa tcgccatccc ggtactgggc   2100
   acctttgctc tggtttctta cattgcaaac aaggttctga ctgtacaaac catcgacaac   2160
   gcgctgagca aacgtaacga aaaatgggat gaagtttaca aatatatcgt gaccaactgg   2220
   ctggctaagg ttaatactca gatcgacctc atccgcaaaa aaatgaaaga agcactggaa   2280
   aaccaggcgg aagctaccaa ggcaatcatt aactaccagt acaaccagta caccgaggaa   2340
   gaaaaaaaca acatcaactt caacatcgac gatctgtcct ctaaactgaa cgaatccatc   2400
   aacaaagcta tgatcaacat caacaagttc ctgaaccagt gctctgtaag ctatctgatg   2460
   aactccatga tcccgtacgg tgttaaacgt ctggaggact tcgatgcgtc tctgaaagac   2520
   gccctgctga aatacattta cgacaaccgt ggcactctga tcggtcaggt tgatcgtctg   2580
   aaggacaaag tgaacaatac cttatcgacc gacatccctt ttcagctcag taaatatgtc   2640
   gataaccaac gccttttgtc cactctagac tag                                2673
<210> 48
   <211> 890
   <212> PRT
   <213> Artificial Sequence
<220>
   <223> Synthetic
<400> 48
   Gly Ser Met Glu Phe Val Asn Lys Gln Phe Asn Tyr Lys Asp Pro Val
   1               5                   10                  15
   Asn Gly Val Asp Ile Ala Tyr Ile Lys Ile Pro Asn Ala Gly Gln Met
               20                  25                  30
   Gln Pro Val Lys Ala Phe Lys Ile His Asn Lys Ile Trp Val Ile Pro
           35                  40                  45
   Glu Arg Asp Thr Phe Thr Asn Pro Glu Glu Gly Asp Leu Asn Pro Pro
       50                  55                  60
   Pro Glu Ala Lys Gln Val Pro Val Ser Tyr Tyr Asp Ser Thr Tyr Leu
   65                  70                  75                  80
   Ser Thr Asp Asn Glu Lys Asp Asn Tyr Leu Lys Gly Val Thr Lys Leu
                   85                  90                  95
   Phe Glu Arg Ile Tyr Ser Thr Asp Leu Gly Arg Met Leu Leu Thr Ser
               100                 105                 110
   Ile Val Arg Gly Ile Pro Phe Trp Gly Gly Ser Thr Ile Asp Thr Glu
           115                 120                 125
   Leu Lys Val Ile Asp Thr Asn Cys Ile Asn Val Ile Gln Pro Asp Gly
       130                 135                 140
   Ser Tyr Arg Ser Glu Glu Leu Asn Leu Val Ile Ile Gly Pro Ser Ala
   145                 150                 155                 160
   Asp Ile Ile Gln Phe Glu Cys Lys Ser Phe Gly His Glu Val Leu Asn
                   165                 170                 175
   Leu Thr Arg Asn Gly Tyr Gly Ser Thr Gln Tyr Ile Arg Phe Ser Pro
               180                 185                 190
   Asp Phe Thr Phe Gly Phe Glu Glu Ser Leu Glu Val Asp Thr Asn Pro
           195                 200                 205
   Leu Leu Gly Ala Gly Lys Phe Ala Thr Asp Pro Ala Val Thr Leu Ala
       210                 215                 220
   His Glu Leu Ile His Ala Gly His Arg Leu Tyr Gly Ile Ala Ile Asn
   225                 230                 235                 240
   Pro Asn Arg Val Phe Lys Val Asn Thr Asn Ala Tyr Tyr Glu Met Ser
   245 250 255
   Gly Leu Glu Val Ser Phe Glu Glu Leu Arg Thr Phe Gly Gly His Asp
   260 265 270
   Ala Lys Phe Ile Asp Ser Leu Gln Glu Asn Glu Phe Arg Leu Tyr Tyr
   275 280 285
   Tyr Asn Lys Phe Lys Asp Ile Ala Ser Thr Leu Asn Lys Ala Lys Ser
   290 295 300
   Ile Val Gly Thr Thr Ala Ser Leu Gln Tyr Met Lys Asn Val Phe Lys
   305 310 315 320
   Glu Lys Tyr Leu Leu Ser Glu Asp Thr Ser Gly Lys Phe Ser Val Asp
   325 330 335
   Lys Leu Lys Phe Asp Lys Leu Tyr Lys Met Leu Thr Glu Ile Tyr Thr
   340 345 350
   Glu Asp Asn Phe Val Lys Phe Phe Lys Val Leu Asn Arg Lys Thr Tyr
   355 360 365
   Leu Asn Phe Asp Lys Ala Val Phe Lys Ile Asn Ile Val Pro Lys Val
   370 375 380
   Asn Tyr Thr Ile Tyr Asp Gly Phe Asn Leu Arg Asn Thr Asn Leu Ala
   385 390 395 400
   Ala Asn Phe Asn Gly Gln Asn Thr Glu Ile Asn Asn Met Asn Phe Thr
   405 410 415
   Lys Leu Lys Asn Phe Thr Gly Leu Phe Glu Phe Tyr Lys Leu Leu Cys
   420 425 430
   Val Asp Gly Ile Ile Thr Ser Lys Thr Lys Ser Leu Ile Glu Gly Arg
   435 440 445
   Tyr Gly Gly Phe Met Ala Leu Ala Gly Gly Gly Gly Ser Gly Gly Gly
   450 455 460
   Gly Ser Gly Gly Gly Gly Ser Ala Leu Val Leu Gln Cys Ile Lys Val
   465 470 475 480
   Asn Asn Trp Asp Leu Phe Phe Ser Pro Ser Glu Asp Asn Phe Thr Asn
   485 490 495
   Asp Leu Asn Lys Gly Glu Glu Ile Thr Ser Asp Thr Asn Ile Glu Ala
   500 505 510
   Ala Glu Glu Asn Ile Ser Leu Asp Leu Ile Gln Gln Tyr Tyr Leu Thr
   515 520 525
   Phe Asn Phe Asp Asn Glu Pro Glu Asn Ile Ser Ile Glu Asn Leu Ser
   530 535 540
   Ser Asp Ile Ile Gly Gln Leu Glu Leu Met Pro Asn Ile Glu Arg Phe
   545 550 555 560
   Pro Asn Gly Lys Lys Tyr Glu Leu Asp Lys Tyr Thr Met Phe His Tyr
   565 570 575
   Leu Arg Ala Gln Glu Phe Glu His Gly Lys Ser Arg Ile Ala Leu Thr
   580 585 590
   Asn Ser Val Asn Glu Ala Leu Leu Asn Pro Ser Arg Val Tyr Thr Phe
   595 600 605
   Phe Ser Ser Asp Tyr Val Lys Lys Val Asn Lys Ala Thr Glu Ala Ala
   610 615 620
   Met Phe Leu Gly Trp Val Glu Gln Leu Val Tyr Asp Phe Thr Asp Glu
   625 630 635 640
   Thr Ser Glu Val Ser Thr Thr Asp Lys Ile Ala Asp Ile Thr Ile Ile
   645 650 655
   Ile Pro Tyr Ile Gly Pro Ala Leu Asn Ile Gly Asn Met Leu Tyr Lys
   660 665 670
   Asp Asp Phe Val Gly Ala Leu Ile Phe Ser Gly Ala Val Ile Leu Leu
   675 680 685
   Glu Phe Ile Pro Glu Ile Ala Ile Pro Val Leu Gly Thr Phe Ala Leu
   690 695 700
   Val Ser Tyr Ile Ala Asn Lys Val Leu Thr Val Gln Thr Ile Asp Asn
   705 710 715 720
   Ala Leu Ser Lys Arg Asn Glu Lys Trp Asp Glu Val Tyr Lys Tyr Ile
   725 730 735
   Val Thr Asn Trp Leu Ala Lys Val Asn Thr Gln Ile Asp Leu Ile Arg
   740 745 750
   Lys Lys Met Lys Glu Ala Leu Glu Asn Gln Ala Glu Ala Thr Lys Ala
   755 760 765
   Ile Ile Asn Tyr Gln Tyr Asn Gln Tyr Thr Glu Glu Glu Lys Asn Asn
   770 775 780
   Ile Asn Phe Asn Ile Asp Asp Leu Ser Ser Lys Leu Asn Glu Ser Ile
   785 790 795 800
   Asn Lys Ala Met Ile Asn Ile Asn Lys Phe Leu Asn Gln Cys Ser Val
   805 810 815
   Ser Tyr Leu Met Asn Ser Met Ile Pro Tyr Gly Val Lys Arg Leu Glu
   820 825 830
   Asp Phe Asp Ala Ser Leu Lys Asp Ala Leu Leu Lys Tyr Ile Tyr Asp
   835 840 845
   Asn Arg Gly Thr Leu Ile Gly Gln Val Asp Arg Leu Lys Asp Lys Val
   850 855 860
   Asn Asn Thr Leu Ser Thr Asp Ile Pro Phe Gln Leu Ser Lys Tyr Val
   865 870 875 880
   Asp Asn Gln Arg Leu Leu Ser Thr Leu Asp
   885 890
<210> 49
   <211> 2751
   <212> DNA
   <213> Artificial Sequence
<220>
   <223> Synthetic
<400> 49
   ggatccatgg agttcgttaa caaacagttc aactataaag acccagttaa cggtgttgac     60
   attgcttaca tcaaaatccc gaacgctggc cagatgcagc cggtaaaggc attcaaaatc    120
   cacaacaaaa tctgggttat cccggaacgt gataccttta ctaacccgga agaaggtgac    180
   ctgaacccgc caccggaagc gaaacaggtg ccggtatctt actatgactc cacctacctg    240
   tctaccgata acgaaaagga caactacctg aaaggtgtta ctaaactgtt cgagcgtatt    300
   tactccaccg acctgggccg tatgctgctg actagcatcg ttcgcggtat cccgttctgg    360
   ggcggttcta ccatcgatac cgaactgaaa gtaatcgaca ctaactgcat caacgttatt    420
   cagccggacg gttcctatcg ttccgaagaa ctgaacctgg tgatcatcgg cccgtctgct    480
   gatatcatcc agttcgagtg taagagcttt ggtcacgaag ttctgaacct cacccgtaac    540
   ggctacggtt ccactcagta catccgtttc tctccggact tcaccttcgg ttttgaagaa    600
   tccctggaag tagacacgaa cccactgctg ggcgctggta aattcgcaac tgatcctgcg    660
   gttaccctgg ctcacgaact gattcatgca ggccaccgcc tgtacggtat cgccatcaat    720
   ccgaaccgtg tcttcaaagt taacaccaac gcgtattacg agatgtccgg tctggaagtt    780
   agcttcgaag aactgcgtac ttttggcggt cacgacgcta aattcatcga ctctctgcaa    840
   gaaaacgagt tccgtctgta ctactataac aagttcaaag atatcgcatc caccctgaac    900
   aaagcgaaat ccatcgtggg taccactgct tctctccagt acatgaagaa cgtttttaaa    960
   gaaaaatacc tgctcagcga agacacctcc ggcaaattct ctgtagacaa gttgaaattc   1020
   gataaacttt acaaaatgct gactgaaatt tacaccgaag acaacttcgt taagttcttt   1080
   aaagttctga accgcaaaac ctatctgaac ttcgacaagg cagtattcaa aatcaacatc   1140
   gtgccgaaag ttaactacac tatctacgat ggtttcaacc tgcgtaacac caacctggct   1200
   gctaatttta acggccagaa cacggaaatc aacaacatga acttcacaaa actgaaaaac   1260
   ttcactggtc tgttcgagtt ttacaagctg ctgtgcgtcg acggcatcat tacctccaaa   1320
   actaaatctc tgatcgaagg tcgttacggt ggtttcatga cctctgaaaa atctcagacc   1380
   ccgctggtta ccctgttcaa aaacgctatc atcaaaaacg cttacaaaaa aggtgaagcg   1440
   ctagcgggtg gtggtggttc tggtggtggt ggttctggtg gtggtggttc tgcactagtg   1500
   ctgcagtgta tcaaggttaa caactgggat ttattcttca gcccgagtga agacaacttc   1560
   accaacgacc tgaacaaagg tgaagaaatc acctcagata ctaacatcga agcagccgaa   1620
   gaaaacatct cgctggacct gatccagcag tactacctga cctttaattt cgacaacgag   1680
   ccggaaaaca tttctatcga aaacctgagc tctgatatca tcggccagct ggaactgatg   1740
   ccgaacatcg aacgtttccc aaacggtaaa aagtacgagc tggacaaata taccatgttc   1800
   cactacctgc gcgcgcagga atttgaacac ggcaaatccc gtatcgcact gactaactcc   1860
   gttaacgaag ctctgctcaa cccgtcccgt gtatacacct tcttctctag cgactacgtg   1920
   aaaaaggtca acaaagcgac tgaagctgca atgttcttgg gttgggttga acagcttgtt   1980
   tatgatttta ccgacgagac gtccgaagta tctactaccg acaaaattgc ggatatcact   2040
   atcatcatcc cgtacatcgg tccggctctg aacattggca acatgctgta caaagacgac   2100
   ttcgttggcg cactgatctt ctccggtgcg gtgatcctgc tggagttcat cccggaaatc   2160
   gccatcccgg tactgggcac ctttgctctg gtttcttaca ttgcaaacaa ggttctgact 2220
   gtacaaacca tcgacaacgc gctgagcaaa cgtaacgaaa aatgggatga agtttacaaa 2280
   tatatcgtga ccaactggct ggctaaggtt aatactcaga tcgacctcat ccgcaaaaaa 2340
   atgaaagaag cactggaaaa ccaggcggaa gctaccaagg caatcattaa ctaccagtac 2400
   aaccagtaca ccgaggaaga aaaaaacaac atcaacttca acatcgacga tctgtcctct 2460
   aaactgaacg aatccatcaa caaagctatg atcaacatca acaagttcct gaaccagtgc 2520
   tctgtaagct atctgatgaa ctccatgatc ccgtacggtg ttaaacgtct ggaggacttc 2580
   gatgcgtctc tgaaagacgc cctgctgaaa tacatttacg acaaccgtgg cactctgatc 2640
   ggtcaggttg atcgtctgaa ggacaaagtg aacaatacct tatcgaccga catccctttt 2700
   cagctcagta aatatgtcga taaccaacgc cttttgtcca ctctagacta g 2751
<210> 50
   <211> 916
   <212> PRT
   <213> Artificial Sequence
<220>
   <223> Synthetic
<400> 50
   Gly Ser Met Glu Phe Val Asn Lys Gln Phe Asn Tyr Lys Asp Pro Val
   1               5                   10                  15
   Asn Gly Val Asp Ile Ala Tyr Ile Lys Ile Pro Asn Ala Gly Gln Met
               20                  25                  30
   Gln Pro Val Lys Ala Phe Lys Ile His Asn Lys Ile Trp Val Ile Pro
           35                  40                  45
   Glu Arg Asp Thr Phe Thr Asn Pro Glu Glu Gly Asp Leu Asn Pro Pro
       50                  55                  60
   Pro Glu Ala Lys Gln Val Pro Val Ser Tyr Tyr Asp Ser Thr Tyr Leu
   65                  70                  75                  80
   Ser Thr Asp Asn Glu Lys Asp Asn Tyr Leu Lys Gly Val Thr Lys Leu
                   85                  90                  95
   Phe Glu Arg Ile Tyr Ser Thr Asp Leu Gly Arg Met Leu Leu Thr Ser
               100                 105                 110
   Ile Val Arg Gly Ile Pro Phe Trp Gly Gly Ser Thr Ile Asp Thr Glu
           115                 120                 125
   Leu Lys Val Ile Asp Thr Asn Cys Ile Asn Val Ile Gln Pro Asp Gly
       130                 135                 140
   Ser Tyr Arg Ser Glu Glu Leu Asn Leu Val Ile Ile Gly Pro Ser Ala
   145                 150                 155                 160
   Asp Ile Ile Gln Phe Glu Cys Lys Ser Phe Gly His Glu Val Leu Asn
                   165                 170                 175
   Leu Thr Arg Asn Gly Tyr Gly Ser Thr Gln Tyr Ile Arg Phe Ser Pro
               180                 185                 190
   Asp Phe Thr Phe Gly Phe Glu Glu Ser Leu Glu Val Asp Thr Asn Pro
           195                 200                 205
   Leu Leu Gly Ala Gly Lys Phe Ala Thr Asp Pro Ala Val Thr Leu Ala
       210                 215                 220
   His Glu Leu Ile His Ala Gly His Arg Leu Tyr Gly Ile Ala Ile Asn
   225                 230                 235                 240
   Pro Asn Arg Val Phe Lys Val Asn Thr Asn Ala Tyr Tyr Glu Met Ser
                   245                 250                 255
   Gly Leu Glu Val Ser Phe Glu Glu Leu Arg Thr Phe Gly Gly His Asp
               260                 265                 270
   Ala Lys Phe Ile Asp Ser Leu Gln Glu Asn Glu Phe Arg Leu Tyr Tyr
           275                 280                 285
   Tyr Asn Lys Phe Lys Asp Ile Ala Ser Thr Leu Asn Lys Ala Lys Ser
       290                 295                 300
   Ile Val Gly Thr Thr Ala Ser Leu Gln Tyr Met Lys Asn Val Phe Lys
   305                 310                 315                 320
   Glu Lys Tyr Leu Leu Ser Glu Asp Thr Ser Gly Lys Phe Ser Val Asp
                   325                 330                 335
   Lys Leu Lys Phe Asp Lys Leu Tyr Lys Met Leu Thr Glu Ile Tyr Thr
               340                 345                 350
   Glu Asp Asn Phe Val Lys Phe Phe Lys Val Leu Asn Arg Lys Thr Tyr
           355                 360                 365
   Leu Asn Phe Asp Lys Ala Val Phe Lys Ile Asn Ile Val Pro Lys Val
       370                 375                 380
   Asn Tyr Thr Ile Tyr Asp Gly Phe Asn Leu Arg Asn Thr Asn Leu Ala
   385                 390                 395                 400
   Ala Asn Phe Asn Gly Gln Asn Thr Glu Ile Asn Asn Met Asn Phe Thr
                   405                 410                 415
   Lys Leu Lys Asn Phe Thr Gly Leu Phe Glu Phe Tyr Lys Leu Leu Cys
               420                 425                 430
   Val Asp Gly Ile Ile Thr Ser Lys Thr Lys Ser Leu Ile Glu Gly Arg
           435                 440                 445
   Tyr Gly Gly Phe Met Thr Ser Glu Lys Ser Gln Thr Pro Leu Val Thr
       450                 455                 460
   Leu Phe Lys Asn Ala Ile Ile Lys Asn Ala Tyr Lys Lys Gly Glu Ala
   465                 470                 475                 480
   Leu Ala Gly Gly Gly Gly Ser Gly Gly Gly Gly Ser Gly Gly Gly Gly
                   485                 490                 495
   Ser Ala Leu Val Leu Gln Cys Ile Lys Val Asn Asn Trp Asp Leu Phe
               500                 505                 510
   Phe Ser Pro Ser Glu Asp Asn Phe Thr Asn Asp Leu Asn Lys Gly Glu
           515                 520                 525
   Glu Ile Thr Ser Asp Thr Asn Ile Glu Ala Ala Glu Glu Asn Ile Ser
       530                 535                 540
   Leu Asp Leu Ile Gln Gln Tyr Tyr Leu Thr Phe Asn Phe Asp Asn Glu
   545                 550                 555                 560
   Pro Glu Asn Ile Ser Ile Glu Asn Leu Ser Ser Asp Ile Ile Gly Gln
                   565                 570                 575
   Leu Glu Leu Met Pro Asn Ile Glu Arg Phe Pro Asn Gly Lys Lys Tyr
               580                 585                 590
   Glu Leu Asp Lys Tyr Thr Met Phe His Tyr Leu Arg Ala Gln Glu Phe
           595                 600                 605
   Glu His Gly Lys Ser Arg Ile Ala Leu Thr Asn Ser Val Asn Glu Ala
   610 615 620
   Leu Leu Asn Pro Ser Arg Val Tyr Thr Phe Phe Ser Ser Asp Tyr Val
   625 630 635 640
   Lys Lys Val Asn Lys Ala Thr Glu Ala Ala Met Phe Leu Gly Trp Val
   645 650 655
   Glu Gln Leu Val Tyr Asp Phe Thr Asp Glu Thr Ser Glu Val Ser Thr
   660 665 670
   Thr Asp Lys Ile Ala Asp Ile Thr Ile Ile Ile Pro Tyr Ile Gly Pro
   675 680 685
   Ala Leu Asn Ile Gly Asn Met Leu Tyr Lys Asp Asp Phe Val Gly Ala
   690 695 700
   Leu Ile Phe Ser Gly Ala Val Ile Leu Leu Glu Phe Ile Pro Glu Ile
   705 710 715 720
   Ala Ile Pro Val Leu Gly Thr Phe Ala Leu Val Ser Tyr Ile Ala Asn
   725 730 735
   Lys Val Leu Thr Val Gln Thr Ile Asp Asn Ala Leu Ser Lys Arg Asn
   740 745 750
   Glu Lys Trp Asp Glu Val Tyr Lys Tyr Ile Val Thr Asn Trp Leu Ala
   755 760 765
   Lys Val Asn Thr Gln Ile Asp Leu Ile Arg Lys Lys Met Lys Glu Ala
   770 775 780
   Leu Glu Asn Gln Ala Glu Ala Thr Lys Ala Ile Ile Asn Tyr Gln Tyr
   785 790 795 800
   Asn Gln Tyr Thr Glu Glu Glu Lys Asn Asn Ile Asn Phe Asn Ile Asp
   805 810 815
   Asp Leu Ser Ser Lys Leu Asn Glu Ser Ile Asn Lys Ala Met Ile Asn
   820 825 830
   Ile Asn Lys Phe Leu Asn Gln Cys Ser Val Ser Tyr Leu Met Asn Ser
   835 840 845
   Met Ile Pro Tyr Gly Val Lys Arg Leu Glu Asp Phe Asp Ala Ser Leu
   850 855 860
   Lys Asp Ala Leu Leu Lys Tyr Ile Tyr Asp Asn Arg Gly Thr Leu Ile
   865 870 875 880
   Gly Gln Val Asp Arg Leu Lys Asp Lys Val Asn Asn Thr Leu Ser Thr
   885 890 895
   Asp Ile Pro Phe Gln Leu Ser Lys Tyr Val Asp Asn Gln Arg Leu Leu
   900 905 910
   Ser Thr Leu Asp
   915
<210> 51
   <211> 2709
   <212> DNA
   <213> Artificial Sequence
<220>
   <223> Synthetic
<400> 51
   ggatccatgg agttcgttaa caaacagttc aactataaag acccagttaa cggtgttgac      60
   attgcttaca tcaaaatccc gaacgctggc cagatgcagc cggtaaaggc attcaaaatc     120
   cacaacaaaa tctgggttat cccggaacgt gataccttta ctaacccgga agaaggtgac    180
   ctgaacccgc caccggaagc gaaacaggtg ccggtatctt actatgactc cacctacctg     240
   tctaccgata acgaaaagga caactacctg aaaggtgtta ctaaactgtt cgagcgtatt     300
   tactccaccg acctgggccg tatgctgctg actagcatcg ttcgcggtat cccgttctgg     360
   ggcggttcta ccatcgatac cgaactgaaa gtaatcgaca ctaactgcat caacgttatt     420
   cagccggacg gttcctatcg ttccgaagaa ctgaacctgg tgatcatcgg cccgtctgct     480
   gatatcatcc agttcgagtg taagagcttt ggtcacgaag ttctgaacct cacccgtaac     540
   ggctacggtt ccactcagta catccgtttc tctccggact tcaccttcgg ttttgaagaa     600
   tccctggaag tagacacgaa cccactgctg ggcgctggta aattcgcaac tgatcctgcg     660
   gttaccctgg ctcacgaact gattcatgca ggccaccgcc tgtacggtat cgccatcaat     720
   ccgaaccgtg tcttcaaagt taacaccaac gcgtattacg agatgtccgg tctggaagtt     780
   agcttcgaag aactgcgtac ttttggcggt cacgacgcta aattcatcga ctctctgcaa     840
   gaaaacgagt tccgtctgta ctactataac aagttcaaag atatcgcatc caccctgaac     900
   aaagcgaaat ccatcgtggg taccactgct tctctccagt acatgaagaa cgtttttaaa     960
   gaaaaatacc tgctcagcga agacacctcc ggcaaattct ctgtagacaa gttgaaattc    1020
   gataaacttt acaaaatgct gactgaaatt tacaccgaag acaacttcgt taagttcttt 1080
   aaagttctga accgcaaaac ctatctgaac ttcgacaagg cagtattcaa aatcaacatc 1140
   gtgccgaaag ttaactacac tatctacgat ggtttcaacc tgcgtaacac caacctggct 1200
   gctaatttta acggccagaa cacggaaatc aacaacatga acttcacaaa actgaaaaac 1260
   ttcactggtc tgttcgagtt ttacaagctg ctgtgcgtcg acggcatcat tacctccaaa 1320
   actaaatctc tgatagaagg tagatttggc ggtttcacgg gcgcacgcaa atcagcgcgt 1380
   aaacgtaaga accaggcgct agcgggcggt ggcggtagcg gcggtggcgg tagcggcggt 1440
   ggcggtagcg cactagtgct gcagtgtatc aaggttaaca actgggattt attcttcagc 1500
   ccgagtgaag acaacttcac caacgacctg aacaaaggtg aagaaatcac ctcagatact 1560
   aacatcgaag cagccgaaga aaacatctcg ctggacctga tccagcagta ctacctgacc 1620
   tttaatttcg acaacgagcc ggaaaacatt tctatcgaaa acctgagctc tgatatcatc 1680
   ggccagctgg aactgatgcc gaacatcgaa cgtttcccaa acggtaaaaa gtacgagctg 1740
   gacaaatata ccatgttcca ctacctgcgc gcgcaggaat ttgaacacgg caaatcccgt 1800
   atcgcactga ctaactccgt taacgaagct ctgctcaacc cgtcccgtgt atacaccttc 1860
   ttctctagcg actacgtgaa aaaggtcaac aaagcgactg aagctgcaat gttcttgggt 1920
   tgggttgaac agcttgttta tgattttacc gacgagacgt ccgaagtatc tactaccgac 1980
   aaaattgcgg atatcactat catcatcccg tacatcggtc cggctctgaa cattggcaac 2040
   atgctgtaca aagacgactt cgttggcgca ctgatcttct ccggtgcggt gatcctgctg 2100
   gagttcatcc cggaaatcgc catcccggta ctgggcacct ttgctctggt ttcttacatt 2160
   gcaaacaagg ttctgactgt acaaaccatc gacaacgcgc tgagcaaacg taacgaaaaa 2220
   tgggatgaag tttacaaata tatcgtgacc aactggctgg ctaaggttaa tactcagatc 2280
   gacctcatcc gcaaaaaaat gaaagaagca ctggaaaacc aggcggaagc taccaaggca 2340
   atcattaact accagtacaa ccagtacacc gaggaagaaa aaaacaacat caacttcaac 2400
   atcgacgatc tgtcctctaa actgaacgaa tccatcaaca aagctatgat caacatcaac 2460
   aagttcctga accagtgctc tgtaagctat ctgatgaact ccatgatccc gtacggtgtt 2520
   aaacgtctgg aggacttcga tgcgtctctg aaagacgccc tgctgaaata catttacgac 2580
   aaccgtggca ctctgatcgg tcaggttgat cgtctgaagg acaaagtgaa caatacctta 2640
   tcgaccgaca tcccttttca gctcagtaaa tatgtcgata accaacgcct tttgtccact 2700
   ctagactag 2709
<210> 52
   <211> 902
   <212> PRT
   <213> Artificial Sequence
<220>
   <223> Synthetic
<400> 52
   Gly Ser Met Glu Phe Val Asn Lys Gln Phe Asn Tyr Lys Asp Pro Val
   1               5                   10                  15
   Asn Gly Val Asp Ile Ala Tyr Ile Lys Ile Pro Asn Ala Gly Gln Met
               20                  25                  30
   Gln Pro Val Lys Ala Phe Lys Ile His Asn Lys Ile Trp Val Ile Pro
           35                  40                  45
   Glu Arg Asp Thr Phe Thr Asn Pro Glu Glu Gly Asp Leu Asn Pro Pro
       50                  55                  60
   Pro Glu Ala Lys Gln Val Pro Val Ser Tyr Tyr Asp Ser Thr Tyr Leu
   65                  70                  75                  80
   Ser Thr Asp Asn Glu Lys Asp Asn Tyr Leu Lys Gly Val Thr Lys Leu
                   85                  90                  95
   Phe Glu Arg Ile Tyr Ser Thr Asp Leu Gly Arg Met Leu Leu Thr Ser
               100                 105                 110
   Ile Val Arg Gly Ile Pro Phe Trp Gly Gly Ser Thr Ile Asp Thr Glu
           115                 120                 125
   Leu Lys Val Ile Asp Thr Asn Cys Ile Asn Val Ile Gln Pro Asp Gly
       130                 135                 140
   Ser Tyr Arg Ser Glu Glu Leu Asn Leu Val Ile Ile Gly Pro Ser Ala
   145                 150                 155                 160
   Asp Ile Ile Gln Phe Glu Cys Lys Ser Phe Gly His Glu Val Leu Asn
                   165                 170                 175
   Leu Thr Arg Asn Gly Tyr Gly Ser Thr Gln Tyr Ile Arg Phe Ser Pro
               180                 185                 190
   Asp Phe Thr Phe Gly Phe Glu Glu Ser Leu Glu Val Asp Thr Asn Pro
           195                 200                 205
   Leu Leu Gly Ala Gly Lys Phe Ala Thr Asp Pro Ala Val Thr Leu Ala
       210                 215                 220
   His Glu Leu Ile His Ala Gly His Arg Leu Tyr Gly Ile Ala Ile Asn
   225                 230                 235                 240
   Pro Asn Arg Val Phe Lys Val Asn Thr Asn Ala Tyr Tyr Glu Met Ser
                   245                 250                 255
   Gly Leu Glu Val Ser Phe Glu Glu Leu Arg Thr Phe Gly Gly His Asp
               260                 265                 270
   Ala Lys Phe Ile Asp Ser Leu Gln Glu Asn Glu Phe Arg Leu Tyr Tyr
           275                 280                 285
   Tyr Asn Lys Phe Lys Asp Ile Ala Ser Thr Leu Asn Lys Ala Lys Ser
       290                 295                 300
   Ile Val Gly Thr Thr Ala Ser Leu Gln Tyr Met Lys Asn Val Phe Lys
   305                 310                 315                 320
   Glu Lys Tyr Leu Leu Ser Glu Asp Thr Ser Gly Lys Phe Ser Val Asp
                   325                 330                 335
   Lys Leu Lys Phe Asp Lys Leu Tyr Lys Met Leu Thr Glu Ile Tyr Thr
               340                 345                 350
   Glu Asp Asn Phe Val Lys Phe Phe Lys Val Leu Asn Arg Lys Thr Tyr
           355                 360                 365
   Leu Asn Phe Asp Lys Ala Val Phe Lys Ile Asn Ile Val Pro Lys Val
       370                 375                 380
   Asn Tyr Thr Ile Tyr Asp Gly Phe Asn Leu Arg Asn Thr Asn Leu Ala
   385                 390                 395                 400
   Ala Asn Phe Asn Gly Gln Asn Thr Glu Ile Asn Asn Met Asn Phe Thr
                   405                 410                 415
   Lys Leu Lys Asn Phe Thr Gly Leu Phe Glu Phe Tyr Lys Leu Leu Cys
               420                 425                 430
   Val Asp Gly Ile Ile Thr Ser Lys Thr Lys Ser Leu Ile Glu Gly Arg
           435                 440                 445
   Phe Gly Gly Phe Thr Gly Ala Arg Lys Ser Ala Arg Lys Arg Lys Asn
       450                 455                 460
   Gln Ala Leu Ala Gly Gly Gly Gly Ser Gly Gly Gly Gly Ser Gly Gly
   465                 470                 475                 480
   Gly Gly Ser Ala Leu Val Leu Gln Cys Ile Lys Val Asn Asn Trp Asp
                   485                 490                 495
   Leu Phe Phe Ser Pro Ser Glu Asp Asn Phe Thr Asn Asp Leu Asn Lys
               500                 505                 510
   Gly Glu Glu Ile Thr Ser Asp Thr Asn Ile Glu Ala Ala Glu Glu Asn
           515                 520                 525
   Ile Ser Leu Asp Leu Ile Gln Gln Tyr Tyr Leu Thr Phe Asn Phe Asp
       530                 535                 540
   Asn Glu Pro Glu Asn Ile Ser Ile Glu Asn Leu Ser Ser Asp Ile Ile
   545                 550                 555                 560
   Gly Gln Leu Glu Leu Met Pro Asn Ile Glu Arg Phe Pro Asn Gly Lys
                   565                 570                 575
   Lys Tyr Glu Leu Asp Lys Tyr Thr Met Phe His Tyr Leu Arg Ala Gln
               580                 585                 590
   Glu Phe Glu His Gly Lys Ser Arg Ile Ala Leu Thr Asn Ser Val Asn
           595                 600                 605
   Glu Ala Leu Leu Asn Pro Ser Arg Val Tyr Thr Phe Phe Ser Ser Asp
       610                 615                 620
   Tyr Val Lys Lys Val Asn Lys Ala Thr Glu Ala Ala Met Phe Leu Gly
   625                 630                 635                 640
   Trp Val Glu Gln Leu Val Tyr Asp Phe Thr Asp Glu Thr Ser Glu Val
                   645                 650                 655
   Ser Thr Thr Asp Lys Ile Ala Asp Ile Thr Ile Ile Ile Pro Tyr Ile
               660                 665                 670
   Gly Pro Ala Leu Asn Ile Gly Asn Met Leu Tyr Lys Asp Asp Phe Val
           675                 680                 685
   Gly Ala Leu Ile Phe Ser Gly Ala Val Ile Leu Leu Glu Phe Ile Pro
       690                 695                 700
   Glu Ile Ala Ile Pro Val Leu Gly Thr Phe Ala Leu Val Ser Tyr Ile
   705 710 715 720
   Ala Asn Lys Val Leu Thr Val Gln Thr Ile Asp Asn Ala Leu Ser Lys
   725 730 735
   Arg Asn Glu Lys Trp Asp Glu Val Tyr Lys Tyr Ile Val Thr Asn Trp
   740 745 750
   Leu Ala Lys Val Asn Thr Gln Ile Asp Leu Ile Arg Lys Lys Met Lys
   755 760 765
   Glu Ala Leu Glu Asn Gln Ala Glu Ala Thr Lys Ala Ile Ile Asn Tyr
   770 775 780
   Gln Tyr Asn Gln Tyr Thr Glu Glu Glu Lys Asn Asn Ile Asn Phe Asn
   785 790 795 800
   Ile Asp Asp Leu Ser Ser Lys Leu Asn Glu Ser Ile Asn Lys Ala Met
   805 810 815
   Ile Asn Ile Asn Lys Phe Leu Asn Gln Cys Ser Val Ser Tyr Leu Met
   820 825 830
   Asn Ser Met Ile Pro Tyr Gly Val Lys Arg Leu Glu Asp Phe Asp Ala
   835 840 845
   Ser Leu Lys Asp Ala Leu Leu Lys Tyr Ile Tyr Asp Asn Arg Gly Thr
   850 855 860
   Leu Ile Gly Gln Val Asp Arg Leu Lys Asp Lys Val Asn Asn Thr Leu
   865 870 875 880
   Ser Thr Asp Ile Pro Phe Gln Leu Ser Lys Tyr Val Asp Asn Gln Arg
   885 890 895
   Leu Leu Ser Thr Leu Asp
   900
<210> 53
   <211> 2691
   <212> DNA
   <213> Artificial Sequence
<220>
   <223> Synthetic
<400> 53
   ggatccatgg agttcgttaa caaacagttc aactataaag acccagttaa cggtgttgac      60
   attgcttaca tcaaaatccc gaacgctggc cagatgcagc cggtaaaggc attcaaaatc     120
   cacaacaaaa tctgggttat cccggaacgt gataccttta ctaacccgga agaaggtgac     180
   ctgaacccgc caccggaagc gaaacaggtg ccggtatctt actatgactc cacctacctg     240
   tctaccgata acgaaaagga caactacctg aaaggtgtta ctaaactgtt cgagcgtatt     300
   tactccaccg acctgggccg tatgctgctg actagcatcg ttcgcggtat cccgttctgg     360
   ggcggttcta ccatcgatac cgaactgaaa gtaatcgaca ctaactgcat caacgttatt     420
   cagccggacg gttcctatcg ttccgaagaa ctgaacctgg tgatcatcgg cccgtctgct     480
   gatatcatcc agttcgagtg taagagcttt ggtcacgaag ttctgaacct cacccgtaac     540
   ggctacggtt ccactcagta catccgtttc tctccggact tcaccttcgg ttttgaagaa     600
   tccctggaag tagacacgaa cccactgctg ggcgctggta aattcgcaac tgatcctgcg     660
   gttaccctgg ctcacgaact gattcatgca ggccaccgcc tgtacggtat cgccatcaat     720
   ccgaaccgtg tcttcaaagt taacaccaac gcgtattacg agatgtccgg tctggaagtt     780
   agcttcgaag aactgcgtac ttttggcggt cacgacgcta aattcatcga ctctctgcaa     840
   gaaaacgagt tccgtctgta ctactataac aagttcaaag atatcgcatc caccctgaac     900
   aaagcgaaat ccatcgtggg taccactgct tctctccagt acatgaagaa cgtttttaaa     960
   gaaaaatacc tgctcagcga agacacctcc ggcaaattct ctgtagacaa gttgaaattc    1020
   gataaacttt acaaaatgct gactgaaatt tacaccgaag acaacttcgt taagttcttt    1080
   aaagttctga accgcaaaac ctatctgaac ttcgacaagg cagtattcaa aatcaacatc    1140
   gtgccgaaag ttaactacac tatctacgat ggtttcaacc tgcgtaacac caacctggct    1200
   gctaatttta acggccagaa cacggaaatc aacaacatga acttcacaaa actgaaaaac    1260
   ttcactggtc tgttcgagtt ttacaagctg ctgtgcgtcg acggcatcat tacctccaaa    1320
   actaaatctc tgatagaagg tagatttggc ggtttcacgg gcgcacgcaa atcagcggcg    1380
   ctagcgggcg gtggcggtag cggcggtggc ggtagcggcg gtggcggtag cgcactagtg    1440
   ctgcagtgta tcaaggttaa caactgggat ttattcttca gcccgagtga agacaacttc    1500
   accaacgacc tgaacaaagg tgaagaaatc acctcagata ctaacatcga agcagccgaa    1560
   gaaaacatct cgctggacct gatccagcag tactacctga cctttaattt cgacaacgag    1620
   ccggaaaaca tttctatcga aaacctgagc tctgatatca tcggccagct ggaactgatg    1680
   ccgaacatcg aacgtttccc aaacggtaaa aagtacgagc tggacaaata taccatgttc    1740
   cactacctgc gcgcgcagga atttgaacac ggcaaatccc gtatcgcact gactaactcc    1800
   gttaacgaag ctctgctcaa cccgtcccgt gtatacacct tcttctctag cgactacgtg 1860
   aaaaaggtca acaaagcgac tgaagctgca atgttcttgg gttgggttga acagcttgtt 1920
   tatgatttta ccgacgagac gtccgaagta tctactaccg acaaaattgc ggatatcact 1980
   atcatcatcc cgtacatcgg tccggctctg aacattggca acatgctgta caaagacgac 2040
   ttcgttggcg cactgatctt ctccggtgcg gtgatcctgc tggagttcat cccggaaatc 2100
   gccatcccgg tactgggcac ctttgctctg gtttcttaca ttgcaaacaa ggttctgact 2160
   gtacaaacca tcgacaacgc gctgagcaaa cgtaacgaaa aatgggatga agtttacaaa 2220
   tatatcgtga ccaactggct ggctaaggtt aatactcaga tcgacctcat ccgcaaaaaa 2280
   atgaaagaag cactggaaaa ccaggcggaa gctaccaagg caatcattaa ctaccagtac 2340
   aaccagtaca ccgaggaaga aaaaaacaac atcaacttca acatcgacga tctgtcctct 2400
   aaactgaacg aatccatcaa caaagctatg atcaacatca acaagttcct gaaccagtgc 2460
   tctgtaagct atctgatgaa ctccatgatc ccgtacggtg ttaaacgtct ggaggacttc 2520
   gatgcgtctc tgaaagacgc cctgctgaaa tacatttacg acaaccgtgg cactctgatc 2580
   ggtcaggttg atcgtctgaa ggacaaagtg aacaatacct tatcgaccga catccctttt 2640
   cagctcagta aatatgtcga taaccaacgc cttttgtcca ctctagacta g 2691
<210> 54
   <211> 896
   <212> PRT
   <213> Artificial Sequence
<220>
   <223> Synthetic
<400> 54
   Gly Ser Met Glu Phe Val Asn Lys Gln Phe Asn Tyr Lys Asp Pro Val
   1 5 10 15
   Asn Gly Val Asp Ile Ala Tyr Ile Lys Ile Pro Asn Ala Gly Gln Met
   20 25 30
   Gln Pro Val Lys Ala Phe Lys Ile His Asn Lys Ile Trp Val Ile Pro
   35 40 45
   Glu Arg Asp Thr Phe Thr Asn Pro Glu Glu Gly Asp Leu Asn Pro Pro
   50 55 60
   Pro Glu Ala Lys Gln Val Pro Val Ser Tyr Tyr Asp Ser Thr Tyr Leu
   65 70 75 80
   Ser Thr Asp Asn Glu Lys Asp Asn Tyr Leu Lys Gly Val Thr Lys Leu
   85 90 95
   Phe Glu Arg Ile Tyr Ser Thr Asp Leu Gly Arg Met Leu Leu Thr Ser
   100 105 110
   Ile Val Arg Gly Ile Pro Phe Trp Gly Gly Ser Thr Ile Asp Thr Glu
   115 120 125
   Leu Lys Val Ile Asp Thr Asn Cys Ile Asn Val Ile Gln Pro Asp Gly
   130 135 140
   Ser Tyr Arg Ser Glu Glu Leu Asn Leu Val Ile Ile Gly Pro Ser Ala
   145 150 155 160
   Asp Ile Ile Gln Phe Glu Cys Lys Ser Phe Gly His Glu Val Leu Asn
   165 170 175
   Leu Thr Arg Asn Gly Tyr Gly Ser Thr Gln Tyr Ile Arg Phe Ser Pro
   180 185 190
   Asp Phe Thr Phe Gly Phe Glu Glu Ser Leu Glu Val Asp Thr Asn Pro
   195 200 205
   Leu Leu Gly Ala Gly Lys Phe Ala Thr Asp Pro Ala Val Thr Leu Ala
   210 215 220
   His Glu Leu Ile His Ala Gly His Arg Leu Tyr Gly Ile Ala Ile Asn
   225 230 235 240
   Pro Asn Arg Val Phe Lys Val Asn Thr Asn Ala Tyr Tyr Glu Met Ser
   245 250 255
   Gly Leu Glu Val Ser Phe Glu Glu Leu Arg Thr Phe Gly Gly His Asp
   260 265 270
   Ala Lys Phe Ile Asp Ser Leu Gln Glu Asn Glu Phe Arg Leu Tyr Tyr
   275 280 285
   Tyr Asn Lys Phe Lys Asp Ile Ala Ser Thr Leu Asn Lys Ala Lys Ser
   290 295 300
   Ile Val Gly Thr Thr Ala Ser Leu Gln Tyr Met Lys Asn Val Phe Lys
   305 310 315 320
   Glu Lys Tyr Leu Leu Ser Glu Asp Thr Ser Gly Lys Phe Ser Val Asp
   325 330 335
   Lys Leu Lys Phe Asp Lys Leu Tyr Lys Met Leu Thr Glu Ile Tyr Thr
   340 345 350
   Glu Asp Asn Phe Val Lys Phe Phe Lys Val Leu Asn Arg Lys Thr Tyr
   355 360 365
   Leu Asn Phe Asp Lys Ala Val Phe Lys Ile Asn Ile Val Pro Lys Val
   370 375 380
   Asn Tyr Thr Ile Tyr Asp Gly Phe Asn Leu Arg Asn Thr Asn Leu Ala
   385 390 395 400
   Ala Asn Phe Asn Gly Gln Asn Thr Glu Ile Asn Asn Met Asn Phe Thr
   405 410 415
   Lys Leu Lys Asn Phe Thr Gly Leu Phe Glu Phe Tyr Lys Leu Leu Cys
   420 425 430
   Val Asp Gly Ile Ile Thr Ser Lys Thr Lys Ser Leu Ile Glu Gly Arg
   435 440 445
   Phe Gly Gly Phe Thr Gly Ala Arg Lys Ser Ala Ala Leu Ala Gly Gly
   450 455 460
   Gly Gly Ser Gly Gly Gly Gly Ser Gly Gly Gly Gly Ser Ala Leu Val
   465 470 475 480
   Leu Gln Cys Ile Lys Val Asn Asn Trp Asp Leu Phe Phe Ser Pro Ser
   485 490 495
   Glu Asp Asn Phe Thr Asn Asp Leu Asn Lys Gly Glu Glu Ile Thr Ser
   500 505 510
   Asp Thr Asn Ile Glu Ala Ala Glu Glu Asn Ile Ser Leu Asp Leu Ile
   515 520 525
   Gln Gln Tyr Tyr Leu Thr Phe Asn Phe Asp Asn Glu Pro Glu Asn Ile
   530 535 540
   Ser Ile Glu Asn Leu Ser Ser Asp Ile Ile Gly Gln Leu Glu Leu Met
   545 550 555 560
   Pro Asn Ile Glu Arg Phe Pro Asn Gly Lys Lys Tyr Glu Leu Asp Lys
   565 570 575
   Tyr Thr Met Phe His Tyr Leu Arg Ala Gln Glu Phe Glu His Gly Lys
   580 585 590
   Ser Arg Ile Ala Leu Thr Asn Ser Val Asn Glu Ala Leu Leu Asn Pro
   595 600 605
   Ser Arg Val Tyr Thr Phe Phe Ser Ser Asp Tyr Val Lys Lys Val Asn
   610 615 620
   Lys Ala Thr Glu Ala Ala Met Phe Leu Gly Trp Val Glu Gln Leu Val
   625 630 635 640
   Tyr Asp Phe Thr Asp Glu Thr Ser Glu Val Ser Thr Thr Asp Lys Ile
   645 650 655
   Ala Asp Ile Thr Ile Ile Ile Pro Tyr Ile Gly Pro Ala Leu Asn Ile
   660 665 670
   Gly Asn Met Leu Tyr Lys Asp Asp Phe Val Gly Ala Leu Ile Phe Ser
   675 680 685
   Gly Ala Val Ile Leu Leu Glu Phe Ile Pro Glu Ile Ala Ile Pro Val
   690 695 700
   Leu Gly Thr Phe Ala Leu Val Ser Tyr Ile Ala Asn Lys Val Leu Thr
   705 710 715 720
   Val Gln Thr Ile Asp Asn Ala Leu Ser Lys Arg Asn Glu Lys Trp Asp
   725 730 735
   Glu Val Tyr Lys Tyr Ile Val Thr Asn Trp Leu Ala Lys Val Asn Thr
   740 745 750
   Gln Ile Asp Leu Ile Arg Lys Lys Met Lys Glu Ala Leu Glu Asn Gln
   755 760 765
   Ala Glu Ala Thr Lys Ala Ile Ile Asn Tyr Gln Tyr Asn Gln Tyr Thr
   770 775 780
   Glu Glu Glu Lys Asn Asn Ile Asn Phe Asn Ile Asp Asp Leu Ser Ser
   785 790 795 800
   Lys Leu Asn Glu Ser Ile Asn Lys Ala Met Ile Asn Ile Asn Lys Phe
   805 810 815
   Leu Asn Gln Cys Ser Val Ser Tyr Leu Met Asn Ser Met Ile Pro Tyr
   820 825 830
   Gly Val Lys Arg Leu Glu Asp Phe Asp Ala Ser Leu Lys Asp Ala Leu
   835 840 845
   Leu Lys Tyr Ile Tyr Asp Asn Arg Gly Thr Leu Ile Gly Gln Val Asp
   850 855 860
   Arg Leu Lys Asp Lys Val Asn Asn Thr Leu Ser Thr Asp Ile Pro Phe
   865 870 875 880
   Gln Leu Ser Lys Tyr Val Asp Asn Gln Arg Leu Leu Ser Thr Leu Asp
   885 890 895
<210> 55
   <211> 2691
   <212> DNA
   <213> Artificial Sequence
<220>
   <223> Synthetic
<400> 55
   ggatccatgg agttcgttaa caaacagttc aactataaag acccagttaa cggtgttgac 60
   attgcttaca tcaaaatccc gaacgctggc cagatgcagc cggtaaaggc attcaaaatc 120
   cacaacaaaa tctgggttat cccggaacgt gataccttta ctaacccgga agaaggtgac 180
   ctgaacccgc caccggaagc gaaacaggtg ccggtatctt actatgactc cacctacctg 240
   tctaccgata acgaaaagga caactacctg aaaggtgtta ctaaactgtt cgagcgtatt 300
   tactccaccg acctgggccg tatgctgctg actagcatcg ttcgcggtat cccgttctgg 360
   ggcggttcta ccatcgatac cgaactgaaa gtaatcgaca ctaactgcat caacgttatt 420
   cagccggacg gttcctatcg ttccgaagaa ctgaacctgg tgatcatcgg cccgtctgct 480
   gatatcatcc agttcgagtg taagagcttt ggtcacgaag ttctgaacct cacccgtaac 540
   ggctacggtt ccactcagta catccgtttc tctccggact tcaccttcgg ttttgaagaa 600
   tccctggaag tagacacgaa cccactgctg ggcgctggta aattcgcaac tgatcctgcg 660
   gttaccctgg ctcacgaact gattcatgca ggccaccgcc tgtacggtat cgccatcaat 720
   ccgaaccgtg tcttcaaagt taacaccaac gcgtattacg agatgtccgg tctggaagtt 780
   agcttcgaag aactgcgtac ttttggcggt cacgacgcta aattcatcga ctctctgcaa 840
   gaaaacgagt tccgtctgta ctactataac aagttcaaag atatcgcatc caccctgaac 900
   aaagcgaaat ccatcgtggg taccactgct tctctccagt acatgaagaa cgtttttaaa    960
   gaaaaatacc tgctcagcga agacacctcc ggcaaattct ctgtagacaa gttgaaattc   1020
   gataaacttt acaaaatgct gactgaaatt tacaccgaag acaacttcgt taagttcttt   1080
   aaagttctga accgcaaaac ctatctgaac ttcgacaagg cagtattcaa aatcaacatc   1140
   gtgccgaaag ttaactacac tatctacgat ggtttcaacc tgcgtaacac caacctggct   1200
   gctaatttta acggccagaa cacggaaatc aacaacatga acttcacaaa actgaaaaac   1260
   ttcactggtc tgttcgagtt ttacaagctg ctgtgcgtcg acggcatcat tacctccaaa   1320
   actaaatctc tgatagaagg tagatttggc ggtttcacgg gcgcacgcaa atatgcggcg   1380
   ctagcgggcg gtggcggtag cggcggtggc ggtagcggcg gtggcggtag cgcactagtg   1440
   ctgcagtgta tcaaggttaa caactgggat ttattcttca gcccgagtga agacaacttc   1500
   accaacgacc tgaacaaagg tgaagaaatc acctcagata ctaacatcga agcagccgaa   1560
   gaaaacatct cgctggacct gatccagcag tactacctga cctttaattt cgacaacgag   1620
   ccggaaaaca tttctatcga aaacctgagc tctgatatca tcggccagct ggaactgatg   1680
   ccgaacatcg aacgtttccc aaacggtaaa aagtacgagc tggacaaata taccatgttc   1740
   cactacctgc gcgcgcagga atttgaacac ggcaaatccc gtatcgcact gactaactcc   1800
   gttaacgaag ctctgctcaa cccgtcccgt gtatacacct tcttctctag cgactacgtg   1860
   aaaaaggtca acaaagcgac tgaagctgca atgttcttgg gttgggttga acagcttgtt   1920
   tatgatttta ccgacgagac gtccgaagta tctactaccg acaaaattgc ggatatcact   1980
   atcatcatcc cgtacatcgg tccggctctg aacattggca acatgctgta caaagacgac   2040
   ttcgttggcg cactgatctt ctccggtgcg gtgatcctgc tggagttcat cccggaaatc   2100
   gccatcccgg tactgggcac ctttgctctg gtttcttaca ttgcaaacaa ggttctgact   2160
   gtacaaacca tcgacaacgc gctgagcaaa cgtaacgaaa aatgggatga agtttacaaa   2220
   tatatcgtga ccaactggct ggctaaggtt aatactcaga tcgacctcat ccgcaaaaaa   2280
   atgaaagaag cactggaaaa ccaggcggaa gctaccaagg caatcattaa ctaccagtac   2340
   aaccagtaca ccgaggaaga aaaaaacaac atcaacttca acatcgacga tctgtcctct   2400
   aaactgaacg aatccatcaa caaagctatg atcaacatca acaagttcct gaaccagtgc   2460
   tctgtaagct atctgatgaa ctccatgatc ccgtacggtg ttaaacgtct ggaggacttc   2520
   gatgcgtctc tgaaagacgc cctgctgaaa tacatttacg acaaccgtgg cactctgatc   2580
   ggtcaggttg atcgtctgaa ggacaaagtg aacaatacct tatcgaccga catccctttt   2640
   cagctcagta aatatgtcga taaccaacgc cttttgtcca ctctagacta g            2691
<210> 56
   <211> 896
   <212> PRT
   <213> Artificial Sequence
<220>
   <223> Synthetic
<400> 56
   Gly Ser Met Glu Phe Val Asn Lys Gln Phe Asn Tyr Lys Asp Pro Val
   1               5                   10                  15
   Asn Gly Val Asp Ile Ala Tyr Ile Lys Ile Pro Asn Ala Gly Gln Met
               20                  25                  30
   Gln Pro Val Lys Ala Phe Lys Ile His Asn Lys Ile Trp Val Ile Pro
           35                  40                  45
   Glu Arg Asp Thr Phe Thr Asn Pro Glu Glu Gly Asp Leu Asn Pro Pro
       50                  55                  60
   Pro Glu Ala Lys Gln Val Pro Val Ser Tyr Tyr Asp Ser Thr Tyr Leu
   65                  70                  75                  80
   Ser Thr Asp Asn Glu Lys Asp Asn Tyr Leu Lys Gly Val Thr Lys Leu
                   85                  90                  95
   Phe Glu Arg Ile Tyr Ser Thr Asp Leu Gly Arg Met Leu Leu Thr Ser
               100                 105                 110
   Ile Val Arg Gly Ile Pro Phe Trp Gly Gly Ser Thr Ile Asp Thr Glu
           115                 120                 125
   Leu Lys Val Ile Asp Thr Asn Cys Ile Asn Val Ile Gln Pro Asp Gly
       130                 135                 140
   Ser Tyr Arg Ser Glu Glu Leu Asn Leu Val Ile Ile Gly Pro Ser Ala
   145                 150                 155                 160
   Asp Ile Ile Gln Phe Glu Cys Lys Ser Phe Gly His Glu Val Leu Asn
                   165                 170                 175
   Leu Thr Arg Asn Gly Tyr Gly Ser Thr Gln Tyr Ile Arg Phe Ser Pro
               180                 185                 190
   Asp Phe Thr Phe Gly Phe Glu Glu Ser Leu Glu Val Asp Thr Asn Pro
           195                 200                 205
   Leu Leu Gly Ala Gly Lys Phe Ala Thr Asp Pro Ala Val Thr Leu Ala
       210                 215                 220
   His Glu Leu Ile His Ala Gly His Arg Leu Tyr Gly Ile Ala Ile Asn
   225                 230                 235                 240
   Pro Asn Arg Val Phe Lys Val Asn Thr Asn Ala Tyr Tyr Glu Met Ser
                   245                 250                 255
   Gly Leu Glu Val Ser Phe Glu Glu Leu Arg Thr Phe Gly Gly His Asp
               260                 265                 270
   Ala Lys Phe Ile Asp Ser Leu Gln Glu Asn Glu Phe Arg Leu Tyr Tyr
           275                 280                 285
   Tyr Asn Lys Phe Lys Asp Ile Ala Ser Thr Leu Asn Lys Ala Lys Ser
       290                 295                 300
   Ile Val Gly Thr Thr Ala Ser Leu Gln Tyr Met Lys Asn Val Phe Lys
   305                 310                 315                 320
   Glu Lys Tyr Leu Leu Ser Glu Asp Thr Ser Gly Lys Phe Ser Val Asp
                   325                 330                 335
   Lys Leu Lys Phe Asp Lys Leu Tyr Lys Met Leu Thr Glu Ile Tyr Thr
               340                 345                 350
   Glu Asp Asn Phe Val Lys Phe Phe Lys Val Leu Asn Arg Lys Thr Tyr
           355                 360                 365
   Leu Asn Phe Asp Lys Ala Val Phe Lys Ile Asn Ile Val Pro Lys Val
       370                 375                 380
   Asn Tyr Thr Ile Tyr Asp Gly Phe Asn Leu Arg Asn Thr Asn Leu Ala
   385                 390                 395                 400
   Ala Asn Phe Asn Gly Gln Asn Thr Glu Ile Asn Asn Met Asn Phe Thr
                   405                 410                 415
   Lys Leu Lys Asn Phe Thr Gly Leu Phe Glu Phe Tyr Lys Leu Leu Cys
               420                 425                 430
   Val Asp Gly Ile Ile Thr Ser Lys Thr Lys Ser Leu Ile Glu Gly Arg
           435                 440                 445
   Phe Gly Gly Phe Thr Gly Ala Arg Lys Tyr Ala Ala Leu Ala Gly Gly
   450 455 460
   Gly Gly Ser Gly Gly Gly Gly Ser Gly Gly Gly Gly Ser Ala Leu Val
   465 470 475 480
   Leu Gln Cys Ile Lys Val Asn Asn Trp Asp Leu Phe Phe Ser Pro Ser
   485 490 495
   Glu Asp Asn Phe Thr Asn Asp Leu Asn Lys Gly Glu Glu Ile Thr Ser
   500 505 510
   Asp Thr Asn Ile Glu Ala Ala Glu Glu Asn Ile Ser Leu Asp Leu Ile
   515 520 525
   Gln Gln Tyr Tyr Leu Thr Phe Asn Phe Asp Asn Glu Pro Glu Asn Ile
   530 535 540
   Ser Ile Glu Asn Leu Ser Ser Asp Ile Ile Gly Gln Leu Glu Leu Met
   545 550 555 560
   Pro Asn Ile Glu Arg Phe Pro Asn Gly Lys Lys Tyr Glu Leu Asp Lys
   565 570 575
   Tyr Thr Met Phe His Tyr Leu Arg Ala Gln Glu Phe Glu His Gly Lys
   580 585 590
   Ser Arg Ile Ala Leu Thr Asn Ser Val Asn Glu Ala Leu Leu Asn Pro
   595 600 605
   Ser Arg Val Tyr Thr Phe Phe Ser Ser Asp Tyr Val Lys Lys Val Asn
   610 615 620
   Lys Ala Thr Glu Ala Ala Met Phe Leu Gly Trp Val Glu Gln Leu Val
   625 630 635 640
   Tyr Asp Phe Thr Asp Glu Thr Ser Glu Val Ser Thr Thr Asp Lys Ile
   645 650 655
   Ala Asp Ile Thr Ile Ile Ile Pro Tyr Ile Gly Pro Ala Leu Asn Ile
   660 665 670
   Gly Asn Met Leu Tyr Lys Asp Asp Phe Val Gly Ala Leu Ile Phe Ser
   675 680 685
   Gly Ala Val Ile Leu Leu Glu Phe Ile Pro Glu Ile Ala Ile Pro Val
   690 695 700
   Leu Gly Thr Phe Ala Leu Val Ser Tyr Ile Ala Asn Lys Val Leu Thr
   705 710 715 720
   Val Gln Thr Ile Asp Asn Ala Leu Ser Lys Arg Asn Glu Lys Trp Asp
   725 730 735
   Glu Val Tyr Lys Tyr Ile Val Thr Asn Trp Leu Ala Lys Val Asn Thr
   740 745 750
   Gln Ile Asp Leu Ile Arg Lys Lys Met Lys Glu Ala Leu Glu Asn Gln
   755 760 765
   Ala Glu Ala Thr Lys Ala Ile Ile Asn Tyr Gln Tyr Asn Gln Tyr Thr
   770 775 780
   Glu Glu Glu Lys Asn Asn Ile Asn Phe Asn Ile Asp Asp Leu Ser Ser
   785 790 795 800
   Lys Leu Asn Glu Ser Ile Asn Lys Ala Met Ile Asn Ile Asn Lys Phe
   805 810 815
   Leu Asn Gln Cys Ser Val Ser Tyr Leu Met Asn Ser Met Ile Pro Tyr
   820 825 830
   Gly Val Lys Arg Leu Glu Asp Phe Asp Ala Ser Leu Lys Asp Ala Leu
   835 840 845
   Leu Lys Tyr Ile Tyr Asp Asn Arg Gly Thr Leu Ile Gly Gln Val Asp
   850 855 860
   Arg Leu Lys Asp Lys Val Asn Asn Thr Leu Ser Thr Asp Ile Pro Phe
   865 870 875 880
   Gln Leu Ser Lys Tyr Val Asp Asn Gln Arg Leu Leu Ser Thr Leu Asp
   885 890 895
<210> 57
   <211> 2691
   <212> DNA
   <213> Artificial Sequence
<220>
   <223> Synthetic
<400> 57
   ggatccatgg agttcgttaa caaacagttc aactataaag acccagttaa cggtgttgac 60
   attgcttaca tcaaaatccc gaacgctggc cagatgcagc cggtaaaggc attcaaaatc     120
   cacaacaaaa tctgggttat cccggaacgt gataccttta ctaacccgga agaaggtgac     180
   ctgaacccgc caccggaagc gaaacaggtg ccggtatctt actatgactc cacctacctg     240
   tctaccgata acgaaaagga caactacctg aaaggtgtta ctaaactgtt cgagcgtatt     300
   tactccaccg acctgggccg tatgctgctg actagcatcg ttcgcggtat cccgttctgg     360
   ggcggttcta ccatcgatac cgaactgaaa gtaatcgaca ctaactgcat caacgttatt     420
   cagccggacg gttcctatcg ttccgaagaa ctgaacctgg tgatcatcgg cccgtctgct     480
   gatatcatcc agttcgagtg taagagcttt ggtcacgaag ttctgaacct cacccgtaac     540
   ggctacggtt ccactcagta catccgtttc tctccggact tcaccttcgg ttttgaagaa     600
   tccctggaag tagacacgaa cccactgctg ggcgctggta aattcgcaac tgatcctgcg     660
   gttaccctgg ctcacgaact gattcatgca ggccaccgcc tgtacggtat cgccatcaat     720
   ccgaaccgtg tcttcaaagt taacaccaac gcgtattacg agatgtccgg tctggaagtt     780
   agcttcgaag aactgcgtac ttttggcggt cacgacgcta aattcatcga ctctctgcaa     840
   gaaaacgagt tccgtctgta ctactataac aagttcaaag atatcgcatc caccctgaac     900
   aaagcgaaat ccatcgtggg taccactgct tctctccagt acatgaagaa cgtttttaaa     960
   gaaaaatacc tgctcagcga agacacctcc ggcaaattct ctgtagacaa gttgaaattc    1020
   gataaacttt acaaaatgct gactgaaatt tacaccgaag acaacttcgt taagttcttt    1080
   aaagttctga accgcaaaac ctatctgaac ttcgacaagg cagtattcaa aatcaacatc    1140
   gtgccgaaag ttaactacac tatctacgat ggtttcaacc tgcgtaacac caacctggct    1200
   gctaatttta acggccagaa cacggaaatc aacaacatga acttcacaaa actgaaaaac    1260
   ttcactggtc tgttcgagtt ttacaagctg ctgtgcgtcg acggcatcat tacctccaaa    1320
   actaaatctc tgatagaagg tagatttggc ggtttcacgg gcgcacgcaa atcatatgcg    1380
   ctagcgggcg gtggcggtag cggcggtggc ggtagcggcg gtggcggtag cgcactagtg    1440
   ctgcagtgta tcaaggttaa caactgggat ttattcttca gcccgagtga agacaacttc    1500
   accaacgacc tgaacaaagg tgaagaaatc acctcagata ctaacatcga agcagccgaa    1560
   gaaaacatct cgctggacct gatccagcag tactacctga cctttaattt cgacaacgag    1620
   ccggaaaaca tttctatcga aaacctgagc tctgatatca tcggccagct ggaactgatg    1680
   ccgaacatcg aacgtttccc aaacggtaaa aagtacgagc tggacaaata taccatgttc    1740
   cactacctgc gcgcgcagga atttgaacac ggcaaatccc gtatcgcact gactaactcc    1800
   gttaacgaag ctctgctcaa cccgtcccgt gtatacacct tcttctctag cgactacgtg    1860
   aaaaaggtca acaaagcgac tgaagctgca atgttcttgg gttgggttga acagcttgtt 1920
   tatgatttta ccgacgagac gtccgaagta tctactaccg acaaaattgc ggatatcact 1980
   atcatcatcc cgtacatcgg tccggctctg aacattggca acatgctgta caaagacgac 2040
   ttcgttggcg cactgatctt ctccggtgcg gtgatcctgc tggagttcat cccggaaatc 2100
   gccatcccgg tactgggcac ctttgctctg gtttcttaca ttgcaaacaa ggttctgact 2160
   gtacaaacca tcgacaacgc gctgagcaaa cgtaacgaaa aatgggatga agtttacaaa 2220
   tatatcgtga ccaactggct ggctaaggtt aatactcaga tcgacctcat ccgcaaaaaa 2280
   atgaaagaag cactggaaaa ccaggcggaa gctaccaagg caatcattaa ctaccagtac 2340
   aaccagtaca ccgaggaaga aaaaaacaac atcaacttca acatcgacga tctgtcctct 2400
   aaactgaacg aatccatcaa caaagctatg atcaacatca acaagttcct gaaccagtgc 2460
   tctgtaagct atctgatgaa ctccatgatc ccgtacggtg ttaaacgtct ggaggacttc 2520
   gatgcgtctc tgaaagacgc cctgctgaaa tacatttacg acaaccgtgg cactctgatc 2580
   ggtcaggttg atcgtctgaa ggacaaagtg aacaatacct tatcgaccga catccctttt 2640
   cagctcagta aatatgtcga taaccaacgc cttttgtcca ctctagacta g 2691
<210> 58
   <211> 896
   <212> PRT
   <213> Artificial Sequence
<220>
   <223> Synthetic
<400> 58
   Gly Ser Met Glu Phe Val Asn Lys Gln Phe Asn Tyr Lys Asp Pro Val
   1               5                   10                  15
   Asn Gly Val Asp Ile Ala Tyr Ile Lys Ile Pro Asn Ala Gly Gln Met
               20                  25                  30
   Gln Pro Val Lys Ala Phe Lys Ile His Asn Lys Ile Trp Val Ile Pro
           35                  40                  45
   Glu Arg Asp Thr Phe Thr Asn Pro Glu Glu Gly Asp Leu Asn Pro Pro
       50                  55                  60
   Pro Glu Ala Lys Gln Val Pro Val Ser Tyr Tyr Asp Ser Thr Tyr Leu
   65                  70                  75                  80
   Ser Thr Asp Asn Glu Lys Asp Asn Tyr Leu Lys Gly Val Thr Lys Leu
                   85                  90                  95
   Phe Glu Arg Ile Tyr Ser Thr Asp Leu Gly Arg Met Leu Leu Thr Ser
   100 105 110
   Ile Val Arg Gly Ile Pro Phe Trp Gly Gly Ser Thr Ile Asp Thr Glu
   115 120 125
   Leu Lys Val Ile Asp Thr Asn Cys Ile Asn Val Ile Gln Pro Asp Gly
   130 135 140
   Ser Tyr Arg Ser Glu Glu Leu Asn Leu Val Ile Ile Gly Pro Ser Ala
   145 150 155 160
   Asp Ile Ile Gln Phe Glu Cys Lys Ser Phe Gly His Glu Val Leu Asn
   165 170 175
   Leu Thr Arg Asn Gly Tyr Gly Ser Thr Gln Tyr Ile Arg Phe Ser Pro
   180 185 190
   Asp Phe Thr Phe Gly Phe Glu Glu Ser Leu Glu Val Asp Thr Asn Pro
   195 200 205
   Leu Leu Gly Ala Gly Lys Phe Ala Thr Asp Pro Ala Val Thr Leu Ala
   210 215 220
   His Glu Leu Ile His Ala Gly His Arg Leu Tyr Gly Ile Ala Ile Asn
   225 230 235 240
   Pro Asn Arg Val Phe Lys Val Asn Thr Asn Ala Tyr Tyr Glu Met Ser
   245 250 255
   Gly Leu Glu Val Ser Phe Glu Glu Leu Arg Thr Phe Gly Gly His Asp
   260 265 270
   Ala Lys Phe Ile Asp Ser Leu Gln Glu Asn Glu Phe Arg Leu Tyr Tyr
   275 280 285
   Tyr Asn Lys Phe Lys Asp Ile Ala Ser Thr Leu Asn Lys Ala Lys Ser
   290 295 300
   Ile Val Gly Thr Thr Ala Ser Leu Gln Tyr Met Lys Asn Val Phe Lys
   305 310 315 320
   Glu Lys Tyr Leu Leu Ser Glu Asp Thr Ser Gly Lys Phe Ser Val Asp
   325 330 335
   Lys Leu Lys Phe Asp Lys Leu Tyr Lys Met Leu Thr Glu Ile Tyr Thr
   340 345 350
   Glu Asp Asn Phe Val Lys Phe Phe Lys Val Leu Asn Arg Lys Thr Tyr
   355 360 365
   Leu Asn Phe Asp Lys Ala Val Phe Lys Ile Asn Ile Val Pro Lys Val
   370 375 380
   Asn Tyr Thr Ile Tyr Asp Gly Phe Asn Leu Arg Asn Thr Asn Leu Ala
   385 390 395 400
   Ala Asn Phe Asn Gly Gln Asn Thr Glu Ile Asn Asn Met Asn Phe Thr
   405 410 415
   Lys Leu Lys Asn Phe Thr Gly Leu Phe Glu Phe Tyr Lys Leu Leu Cys
   420 425 430
   Val Asp Gly Ile Ile Thr Ser Lys Thr Lys Ser Leu Ile Glu Gly Arg
   435 440 445
   Phe Gly Gly Phe Thr Gly Ala Arg Lys Ser Tyr Ala Leu Ala Gly Gly
   450 455 460
   Gly Gly Ser Gly Gly Gly Gly Ser Gly Gly Gly Gly Ser Ala Leu Val
   465 470 475 480
   Leu Gln Cys Ile Lys Val Asn Asn Trp Asp Leu Phe Phe Ser Pro Ser
   485 490 495
   Glu Asp Asn Phe Thr Asn Asp Leu Asn Lys Gly Glu Glu Ile Thr Ser
   500 505 510
   Asp Thr Asn Ile Glu Ala Ala Glu Glu Asn Ile Ser Leu Asp Leu Ile
   515 520 525
   Gln Gln Tyr Tyr Leu Thr Phe Asn Phe Asp Asn Glu Pro Glu Asn Ile
   530 535 540
   Ser Ile Glu Asn Leu Ser Ser Asp Ile Ile Gly Gln Leu Glu Leu Met
   545 550 555 560
   Pro Asn Ile Glu Arg Phe Pro Asn Gly Lys Lys Tyr Glu Leu Asp Lys
   565 570 575
   Tyr Thr Met Phe His Tyr Leu Arg Ala Gln Glu Phe Glu His Gly Lys
   580 585 590
   Ser Arg Ile Ala Leu Thr Asn Ser Val Asn Glu Ala Leu Leu Asn Pro
   595 600 605
   Ser Arg Val Tyr Thr Phe Phe Ser Ser Asp Tyr Val Lys Lys Val Asn
   610 615 620
   Lys Ala Thr Glu Ala Ala Met Phe Leu Gly Trp Val Glu Gln Leu Val
   625 630 635 640
   Tyr Asp Phe Thr Asp Glu Thr Ser Glu Val Ser Thr Thr Asp Lys Ile
   645 650 655
   Ala Asp Ile Thr Ile Ile Ile Pro Tyr Ile Gly Pro Ala Leu Asn Ile
   660 665 670
   Gly Asn Met Leu Tyr Lys Asp Asp Phe Val Gly Ala Leu Ile Phe Ser
   675 680 685
   Gly Ala Val Ile Leu Leu Glu Phe Ile Pro Glu Ile Ala Ile Pro Val
   690 695 700
   Leu Gly Thr Phe Ala Leu Val Ser Tyr Ile Ala Asn Lys Val Leu Thr
   705 710 715 720
   Val Gln Thr Ile Asp Asn Ala Leu Ser Lys Arg Asn Glu Lys Trp Asp
   725 730 735
   Glu Val Tyr Lys Tyr Ile Val Thr Asn Trp Leu Ala Lys Val Asn Thr
   740 745 750
   Gln Ile Asp Leu Ile Arg Lys Lys Met Lys Glu Ala Leu Glu Asn Gln
   755 760 765
   Ala Glu Ala Thr Lys Ala Ile Ile Asn Tyr Gln Tyr Asn Gln Tyr Thr
   770 775 780
   Glu Glu Glu Lys Asn Asn Ile Asn Phe Asn Ile Asp Asp Leu Ser Ser
   785 790 795 800
   Lys Leu Asn Glu Ser Ile Asn Lys Ala Met Ile Asn Ile Asn Lys Phe
   805 810 815
   Leu Asn Gln Cys Ser Val Ser Tyr Leu Met Asn Ser Met Ile Pro Tyr
   820 825 830
   Gly Val Lys Arg Leu Glu Asp Phe Asp Ala Ser Leu Lys Asp Ala Leu
   835 840 845
   Leu Lys Tyr Ile Tyr Asp Asn Arg Gly Thr Leu Ile Gly Gln Val Asp
   850 855 860
   Arg Leu Lys Asp Lys Val Asn Asn Thr Leu Ser Thr Asp Ile Pro Phe
   865 870 875 880
   Gln Leu Ser Lys Tyr Val Asp Asn Gln Arg Leu Leu Ser Thr Leu Asp
   885 890 895
<210> 59
   <211> 2709
   <212> DNA
   <213> Artificial Sequence
<220>
   <223> Synthetic
<400> 59
   ggatccatgg agttcgttaa caaacagttc aactataaag acccagttaa cggtgttgac      60
   attgcttaca tcaaaatccc gaacgctggc cagatgcagc cggtaaaggc attcaaaatc     120
   cacaacaaaa tctgggttat cccggaacgt gataccttta ctaacccgga agaaggtgac     180
   ctgaacccgc caccggaagc gaaacaggtg ccggtatctt actatgactc cacctacctg     240
   tctaccgata acgaaaagga caactacctg aaaggtgtta ctaaactgtt cgagcgtatt     300
   tactccaccg acctgggccg tatgctgctg actagcatcg ttcgcggtat cccgttctgg     360
   ggcggttcta ccatcgatac cgaactgaaa gtaatcgaca ctaactgcat caacgttatt     420
   cagccggacg gttcctatcg ttccgaagaa ctgaacctgg tgatcatcgg cccgtctgct     480
   gatatcatcc agttcgagtg taagagcttt ggtcacgaag ttctgaacct cacccgtaac     540
   ggctacggtt ccactcagta catccgtttc tctccggact tcaccttcgg ttttgaagaa     600
   tccctggaag tagacacgaa cccactgctg ggcgctggta aattcgcaac tgatcctgcg     660
   gttaccctgg ctcacgaact gattcatgca ggccaccgcc tgtacggtat cgccatcaat     720
   ccgaaccgtg tcttcaaagt taacaccaac gcgtattacg agatgtccgg tctggaagtt     780
   agcttcgaag aactgcgtac ttttggcggt cacgacgcta aattcatcga ctctctgcaa     840
   gaaaacgagt tccgtctgta ctactataac aagttcaaag atatcgcatc caccctgaac     900
   aaagcgaaat ccatcgtggg taccactgct tctctccagt acatgaagaa cgtttttaaa     960
   gaaaaatacc tgctcagcga agacacctcc ggcaaattct ctgtagacaa gttgaaattc    1020
   gataaacttt acaaaatgct gactgaaatt tacaccgaag acaacttcgt taagttcttt 1080
   aaagttctga accgcaaaac ctatctgaac ttcgacaagg cagtattcaa aatcaacatc 1140
   gtgccgaaag ttaactacac tatctacgat ggtttcaacc tgcgtaacac caacctggct 1200
   gctaatttta acggccagaa cacggaaatc aacaacatga acttcacaaa actgaaaaac 1260
   ttcactggtc tgttcgagtt ttacaagctg ctgtgcgtcg acggcatcat tacctccaaa 1320
   actaaatctc tgatagaagg tagatttggc ggtttcacgg gcgcacgcaa atcagcgcgt 1380
   aaatatgcta accaggcgct agcgggcggt ggcggtagcg gcggtggcgg tagcggcggt 1440
   ggcggtagcg cactagtgct gcagtgtatc aaggttaaca actgggattt attcttcagc 1500
   ccgagtgaag acaacttcac caacgacctg aacaaaggtg aagaaatcac ctcagatact 1560
   aacatcgaag cagccgaaga aaacatctcg ctggacctga tccagcagta ctacctgacc 1620
   tttaatttcg acaacgagcc ggaaaacatt tctatcgaaa acctgagctc tgatatcatc 1680
   ggccagctgg aactgatgcc gaacatcgaa cgtttcccaa acggtaaaaa gtacgagctg 1740
   gacaaatata ccatgttcca ctacctgcgc gcgcaggaat ttgaacacgg caaatcccgt 1800
   atcgcactga ctaactccgt taacgaagct ctgctcaacc cgtcccgtgt atacaccttc 1860
   ttctctagcg actacgtgaa aaaggtcaac aaagcgactg aagctgcaat gttcttgggt 1920
   tgggttgaac agcttgttta tgattttacc gacgagacgt ccgaagtatc tactaccgac 1980
   aaaattgcgg atatcactat catcatcccg tacatcggtc cggctctgaa cattggcaac 2040
   atgctgtaca aagacgactt cgttggcgca ctgatcttct ccggtgcggt gatcctgctg 2100
   gagttcatcc cggaaatcgc catcccggta ctgggcacct ttgctctggt ttcttacatt 2160
   gcaaacaagg ttctgactgt acaaaccatc gacaacgcgc tgagcaaacg taacgaaaaa 2220
   tgggatgaag tttacaaata tatcgtgacc aactggctgg ctaaggttaa tactcagatc 2280
   gacctcatcc gcaaaaaaat gaaagaagca ctggaaaacc aggcggaagc taccaaggca 2340
   atcattaact accagtacaa ccagtacacc gaggaagaaa aaaacaacat caacttcaac 2400
   atcgacgatc tgtcctctaa actgaacgaa tccatcaaca aagctatgat caacatcaac 2460
   aagttcctga accagtgctc tgtaagctat ctgatgaact ccatgatccc gtacggtgtt 2520
   aaacgtctgg aggacttcga tgcgtctctg aaagacgccc tgctgaaata catttacgac 2580
   aaccgtggca ctctgatcgg tcaggttgat cgtctgaagg acaaagtgaa caatacctta 2640
   tcgaccgaca tcccttttca gctcagtaaa tatgtcgata accaacgcct tttgtccact 2700
   ctagactag 2709
<210> 60
   <211> 902
   <212> PRT
   <213> Artificial Sequence
<220>
   <223> Synthetic
<400> 60
   Gly Ser Met Glu Phe Val Asn Lys Gln Phe Asn Tyr Lys Asp Pro Val
   1               5                   10                  15
   Asn Gly Val Asp Ile Ala Tyr Ile Lys Ile Pro Asn Ala Gly Gln Met
               20                  25                  30
   Gln Pro Val Lys Ala Phe Lys Ile His Asn Lys Ile Trp Val Ile Pro
           35                  40                  45
   Glu Arg Asp Thr Phe Thr Asn Pro Glu Glu Gly Asp Leu Asn Pro Pro
       50                  55                  60
   Pro Glu Ala Lys Gln Val Pro Val Ser Tyr Tyr Asp Ser Thr Tyr Leu
   65                  70                  75                  80
   Ser Thr Asp Asn Glu Lys Asp Asn Tyr Leu Lys Gly Val Thr Lys Leu
                   85                  90                  95
   Phe Glu Arg Ile Tyr Ser Thr Asp Leu Gly Arg Met Leu Leu Thr Ser
               100                 105                 110
   Ile Val Arg Gly Ile Pro Phe Trp Gly Gly Ser Thr Ile Asp Thr Glu
           115                 120                 125
   Leu Lys Val Ile Asp Thr Asn Cys Ile Asn Val Ile Gln Pro Asp Gly
       130                 135                 140
   Ser Tyr Arg Ser Glu Glu Leu Asn Leu Val Ile Ile Gly Pro Ser Ala
   145                 150                 155                 160
   Asp Ile Ile Gln Phe Glu Cys Lys Ser Phe Gly His Glu Val Leu Asn
                   165                 170                 175
   Leu Thr Arg Asn Gly Tyr Gly Ser Thr Gln Tyr Ile Arg Phe Ser Pro
               180                 185                 190
   Asp Phe Thr Phe Gly Phe Glu Glu Ser Leu Glu Val Asp Thr Asn Pro
           195                 200                 205
   Leu Leu Gly Ala Gly Lys Phe Ala Thr Asp Pro Ala Val Thr Leu Ala
       210                 215                 220
   His Glu Leu Ile His Ala Gly His Arg Leu Tyr Gly Ile Ala Ile Asn
   225                 230                 235                 240
   Pro Asn Arg Val Phe Lys Val Asn Thr Asn Ala Tyr Tyr Glu Met Ser
                   245                 250                 255
   Gly Leu Glu Val Ser Phe Glu Glu Leu Arg Thr Phe Gly Gly His Asp
               260                 265                 270
   Ala Lys Phe Ile Asp Ser Leu Gln Glu Asn Glu Phe Arg Leu Tyr Tyr
           275                 280                 285
   Tyr Asn Lys Phe Lys Asp Ile Ala Ser Thr Leu Asn Lys Ala Lys Ser
       290                 295                 300
   Ile Val Gly Thr Thr Ala Ser Leu Gln Tyr Met Lys Asn Val Phe Lys
   305                 310                 315                 320
   Glu Lys Tyr Leu Leu Ser Glu Asp Thr Ser Gly Lys Phe Ser Val Asp
                   325                 330                 335
   Lys Leu Lys Phe Asp Lys Leu Tyr Lys Met Leu Thr Glu Ile Tyr Thr
               340                 345                 350
   Glu Asp Asn Phe Val Lys Phe Phe Lys Val Leu Asn Arg Lys Thr Tyr
           355                 360                 365
   Leu Asn Phe Asp Lys Ala Val Phe Lys Ile Asn Ile Val Pro Lys Val
       370                 375                 380
   Asn Tyr Thr Ile Tyr Asp Gly Phe Asn Leu Arg Asn Thr Asn Leu Ala
   385                 390                 395                 400
   Ala Asn Phe Asn Gly Gln Asn Thr Glu Ile Asn Asn Met Asn Phe Thr
                   405                 410                 415
   Lys Leu Lys Asn Phe Thr Gly Leu Phe Glu Phe Tyr Lys Leu Leu Cys
               420                 425                 430
   Val Asp Gly Ile Ile Thr Ser Lys Thr Lys Ser Leu Ile Glu Gly Arg
           435                 440                 445
   Phe Gly Gly Phe Thr Gly Ala Arg Lys Ser Ala Arg Lys Tyr Ala Asn
   450 455 460
   Gln Ala Leu Ala Gly Gly Gly Gly Ser Gly Gly Gly Gly Ser Gly Gly
   465 470 475 480
   Gly Gly Ser Ala Leu Val Leu Gln Cys Ile Lys Val Asn Asn Trp Asp
   485 490 495
   Leu Phe Phe Ser Pro Ser Glu Asp Asn Phe Thr Asn Asp Leu Asn Lys
   500 505 510
   Gly Glu Glu Ile Thr Ser Asp Thr Asn Ile Glu Ala Ala Glu Glu Asn
   515 520 525
   Ile Ser Leu Asp Leu Ile Gln Gln Tyr Tyr Leu Thr Phe Asn Phe Asp
   530 535 540
   Asn Glu Pro Glu Asn Ile Ser Ile Glu Asn Leu Ser Ser Asp Ile Ile
   545 550 555 560
   Gly Gln Leu Glu Leu Met Pro Asn Ile Glu Arg Phe Pro Asn Gly Lys
   565 570 575
   Lys Tyr Glu Leu Asp Lys Tyr Thr Met Phe His Tyr Leu Arg Ala Gln
   580 585 590
   Glu Phe Glu His Gly Lys Ser Arg Ile Ala Leu Thr Asn Ser Val Asn
   595 600 605
   Glu Ala Leu Leu Asn Pro Ser Arg Val Tyr Thr Phe Phe Ser Ser Asp
   610 615 620
   Tyr Val Lys Lys Val Asn Lys Ala Thr Glu Ala Ala Met Phe Leu Gly
   625 630 635 640
   Trp Val Glu Gln Leu Val Tyr Asp Phe Thr Asp Glu Thr Ser Glu Val
   645 650 655
   Ser Thr Thr Asp Lys Ile Ala Asp Ile Thr Ile Ile Ile Pro Tyr Ile
   660 665 670
   Gly Pro Ala Leu Asn Ile Gly Asn Met Leu Tyr Lys Asp Asp Phe Val
   675 680 685
   Gly Ala Leu Ile Phe Ser Gly Ala Val Ile Leu Leu Glu Phe Ile Pro
   690 695 700
   Glu Ile Ala Ile Pro Val Leu Gly Thr Phe Ala Leu Val Ser Tyr Ile
   705 710 715 720
   Ala Asn Lys Val Leu Thr Val Gln Thr Ile Asp Asn Ala Leu Ser Lys
   725 730 735
   Arg Asn Glu Lys Trp Asp Glu Val Tyr Lys Tyr Ile Val Thr Asn Trp
   740 745 750
   Leu Ala Lys Val Asn Thr Gln Ile Asp Leu Ile Arg Lys Lys Met Lys
   755 760 765
   Glu Ala Leu Glu Asn Gln Ala Glu Ala Thr Lys Ala Ile Ile Asn Tyr
   770 775 780
   Gln Tyr Asn Gln Tyr Thr Glu Glu Glu Lys Asn Asn Ile Asn Phe Asn
   785 790 795 800
   Ile Asp Asp Leu Ser Ser Lys Leu Asn Glu Ser Ile Asn Lys Ala Met
   805 810 815
   Ile Asn Ile Asn Lys Phe Leu Asn Gln Cys Ser Val Ser Tyr Leu Met
   820 825 830
   Asn Ser Met Ile Pro Tyr Gly Val Lys Arg Leu Glu Asp Phe Asp Ala
   835 840 845
   Ser Leu Lys Asp Ala Leu Leu Lys Tyr Ile Tyr Asp Asn Arg Gly Thr
   850 855 860
   Leu Ile Gly Gln Val Asp Arg Leu Lys Asp Lys Val Asn Asn Thr Leu
   865 870 875 880
   Ser Thr Asp Ile Pro Phe Gln Leu Ser Lys Tyr Val Asp Asn Gln Arg
   885 890 895
   Leu Leu Ser Thr Leu Asp
   900
<210> 61
   <211> 2697
   <212> DNA
   <213> Artificial Sequence
<220>
   <223> Synthetic
<400> 61
   ggatccatgg agttcgttaa caaacagttc aactataaag acccagttaa cggtgttgac      60
   attgcttaca tcaaaatccc gaacgctggc cagatgcagc cggtaaaggc attcaaaatc     120
   cacaacaaaa tctgggttat cccggaacgt gataccttta ctaacccgga agaaggtgac     180
   ctgaacccgc caccggaagc gaaacaggtg ccggtatctt actatgactc cacctacctg     240
   tctaccgata acgaaaagga caactacctg aaaggtgtta ctaaactgtt cgagcgtatt     300
   tactccaccg acctgggccg tatgctgctg actagcatcg ttcgcggtat cccgttctgg     360
   ggcggttcta ccatcgatac cgaactgaaa gtaatcgaca ctaactgcat caacgttatt     420
   cagccggacg gttcctatcg ttccgaagaa ctgaacctgg tgatcatcgg cccgtctgct     480
   gatatcatcc agttcgagtg taagagcttt ggtcacgaag ttctgaacct cacccgtaac     540
   ggctacggtt ccactcagta catccgtttc tctccggact tcaccttcgg ttttgaagaa     600
   tccctggaag tagacacgaa cccactgctg ggcgctggta aattcgcaac tgatcctgcg     660
   gttaccctgg ctcacgaact gattcatgca ggccaccgcc tgtacggtat cgccatcaat     720
   ccgaaccgtg tcttcaaagt taacaccaac gcgtattacg agatgtccgg tctggaagtt     780
   agcttcgaag aactgcgtac ttttggcggt cacgacgcta aattcatcga ctctctgcaa     840
   gaaaacgagt tccgtctgta ctactataac aagttcaaag atatcgcatc caccctgaac     900
   aaagcgaaat ccatcgtggg taccactgct tctctccagt acatgaagaa cgtttttaaa     960
   gaaaaatacc tgctcagcga agacacctcc ggcaaattct ctgtagacaa gttgaaattc    1020
   gataaacttt acaaaatgct gactgaaatt tacaccgaag acaacttcgt taagttcttt    1080
   aaagttctga accgcaaaac ctatctgaac ttcgacaagg cagtattcaa aatcaacatc    1140
   gtgccgaaag ttaactacac tatctacgat ggtttcaacc tgcgtaacac caacctggct    1200
   gctaatttta acggccagaa cacggaaatc aacaacatga acttcacaaa actgaaaaac    1260
   ttcactggtc tgttcgagtt ttacaagctg ctgtgcgtcg acggcatcat tacctccaaa    1320
   actaaatctc tgatagaagg tagatttggc ggtttcacgg gcgcacgcaa atcagcgcgt    1380
   aaagcgctag cgggcggtgg cggtagcggc ggtggcggta gcggcggtgg cggtagcgca    1440
   ctagtgctgc agtgtatcaa ggttaacaac tgggatttat tcttcagccc gagtgaagac    1500
   aacttcacca acgacctgaa caaaggtgaa gaaatcacct cagatactaa catcgaagca    1560
   gccgaagaaa acatctcgct ggacctgatc cagcagtact acctgacctt taatttcgac    1620
   aacgagccgg aaaacatttc tatcgaaaac ctgagctctg atatcatcgg ccagctggaa    1680
   ctgatgccga acatcgaacg tttcccaaac ggtaaaaagt acgagctgga caaatatacc    1740
   atgttccact acctgcgcgc gcaggaattt gaacacggca aatcccgtat cgcactgact    1800
   aactccgtta acgaagctct gctcaacccg tcccgtgtat acaccttctt ctctagcgac   1860
   tacgtgaaaa aggtcaacaa agcgactgaa gctgcaatgt tcttgggttg ggttgaacag   1920
   cttgtttatg attttaccga cgagacgtcc gaagtatcta ctaccgacaa aattgcggat   1980
   atcactatca tcatcccgta catcggtccg gctctgaaca ttggcaacat gctgtacaaa   2040
   gacgacttcg ttggcgcact gatcttctcc ggtgcggtga tcctgctgga gttcatcccg   2100
   gaaatcgcca tcccggtact gggcaccttt gctctggttt cttacattgc aaacaaggtt   2160
   ctgactgtac aaaccatcga caacgcgctg agcaaacgta acgaaaaatg ggatgaagtt   2220
   tacaaatata tcgtgaccaa ctggctggct aaggttaata ctcagatcga cctcatccgc   2280
   aaaaaaatga aagaagcact ggaaaaccag gcggaagcta ccaaggcaat cattaactac   2340
   cagtacaacc agtacaccga ggaagaaaaa aacaacatca acttcaacat cgacgatctg   2400
   tcctctaaac tgaacgaatc catcaacaaa gctatgatca acatcaacaa gttcctgaac   2460
   cagtgctctg taagctatct gatgaactcc atgatcccgt acggtgttaa acgtctggag   2520
   gacttcgatg cgtctctgaa agacgccctg ctgaaataca tttacgacaa ccgtggcact   2580
   ctgatcggtc aggttgatcg tctgaaggac aaagtgaaca ataccttatc gaccgacatc   2640
   ccttttcagc tcagtaaata tgtcgataac caacgccttt tgtccactct agactag      2697
<210> 62
   <211> 898
   <212> PRT
   <213> Artificial Sequence
<220>
   <223> Synthetic
<400> 62
   Gly Ser Met Glu Phe Val Asn Lys Gln Phe Asn Tyr Lys Asp Pro Val
   1               5                   10                  15
   Asn Gly Val Asp Ile Ala Tyr Ile Lys Ile Pro Asn Ala Gly Gln Met
               20                  25                  30
   Gln Pro Val Lys Ala Phe Lys Ile His Asn Lys Ile Trp Val Ile Pro
           35                  40                  45
   Glu Arg Asp Thr Phe Thr Asn Pro Glu Glu Gly Asp Leu Asn Pro Pro
       50                  55                  60
   Pro Glu Ala Lys Gln Val Pro Val Ser Tyr Tyr Asp Ser Thr Tyr Leu
   65                  70                  75                  80
   Ser Thr Asp Asn Glu Lys Asp Asn Tyr Leu Lys Gly Val Thr Lys Leu
                   85                  90                  95
   Phe Glu Arg Ile Tyr Ser Thr Asp Leu Gly Arg Met Leu Leu Thr Ser
               100                 105                 110
   Ile Val Arg Gly Ile Pro Phe Trp Gly Gly Ser Thr Ile Asp Thr Glu
           115                 120                 125
   Leu Lys Val Ile Asp Thr Asn Cys Ile Asn Val Ile Gln Pro Asp Gly
       130                 135                 140
   Ser Tyr Arg Ser Glu Glu Leu Asn Leu Val Ile Ile Gly Pro Ser Ala
   145                 150                 155                 160
   Asp Ile Ile Gln Phe Glu Cys Lys Ser Phe Gly His Glu Val Leu Asn
                   165                 170                 175
   Leu Thr Arg Asn Gly Tyr Gly Ser Thr Gln Tyr Ile Arg Phe Ser Pro
               180                 185                 190
   Asp Phe Thr Phe Gly Phe Glu Glu Ser Leu Glu Val Asp Thr Asn Pro
           195                 200                 205
   Leu Leu Gly Ala Gly Lys Phe Ala Thr Asp Pro Ala Val Thr Leu Ala
       210                 215                 220
   His Glu Leu Ile His Ala Gly His Arg Leu Tyr Gly Ile Ala Ile Asn
   225                 230                 235                 240
   Pro Asn Arg Val Phe Lys Val Asn Thr Asn Ala Tyr Tyr Glu Met Ser
                   245                 250                 255
   Gly Leu Glu Val Ser Phe Glu Glu Leu Arg Thr Phe Gly Gly His Asp
               260                 265                 270
   Ala Lys Phe Ile Asp Ser Leu Gln Glu Asn Glu Phe Arg Leu Tyr Tyr
           275                 280                 285
   Tyr Asn Lys Phe Lys Asp Ile Ala Ser Thr Leu Asn Lys Ala Lys Ser
       290                 295                 300
   Ile Val Gly Thr Thr Ala Ser Leu Gln Tyr Met Lys Asn Val Phe Lys
   305                 310                 315                 320
   Glu Lys Tyr Leu Leu Ser Glu Asp Thr Ser Gly Lys Phe Ser Val Asp
   325 330 335
   Lys Leu Lys Phe Asp Lys Leu Tyr Lys Met Leu Thr Glu Ile Tyr Thr
   340 345 350
   Glu Asp Asn Phe Val Lys Phe Phe Lys Val Leu Asn Arg Lys Thr Tyr
   355 360 365
   Leu Asn Phe Asp Lys Ala Val Phe Lys Ile Asn Ile Val Pro Lys Val
   370 375 380
   Asn Tyr Thr Ile Tyr Asp Gly Phe Asn Leu Arg Asn Thr Asn Leu Ala
   385 390 395 400
   Ala Asn Phe Asn Gly Gln Asn Thr Glu Ile Asn Asn Met Asn Phe Thr
   405 410 415
   Lys Leu Lys Asn Phe Thr Gly Leu Phe Glu Phe Tyr Lys Leu Leu Cys
   420 425 430
   Val Asp Gly Ile Ile Thr Ser Lys Thr Lys Ser Leu Ile Glu Gly Arg
   435 440 445
   Phe Gly Gly Phe Thr Gly Ala Arg Lys Ser Ala Arg Lys Ala Leu Ala
   450 455 460
   Gly Gly Gly Gly Ser Gly Gly Gly Gly Ser Gly Gly Gly Gly Ser Ala
   465 470 475 480
   Leu Val Leu Gln Cys Ile Lys Val Asn Asn Trp Asp Leu Phe Phe Ser
   485 490 495
   Pro Ser Glu Asp Asn Phe Thr Asn Asp Leu Asn Lys Gly Glu Glu Ile
   500 505 510
   Thr Ser Asp Thr Asn Ile Glu Ala Ala Glu Glu Asn Ile Ser Leu Asp
   515 520 525
   Leu Ile Gln Gln Tyr Tyr Leu Thr Phe Asn Phe Asp Asn Glu Pro Glu
   530 535 540
   Asn Ile Ser Ile Glu Asn Leu Ser Ser Asp Ile Ile Gly Gln Leu Glu
   545 550 555 560
   Leu Met Pro Asn Ile Glu Arg Phe Pro Asn Gly Lys Lys Tyr Glu Leu
   565 570 575
   Asp Lys Tyr Thr Met Phe His Tyr Leu Arg Ala Gln Glu Phe Glu His
   580 585 590
   Gly Lys Ser Arg Ile Ala Leu Thr Asn Ser Val Asn Glu Ala Leu Leu
   595 600 605
   Asn Pro Ser Arg Val Tyr Thr Phe Phe Ser Ser Asp Tyr Val Lys Lys
   610 615 620
   Val Asn Lys Ala Thr Glu Ala Ala Met Phe Leu Gly Trp Val Glu Gln
   625 630 635 640
   Leu Val Tyr Asp Phe Thr Asp Glu Thr Ser Glu Val Ser Thr Thr Asp
   645 650 655
   Lys Ile Ala Asp Ile Thr Ile Ile Ile Pro Tyr Ile Gly Pro Ala Leu
   660 665 670
   Asn Ile Gly Asn Met Leu Tyr Lys Asp Asp Phe Val Gly Ala Leu Ile
   675 680 685
   Phe Ser Gly Ala Val Ile Leu Leu Glu Phe Ile Pro Glu Ile Ala Ile
   690 695 700
   Pro Val Leu Gly Thr Phe Ala Leu Val Ser Tyr Ile Ala Asn Lys Val
   705 710 715 720
   Leu Thr Val Gln Thr Ile Asp Asn Ala Leu Ser Lys Arg Asn Glu Lys
   725 730 735
   Trp Asp Glu Val Tyr Lys Tyr Ile Val Thr Asn Trp Leu Ala Lys Val
   740 745 750
   Asn Thr Gln Ile Asp Leu Ile Arg Lys Lys Met Lys Glu Ala Leu Glu
   755 760 765
   Asn Gln Ala Glu Ala Thr Lys Ala Ile Ile Asn Tyr Gln Tyr Asn Gln
   770 775 780
   Tyr Thr Glu Glu Glu Lys Asn Asn Ile Asn Phe Asn Ile Asp Asp Leu
   785 790 795 800
   Ser Ser Lys Leu Asn Glu Ser Ile Asn Lys Ala Met Ile Asn Ile Asn
   805 810 815
   Lys Phe Leu Asn Gln Cys Ser Val Ser Tyr Leu Met Asn Ser Met Ile
   820 825 830
   Pro Tyr Gly Val Lys Arg Leu Glu Asp Phe Asp Ala Ser Leu Lys Asp
   835 840 845
   Ala Leu Leu Lys Tyr Ile Tyr Asp Asn Arg Gly Thr Leu Ile Gly Gln
   850 855 860
   Val Asp Arg Leu Lys Asp Lys Val Asn Asn Thr Leu Ser Thr Asp Ile
   865 870 875 880
   Pro Phe Gln Leu Ser Lys Tyr Val Asp Asn Gln Arg Leu Leu Ser Thr
   885 890 895
   Leu Asp
<210> 63
   <211> 2736
   <212> DNA
   <213> Artificial Sequence
<220>
   <223> Synthetic
<400> 63
   ctcgggattg agggtcgttt tggcggtttc acgggcgcac gcaaatcagc gcgtaaatta 60
   gctaaccaga ctagtggcgg tgggggtagt ggcggtggcg gttcgggcgg gggtgggagc 120
   cctaggggat ccatggagtt cgttaacaaa cagttcaact ataaagaccc agttaacggt 180
   gttgacattg cttacatcaa aatcccgaac gctggccaga tgcagccggt aaaggcattc 240
   aaaatccaca acaaaatctg ggttatcccg gaacgtgata cctttactaa cccggaagaa 300
   ggtgacctga acccgccacc ggaagcgaaa caggtgccgg tatcttacta tgactccacc 360
   tacctgtcta ccgataacga aaaggacaac tacctgaaag gtgttactaa actgttcgag 420
   cgtatttact ccaccgacct gggccgtatg ctgctgacta gcatcgttcg cggtatcccg 480
   ttctggggcg gttctaccat cgataccgaa ctgaaagtaa tcgacactaa ctgcatcaac 540
   gttattcagc cggacggttc ctatcgttcc gaagaactga acctggtgat catcggcccg 600
   tctgctgata tcatccagtt cgagtgtaag agctttggtc acgaagttct gaacctcacc 660
   cgtaacggct acggttccac tcagtacatc cgtttctctc cggacttcac cttcggtttt 720
   gaagaatccc tggaagtaga cacgaaccca ctgctgggcg ctggtaaatt cgcaactgat 780
   cctgcggtta ccctggctca cgaactgatt catgcaggcc accgcctgta cggtatcgcc 840
   atcaatccga accgtgtctt caaagttaac accaacgcgt attacgagat gtccggtctg 900
   gaagttagct tcgaagaact gcgtactttt ggcggtcacg acgctaaatt catcgactct 960
   ctgcaagaaa acgagttccg tctgtactac tataacaagt tcaaagatat cgcatccacc 1020
   ctgaacaaag cgaaatccat cgtgggtacc actgcttctc tccagtacat gaagaacgtt 1080
   tttaaagaaa aatacctgct cagcgaagac acctccggca aattctctgt agacaagttg 1140
   aaattcgata aactttacaa aatgctgact gaaatttaca ccgaagacaa cttcgttaag 1200
   ttctttaaag ttctgaaccg caaaacctat ctgaacttcg acaaggcagt attcaaaatc 1260
   aacatcgtgc cgaaagttaa ctacactatc tacgatggtt tcaacctgcg taacaccaac 1320
   ctggctgcta attttaacgg ccagaacacg gaaatcaaca acatgaactt cacaaaactg 1380
   aaaaacttca ctggtctgtt cgagttttac aagctgctgt gcgtcgacgg catcattacc 1440
   tccaaaacta aatctctgat agaaggtaga aacaaagcgc tgaacctgca gtgtatcaag 1500
   gttaacaact gggatttatt cttcagcccg agtgaagaca acttcaccaa cgacctgaac 1560
   aaaggtgaag aaatcacctc agatactaac atcgaagcag ccgaagaaaa catctcgctg 1620
   gacctgatcc agcagtacta cctgaccttt aatttcgaca acgagccgga aaacatttct 1680
   atcgaaaacc tgagctctga tatcatcggc cagctggaac tgatgccgaa catcgaacgt 1740
   ttcccaaacg gtaaaaagta cgagctggac aaatatacca tgttccacta cctgcgcgcg 1800
   caggaatttg aacacggcaa atcccgtatc gcactgacta actccgttaa cgaagctctg 1860
   ctcaacccgt cccgtgtata caccttcttc tctagcgact acgtgaaaaa ggtcaacaaa 1920
   gcgactgaag ctgcaatgtt cttgggttgg gttgaacagc ttgtttatga ttttaccgac 1980
   gagacgtccg aagtatctac taccgacaaa attgcggata tcactatcat catcccgtac 2040
   atcggtccgg ctctgaacat tggcaacatg ctgtacaaag acgacttcgt tggcgcactg 2100
   atcttctccg gtgcggtgat cctgctggag ttcatcccgg aaatcgccat cccggtactg 2160
   ggcacctttg ctctggtttc ttacattgca aacaaggttc tgactgtaca aaccatcgac 2220
   aacgcgctga gcaaacgtaa cgaaaaatgg gatgaagttt acaaatatat cgtgaccaac 2280
   tggctggcta aggttaatac tcagatcgac ctcatccgca aaaaaatgaa agaagcactg 2340
   gaaaaccagg cggaagctac caaggcaatc attaactacc agtacaacca gtacaccgag 2400
   gaagaaaaaa acaacatcaa cttcaacatc gacgatctgt cctctaaact gaacgaatcc 2460
   atcaacaaag ctatgatcaa catcaacaag ttcctgaacc agtgctctgt aagctatctg 2520
   atgaactcca tgatcccgta cggtgttaaa cgtctggagg acttcgatgc gtctctgaaa 2580
   gacgccctgc tgaaatacat ttacgacaac cgtggcactc tgatcggtca ggttgatcgt 2640
   ctgaaggaca aagtgaacaa taccttatcg accgacatcc cttttcagct cagtaaatat 2700
   gtcgataacc aacgcctttt gtccactcta gactag 2736
<210> 64
   <211> 911
   <212> PRT
   <213> Artificial Sequence
<220>
   <223> Synthetic
<400> 64
   Leu Gly Ile Glu Gly Arg Phe Gly Gly Phe Thr Gly Ala Arg Lys Ser
   1               5                   10                  15
   Ala Arg Lys Leu Ala Asn Gln Thr Ser Gly Gly Gly Gly Ser Gly Gly
               20                  25                  30
   Gly Gly Ser Gly Gly Gly Gly Ser Pro Arg Gly Ser Met Glu Phe Val
           35                  40                  45
   Asn Lys Gln Phe Asn Tyr Lys Asp Pro Val Asn Gly Val Asp Ile Ala
       50                  55                  60
   Tyr Ile Lys Ile Pro Asn Ala Gly Gln Met Gln Pro Val Lys Ala Phe
   65                  70                  75                  80
   Lys Ile His Asn Lys Ile Trp Val Ile Pro Glu Arg Asp Thr Phe Thr
                   85                  90                  95
   Asn Pro Glu Glu Gly Asp Leu Asn Pro Pro Pro Glu Ala Lys Gln Val
               100                 105                 110
   Pro Val Ser Tyr Tyr Asp Ser Thr Tyr Leu Ser Thr Asp Asn Glu Lys
           115                 120                 125
   Asp Asn Tyr Leu Lys Gly Val Thr Lys Leu Phe Glu Arg Ile Tyr Ser
       130                 135                 140
   Thr Asp Leu Gly Arg Met Leu Leu Thr Ser Ile Val Arg Gly Ile Pro
   145                 150                 155                 160
   Phe Trp Gly Gly Ser Thr Ile Asp Thr Glu Leu Lys Val Ile Asp Thr
                   165                 170                 175
   Asn Cys Ile Asn Val Ile Gln Pro Asp Gly Ser Tyr Arg Ser Glu Glu
   180 185 190
   Leu Asn Leu Val Ile Ile Gly Pro Ser Ala Asp Ile Ile Gln Phe Glu
   195 200 205
   Cys Lys Ser Phe Gly His Glu Val Leu Asn Leu Thr Arg Asn Gly Tyr
   210 215 220
   Gly Ser Thr Gln Tyr Ile Arg Phe Ser Pro Asp Phe Thr Phe Gly Phe
   225 230 235 240
   Glu Glu Ser Leu Glu Val Asp Thr Asn Pro Leu Leu Gly Ala Gly Lys
   245 250 255
   Phe Ala Thr Asp Pro Ala Val Thr Leu Ala His Glu Leu Ile His Ala
   260 265 270
   Gly His Arg Leu Tyr Gly Ile Ala Ile Asn Pro Asn Arg Val Phe Lys
   275 280 285
   Val Asn Thr Asn Ala Tyr Tyr Glu Met Ser Gly Leu Glu Val Ser Phe
   290 295 300
   Glu Glu Leu Arg Thr Phe Gly Gly His Asp Ala Lys Phe Ile Asp Ser
   305 310 315 320
   Leu Gln Glu Asn Glu Phe Arg Leu Tyr Tyr Tyr Asn Lys Phe Lys Asp
   325 330 335
   Ile Ala Ser Thr Leu Asn Lys Ala Lys Ser Ile Val Gly Thr Thr Ala
   340 345 350
   Ser Leu Gln Tyr Met Lys Asn Val Phe Lys Glu Lys Tyr Leu Leu Ser
   355 360 365
   Glu Asp Thr Ser Gly Lys Phe Ser Val Asp Lys Leu Lys Phe Asp Lys
   370 375 380
   Leu Tyr Lys Met Leu Thr Glu Ile Tyr Thr Glu Asp Asn Phe Val Lys
   385 390 395 400
   Phe Phe Lys Val Leu Asn Arg Lys Thr Tyr Leu Asn Phe Asp Lys Ala
   405 410 415
   Val Phe Lys Ile Asn Ile Val Pro Lys Val Asn Tyr Thr Ile Tyr Asp
   420 425 430
   Gly Phe Asn Leu Arg Asn Thr Asn Leu Ala Ala Asn Phe Asn Gly Gln
           435                 440                 445
   Asn Thr Glu Ile Asn Asn Met Asn Phe Thr Lys Leu Lys Asn Phe Thr
       450                 455                 460
   Gly Leu Phe Glu Phe Tyr Lys Leu Leu Cys Val Asp Gly Ile Ile Thr
   465                 470                 475                 480
   Ser Lys Thr Lys Ser Leu Ile Glu Gly Arg Asn Lys Ala Leu Asn Leu
                   485                 490                 495
   Gln Cys Ile Lys Val Asn Asn Trp Asp Leu Phe Phe Ser Pro Ser Glu
               500                 505                 510
   Asp Asn Phe Thr Asn Asp Leu Asn Lys Gly Glu Glu Ile Thr Ser Asp
           515                 520                 525
   Thr Asn Ile Glu Ala Ala Glu Glu Asn Ile Ser Leu Asp Leu Ile Gln
       530                 535                 540
   Gln Tyr Tyr Leu Thr Phe Asn Phe Asp Asn Glu Pro Glu Asn Ile Ser
   545                 550                 555                 560
   Ile Glu Asn Leu Ser Ser Asp Ile Ile Gly Gln Leu Glu Leu Met Pro
                   565                 570                 575
   Asn Ile Glu Arg Phe Pro Asn Gly Lys Lys Tyr Glu Leu Asp Lys Tyr
               580                 585                 590
   Thr Met Phe His Tyr Leu Arg Ala Gln Glu Phe Glu His Gly Lys Ser
           595                 600                 605
   Arg Ile Ala Leu Thr Asn Ser Val Asn Glu Ala Leu Leu Asn Pro Ser
       610                 615                 620
   Arg Val Tyr Thr Phe Phe Ser Ser Asp Tyr Val Lys Lys Val Asn Lys
   625                 630                 635                 640
   Ala Thr Glu Ala Ala Met Phe Leu Gly Trp Val Glu Gln Leu Val Tyr
                   645                 650                 655
   Asp Phe Thr Asp Glu Thr Ser Glu Val Ser Thr Thr Asp Lys Ile Ala
               660                 665                 670
   Asp Ile Thr Ile Ile Ile Pro Tyr Ile Gly Pro Ala Leu Asn Ile Gly
           675                 680                 685
   Asn Met Leu Tyr Lys Asp Asp Phe Val Gly Ala Leu Ile Phe Ser Gly
       690                 695                 700
   Ala Val Ile Leu Leu Glu Phe Ile Pro Glu Ile Ala Ile Pro Val Leu
   705                 710                 715                 720
   Gly Thr Phe Ala Leu Val Ser Tyr Ile Ala Asn Lys Val Leu Thr Val
                   725                 730                 735
   Gln Thr Ile Asp Asn Ala Leu Ser Lys Arg Asn Glu Lys Trp Asp Glu
               740                 745                 750
   Val Tyr Lys Tyr Ile Val Thr Asn Trp Leu Ala Lys Val Asn Thr Gln
           755                 760                 765
   Ile Asp Leu Ile Arg Lys Lys Met Lys Glu Ala Leu Glu Asn Gln Ala
       770                 775                 780
   Glu Ala Thr Lys Ala Ile Ile Asn Tyr Gln Tyr Asn Gln Tyr Thr Glu
   785                 790                 795                 800
   Glu Glu Lys Asn Asn Ile Asn Phe Asn Ile Asp Asp Leu Ser Ser Lys
                   805                 810                 815
   Leu Asn Glu Ser Ile Asn Lys Ala Met Ile Asn Ile Asn Lys Phe Leu
               820                 825                 830
   Asn Gln Cys Ser Val Ser Tyr Leu Met Asn Ser Met Ile Pro Tyr Gly
           835                 840                 845
   Val Lys Arg Leu Glu Asp Phe Asp Ala Ser Leu Lys Asp Ala Leu Leu
       850                 855                 860
   Lys Tyr Ile Tyr Asp Asn Arg Gly Thr Leu Ile Gly Gln Val Asp Arg
   865                 870                 875                 880
   Leu Lys Asp Lys Val Asn Asn Thr Leu Ser Thr Asp Ile Pro Phe Gln
                   885                 890                 895
   Leu Ser Lys Tyr Val Asp Asn Gln Arg Leu Leu Ser Thr Leu Asp
               900                 905                 910
<210> 65
   <211> 177
   <212> DNA
   <213> Artificial Sequence
<220>
   <223> Synthetic
<400> 65
   ggatccacgc acgtcgacgg catcattacc tccaaaacta aatctctgat agaaggtaga      60
   tttggcggtt tcacgggcgc acgcaaatca gcgcgtaaat tagctaacca ggcgctagcg     120
   ggtggtggtg gttctgcact agtgctgcag acgcacggtc tagaatgata aaagctt        177
<210> 66
   <211> 192
   <212> DNA
   <213> Artificial Sequence
<220>
   <223> Synthetic
<400> 66
   ggatccacgc acgtcgacgg catcattacc tccaaaacta aatctctgat agaaggtaga      60
   tttggcggtt tcacgggcgc acgcaaatca gcgcgtaaat tagctaacca ggcgctagcg     120
   ggtggtggtg gttctggtgg tggtggttct gcactagtgc tgcagacgca cggtctagaa     180
   tgataaaagc tt                                                         192
<210> 67
   <211> 222
   <212> DNA
   <213> Artificial Sequence
<220>
   <223> Synthetic
<400> 67
   ggatccacgc acgtcgacgg catcattacc tccaaaacta aatctctgat agaaggtaga      60
   tttggcggtt tcacgggcgc acgcaaatca gcgcgtaaat tagctaacca ggcgctagcg     120
   ggtggtggtg gttctggtgg tggtggttct ggtggtggtg gttctggtgg tggtggttct     180
   gcactagtgc tgcagacgca cggtctagaa tgataaaagc tt                        222
<210> 68
   <211> 237
   <212> DNA
   <213> Artificial Sequence
<220>
   <223> Synthetic
<400> 68
   ggatccacgc acgtcgacgg catcattacc tccaaaacta aatctctgat agaaggtaga     60
   tttggcggtt tcacgggcgc acgcaaatca gcgcgtaaat tagctaacca ggcgctagcg    120
   ggtggtggtg gttctggtgg tggtggttct ggtggtggtg gttctggtgg tggtggttct    180
   ggtggtggtg gttctgcact agtgctgcag acgcacggtc tagaatgata aaagctt       237
<210> 69
   <211> 228
   <212> DNA
   <213> Artificial Sequence
<220>
   <223> Synthetic
<400> 69
   ggatccacgc acgtcgacgg catcattacc tccaaaacta aatctctgat agaaggtaga     60
   tttggcggtt tcacgggcgc acgcaaatca gcgcgtaaat tagctaacca ggcgctagcg    120
   gctgaagctg ctgctaaaga agctgctgct aaagaagctg ctgctaaagc tggtggcggt    180
   ggttccgcac tagtgctgca gacgcacggt ctagaatgat aaaagctt                 228
<210> 70
   <211> 2694
   <212> DNA
   <213> Artificial Sequence
<220>
   <223> Synthetic
<400> 70
   ggatccatgg agttcgttaa caaacagttc aactataaag acccagttaa cggtgttgac      60
   attgcttaca tcaaaatccc gaacgctggc cagatgcagc cggtaaaggc attcaaaatc     120
   cacaacaaaa tctgggttat cccggaacgt gataccttta ctaacccgga agaaggtgac     180
   ctgaacccgc caccggaagc gaaacaggtg ccggtatctt actatgactc cacctacctg     240
   tctaccgata acgaaaagga caactacctg aaaggtgtta ctaaactgtt cgagcgtatt     300
   tactccaccg acctgggccg tatgctgctg actagcatcg ttcgcggtat cccgttctgg     360
   ggcggttcta ccatcgatac cgaactgaaa gtaatcgaca ctaactgcat caacgttatt     420
   cagccggacg gttcctatcg ttccgaagaa ctgaacctgg tgatcatcgg cccgtctgct     480
   gatatcatcc agttcgagtg taagagcttt ggtcacgaag ttctgaacct cacccgtaac     540
   ggctacggtt ccactcagta catccgtttc tctccggact tcaccttcgg ttttgaagaa     600
   tccctggaag tagacacgaa cccactgctg ggcgctggta aattcgcaac tgatcctgcg     660
   gttaccctgg ctcacgaact gattcatgca ggccaccgcc tgtacggtat cgccatcaat     720
   ccgaaccgtg tcttcaaagt taacaccaac gcgtattacg agatgtccgg tctggaagtt     780
   agcttcgaag aactgcgtac ttttggcggt cacgacgcta aattcatcga ctctctgcaa 840
   gaaaacgagt tccgtctgta ctactataac aagttcaaag atatcgcatc caccctgaac 900
   aaagcgaaat ccatcgtggg taccactgct tctctccagt acatgaagaa cgtttttaaa 960
   gaaaaatacc tgctcagcga agacacctcc ggcaaattct ctgtagacaa gttgaaattc 1020
   gataaacttt acaaaatgct gactgaaatt tacaccgaag acaacttcgt taagttcttt 1080
   aaagttctga accgcaaaac ctatctgaac ttcgacaagg cagtattcaa aatcaacatc 1140
   gtgccgaaag ttaactacac tatctacgat ggtttcaacc tgcgtaacac caacctggct 1200
   gctaatttta acggccagaa cacggaaatc aacaacatga acttcacaaa actgaaaaac 1260
   ttcactggtc tgttcgagtt ttacaagctg ctgtgcgtcg acggcatcat tacctccaaa 1320
   actaaatctc tgatagaagg tagatttggc ggtttcacgg gcgcacgcaa atcagcgcgt 1380
   aaattagcta accaggcgct agcgggtggt ggtggttctg gtggtggtgg ttctgcacta 1440
   gtgctgcagt gtatcaaggt taacaactgg gatttattct tcagcccgag tgaagacaac 1500
   ttcaccaacg acctgaacaa aggtgaagaa atcacctcag atactaacat cgaagcagcc 1560
   gaagaaaaca tctcgctgga cctgatccag cagtactacc tgacctttaa tttcgacaac 1620
   gagccggaaa acatttctat cgaaaacctg agctctgata tcatcggcca gctggaactg 1680
   atgccgaaca tcgaacgttt cccaaacggt aaaaagtacg agctggacaa atataccatg 1740
   ttccactacc tgcgcgcgca ggaatttgaa cacggcaaat cccgtatcgc actgactaac 1800
   tccgttaacg aagctctgct caacccgtcc cgtgtataca ccttcttctc tagcgactac 1860
   gtgaaaaagg tcaacaaagc gactgaagct gcaatgttct tgggttgggt tgaacagctt 1920
   gtttatgatt ttaccgacga gacgtccgaa gtatctacta ccgacaaaat tgcggatatc 1980
   actatcatca tcccgtacat cggtccggct ctgaacattg gcaacatgct gtacaaagac 2040
   gacttcgttg gcgcactgat cttctccggt gcggtgatcc tgctggagtt catcccggaa 2100
   atcgccatcc cggtactggg cacctttgct ctggtttctt acattgcaaa caaggttctg 2160
   actgtacaaa ccatcgacaa cgcgctgagc aaacgtaacg aaaaatggga tgaagtttac 2220
   aaatatatcg tgaccaactg gctggctaag gttaatactc agatcgacct catccgcaaa 2280
   aaaatgaaag aagcactgga aaaccaggcg gaagctacca aggcaatcat taactaccag 2340
   tacaaccagt acaccgagga agaaaaaaac aacatcaact tcaacatcga cgatctgtcc 2400
   tctaaactga acgaatccat caacaaagct atgatcaaca tcaacaagtt cctgaaccag 2460
   tgctctgtaa gctatctgat gaactccatg atcccgtacg gtgttaaacg tctggaggac 2520
   ttcgatgcgt ctctgaaaga cgccctgctg aaatacattt acgacaaccg tggcactctg 2580
   atcggtcagg ttgatcgtct gaaggacaaa gtgaacaata ccttatcgac cgacatccct 2640
   tttcagctca gtaaatatgt cgataaccaa cgccttttgt ccactctaga ctag 2694
<210> 71
   <211> 897
   <212> PRT
   <213> Artificial Sequence
<220>
   <223> Synthetic
<400> 71
   Gly Ser Met Glu Phe Val Asn Lys Gln Phe Asn Tyr Lys Asp Pro Val
   1 5 10 15
   Asn Gly Val Asp Ile Ala Tyr Ile Lys Ile Pro Asn Ala Gly Gln Met
   20 25 30
   Gln Pro Val Lys Ala Phe Lys Ile His Asn Lys Ile Trp Val Ile Pro
   35 40 45
   Glu Arg Asp Thr Phe Thr Asn Pro Glu Glu Gly Asp Leu Asn Pro Pro
   50 55 60
   Pro Glu Ala Lys Gln Val Pro Val Ser Tyr Tyr Asp Ser Thr Tyr Leu
   65 70 75 80
   Ser Thr Asp Asn Glu Lys Asp Asn Tyr Leu Lys Gly Val Thr Lys Leu
   85 90 95
   Phe Glu Arg Ile Tyr Ser Thr Asp Leu Gly Arg Met Leu Leu Thr Ser
   100 105 110
   Ile Val Arg Gly Ile Pro Phe Trp Gly Gly Ser Thr Ile Asp Thr Glu
   115 120 125
   Leu Lys Val Ile Asp Thr Asn Cys Ile Asn Val Ile Gln Pro Asp Gly
   130 135 140
   Ser Tyr Arg Ser Glu Glu Leu Asn Leu Val Ile Ile Gly Pro Ser Ala
   145 150 155 160
   Asp Ile Ile Gln Phe Glu Cys Lys Ser Phe Gly His Glu Val Leu Asn
   165 170 175
   Leu Thr Arg Asn Gly Tyr Gly Ser Thr Gln Tyr Ile Arg Phe Ser Pro
   180 185 190
   Asp Phe Thr Phe Gly Phe Glu Glu Ser Leu Glu Val Asp Thr Asn Pro
           195                 200                 205
   Leu Leu Gly Ala Gly Lys Phe Ala Thr Asp Pro Ala Val Thr Leu Ala
       210                 215                 220
   His Glu Leu Ile His Ala Gly His Arg Leu Tyr Gly Ile Ala Ile Asn
   225                 230                 235                 240
   Pro Asn Arg Val Phe Lys Val Asn Thr Asn Ala Tyr Tyr Glu Met Ser
                   245                 250                 255
   Gly Leu Glu Val Ser Phe Glu Glu Leu Arg Thr Phe Gly Gly His Asp
               260                 265                 270
   Ala Lys Phe Ile Asp Ser Leu Gln Glu Asn Glu Phe Arg Leu Tyr Tyr
           275                 280                 285
   Tyr Asn Lys Phe Lys Asp Ile Ala Ser Thr Leu Asn Lys Ala Lys Ser
       290                 295                 300
   Ile Val Gly Thr Thr Ala Ser Leu Gln Tyr Met Lys Asn Val Phe Lys
   305                 310                 315                 320
   Glu Lys Tyr Leu Leu Ser Glu Asp Thr Ser Gly Lys Phe Ser Val Asp
                   325                 330                 335
   Lys Leu Lys Phe Asp Lys Leu Tyr Lys Met Leu Thr Glu Ile Tyr Thr
               340                 345                 350
   Glu Asp Asn Phe Val Lys Phe Phe Lys Val Leu Asn Arg Lys Thr Tyr
           355                 360                 365
   Leu Asn Phe Asp Lys Ala Val Phe Lys Ile Asn Ile Val Pro Lys Val
       370                 375                 380
   Asn Tyr Thr Ile Tyr Asp Gly Phe Asn Leu Arg Asn Thr Asn Leu Ala
   385                 390                 395                 400
   Ala Asn Phe Asn Gly Gln Asn Thr Glu Ile Asn Asn Met Asn Phe Thr
                   405                 410                 415
   Lys Leu Lys Asn Phe Thr Gly Leu Phe Glu Phe Tyr Lys Leu Leu Cys
               420                 425                 430
   Val Asp Gly Ile Ile Thr Ser Lys Thr Lys Ser Leu Ile Glu Gly Arg
           435                     440                     445
   Phe Gly Gly Phe Thr Gly Ala Arg Lys Ser Ala Arg Lys Leu Ala Asn
       450                     455                     460
   Gln Ala Leu Ala Gly Gly Gly Gly Ser Gly Gly Gly Gly Ser Ala Leu
   465                     470                     475                     480
   Val Leu Gln Cys Ile Lys Val Asn Asn Trp Asp Leu Phe Phe Ser Pro
                   485                     490                     495
   Ser Glu Asp Asn Phe Thr Asn Asp Leu Asn Lys Gly Glu Glu Ile Thr
               500                     505                     510
   Ser Asp Thr Asn Ile Glu Ala Ala Glu Glu Asn Ile Ser Leu Asp Leu
           515                     520                     525
   Ile Gln Gln Tyr Tyr Leu Thr Phe Asn Phe Asp Asn Glu Pro Glu Asn
       530                     535                     540
   Ile Ser Ile Glu Asn Leu Ser Ser Asp Ile Ile Gly Gln Leu Glu Leu
   545                     550                     555                     560
   Met Pro Asn Ile Glu Arg Phe Pro Asn Gly Lys Lys Tyr Glu Leu Asp
                   565                     570                     575
   Lys Tyr Thr Met Phe His Tyr Leu Arg Ala Gln Glu Phe Glu His Gly
               580                     585                     590
   Lys Ser Arg Ile Ala Leu Thr Asn Ser Val Asn Glu Ala Leu Leu Asn
           595                     600                     605
   Pro Ser Arg Val Tyr Thr Phe Phe Ser Ser Asp Tyr Val Lys Lys Val
       610                     615                     620
   Asn Lys Ala Thr Glu Ala Ala Met Phe Leu Gly Trp Val Glu Gln Leu
   625                     630                     635                     640
   Val Tyr Asp Phe Thr Asp Glu Thr Ser Glu Val Ser Thr Thr Asp Lys
                   645                     650                     655
   Ile Ala Asp Ile Thr Ile Ile Ile Pro Tyr Ile Gly Pro Ala Leu Asn
               660                     665                     670
   Ile Gly Asn Met Leu Tyr Lys Asp Asp Phe Val Gly Ala Leu Ile Phe
   675 680 685
   Ser Gly Ala Val Ile Leu Leu Glu Phe Ile Pro Glu Ile Ala Ile Pro
   690 695 700
   Val Leu Gly Thr Phe Ala Leu Val Ser Tyr Ile Ala Asn Lys Val Leu
   705 710 715 720
   Thr Val Gln Thr Ile Asp Asn Ala Leu Ser Lys Arg Asn Glu Lys Trp
   725 730 735
   Asp Glu Val Tyr Lys Tyr Ile Val Thr Asn Trp Leu Ala Lys Val Asn
   740 745 750
   Thr Gln Ile Asp Leu Ile Arg Lys Lys Met Lys Glu Ala Leu Glu Asn
   755 760 765
   Gln Ala Glu Ala Thr Lys Ala Ile Ile Asn Tyr Gln Tyr Asn Gln Tyr
   770 775 780
   Thr Glu Glu Glu Lys Asn Asn Ile Asn Phe Asn Ile Asp Asp Leu Ser
   785 790 795 800
   Ser Lys Leu Asn Glu Ser Ile Asn Lys Ala Met Ile Asn Ile Asn Lys
   805 810 815
   Phe Leu Asn Gln Cys Ser Val Ser Tyr Leu Met Asn Ser Met Ile Pro
   820 825 830
   Tyr Gly Val Lys Arg Leu Glu Asp Phe Asp Ala Ser Leu Lys Asp Ala
   835 840 845
   Leu Leu Lys Tyr Ile Tyr Asp Asn Arg Gly Thr Leu Ile Gly Gln Val
   850 855 860
   Asp Arg Leu Lys Asp Lys Val Asn Asn Thr Leu Ser Thr Asp Ile Pro
   865 870 875 880
   Phe Gln Leu Ser Lys Tyr Val Asp Asn Gln Arg Leu Leu Ser Thr Leu
   885 890 895
   Asp
<210> 72
   <211> 2724
   <212> DNA
   <213> Artificial Sequence
<220>
   <223> Synthetic
<400> 72
   ggatccatgg agttcgttaa caaacagttc aactataaag acccagttaa cggtgttgac     60
   attgcttaca tcaaaatccc gaacgctggc cagatgcagc cggtaaaggc attcaaaatc    120
   cacaacaaaa tctgggttat cccggaacgt gataccttta ctaacccgga agaaggtgac    180
   ctgaacccgc caccggaagc gaaacaggtg ccggtatctt actatgactc cacctacctg    240
   tctaccgata acgaaaagga caactacctg aaaggtgtta ctaaactgtt cgagcgtatt    300
   tactccaccg acctgggccg tatgctgctg actagcatcg ttcgcggtat cccgttctgg    360
   ggcggttcta ccatcgatac cgaactgaaa gtaatcgaca ctaactgcat caacgttatt    420
   cagccggacg gttcctatcg ttccgaagaa ctgaacctgg tgatcatcgg cccgtctgct    480
   gatatcatcc agttcgagtg taagagcttt ggtcacgaag ttctgaacct cacccgtaac    540
   ggctacggtt ccactcagta catccgtttc tctccggact tcaccttcgg ttttgaagaa    600
   tccctggaag tagacacgaa cccactgctg ggcgctggta aattcgcaac tgatcctgcg    660
   gttaccctgg ctcacgaact gattcatgca ggccaccgcc tgtacggtat cgccatcaat    720
   ccgaaccgtg tcttcaaagt taacaccaac gcgtattacg agatgtccgg tctggaagtt    780
   agcttcgaag aactgcgtac ttttggcggt cacgacgcta aattcatcga ctctctgcaa    840
   gaaaacgagt tccgtctgta ctactataac aagttcaaag atatcgcatc caccctgaac    900
   aaagcgaaat ccatcgtggg taccactgct tctctccagt acatgaagaa cgtttttaaa    960
   gaaaaatacc tgctcagcga agacacctcc ggcaaattct ctgtagacaa gttgaaattc   1020
   gataaacttt acaaaatgct gactgaaatt tacaccgaag acaacttcgt taagttcttt   1080
   aaagttctga accgcaaaac ctatctgaac ttcgacaagg cagtattcaa aatcaacatc   1140
   gtgccgaaag ttaactacac tatctacgat ggtttcaacc tgcgtaacac caacctggct   1200
   gctaatttta acggccagaa cacggaaatc aacaacatga acttcacaaa actgaaaaac   1260
   ttcactggtc tgttcgagtt ttacaagctg ctgtgcgtcg acggcatcat tacctccaaa   1320
   actaaatctc tgatagaagg tagatttggc ggtttcacgg gcgcacgcaa atcagcgcgt   1380
   aaattagcta accaggcgct agcgggtggt ggtggttctg gtggtggtgg ttctggtggt   1440
   ggtggttctg gtggtggtgg ttctgcacta gtgctgcagt gtatcaaggt taacaactgg   1500
   gatttattct tcagcccgag tgaagacaac ttcaccaacg acctgaacaa aggtgaagaa   1560
   atcacctcag atactaacat cgaagcagcc gaagaaaaca tctcgctgga cctgatccag   1620
   cagtactacc tgacctttaa tttcgacaac gagccggaaa acatttctat cgaaaacctg 1680
   agctctgata tcatcggcca gctggaactg atgccgaaca tcgaacgttt cccaaacggt 1740
   aaaaagtacg agctggacaa atataccatg ttccactacc tgcgcgcgca ggaatttgaa 1800
   cacggcaaat cccgtatcgc actgactaac tccgttaacg aagctctgct caacccgtcc 1860
   cgtgtataca ccttcttctc tagcgactac gtgaaaaagg tcaacaaagc gactgaagct 1920
   gcaatgttct tgggttgggt tgaacagctt gtttatgatt ttaccgacga gacgtccgaa 1980
   gtatctacta ccgacaaaat tgcggatatc actatcatca tcccgtacat cggtccggct 2040
   ctgaacattg gcaacatgct gtacaaagac gacttcgttg gcgcactgat cttctccggt 2100
   gcggtgatcc tgctggagtt catcccggaa atcgccatcc cggtactggg cacctttgct 2160
   ctggtttctt acattgcaaa caaggttctg actgtacaaa ccatcgacaa cgcgctgagc 2220
   aaacgtaacg aaaaatggga tgaagtttac aaatatatcg tgaccaactg gctggctaag 2280
   gttaatactc agatcgacct catccgcaaa aaaatgaaag aagcactgga aaaccaggcg 2340
   gaagctacca aggcaatcat taactaccag tacaaccagt acaccgagga agaaaaaaac 2400
   aacatcaact tcaacatcga cgatctgtcc tctaaactga acgaatccat caacaaagct 2460
   atgatcaaca tcaacaagtt cctgaaccag tgctctgtaa gctatctgat gaactccatg 2520
   atcccgtacg gtgttaaacg tctggaggac ttcgatgcgt ctctgaaaga cgccctgctg 2580
   aaatacattt acgacaaccg tggcactctg atcggtcagg ttgatcgtct gaaggacaaa 2640
   gtgaacaata ccttatcgac cgacatccct tttcagctca gtaaatatgt cgataaccaa 2700
   cgccttttgt ccactctaga ctag 2724
<210> 73
   <211> 907
   <212> PRT
   <213> Artificial Sequence
<220>
   <223> Synthetic
<400> 73
   Gly Ser Met Glu Phe Val Asn Lys Gln Phe Asn Tyr Lys Asp Pro Val
   1 5 10 15
   Asn Gly Val Asp Ile Ala Tyr Ile Lys Ile Pro Asn Ala Gly Gln Met
   20 25 30
   Gln Pro Val Lys Ala Phe Lys Ile His Asn Lys Ile Trp Val Ile Pro
   35 40 45
   Glu Arg Asp Thr Phe Thr Asn Pro Glu Glu Gly Asp Leu Asn Pro Pro
       50                  55                  60
   Pro Glu Ala Lys Gln Val Pro Val Ser Tyr Tyr Asp Ser Thr Tyr Leu
   65                  70                  75                  80
   Ser Thr Asp Asn Glu Lys Asp Asn Tyr Leu Lys Gly Val Thr Lys Leu
                   85                  90                  95
   Phe Glu Arg Ile Tyr Ser Thr Asp Leu Gly Arg Met Leu Leu Thr Ser
               100                 105                 110
   Ile Val Arg Gly Ile Pro Phe Trp Gly Gly Ser Thr Ile Asp Thr Glu
           115                 120                 125
   Leu Lys Val Ile Asp Thr Asn Cys Ile Asn Val Ile Gln Pro Asp Gly
       130                 135                 140
   Ser Tyr Arg Ser Glu Glu Leu Asn Leu Val Ile Ile Gly Pro Ser Ala
   145                 150                 155                 160
   Asp Ile Ile Gln Phe Glu Cys Lys Ser Phe Gly His Glu Val Leu Asn
                   165                 170                 175
   Leu Thr Arg Asn Gly Tyr Gly Ser Thr Gln Tyr Ile Arg Phe Ser Pro
               180                 185                 190
   Asp Phe Thr Phe Gly Phe Glu Glu Ser Leu Glu Val Asp Thr Asn Pro
           195                 200                 205
   Leu Leu Gly Ala Gly Lys Phe Ala Thr Asp Pro Ala Val Thr Leu Ala
       210                 215                 220
   His Glu Leu Ile His Ala Gly His Arg Leu Tyr Gly Ile Ala Ile Asn
   225                 230                 235                 240
   Pro Asn Arg Val Phe Lys Val Asn Thr Asn Ala Tyr Tyr Glu Met Ser
                   245                 250                 255
   Gly Leu Glu Val Ser Phe Glu Glu Leu Arg Thr Phe Gly Gly His Asp
               260                 265                 270
   Ala Lys Phe Ile Asp Ser Leu Gln Glu Asn Glu Phe Arg Leu Tyr Tyr
           275                 280                 285
   Tyr Asn Lys Phe Lys Asp Ile Ala Ser Thr Leu Asn Lys Ala Lys Ser
   290 295 300
   Ile Val Gly Thr Thr Ala Ser Leu Gln Tyr Met Lys Asn Val Phe Lys
   305 310 315 320
   Glu Lys Tyr Leu Leu Ser Glu Asp Thr Ser Gly Lys Phe Ser Val Asp
   325 330 335
   Lys Leu Lys Phe Asp Lys Leu Tyr Lys Met Leu Thr Glu Ile Tyr Thr
   340 345 350
   Glu Asp Asn Phe Val Lys Phe Phe Lys Val Leu Asn Arg Lys Thr Tyr
   355 360 365
   Leu Asn Phe Asp Lys Ala Val Phe Lys Ile Asn Ile Val Pro Lys Val
   370 375 380
   Asn Tyr Thr Ile Tyr Asp Gly Phe Asn Leu Arg Asn Thr Asn Leu Ala
   385 390 395 400
   Ala Asn Phe Asn Gly Gln Asn Thr Glu Ile Asn Asn Met Asn Phe Thr
   405 410 415
   Lys Leu Lys Asn Phe Thr Gly Leu Phe Glu Phe Tyr Lys Leu Leu Cys
   420 425 430
   Val Asp Gly Ile Ile Thr Ser Lys Thr Lys Ser Leu Ile Glu Gly Arg
   435 440 445
   Phe Gly Gly Phe Thr Gly Ala Arg Lys Ser Ala Arg Lys Leu Ala Asn
   450 455 460
   Gln Ala Leu Ala Gly Gly Gly Gly Ser Gly Gly Gly Gly Ser Gly Gly
   465 470 475 480
   Gly Gly Ser Gly Gly Gly Gly Ser Ala Leu Val Leu Gln Cys Ile Lys
   485 490 495
   Val Asn Asn Trp Asp Leu Phe Phe Ser Pro Ser Glu Asp Asn Phe Thr
   500 505 510
   Asn Asp Leu Asn Lys Gly Glu Glu Ile Thr Ser Asp Thr Asn Ile Glu
   515 520 525
   Ala Ala Glu Glu Asn Ile Ser Leu Asp Leu Ile Gln Gln Tyr Tyr Leu
   530 535 540
   Thr Phe Asn Phe Asp Asn Glu Pro Glu Asn Ile Ser Ile Glu Asn Leu
   545 550 555 560
   Ser Ser Asp Ile Ile Gly Gln Leu Glu Leu Met Pro Asn Ile Glu Arg
   565 570 575
   Phe Pro Asn Gly Lys Lys Tyr Glu Leu Asp Lys Tyr Thr Met Phe His
   580 585 590
   Tyr Leu Arg Ala Gln Glu Phe Glu His Gly Lys Ser Arg Ile Ala Leu
   595 600 605
   Thr Asn Ser Val Asn Glu Ala Leu Leu Asn Pro Ser Arg Val Tyr Thr
   610 615 620
   Phe Phe Ser Ser Asp Tyr Val Lys Lys Val Asn Lys Ala Thr Glu Ala
   625 630 635 640
   Ala Met Phe Leu Gly Trp Val Glu Gln Leu Val Tyr Asp Phe Thr Asp
   645 650 655
   Glu Thr Ser Glu Val Ser Thr Thr Asp Lys Ile Ala Asp Ile Thr Ile
   660 665 670
   Ile Ile Pro Tyr Ile Gly Pro Ala Leu Asn Ile Gly Asn Met Leu Tyr
   675 680 685
   Lys Asp Asp Phe Val Gly Ala Leu Ile Phe Ser Gly Ala Val Ile Leu
   690 695 700
   Leu Glu Phe Ile Pro Glu Ile Ala Ile Pro Val Leu Gly Thr Phe Ala
   705 710 715 720
   Leu Val Ser Tyr Ile Ala Asn Lys Val Leu Thr Val Gln Thr Ile Asp
   725 730 735
   Asn Ala Leu Ser Lys Arg Asn Glu Lys Trp Asp Glu Val Tyr Lys Tyr
   740 745 750
   Ile Val Thr Asn Trp Leu Ala Lys Val Asn Thr Gln Ile Asp Leu Ile
   755 760 765
   Arg Lys Lys Met Lys Glu Ala Leu Glu Asn Gln Ala Glu Ala Thr Lys
   770 775 780
   Ala Ile Ile Asn Tyr Gln Tyr Asn Gln Tyr Thr Glu Glu Glu Lys Asn
   785 790 795 800
   Asn Ile Asn Phe Asn Ile Asp Asp Leu Ser Ser Lys Leu Asn Glu Ser
   805 810 815
   Ile Asn Lys Ala Met Ile Asn Ile Asn Lys Phe Leu Asn Gln Cys Ser
   820 825 830
   Val Ser Tyr Leu Met Asn Ser Met Ile Pro Tyr Gly Val Lys Arg Leu
   835 840 845
   Glu Asp Phe Asp Ala Ser Leu Lys Asp Ala Leu Leu Lys Tyr Ile Tyr
   850 855 860
   Asp Asn Arg Gly Thr Leu Ile Gly Gln Val Asp Arg Leu Lys Asp Lys
   865 870 875 880
   Val Asn Asn Thr Leu Ser Thr Asp Ile Pro Phe Gln Leu Ser Lys Tyr
   885 890 895
   Val Asp Asn Gln Arg Leu Leu Ser Thr Leu Asp
   900 905
<210> 74
   <211> 207
   <212> DNA
   <213> Artificial Sequence
<220>
   <223> Synthetic
<400> 74
   ggatccacgc acgtcgacgg catcattacc tccaaaacta aatctgacga tgacgataaa 60
   tttggcggtt tcacgggcgc acgcaaatca gcgcgtaaac gtaagaacca ggcgctagcg 120
   ggcggtggcg gtagcggcgg tggcggtagc ggcggtggcg gtagcgcact agtgctgcag 180
   acgcacggtc tagaatgata aaagctt 207
<210> 75
   <211> 2709
   <212> DNA Artificial Sequence
<220>
   <223> Synthetic
<400> 75
   ggatccatgg agttcgttaa caaacagttc aactataaag acccagttaa cggtgttgac      60
   attgcttaca tcaaaatccc gaacgctggc cagatgcagc cggtaaaggc attcaaaatc     120
   cacaacaaaa tctgggttat cccggaacgt gataccttta ctaacccgga agaaggtgac     180
   ctgaacccgc caccggaagc gaaacaggtg ccggtatctt actatgactc cacctacctg     240
   tctaccgata acgaaaagga caactacctg aaaggtgtta ctaaactgtt cgagcgtatt     300
   tactccaccg acctgggccg tatgctgctg actagcatcg ttcgcggtat cccgttctgg     360
   ggcggttcta ccatcgatac cgaactgaaa gtaatcgaca ctaactgcat caacgttatt     420
   cagccggacg gttcctatcg ttccgaagaa ctgaacctgg tgatcatcgg cccgtctgct     480
   gatatcatcc agttcgagtg taagagcttt ggtcacgaag ttctgaacct cacccgtaac     540
   ggctacggtt ccactcagta catccgtttc tctccggact tcaccttcgg ttttgaagaa     600
   tccctggaag tagacacgaa cccactgctg ggcgctggta aattcgcaac tgatcctgcg     660
   gttaccctgg ctcacgaact gattcatgca ggccaccgcc tgtacggtat cgccatcaat     720
   ccgaaccgtg tcttcaaagt taacaccaac gcgtattacg agatgtccgg tctggaagtt     780
   agcttcgaag aactgcgtac ttttggcggt cacgacgcta aattcatcga ctctctgcaa     840
   gaaaacgagt tccgtctgta ctactataac aagttcaaag atatcgcatc caccctgaac     900
   aaagcgaaat ccatcgtggg taccactgct tctctccagt acatgaagaa cgtttttaaa     960
   gaaaaatacc tgctcagcga agacacctcc ggcaaattct ctgtagacaa gttgaaattc    1020
   gataaacttt acaaaatgct gactgaaatt tacaccgaag acaacttcgt taagttcttt    1080
   aaagttctga accgcaaaac ctatctgaac ttcgacaagg cagtattcaa aatcaacatc    1140
   gtgccgaaag ttaactacac tatctacgat ggtttcaacc tgcgtaacac caacctggct    1200
   gctaatttta acggccagaa cacggaaatc aacaacatga acttcacaaa actgaaaaac    1260
   ttcactggtc tgttcgagtt ttacaagctg ctgtgcgtcg acggcatcat tacctccaaa    1320
   actaaatctg acgatgacga taaatttggc ggtttcacgg gcgcacgcaa atcagcgcgt    1380
   aaacgtaaga accaggcgct agcgggcggt ggcggtagcg gcggtggcgg tagcggcggt    1440
   ggcggtagcg cactagtgct gcagtgtatc aaggttaaca actgggattt attcttcagc    1500
   ccgagtgaag acaacttcac caacgacctg aacaaaggtg aagaaatcac ctcagatact    1560
   aacatcgaag cagccgaaga aaacatctcg ctggacctga tccagcagta ctacctgacc    1620
   tttaatttcg acaacgagcc ggaaaacatt tctatcgaaa acctgagctc tgatatcatc    1680
   ggccagctgg aactgatgcc gaacatcgaa cgtttcccaa acggtaaaaa gtacgagctg    1740
   gacaaatata ccatgttcca ctacctgcgc gcgcaggaat ttgaacacgg caaatcccgt    1800
   atcgcactga ctaactccgt taacgaagct ctgctcaacc cgtcccgtgt atacaccttc    1860
   ttctctagcg actacgtgaa aaaggtcaac aaagcgactg aagctgcaat gttcttgggt   1920
   tgggttgaac agcttgttta tgattttacc gacgagacgt ccgaagtatc tactaccgac   1980
   aaaattgcgg atatcactat catcatcccg tacatcggtc cggctctgaa cattggcaac   2040
   atgctgtaca aagacgactt cgttggcgca ctgatcttct ccggtgcggt gatcctgctg   2100
   gagttcatcc cggaaatcgc catcccggta ctgggcacct ttgctctggt ttcttacatt   2160
   gcaaacaagg ttctgactgt acaaaccatc gacaacgcgc tgagcaaacg taacgaaaaa   2220
   tgggatgaag tttacaaata tatcgtgacc aactggctgg ctaaggttaa tactcagatc   2280
   gacctcatcc gcaaaaaaat gaaagaagca ctggaaaacc aggcggaagc taccaaggca   2340
   atcattaact accagtacaa ccagtacacc gaggaagaaa aaaacaacat caacttcaac   2400
   atcgacgatc tgtcctctaa actgaacgaa tccatcaaca aagctatgat caacatcaac   2460
   aagttcctga accagtgctc tgtaagctat ctgatgaact ccatgatccc gtacggtgtt   2520
   aaacgtctgg aggacttcga tgcgtctctg aaagacgccc tgctgaaata catttacgac   2580
   aaccgtggca ctctgatcgg tcaggttgat cgtctgaagg acaaagtgaa caatacctta   2640
   tcgaccgaca tcccttttca gctcagtaaa tatgtcgata accaacgcct tttgtccact   2700
   ctagactag                                                           2709
<210> 76
   <211> 902
   <212> PRT
   <213> Artificial Sequence
<220>
   <223> Synthetic
<400> 76
   Gly Ser Met Glu Phe Val Asn Lys Gln Phe Asn Tyr Lys Asp Pro Val
   1               5                   10                  15
   Asn Gly Val Asp Ile Ala Tyr Ile Lys Ile Pro Asn Ala Gly Gln Met
               20                  25                  30
   Gln Pro Val Lys Ala Phe Lys Ile His Asn Lys Ile Trp Val Ile Pro
           35                  40                  45
   Glu Arg Asp Thr Phe Thr Asn Pro Glu Glu Gly Asp Leu Asn Pro Pro
       50                  55                  60
   Pro Glu Ala Lys Gln Val Pro Val Ser Tyr Tyr Asp Ser Thr Tyr Leu
   65                  70                  75                  80
   Ser Thr Asp Asn Glu Lys Asp Asn Tyr Leu Lys Gly Val Thr Lys Leu
   85 90 95
   Phe Glu Arg Ile Tyr Ser Thr Asp Leu Gly Arg Met Leu Leu Thr Ser
   100 105 110
   Ile Val Arg Gly Ile Pro Phe Trp Gly Gly Ser Thr Ile Asp Thr Glu
   115 120 125
   Leu Lys Val Ile Asp Thr Asn Cys Ile Asn Val Ile Gln Pro Asp Gly
   130 135 140
   Ser Tyr Arg Ser Glu Glu Leu Asn Leu Val Ile Ile Gly Pro Ser Ala
   145 150 155 160
   Asp Ile Ile Gln Phe Glu Cys Lys Ser Phe Gly His Glu Val Leu Asn
   165 170 175
   Leu Thr Arg Asn Gly Tyr Gly Ser Thr Gln Tyr Ile Arg Phe Ser Pro
   180 185 190
   Asp Phe Thr Phe Gly Phe Glu Glu Ser Leu Glu Val Asp Thr Asn Pro
   195 200 205
   Leu Leu Gly Ala Gly Lys Phe Ala Thr Asp Pro Ala Val Thr Leu Ala
   210 215 220
   His Glu Leu Ile His Ala Gly His Arg Leu Tyr Gly Ile Ala Ile Asn
   225 230 235 240
   Pro Asn Arg Val Phe Lys Val Asn Thr Asn Ala Tyr Tyr Glu Met Ser
   245 250 255
   Gly Leu Glu Val Ser Phe Glu Glu Leu Arg Thr Phe Gly Gly His Asp
   260 265 270
   Ala Lys Phe Ile Asp Ser Leu Gln Glu Asn Glu Phe Arg Leu Tyr Tyr
   275 280 285
   Tyr Asn Lys Phe Lys Asp Ile Ala Ser Thr Leu Asn Lys Ala Lys Ser
   290 295 300
   Ile Val Gly Thr Thr Ala Ser Leu Gln Tyr Met Lys Asn Val Phe Lys
   305 310 315 320
   Glu Lys Tyr Leu Leu Ser Glu Asp Thr Ser Gly Lys Phe Ser Val Asp
   325 330 335
   Lys Leu Lys Phe Asp Lys Leu Tyr Lys Met Leu Thr Glu Ile Tyr Thr
   340 345 350
   Glu Asp Asn Phe Val Lys Phe Phe Lys Val Leu Asn Arg Lys Thr Tyr
   355 360 365
   Leu Asn Phe Asp Lys Ala Val Phe Lys Ile Asn Ile Val Pro Lys Val
   370 375 380
   Asn Tyr Thr Ile Tyr Asp Gly Phe Asn Leu Arg Asn Thr Asn Leu Ala
   385 390 395 400
   Ala Asn Phe Asn Gly Gln Asn Thr Glu Ile Asn Asn Met Asn Phe Thr
   405 410 415
   Lys Leu Lys Asn Phe Thr Gly Leu Phe Glu Phe Tyr Lys Leu Leu Cys
   420 425 430
   Val Asp Gly Ile Ile Thr Ser Lys Thr Lys Ser Asp Asp Asp Asp Lys
   435 440 445
   Phe Gly Gly Phe Thr Gly Ala Arg Lys Ser Ala Arg Lys Arg Lys Asn
   450 455 460
   Gln Ala Leu Ala Gly Gly Gly Gly Ser Gly Gly Gly Gly Ser Gly Gly
   465 470 475 480
   Gly Gly Ser Ala Leu Val Leu Gln Cys Ile Lys Val Asn Asn Trp Asp
   485 490 495
   Leu Phe Phe Ser Pro Ser Glu Asp Asn Phe Thr Asn Asp Leu Asn Lys
   500 505 510
   Gly Glu Glu Ile Thr Ser Asp Thr Asn Ile Glu Ala Ala Glu Glu Asn
   515 520 525
   Ile Ser Leu Asp Leu Ile Gln Gln Tyr Tyr Leu Thr Phe Asn Phe Asp
   530 535 540
   Asn Glu Pro Glu Asn Ile Ser Ile Glu Asn Leu Ser Ser Asp Ile Ile
   545 550 555 560
   Gly Gln Leu Glu Leu Met Pro Asn Ile Glu Arg Phe Pro Asn Gly Lys
   565 570 575
   Lys Tyr Glu Leu Asp Lys Tyr Thr Met Phe His Tyr Leu Arg Ala Gln
   580 585 590
   Glu Phe Glu His Gly Lys Ser Arg Ile Ala Leu Thr Asn Ser Val Asn
   595 600 605
   Glu Ala Leu Leu Asn Pro Ser Arg Val Tyr Thr Phe Phe Ser Ser Asp
   610 615 620
   Tyr Val Lys Lys Val Asn Lys Ala Thr Glu Ala Ala Met Phe Leu Gly
   625 630 635 640
   Trp Val Glu Gln Leu Val Tyr Asp Phe Thr Asp Glu Thr Ser Glu Val
   645 650 655
   Ser Thr Thr Asp Lys Ile Ala Asp Ile Thr Ile Ile Ile Pro Tyr Ile
   660 665 670
   Gly Pro Ala Leu Asn Ile Gly Asn Met Leu Tyr Lys Asp Asp Phe Val
   675 680 685
   Gly Ala Leu Ile Phe Ser Gly Ala Val Ile Leu Leu Glu Phe Ile Pro
   690 695 700
   Glu Ile Ala Ile Pro Val Leu Gly Thr Phe Ala Leu Val Ser Tyr Ile
   705 710 715 720
   Ala Asn Lys Val Leu Thr Val Gln Thr Ile Asp Asn Ala Leu Ser Lys
   725 730 735
   Arg Asn Glu Lys Trp Asp Glu Val Tyr Lys Tyr Ile Val Thr Asn Trp
   740 745 750
   Leu Ala Lys Val Asn Thr Gln Ile Asp Leu Ile Arg Lys Lys Met Lys
   755 760 765
   Glu Ala Leu Glu Asn Gln Ala Glu Ala Thr Lys Ala Ile Ile Asn Tyr
   770 775 780
   Gln Tyr Asn Gln Tyr Thr Glu Glu Glu Lys Asn Asn Ile Asn Phe Asn
   785 790 795 800
   Ile Asp Asp Leu Ser Ser Lys Leu Asn Glu Ser Ile Asn Lys Ala Met
   805 810 815
   Ile Asn Ile Asn Lys Phe Leu Asn Gln Cys Ser Val Ser Tyr Leu Met
               820                 825                 830
   Asn Ser Met Ile Pro Tyr Gly Val Lys Arg Leu Glu Asp Phe Asp Ala
           835                 840                 845
   Ser Leu Lys Asp Ala Leu Leu Lys Tyr Ile Tyr Asp Asn Arg Gly Thr
       850                 855                 860
   Leu Ile Gly Gln Val Asp Arg Leu Lys Asp Lys Val Asn Asn Thr Leu
   865                 870                 875                 880
   Ser Thr Asp Ile Pro Phe Gln Leu Ser Lys Tyr Val Asp Asn Gln Arg
                   885                 890                 895
   Leu Leu Ser Thr Leu Asp
               900
<210> 77
   <211> 207
   <212> DNA
   <213> Artificial Sequence
<220>
   <223> Synthetic
<400> 77
   ggatccacgc acgtcgacgg catcattacc tccaaaacta aatctctgat agaaggtaga     60
   tttggcggtt tcacgggcgc acgcaaatca gcgcgtaaac gtaagaacca ggcgctagcg    120
   ggcggtggcg gtagcggcgg tggcggtagc ggcggtggcg gtagcgcact agtgctgcag    180
   acgcacggtc tagaatgata aaagctt                                        207
<210> 78
   <211> 2742
   <212> DNA
   <213> Artificial Sequence
<220>
   <223> Synthetic
<400> 78
   ggatccgaat tcatgccgat caccatcaac aacttcaact acagcgatcc ggtggataac     60
   aaaaacatcc tgtacctgga tacccatctg aataccctgg cgaacgaacc ggaaaaagcg    120
   tttcgtatca ccggcaacat ttgggttatt ccggatcgtt ttagccgtaa cagcaacccg    180
   aatctgaata aaccgccgcg tgttaccagc ccgaaaagcg gttattacga tccgaactat    240
   ctgagcaccg atagcgataa agataccttc ctgaaagaaa tcatcaaact gttcaaacgc 300
   atcaacagcc gtgaaattgg cgaagaactg atctatcgcc tgagcaccga tattccgttt 360
   ccgggcaaca acaacacccc gatcaacacc tttgatttcg atgtggattt caacagcgtt 420
   gatgttaaaa cccgccaggg taacaattgg gtgaaaaccg gcagcattaa cccgagcgtg 480
   attattaccg gtccgcgcga aaacattatt gatccggaaa ccagcacctt taaactgacc 540
   aacaacacct ttgcggcgca ggaaggtttt ggcgcgctga gcattattag cattagcccg 600
   cgctttatgc tgacctatag caacgcgacc aacgatgttg gtgaaggccg tttcagcaaa 660
   agcgaatttt gcatggaccc gatcctgatc ctgatgcatg aactgaacca tgcgatgcat 720
   aacctgtatg gcatcgcgat tccgaacgat cagaccatta gcagcgtgac cagcaacatc 780
   ttttacagcc agtacaacgt gaaactggaa tatgcggaaa tctatgcgtt tggcggtccg 840
   accattgatc tgattccgaa aagcgcgcgc aaatacttcg aagaaaaagc gctggattac 900
   tatcgcagca ttgcgaaacg tctgaacagc attaccaccg cgaatccgag cagcttcaac 960
   aaatatatcg gcgaatataa acagaaactg atccgcaaat atcgctttgt ggtggaaagc 1020
   agcggcgaag ttaccgttaa ccgcaataaa ttcgtggaac tgtacaacga actgacccag 1080
   atcttcaccg aatttaacta tgcgaaaatc tataacgtgc agaaccgtaa aatctacctg 1140
   agcaacgtgt ataccccggt gaccgcgaat attctggatg ataacgtgta cgatatccag 1200
   aacggcttta acatcccgaa aagcaacctg aacgttctgt ttatgggcca gaacctgagc 1260
   cgtaatccgg cgctgcgtaa agtgaacccg gaaaacatgc tgtacctgtt caccaaattt 1320
   tgcgtcgacg gcatcattac ctccaaaact aaatctctga tagaaggtag atttggcggt 1380
   ttcacgggcg cacgcaaatc agcgcgtaaa cgtaagaacc aggcgctagc gggcggtggc 1440
   ggtagcggcg gtggcggtag cggcggtggc ggtagcgcac tagtgctgca gtgtcgtgaa 1500
   ctgctggtga aaaacaccga tctgccgttt attggcgata tcagcgatgt gaaaaccgat 1560
   atcttcctgc gcaaagatat caacgaagaa accgaagtga tctactaccc ggataacgtg 1620
   agcgttgatc aggtgatcct gagcaaaaac accagcgaac atggtcagct ggatctgctg 1680
   tatccgagca ttgatagcga aagcgaaatt ctgccgggcg aaaaccaggt gttttacgat 1740
   aaccgtaccc agaacgtgga ttacctgaac agctattact acctggaaag ccagaaactg 1800
   agcgataacg tggaagattt tacctttacc cgcagcattg aagaagcgct ggataacagc 1860
   gcgaaagttt acacctattt tccgaccctg gcgaacaaag ttaatgcggg tgttcagggc 1920
   ggtctgtttc tgatgtgggc gaacgatgtg gtggaagatt tcaccaccaa catcctgcgt 1980
   aaagataccc tggataaaat cagcgatgtt agcgcgatta ttccgtatat tggtccggcg 2040
   ctgaacatta gcaatagcgt gcgtcgtggc aattttaccg aagcgtttgc ggttaccggt 2100
   gtgaccattc tgctggaagc gtttccggaa tttaccattc cggcgctggg tgcgtttgtg   2160
   atctatagca aagtgcagga acgcaacgaa atcatcaaaa ccatcgataa ctgcctggaa   2220
   cagcgtatta aacgctggaa agatagctat gaatggatga tgggcacctg gctgagccgt   2280
   attatcaccc agttcaacaa catcagctac cagatgtacg atagcctgaa ctatcaggcg   2340
   ggtgcgatta aagcgaaaat cgatctggaa tacaaaaaat acagcggcag cgataaagaa   2400
   aacatcaaaa gccaggttga aaacctgaaa aacagcctgg atgtgaaaat tagcgaagcg   2460
   atgaataaca tcaacaaatt catccgcgaa tgcagcgtga cctacctgtt caaaaacatg   2520
   ctgccgaaag tgatcgatga actgaacgaa tttgatcgca acaccaaagc gaaactgatc   2580
   aacctgatcg atagccacaa cattattctg gtgggcgaag tggataaact gaaagcgaaa   2640
   gttaacaaca gcttccagaa caccatcccg tttaacatct tcagctatac caacaacagc   2700
   ctgctgaaag atatcatcaa cgaatacttc aatctagact ag                      2742
<210> 79
   <211> 913
   <212> PRT
   <213> Artificial Sequence
<220>
   <223> Synthetic
<400> 79
   Gly Ser Glu Phe Met Pro Ile Thr Ile Asn Asn Phe Asn Tyr Ser Asp
   1               5                   10                  15
   Pro Val Asp Asn Lys Asn Ile Leu Tyr Leu Asp Thr His Leu Asn Thr
               20                  25                  30
   Leu Ala Asn Glu Pro Glu Lys Ala Phe Arg Ile Thr Gly Asn Ile Trp
           35                  40                  45
   Val Ile Pro Asp Arg Phe Ser Arg Asn Ser Asn Pro Asn Leu Asn Lys
       50                  55                  60
   Pro Pro Arg Val Thr Ser Pro Lys Ser Gly Tyr Tyr Asp Pro Asn Tyr
   65                  70                  75                  80
   Leu Ser Thr Asp Ser Asp Lys Asp Thr Phe Leu Lys Glu Ile Ile Lys
                   85                  90                  95
   Leu Phe Lys Arg Ile Asn Ser Arg Glu Ile Gly Glu Glu Leu Ile Tyr
               100                 105                 110
   Arg Leu Ser Thr Asp Ile Pro Phe Pro Gly Asn Asn Asn Thr Pro Ile
   115 120 125
   Asn Thr Phe Asp Phe Asp Val Asp Phe Asn Ser Val Asp Val Lys Thr
   130 135 140
   Arg Gln Gly Asn Asn Trp Val Lys Thr Gly Ser Ile Asn Pro Ser Val
   145 150 155 160
   Ile Ile Thr Gly Pro Arg Glu Asn Ile Ile Asp Pro Glu Thr Ser Thr
   165 170 175
   Phe Lys Leu Thr Asn Asn Thr Phe Ala Ala Gln Glu Gly Phe Gly Ala
   180 185 190
   Leu Ser Ile Ile Ser Ile Ser Pro Arg Phe Met Leu Thr Tyr Ser Asn
   195 200 205
   Ala Thr Asn Asp Val Gly Glu Gly Arg Phe Ser Lys Ser Glu Phe Cys
   210 215 220
   Met Asp Pro Ile Leu Ile Leu Met His Glu Leu Asn His Ala Met His
   225 230 235 240
   Asn Leu Tyr Gly Ile Ala Ile Pro Asn Asp Gln Thr Ile Ser Ser Val
   245 250 255
   Thr Ser Asn Ile Phe Tyr Ser Gln Tyr Asn Val Lys Leu Glu Tyr Ala
   260 265 270
   Glu Ile Tyr Ala Phe Gly Gly Pro Thr Ile Asp Leu Ile Pro Lys Ser
   275 280 285
   Ala Arg Lys Tyr Phe Glu Glu Lys Ala Leu Asp Tyr Tyr Arg Ser Ile
   290 295 300
   Ala Lys Arg Leu Asn Ser Ile Thr Thr Ala Asn Pro Ser Ser Phe Asn
   305 310 315 320
   Lys Tyr Ile Gly Glu Tyr Lys Gln Lys Leu Ile Arg Lys Tyr Arg Phe
   325 330 335
   Val Val Glu Ser Ser Gly Glu Val Thr Val Asn Arg Asn Lys Phe Val
   340 345 350
   Glu Leu Tyr Asn Glu Leu Thr Gln Ile Phe Thr Glu Phe Asn Tyr Ala
   355 360 365
   Lys Ile Tyr Asn Val Gln Asn Arg Lys Ile Tyr Leu Ser Asn Val Tyr
   370 375 380
   Thr Pro Val Thr Ala Asn Ile Leu Asp Asp Asn Val Tyr Asp Ile Gln
   385 390 395 400
   Asn Gly Phe Asn Ile Pro Lys Ser Asn Leu Asn Val Leu Phe Met Gly
   405 410 415
   Gln Asn Leu Ser Arg Asn Pro Ala Leu Arg Lys Val Asn Pro Glu Asn
   420 425 430
   Met Leu Tyr Leu Phe Thr Lys Phe Cys Val Asp Gly Ile Ile Thr Ser
   435 440 445
   Lys Thr Lys Ser Leu Ile Glu Gly Arg Phe Gly Gly Phe Thr Gly Ala
   450 455 460
   Arg Lys Ser Ala Arg Lys Arg Lys Asn Gln Ala Leu Ala Gly Gly Gly
   465 470 475 480
   Gly Ser Gly Gly Gly Gly Ser Gly Gly Gly Gly Ser Ala Leu Val Leu
   485 490 495
   Gln Cys Arg Glu Leu Leu Val Lys Asn Thr Asp Leu Pro Phe Ile Gly
   500 505 510
   Asp Ile Ser Asp Val Lys Thr Asp Ile Phe Leu Arg Lys Asp Ile Asn
   515 520 525
   Glu Glu Thr Glu Val Ile Tyr Tyr Pro Asp Asn Val Ser Val Asp Gln
   530 535 540
   Val Ile Leu Ser Lys Asn Thr Ser Glu His Gly Gln Leu Asp Leu Leu
   545 550 555 560
   Tyr Pro Ser Ile Asp Ser Glu Ser Glu Ile Leu Pro Gly Glu Asn Gln
   565 570 575
   Val Phe Tyr Asp Asn Arg Thr Gln Asn Val Asp Tyr Leu Asn Ser Tyr
   580 585 590
   Tyr Tyr Leu Glu Ser Gln Lys Leu Ser Asp Asn Val Glu Asp Phe Thr
   595 600 605
   Phe Thr Arg Ser Ile Glu Glu Ala Leu Asp Asn Ser Ala Lys Val Tyr
   610 615 620
   Thr Tyr Phe Pro Thr Leu Ala Asn Lys Val Asn Ala Gly Val Gln Gly
   625 630 635 640
   Gly Leu Phe Leu Met Trp Ala Asn Asp Val Val Glu Asp Phe Thr Thr
   645 650 655
   Asn Ile Leu Arg Lys Asp Thr Leu Asp Lys Ile Ser Asp Val Ser Ala
   660 665 670
   Ile Ile Pro Tyr Ile Gly Pro Ala Leu Asn Ile Ser Asn Ser Val Arg
   675 680 685
   Arg Gly Asn Phe Thr Glu Ala Phe Ala Val Thr Gly Val Thr Ile Leu
   690 695 700
   Leu Glu Ala Phe Pro Glu Phe Thr Ile Pro Ala Leu Gly Ala Phe Val
   705 710 715 720
   Ile Tyr Ser Lys Val Gln Glu Arg Asn Glu Ile Ile Lys Thr Ile Asp
   725 730 735
   Asn Cys Leu Glu Gln Arg Ile Lys Arg Trp Lys Asp Ser Tyr Glu Trp
   740 745 750
   Met Met Gly Thr Trp Leu Ser Arg Ile Ile Thr Gln Phe Asn Asn Ile
   755 760 765
   Ser Tyr Gln Met Tyr Asp Ser Leu Asn Tyr Gln Ala Gly Ala Ile Lys
   770 775 780
   Ala Lys Ile Asp Leu Glu Tyr Lys Lys Tyr Ser Gly Ser Asp Lys Glu
   785 790 795 800
   Asn Ile Lys Ser Gln Val Glu Asn Leu Lys Asn Ser Leu Asp Val Lys
   805 810 815
   Ile Ser Glu Ala Met Asn Asn Ile Asn Lys Phe Ile Arg Glu Cys Ser
   820 825 830
   Val Thr Tyr Leu Phe Lys Asn Met Leu Pro Lys Val Ile Asp Glu Leu
   835 840 845
   Asn Glu Phe Asp Arg Asn Thr Lys Ala Lys Leu Ile Asn Leu Ile Asp
       850                 855                 860
   Ser His Asn Ile Ile Leu Val Gly Glu Val Asp Lys Leu Lys Ala Lys
   865                 870                 875                 880
   Val Asn Asn Ser Phe Gln Asn Thr Ile Pro Phe Asn Ile Phe Ser Tyr
                   885                 890                 895
   Thr Asn Asn Ser Leu Leu Lys Asp Ile Ile Asn Glu Tyr Phe Asn Leu
               900                 905                 910
   Asp
<210> 80
   <211> 2673
   <212> DNA
   <213> Artificial Sequence
<220>
   <223> Synthetic
<400> 80
   ggatccatgg agttcgttaa caaacagttc aactataaag acccagttaa cggtgttgac      60
   attgcttaca tcaaaatccc gaacgctggc cagatgcagc cggtaaaggc attcaaaatc     120
   cacaacaaaa tctgggttat cccggaacgt gataccttta ctaacccgga agaaggtgac    180
   ctgaacccgc caccggaagc gaaacaggtg ccggtatctt actatgactc cacctacctg     240
   tctaccgata acgaaaagga caactacctg aaaggtgtta ctaaactgtt cgagcgtatt     300
   tactccaccg acctgggccg tatgctgctg actagcatcg ttcgcggtat cccgttctgg     360
   ggcggttcta ccatcgatac cgaactgaaa gtaatcgaca ctaactgcat caacgttatt     420
   cagccggacg gttcctatcg ttccgaagaa ctgaacctgg tgatcatcgg cccgtctgct     480
   gatatcatcc agttcgagtg taagagcttt ggtcacgaag ttctgaacct cacccgtaac     540
   ggctacggtt ccactcagta catccgtttc tctccggact tcaccttcgg ttttgaagaa     600
   tccctggaag tagacacgaa cccactgctg ggcgctggta aattcgcaac tgatcctgcg     660
   gttaccctgg ctcacgaact gattcatgca ggccaccgcc tgtacggtat cgccatcaat     720
   ccgaaccgtg tcttcaaagt taacaccaac gcgtattacg agatgtccgg tctggaagtt     780
   agcttcgaag aactgcgtac ttttggcggt cacgacgcta aattcatcga ctctctgcaa     840
   gaaaacgagt tccgtctgta ctactataac aagttcaaag atatcgcatc caccctgaac     900
   aaagcgaaat ccatcgtggg taccactgct tctctccagt acatgaagaa cgtttttaaa 960
   gaaaaatacc tgctcagcga agacacctcc ggcaaattct ctgtagacaa gttgaaattc 1020
   gataaacttt acaaaatgct gactgaaatt tacaccgaag acaacttcgt taagttcttt 1080
   aaagttctga accgcaaaac ctatctgaac ttcgacaagg cagtattcaa aatcaacatc 1140
   gtgccgaaag ttaactacac tatctacgat ggtttcaacc tgcgtaacac caacctggct 1200
   gctaatttta acggccagaa cacggaaatc aacaacatga acttcacaaa actgaaaaac 1260
   ttcactggtc tgttcgagtt ttacaagctg ctgtgcgtcg acggcatcat tacctccaaa 1320
   actaaatctc tgatagaagg tagatacggt ggtttcctgg cgctagcggg cggtggcggt 1380
   agcggcggtg gcggtagcgg cggtggcggt agcgcactag tgctgcagtg tatcaaggtt 1440
   aacaactggg atttattctt cagcccgagt gaagacaact tcaccaacga cctgaacaaa 1500
   ggtgaagaaa tcacctcaga tactaacatc gaagcagccg aagaaaacat ctcgctggac 1560
   ctgatccagc agtactacct gacctttaat ttcgacaacg agccggaaaa catttctatc 1620
   gaaaacctga gctctgatat catcggccag ctggaactga tgccgaacat cgaacgtttc 1680
   ccaaacggta aaaagtacga gctggacaaa tataccatgt tccactacct gcgcgcgcag 1740
   gaatttgaac acggcaaatc ccgtatcgca ctgactaact ccgttaacga agctctgctc 1800
   aacccgtccc gtgtatacac cttcttctct agcgactacg tgaaaaaggt caacaaagcg 1860
   actgaagctg caatgttctt gggttgggtt gaacagcttg tttatgattt taccgacgag 1920
   acgtccgaag tatctactac cgacaaaatt gcggatatca ctatcatcat cccgtacatc 1980
   ggtccggctc tgaacattgg caacatgctg tacaaagacg acttcgttgg cgcactgatc 2040
   ttctccggtg cggtgatcct gctggagttc atcccggaaa tcgccatccc ggtactgggc 2100
   acctttgctc tggtttctta cattgcaaac aaggttctga ctgtacaaac catcgacaac 2160
   gcgctgagca aacgtaacga aaaatgggat gaagtttaca aatatatcgt gaccaactgg 2220
   ctggctaagg ttaatactca gatcgacctc atccgcaaaa aaatgaaaga agcactggaa 2280
   aaccaggcgg aagctaccaa ggcaatcatt aactaccagt acaaccagta caccgaggaa 2340
   gaaaaaaaca acatcaactt caacatcgac gatctgtcct ctaaactgaa cgaatccatc 2400
   aacaaagcta tgatcaacat caacaagttc ctgaaccagt gctctgtaag ctatctgatg 2460
   aactccatga tcccgtacgg tgttaaacgt ctggaggact tcgatgcgtc tctgaaagac 2520
   gccctgctga aatacattta cgacaaccgt ggcactctga tcggtcaggt tgatcgtctg 2580
   aaggacaaag tgaacaatac cttatcgacc gacatccctt ttcagctcag taaatatgtc 2640
   gataaccaac gccttttgtc cactctagac tag 2673
<210> 81
   <211> 890
   <212> PRT
   <213> Artificial Sequence
<220>
   <223> Synthetic
<400> 81
   Gly Ser Met Glu Phe Val Asn Lys Gln Phe Asn Tyr Lys Asp Pro Val
   1 5 10 15
   Asn Gly Val Asp Ile Ala Tyr Ile Lys Ile Pro Asn Ala Gly Gln Met
   20 25 30
   Gln Pro Val Lys Ala Phe Lys Ile His Asn Lys Ile Trp Val Ile Pro
   35 40 45
   Glu Arg Asp Thr Phe Thr Asn Pro Glu Glu Gly Asp Leu Asn Pro Pro
   50 55 60
   Pro Glu Ala Lys Gln Val Pro Val Ser Tyr Tyr Asp Ser Thr Tyr Leu
   65 70 75 80
   Ser Thr Asp Asn Glu Lys Asp Asn Tyr Leu Lys Gly Val Thr Lys Leu
   85 90 95
   Phe Glu Arg Ile Tyr Ser Thr Asp Leu Gly Arg Met Leu Leu Thr Ser
   100 105 110
   Ile Val Arg Gly Ile Pro Phe Trp Gly Gly Ser Thr Ile Asp Thr Glu
   115 120 125
   Leu Lys Val Ile Asp Thr Asn Cys Ile Asn Val Ile Gln Pro Asp Gly
   130 135 140
   Ser Tyr Arg Ser Glu Glu Leu Asn Leu Val Ile Ile Gly Pro Ser Ala
   145 150 155 160
   Asp Ile Ile Gln Phe Glu Cys Lys Ser Phe Gly His Glu Val Leu Asn
   165 170 175
   Leu Thr Arg Asn Gly Tyr Gly Ser Thr Gln Tyr Ile Arg Phe Ser Pro
   180 185 190
   Asp Phe Thr Phe Gly Phe Glu Glu Ser Leu Glu Val Asp Thr Asn Pro
   195 200 205
   Leu Leu Gly Ala Gly Lys Phe Ala Thr Asp Pro Ala Val Thr Leu Ala
       210                 215                 220
   His Glu Leu Ile His Ala Gly His Arg Leu Tyr Gly Ile Ala Ile Asn
   225                 230                 235                 240
   Pro Asn Arg Val Phe Lys Val Asn Thr Asn Ala Tyr Tyr Glu Met Ser
                   245                 250                 255
   Gly Leu Glu Val Ser Phe Glu Glu Leu Arg Thr Phe Gly Gly His Asp
               260                 265                 270
   Ala Lys Phe Ile Asp Ser Leu Gln Glu Asn Glu Phe Arg Leu Tyr Tyr
           275                 280                 285
   Tyr Asn Lys Phe Lys Asp Ile Ala Ser Thr Leu Asn Lys Ala Lys Ser
       290                 295                 300
   Ile Val Gly Thr Thr Ala Ser Leu Gln Tyr Met Lys Asn Val Phe Lys
   305                 310                 315                 320
   Glu Lys Tyr Leu Leu Ser Glu Asp Thr Ser Gly Lys Phe Ser Val Asp
                   325                 330                 335
   Lys Leu Lys Phe Asp Lys Leu Tyr Lys Met Leu Thr Glu Ile Tyr Thr
               340                 345                 350
   Glu Asp Asn Phe Val Lys Phe Phe Lys Val Leu Asn Arg Lys Thr Tyr
           355                 360                 365
   Leu Asn Phe Asp Lys Ala Val Phe Lys Ile Asn Ile Val Pro Lys Val
       370                 375                 380
   Asn Tyr Thr Ile Tyr Asp Gly Phe Asn Leu Arg Asn Thr Asn Leu Ala
   385                 390                 395                 400
   Ala Asn Phe Asn Gly Gln Asn Thr Glu Ile Asn Asn Met Asn Phe Thr
                   405                 410                 415
   Lys Leu Lys Asn Phe Thr Gly Leu Phe Glu Phe Tyr Lys Leu Leu Cys
               420                 425                 430
   Val Asp Gly Ile Ile Thr Ser Lys Thr Lys Ser Leu Ile Glu Gly Arg
           435                 440                 445
   Tyr Gly Gly Phe Leu Ala Leu Ala Gly Gly Gly Gly Ser Gly Gly Gly
       450                 455                 460
   Gly Ser Gly Gly Gly Gly Ser Ala Leu Val Leu Gln Cys Ile Lys Val
   465                 470                 475                 480
   Asn Asn Trp Asp Leu Phe Phe Ser Pro Ser Glu Asp Asn Phe Thr Asn
                   485                 490                 495
   Asp Leu Asn Lys Gly Glu Glu Ile Thr Ser Asp Thr Asn Ile Glu Ala
               500                 505                 510
   Ala Glu Glu Asn Ile Ser Leu Asp Leu Ile Gln Gln Tyr Tyr Leu Thr
           515                 520                 525
   Phe Asn Phe Asp Asn Glu Pro Glu Asn Ile Ser Ile Glu Asn Leu Ser
       530                 535                 540
   Ser Asp Ile Ile Gly Gln Leu Glu Leu Met Pro Asn Ile Glu Arg Phe
   545                 550                 555                 560
   Pro Asn Gly Lys Lys Tyr Glu Leu Asp Lys Tyr Thr Met Phe His Tyr
                   565                 570                 575
   Leu Arg Ala Gln Glu Phe Glu His Gly Lys Ser Arg Ile Ala Leu Thr
               580                 585                 590
   Asn Ser Val Asn Glu Ala Leu Leu Asn Pro Ser Arg Val Tyr Thr Phe
           595                 600                 605
   Phe Ser Ser Asp Tyr Val Lys Lys Val Asn Lys Ala Thr Glu Ala Ala
       610                 615                 620
   Met Phe Leu Gly Trp Val Glu Gln Leu Val Tyr Asp Phe Thr Asp Glu
   625                 630                 635                 640
   Thr Ser Glu Val Ser Thr Thr Asp Lys Ile Ala Asp Ile Thr Ile Ile
                   645                 650                 655
   Ile Pro Tyr Ile Gly Pro Ala Leu Asn Ile Gly Asn Met Leu Tyr Lys
               660                 665                 670
   Asp Asp Phe Val Gly Ala Leu Ile Phe Ser Gly Ala Val Ile Leu Leu
           675                 680                 685
   Glu Phe Ile Pro Glu Ile Ala Ile Pro Val Leu Gly Thr Phe Ala Leu
   690 695 700
   Val Ser Tyr Ile Ala Asn Lys Val Leu Thr Val Gln Thr Ile Asp Asn
   705 710 715 720
   Ala Leu Ser Lys Arg Asn Glu Lys Trp Asp Glu Val Tyr Lys Tyr Ile
   725 730 735
   Val Thr Asn Trp Leu Ala Lys Val Asn Thr Gln Ile Asp Leu Ile Arg
   740 745 750
   Lys Lys Met Lys Glu Ala Leu Glu Asn Gln Ala Glu Ala Thr Lys Ala
   755 760 765
   Ile Ile Asn Tyr Gln Tyr Asn Gln Tyr Thr Glu Glu Glu Lys Asn Asn
   770 775 780
   Ile Asn Phe Asn Ile Asp Asp Leu Ser Ser Lys Leu Asn Glu Ser Ile
   785 790 795 800
   Asn Lys Ala Met Ile Asn Ile Asn Lys Phe Leu Asn Gln Cys Ser Val
   805 810 815
   Ser Tyr Leu Met Asn Ser Met Ile Pro Tyr Gly Val Lys Arg Leu Glu
   820 825 830
   Asp Phe Asp Ala Ser Leu Lys Asp Ala Leu Leu Lys Tyr Ile Tyr Asp
   835 840 845
   Asn Arg Gly Thr Leu Ile Gly Gln Val Asp Arg Leu Lys Asp Lys Val
   850 855 860
   Asn Asn Thr Leu Ser Thr Asp Ile Pro Phe Gln Leu Ser Lys Tyr Val
   865 870 875 880
   Asp Asn Gln Arg Leu Leu Ser Thr Leu Asp
   885 890
<210> 82
   <211> 2709
   <212> DNA
   <213> Artificial Sequence
<220>
   <223> Synthetic
<400> 82
   ggatccatgg agttcgttaa caaacagttc aactataaag acccagttaa cggtgttgac 60
   attgcttaca tcaaaatccc gaacgctggc cagatgcagc cggtaaaggc attcaaaatc    120
   cacaacaaaa tctgggttat cccggaacgt gataccttta ctaacccgga agaaggtgac    180
   ctgaacccgc caccggaagc gaaacaggtg ccggtatctt actatgactc cacctacctg    240
   tctaccgata acgaaaagga caactacctg aaaggtgtta ctaaactgtt cgagcgtatt    300
   tactccaccg acctgggccg tatgctgctg actagcatcg ttcgcggtat cccgttctgg    360
   ggcggttcta ccatcgatac cgaactgaaa gtaatcgaca ctaactgcat caacgttatt    420
   cagccggacg gttcctatcg ttccgaagaa ctgaacctgg tgatcatcgg cccgtctgct    480
   gatatcatcc agttcgagtg taagagcttt ggtcacgaag ttctgaacct cacccgtaac    540
   ggctacggtt ccactcagta catccgtttc tctccggact tcaccttcgg ttttgaagaa    600
   tccctggaag tagacacgaa cccactgctg ggcgctggta aattcgcaac tgatcctgcg    660
   gttaccctgg ctcacgaact gattcatgca ggccaccgcc tgtacggtat cgccatcaat    720
   ccgaaccgtg tcttcaaagt taacaccaac gcgtattacg agatgtccgg tctggaagtt    780
   agcttcgaag aactgcgtac ttttggcggt cacgacgcta aattcatcga ctctctgcaa    840
   gaaaacgagt tccgtctgta ctactataac aagttcaaag atatcgcatc caccctgaac    900
   aaagcgaaat ccatcgtggg taccactgct tctctccagt acatgaagaa cgtttttaaa    960
   gaaaaatacc tgctcagcga agacacctcc ggcaaattct ctgtagacaa gttgaaattc   1020
   gataaacttt acaaaatgct gactgaaatt tacaccgaag acaacttcgt taagttcttt   1080
   aaagttctga accgcaaaac ctatctgaac ttcgacaagg cagtattcaa aatcaacatc   1140
   gtgccgaaag ttaactacac tatctacgat ggtttcaacc tgcgtaacac caacctggct   1200
   gctaatttta acggccagaa cacggaaatc aacaacatga acttcacaaa actgaaaaac   1260
   ttcactggtc tgttcgagtt ttacaagctg ctgtgcgtcg acggcatcat tacctccaaa   1320
   actaaatctc tgatagaagg tagatatggc ggtttcacgg gcgcacgcaa atcagcgcgt   1380
   aaattagcta accaggcgct agcgggcggt ggcggtagcg gcggtggcgg tagcggcggt   1440
   ggcggtagcg cactagtgct gcagtgtatc aaggttaaca actgggattt attcttcagc   1500
   ccgagtgaag acaacttcac caacgacctg aacaaaggtg aagaaatcac ctcagatact   1560
   aacatcgaag cagccgaaga aaacatctcg ctggacctga tccagcagta ctacctgacc   1620
   tttaatttcg acaacgagcc ggaaaacatt tctatcgaaa acctgagctc tgatatcatc   1680
   ggccagctgg aactgatgcc gaacatcgaa cgtttcccaa acggtaaaaa gtacgagctg   1740
   gacaaatata ccatgttcca ctacctgcgc gcgcaggaat ttgaacacgg caaatcccgt   1800
   atcgcactga ctaactccgt taacgaagct ctgctcaacc cgtcccgtgt atacaccttc   1860
   ttctctagcg actacgtgaa aaaggtcaac aaagcgactg aagctgcaat gttcttgggt 1920
   tgggttgaac agcttgttta tgattttacc gacgagacgt ccgaagtatc tactaccgac 1980
   aaaattgcgg atatcactat catcatcccg tacatcggtc cggctctgaa cattggcaac 2040
   atgctgtaca aagacgactt cgttggcgca ctgatcttct ccggtgcggt gatcctgctg 2100
   gagttcatcc cggaaatcgc catcccggta ctgggcacct ttgctctggt ttcttacatt 2160
   gcaaacaagg ttctgactgt acaaaccatc gacaacgcgc tgagcaaacg taacgaaaaa 2220
   tgggatgaag tttacaaata tatcgtgacc aactggctgg ctaaggttaa tactcagatc 2280
   gacctcatcc gcaaaaaaat gaaagaagca ctggaaaacc aggcggaagc taccaaggca 2340
   atcattaact accagtacaa ccagtacacc gaggaagaaa aaaacaacat caacttcaac 2400
   atcgacgatc tgtcctctaa actgaacgaa tccatcaaca aagctatgat caacatcaac 2460
   aagttcctga accagtgctc tgtaagctat ctgatgaact ccatgatccc gtacggtgtt 2520
   aaacgtctgg aggacttcga tgcgtctctg aaagacgccc tgctgaaata catttacgac 2580
   aaccgtggca ctctgatcgg tcaggttgat cgtctgaagg acaaagtgaa caatacctta 2640
   tcgaccgaca tcccttttca gctcagtaaa tatgtcgata accaacgcct tttgtccact 2700
   ctagactag 2709
<210> 83
   <211> 902
   <212> PRT
   <213> Artificial Sequence
<220>
   <223> Synthetic
<400> 83
   Gly Ser Met Glu Phe Val Asn Lys Gln Phe Asn Tyr Lys Asp Pro Val
   1 5 10 15
   Asn Gly Val Asp Ile Ala Tyr Ile Lys Ile Pro Asn Ala Gly Gln Met
   20 25 30
   Gln Pro Val Lys Ala Phe Lys Ile His Asn Lys Ile Trp Val Ile Pro
   35 40 45
   Glu Arg Asp Thr Phe Thr Asn Pro Glu Glu Gly Asp Leu Asn Pro Pro
   50 55 60
   Pro Glu Ala Lys Gln Val Pro Val Ser Tyr Tyr Asp Ser Thr Tyr Leu
   65 70 75 80
   Ser Thr Asp Asn Glu Lys Asp Asn Tyr Leu Lys Gly Val Thr Lys Leu
                   85                  90                  95
   Phe Glu Arg Ile Tyr Ser Thr Asp Leu Gly Arg Met Leu Leu Thr Ser
               100                 105                 110
   Ile Val Arg Gly Ile Pro Phe Trp Gly Gly Ser Thr Ile Asp Thr Glu
           115                 120                 125
   Leu Lys Val Ile Asp Thr Asn Cys Ile Asn Val Ile Gln Pro Asp Gly
       130                 135                 140
   Ser Tyr Arg Ser Glu Glu Leu Asn Leu Val Ile Ile Gly Pro Ser Ala
   145                 150                 155                 160
   Asp Ile Ile Gln Phe Glu Cys Lys Ser Phe Gly His Glu Val Leu Asn
                   165                 170                 175
   Leu Thr Arg Asn Gly Tyr Gly Ser Thr Gln Tyr Ile Arg Phe Ser Pro
               180                 185                 190
   Asp Phe Thr Phe Gly Phe Glu Glu Ser Leu Glu Val Asp Thr Asn Pro
           195                 200                 205
   Leu Leu Gly Ala Gly Lys Phe Ala Thr Asp Pro Ala Val Thr Leu Ala
       210                 215                 220
   His Glu Leu Ile His Ala Gly His Arg Leu Tyr Gly Ile Ala Ile Asn
   225                 230                 235                 240
   Pro Asn Arg Val Phe Lys Val Asn Thr Asn Ala Tyr Tyr Glu Met Ser
                   245                 250                 255
   Gly Leu Glu Val Ser Phe Glu Glu Leu Arg Thr Phe Gly Gly His Asp
               260                 265                 270
   Ala Lys Phe Ile Asp Ser Leu Gln Glu Asn Glu Phe Arg Leu Tyr Tyr
           275                 280                 285
   Tyr Asn Lys Phe Lys Asp Ile Ala Ser Thr Leu Asn Lys Ala Lys Ser
       290                 295                 300
   Ile Val Gly Thr Thr Ala Ser Leu Gln Tyr Met Lys Asn Val Phe Lys
   305                 310                 315                 320
   Glu Lys Tyr Leu Leu Ser Glu Asp Thr Ser Gly Lys Phe Ser Val Asp
   325 330 335
   Lys Leu Lys Phe Asp Lys Leu Tyr Lys Met Leu Thr Glu Ile Tyr Thr
   340 345 350
   Glu Asp Asn Phe Val Lys Phe Phe Lys Val Leu Asn Arg Lys Thr Tyr
   355 360 365
   Leu Asn Phe Asp Lys Ala Val Phe Lys Ile Asn Ile Val Pro Lys Val
   370 375 380
   Asn Tyr Thr Ile Tyr Asp Gly Phe Asn Leu Arg Asn Thr Asn Leu Ala
   385 390 395 400
   Ala Asn Phe Asn Gly Gln Asn Thr Glu Ile Asn Asn Met Asn Phe Thr
   405 410 415
   Lys Leu Lys Asn Phe Thr Gly Leu Phe Glu Phe Tyr Lys Leu Leu Cys
   420 425 430
   Val Asp Gly Ile Ile Thr Ser Lys Thr Lys Ser Leu Ile Glu Gly Arg
   435 440 445
   Tyr Gly Gly Phe Thr Gly Ala Arg Lys Ser Ala Arg Lys Leu Ala Asn
   450 455 460
   Gln Ala Leu Ala Gly Gly Gly Gly Ser Gly Gly Gly Gly Ser Gly Gly
   465 470 475 480
   Gly Gly Ser Ala Leu Val Leu Gln Cys Ile Lys Val Asn Asn Trp Asp
   485 490 495
   Leu Phe Phe Ser Pro Ser Glu Asp Asn Phe Thr Asn Asp Leu Asn Lys
   500 505 510
   Gly Glu Glu Ile Thr Ser Asp Thr Asn Ile Glu Ala Ala Glu Glu Asn
   515 520 525
   Ile Ser Leu Asp Leu Ile Gln Gln Tyr Tyr Leu Thr Phe Asn Phe Asp
   530 535 540
   Asn Glu Pro Glu Asn Ile Ser Ile Glu Asn Leu Ser Ser Asp Ile Ile
   545 550 555 560
   Gly Gln Leu Glu Leu Met Pro Asn Ile Glu Arg Phe Pro Asn Gly Lys
   565 570 575
   Lys Tyr Glu Leu Asp Lys Tyr Thr Met Phe His Tyr Leu Arg Ala Gln
   580 585 590
   Glu Phe Glu His Gly Lys Ser Arg Ile Ala Leu Thr Asn Ser Val Asn
   595 600 605
   Glu Ala Leu Leu Asn Pro Ser Arg Val Tyr Thr Phe Phe Ser Ser Asp
   610 615 620
   Tyr Val Lys Lys Val Asn Lys Ala Thr Glu Ala Ala Met Phe Leu Gly
   625 630 635 640
   Trp Val Glu Gln Leu Val Tyr Asp Phe Thr Asp Glu Thr Ser Glu Val
   645 650 655
   Ser Thr Thr Asp Lys Ile Ala Asp Ile Thr Ile Ile Ile Pro Tyr Ile
   660 665 670
   Gly Pro Ala Leu Asn Ile Gly Asn Met Leu Tyr Lys Asp Asp Phe Val
   675 680 685
   Gly Ala Leu Ile Phe Ser Gly Ala Val Ile Leu Leu Glu Phe Ile Pro
   690 695 700
   Glu Ile Ala Ile Pro Val Leu Gly Thr Phe Ala Leu Val Ser Tyr Ile
   705 710 715 720
   Ala Asn Lys Val Leu Thr Val Gln Thr Ile Asp Asn Ala Leu Ser Lys
   725 730 735
   Arg Asn Glu Lys Trp Asp Glu Val Tyr Lys Tyr Ile Val Thr Asn Trp
   740 745 750
   Leu Ala Lys Val Asn Thr Gln Ile Asp Leu Ile Arg Lys Lys Met Lys
   755 760 765
   Glu Ala Leu Glu Asn Gln Ala Glu Ala Thr Lys Ala Ile Ile Asn Tyr
   770 775 780
   Gln Tyr Asn Gln Tyr Thr Glu Glu Glu Lys Asn Asn Ile Asn Phe Asn
   785 790 795 800
   Ile Asp Asp Leu Ser Ser Lys Leu Asn Glu Ser Ile Asn Lys Ala Met
   805 810 815
   Ile Asn Ile Asn Lys Phe Leu Asn Gln Cys Ser Val Ser Tyr Leu Met
               820                 825                 830
   Asn Ser Met Ile Pro Tyr Gly Val Lys Arg Leu Glu Asp Phe Asp Ala
           835                 840                 845
   Ser Leu Lys Asp Ala Leu Leu Lys Tyr Ile Tyr Asp Asn Arg Gly Thr
       850                 855                 860
   Leu Ile Gly Gln Val Asp Arg Leu Lys Asp Lys Val Asn Asn Thr Leu
   865                 870                 875                 880
   Ser Thr Asp Ile Pro Phe Gln Leu Ser Lys Tyr Val Asp Asn Gln Arg
                   885                 890                 895
   Leu Leu Ser Thr Leu Asp
               900
<210> 84
   <211> 2709
   <212> DNA
   <213> Artificial Sequence
<220>
   <223> Synthetic
<400> 84
   ggatccatgg agttcgttaa caaacagttc aactataaag acccagttaa cggtgttgac      60
   attgcttaca tcaaaatccc gaacgctggc cagatgcagc cggtaaaggc attcaaaatc     120
   cacaacaaaa tctgggttat cccggaacgt gataccttta ctaacccgga agaaggtgac     180
   ctgaacccgc caccggaagc gaaacaggtg ccggtatctt actatgactc cacctacctg     240
   tctaccgata acgaaaagga caactacctg aaaggtgtta ctaaactgtt cgagcgtatt     300
   tactccaccg acctgggccg tatgctgctg actagcatcg ttcgcggtat cccgttctgg     360
   ggcggttcta ccatcgatac cgaactgaaa gtaatcgaca ctaactgcat caacgttatt     420
   cagccggacg gttcctatcg ttccgaagaa ctgaacctgg tgatcatcgg cccgtctgct     480
   gatatcatcc agttcgagtg taagagcttt ggtcacgaag ttctgaacct cacccgtaac     540
   ggctacggtt ccactcagta catccgtttc tctccggact tcaccttcgg ttttgaagaa     600
   tccctggaag tagacacgaa cccactgctg ggcgctggta aattcgcaac tgatcctgcg     660
   gttaccctgg ctcacgaact gattcatgca ggccaccgcc tgtacggtat cgccatcaat     720
   ccgaaccgtg tcttcaaagt taacaccaac gcgtattacg agatgtccgg tctggaagtt     780
   agcttcgaag aactgcgtac ttttggcggt cacgacgcta aattcatcga ctctctgcaa     840
   gaaaacgagt tccgtctgta ctactataac aagttcaaag atatcgcatc caccctgaac 900
   aaagcgaaat ccatcgtggg taccactgct tctctccagt acatgaagaa cgtttttaaa 960
   gaaaaatacc tgctcagcga agacacctcc ggcaaattct ctgtagacaa gttgaaattc 1020
   gataaacttt acaaaatgct gactgaaatt tacaccgaag acaacttcgt taagttcttt 1080
   aaagttctga accgcaaaac ctatctgaac ttcgacaagg cagtattcaa aatcaacatc 1140
   gtgccgaaag ttaactacac tatctacgat ggtttcaacc tgcgtaacac caacctggct 1200
   gctaatttta acggccagaa cacggaaatc aacaacatga acttcacaaa actgaaaaac 1260
   ttcactggtc tgttcgagtt ttacaagctg ctgtgcgtcg acggcatcat tacctccaaa 1320
   actaaatctc tgatagaagg tagatatggc ggtttcacgg gcgcacgcaa atcagcgcgt 1380
   aaacgtaaga accaggcgct agcgggcggt ggcggtagcg gcggtggcgg tagcggcggt 1440
   ggcggtagcg cactagtgct gcagtgtatc aaggttaaca actgggattt attcttcagc 1500
   ccgagtgaag acaacttcac caacgacctg aacaaaggtg aagaaatcac ctcagatact 1560
   aacatcgaag cagccgaaga aaacatctcg ctggacctga tccagcagta ctacctgacc 1620
   tttaatttcg acaacgagcc ggaaaacatt tctatcgaaa acctgagctc tgatatcatc 1680
   ggccagctgg aactgatgcc gaacatcgaa cgtttcccaa acggtaaaaa gtacgagctg 1740
   gacaaatata ccatgttcca ctacctgcgc gcgcaggaat ttgaacacgg caaatcccgt 1800
   atcgcactga ctaactccgt taacgaagct ctgctcaacc cgtcccgtgt atacaccttc 1860
   ttctctagcg actacgtgaa aaaggtcaac aaagcgactg aagctgcaat gttcttgggt 1920
   tgggttgaac agcttgttta tgattttacc gacgagacgt ccgaagtatc tactaccgac 1980
   aaaattgcgg atatcactat catcatcccg tacatcggtc cggctctgaa cattggcaac 2040
   atgctgtaca aagacgactt cgttggcgca ctgatcttct ccggtgcggt gatcctgctg 2100
   gagttcatcc cggaaatcgc catcccggta ctgggcacct ttgctctggt ttcttacatt 2160
   gcaaacaagg ttctgactgt acaaaccatc gacaacgcgc tgagcaaacg taacgaaaaa 2220
   tgggatgaag tttacaaata tatcgtgacc aactggctgg ctaaggttaa tactcagatc 2280
   gacctcatcc gcaaaaaaat gaaagaagca ctggaaaacc aggcggaagc taccaaggca 2340
   atcattaact accagtacaa ccagtacacc gaggaagaaa aaaacaacat caacttcaac 2400
   atcgacgatc tgtcctctaa actgaacgaa tccatcaaca aagctatgat caacatcaac 2460
   aagttcctga accagtgctc tgtaagctat ctgatgaact ccatgatccc gtacggtgtt 2520
   aaacgtctgg aggacttcga tgcgtctctg aaagacgccc tgctgaaata catttacgac 2580
   aaccgtggca ctctgatcgg tcaggttgat cgtctgaagg acaaagtgaa caatacctta 2640
   tcgaccgaca tcccttttca gctcagtaaa tatgtcgata accaacgcct tttgtccact 2700
   ctagactag 2709
<210> 85
   <211> 902
   <212> PRT
   <213> Artificial Sequence
<220>
   <223> Synthetic
<400> 85
   Gly Ser Met Glu Phe Val Asn Lys Gln Phe Asn Tyr Lys Asp Pro Val
   1               5                   10                  15
   Asn Gly Val Asp Ile Ala Tyr Ile Lys Ile Pro Asn Ala Gly Gln Met
               20                  25                  30
   Gln Pro Val Lys Ala Phe Lys Ile His Asn Lys Ile Trp Val Ile Pro
           35                  40                  45
   Glu Arg Asp Thr Phe Thr Asn Pro Glu Glu Gly Asp Leu Asn Pro Pro
       50                  55                  60
   Pro Glu Ala Lys Gln Val Pro Val Ser Tyr Tyr Asp Ser Thr Tyr Leu
   65                  70                  75                  80
   Ser Thr Asp Asn Glu Lys Asp Asn Tyr Leu Lys Gly Val Thr Lys Leu
                   85                  90                  95
   Phe Glu Arg Ile Tyr Ser Thr Asp Leu Gly Arg Met Leu Leu Thr Ser
               100                 105                 110
   Ile Val Arg Gly Ile Pro Phe Trp Gly Gly Ser Thr Ile Asp Thr Glu
           115                 120                 125
   Leu Lys Val Ile Asp Thr Asn Cys Ile Asn Val Ile Gln Pro Asp Gly
       130                 135                 140
   Ser Tyr Arg Ser Glu Glu Leu Asn Leu Val Ile Ile Gly Pro Ser Ala
   145                 150                 155                 160
   Asp Ile Ile Gln Phe Glu Cys Lys Ser Phe Gly His Glu Val Leu Asn
                   165                 170                 175
   Leu Thr Arg Asn Gly Tyr Gly Ser Thr Gln Tyr Ile Arg Phe Ser Pro
               180                 185                 190
   Asp Phe Thr Phe Gly Phe Glu Glu Ser Leu Glu Val Asp Thr Asn Pro
   195 200 205
   Leu Leu Gly Ala Gly Lys Phe Ala Thr Asp Pro Ala Val Thr Leu Ala
   210 215 220
   His Glu Leu Ile His Ala Gly His Arg Leu Tyr Gly Ile Ala Ile Asn
   225 230 235 240
   Pro Asn Arg Val Phe Lys Val Asn Thr Asn Ala Tyr Tyr Glu Met Ser
   245 250 255
   Gly Leu Glu Val Ser Phe Glu Glu Leu Arg Thr Phe Gly Gly His Asp
   260 265 270
   Ala Lys Phe Ile Asp Ser Leu Gln Glu Asn Glu Phe Arg Leu Tyr Tyr
   275 280 285
   Tyr Asn Lys Phe Lys Asp Ile Ala Ser Thr Leu Asn Lys Ala Lys Ser
   290 295 300
   Ile Val Gly Thr Thr Ala Ser Leu Gln Tyr Met Lys Asn Val Phe Lys
   305 310 315 320
   Glu Lys Tyr Leu Leu Ser Glu Asp Thr Ser Gly Lys Phe Ser Val Asp
   325 330 335
   Lys Leu Lys Phe Asp Lys Leu Tyr Lys Met Leu Thr Glu Ile Tyr Thr
   340 345 350
   Glu Asp Asn Phe Val Lys Phe Phe Lys Val Leu Asn Arg Lys Thr Tyr
   355 360 365
   Leu Asn Phe Asp Lys Ala Val Phe Lys Ile Asn Ile Val Pro Lys Val
   370 375 380
   Asn Tyr Thr Ile Tyr Asp Gly Phe Asn Leu Arg Asn Thr Asn Leu Ala
   385 390 395 400
   Ala Asn Phe Asn Gly Gln Asn Thr Glu Ile Asn Asn Met Asn Phe Thr
   405 410 415
   Lys Leu Lys Asn Phe Thr Gly Leu Phe Glu Phe Tyr Lys Leu Leu Cys
   420 425 430
   Val Asp Gly Ile Ile Thr Ser Lys Thr Lys Ser Leu Ile Glu Gly Arg
   435 440 445
   Tyr Gly Gly Phe Thr Gly Ala Arg Lys Ser Ala Arg Lys Arg Lys Asn
   450 455 460
   Gln Ala Leu Ala Gly Gly Gly Gly Ser Gly Gly Gly Gly Ser Gly Gly
   465 470 475 480
   Gly Gly Ser Ala Leu Val Leu Gln Cys Ile Lys Val Asn Asn Trp Asp
   485 490 495
   Leu Phe Phe Ser Pro Ser Glu Asp Asn Phe Thr Asn Asp Leu Asn Lys
   500 505 510
   Gly Glu Glu Ile Thr Ser Asp Thr Asn Ile Glu Ala Ala Glu Glu Asn
   515 520 525
   Ile Ser Leu Asp Leu Ile Gln Gln Tyr Tyr Leu Thr Phe Asn Phe Asp
   530 535 540
   Asn Glu Pro Glu Asn Ile Ser Ile Glu Asn Leu Ser Ser Asp Ile Ile
   545 550 555 560
   Gly Gln Leu Glu Leu Met Pro Asn Ile Glu Arg Phe Pro Asn Gly Lys
   565 570 575
   Lys Tyr Glu Leu Asp Lys Tyr Thr Met Phe His Tyr Leu Arg Ala Gln
   580 585 590
   Glu Phe Glu His Gly Lys Ser Arg Ile Ala Leu Thr Asn Ser Val Asn
   595 600 605
   Glu Ala Leu Leu Asn Pro Ser Arg Val Tyr Thr Phe Phe Ser Ser Asp
   610 615 620
   Tyr Val Lys Lys Val Asn Lys Ala Thr Glu Ala Ala Met Phe Leu Gly
   625 630 635 640
   Trp Val Glu Gln Leu Val Tyr Asp Phe Thr Asp Glu Thr Ser Glu Val
   645 650 655
   Ser Thr Thr Asp Lys Ile Ala Asp Ile Thr Ile Ile Ile Pro Tyr Ile
   660 665 670
   Gly Pro Ala Leu Asn Ile Gly Asn Met Leu Tyr Lys Asp Asp Phe Val
   675 680 685
   Gly Ala Leu Ile Phe Ser Gly Ala Val Ile Leu Leu Glu Phe Ile Pro
   690 695 700
   Glu Ile Ala Ile Pro Val Leu Gly Thr Phe Ala Leu Val Ser Tyr Ile
   705 710 715 720
   Ala Asn Lys Val Leu Thr Val Gln Thr Ile Asp Asn Ala Leu Ser Lys
   725 730 735
   Arg Asn Glu Lys Trp Asp Glu Val Tyr Lys Tyr Ile Val Thr Asn Trp
   740 745 750
   Leu Ala Lys Val Asn Thr Gln Ile Asp Leu Ile Arg Lys Lys Met Lys
   755 760 765
   Glu Ala Leu Glu Asn Gln Ala Glu Ala Thr Lys Ala Ile Ile Asn Tyr
   770 775 780
   Gln Tyr Asn Gln Tyr Thr Glu Glu Glu Lys Asn Asn Ile Asn Phe Asn
   785 790 795 800
   Ile Asp Asp Leu Ser Ser Lys Leu Asn Glu Ser Ile Asn Lys Ala Met
   805 810 815
   Ile Asn Ile Asn Lys Phe Leu Asn Gln Cys Ser Val Ser Tyr Leu Met
   820 825 830
   Asn Ser Met Ile Pro Tyr Gly Val Lys Arg Leu Glu Asp Phe Asp Ala
   835 840 845
   Ser Leu Lys Asp Ala Leu Leu Lys Tyr Ile Tyr Asp Asn Arg Gly Thr
   850 855 860
   Leu Ile Gly Gln Val Asp Arg Leu Lys Asp Lys Val Asn Asn Thr Leu
   865 870 875 880
   Ser Thr Asp Ile Pro Phe Gln Leu Ser Lys Tyr Val Asp Asn Gln Arg
   885 890 895
   Leu Leu Ser Thr Leu Asp
   900
<210> 86
   <211> 2889
   <212> DNA
   <213> Artificial Sequence
<220>
   <223> Synthetic
<400> 86
   ggatccttgg tacgagatga cgttgactat caaattttcc gcgactttgc ggaaaataaa 60
   ggtaagtttt tcgtcggcgc cacagacctg tccgtcaaaa ataagagagg ccagaacatc 120
   ggtaacgcac tgagcaacgt ccctatgatt gattttagtg tagcggacgt taataaacgg 180
   attgcaaccg tcgttgatcc gcagtatgct gtcagcgtca aacatgctaa agcggaagtt 240
   catacgttct attacgggca atataacggc cataacgatg tggctgataa agaaaatgaa 300
   tatcgcgtgg tcgagcagaa caattacgaa ccgcacaaag cgtggggcgc gagtaattta 360
   ggccgcctgg aggactataa catggcccgt ttcaataaat tcgtgaccga ggtagcaccg 420
   atcgccccca cagatgctgg tgggggcctg gatacctaca aagataaaaa ccgcttctct 480
   agcttcgtgc gcattggcgc cggtcgtcag ctcgtgtacg agaagggtgt ctatcaccag 540
   gaaggtaatg aaaaggggta cgacctccgt gatttgtccc aggcgtatcg ctacgctatt 600
   gccggaaccc cgtataaaga tattaatatc gatcaaacca tgaataccga aggcctaatt 660
   ggtttcggga atcataataa gcaatatagc gcagaagagc taaagcaggc cctcagccaa 720
   gatgcgttaa ccaattacgg agtgttaggc gatagcggca gtccgctgtt tgccttcgat 780
   aaacagaaaa atcaatgggt gtttctgggc acttatgatt attgggccgg atatggtaaa 840
   aagagctggc aggaatggaa tatttataaa aaggaattcg cagacaaaat caagcagcat 900
   gacaacgcag gtacggtgaa ggggaacggc gaacatcact ggaagacgac cggcacgaat 960
   agtcatatcg gatcgacggc cgttcgcctg gcgaacaatg agggcgatgc aaacaatggg 1020
   caaaacgtga cctttgagga caacggtacc ctggtcctta accagaacat aaatcagggc 1080
   gcgggaggct tgttctttaa aggcgactat actgttaagg gagcaaacaa tgacatcacc 1140
   tggttagggg ccggtattga cgttgcggat ggaaaaaagg tggtttggca ggttaaaaac 1200
   cctaacgggg accggctggc aaaaatcggc aaagggacat tggaaattaa tggtaccggt 1260
   gtgaatcagg gtcagctgaa agtgggagat gggaccgtga ttctgaacca gaaagcagac 1320
   gctgacaaaa aggtgcaagc ctttagccaa gtaggaattg ttagtggtcg tggcacactc 1380
   gtcttgaact caagcaacca aataaatccg gataacctgt actttggatt tcgtggcgga 1440
   cgcctggatg ctaacgggaa tgatctgacc tttgaacata tccgtaacgt tgacgagggt 1500
   gcgcgcatag ttaatcataa tactgaccat gcatcaacta tcaccttgac cgggaaaagt 1560
   ctgattacaa acccaaactc tctgtcagta cattccatcc agaatgatta tgatgaagac 1620
   gattactcat actattaccg gccgcgtaga ccaattccac aaggtaaaga tctttattac 1680
   aaaaattacc gttattacgc attaaaatcc ggagggcggc tgaatgcacc tatgccggaa 1740
   aatggcgtgg ccgaaaacaa tgactggatt tttatgggtt atactcaaga agaggctcgc 1800
   aaaaatgcaa tgaaccataa aaataaccga aggatcggtg atttcggcgg atttttcgat 1860
   gaggaaaatg gtaaaggtca caatggtgcg ctgaatctaa attttaacgg caaaagtgcc 1920
   cagaaacgtt tccttctgac tggtggcgct aatctgaatg gtaaaatcag tgtgacgcag 1980
   ggtaacgtgc tgctttctgg ccggccaact ccgcatgcac gtgattttgt aaataaatcg 2040
   agcgctcgta aagatgcgca tttttctaaa aataacgagg tcgtgtttga agatgactgg 2100
   ataaatcgca cctttaaagc ggcagaaatc gcggttaatc agagtgcgag cttttcatcg 2160
   ggtaggaatg tatctgatat tacagcaaac attacagcca ctgataatgc gaaggtcaac 2220
   ctgggttata aaaacggtga tgaagtttgt gttcgatcgg attacacggg ctatgttacc 2280
   tgcaacactg gcaatctgtc tgataaagcg cttaactctt ttgacgccac gcgcattaac 2340
   gggaatgtga acctgaacca aaacgctgcc ttggtacttg gtaaggccgc gttgtggggt 2400
   aaaattcagg gccagggcaa ctcccgtgtg tctctgaacc agcactcgaa gtggcacctg 2460
   acgggggact cgcaggtgca caacttgtcc ctggccgata gccatattca ccttaacaat 2520
   gcgtccgatg cccagtcagc taataaatat catacgatca aaatcaatca cctctctggc 2580
   aacggtcact ttcactactt aacggattta gcaaaaaact taggggataa agtcctggta 2640
   aaagaatcag cgagcggaca ttatcagtta catgtacaga acaaaacagg cgagccaaat 2700
   caggaaggcc ttgacttatt tgatgcttca tcggtacaag atcgttccag actgttcgtt 2760
   tcactcgcga atcactacgt tgatctgggt gcgctgcgct atactataaa gacggaaaat 2820
   ggcataacac gcctctataa tccctatgcc ggtaacggcc gtccggtgaa acctgctccc 2880
   tgcgtcgac 2889
<210> 87
   <211> 4296
   <212> DNA
   <213> Artificial Sequence
<220>
   <223> Synthetic
<400> 87
   ggatccttgg tacgagatga cgttgactat caaattttcc gcgactttgc ggaaaataaa     60
   ggtaagtttt tcgtcggcgc cacagacctg tccgtcaaaa ataagagagg ccagaacatc    120
   ggtaacgcac tgagcaacgt ccctatgatt gattttagtg tagcggacgt taataaacgg    180
   attgcaaccg tcgttgatcc gcagtatgct gtcagcgtca aacatgctaa agcggaagtt 240
   catacgttct attacgggca atataacggc cataacgatg tggctgataa agaaaatgaa 300
   tatcgcgtgg tcgagcagaa caattacgaa ccgcacaaag cgtggggcgc gagtaattta 360
   ggccgcctgg aggactataa catggcccgt ttcaataaat tcgtgaccga ggtagcaccg 420
   atcgccccca cagatgctgg tgggggcctg gatacctaca aagataaaaa ccgcttctct 480
   agcttcgtgc gcattggcgc cggtcgtcag ctcgtgtacg agaagggtgt ctatcaccag 540
   gaaggtaatg aaaaggggta cgacctccgt gatttgtccc aggcgtatcg ctacgctatt 600
   gccggaaccc cgtataaaga tattaatatc gatcaaacca tgaataccga aggcctaatt 660
   ggtttcggga atcataataa gcaatatagc gcagaagagc taaagcaggc cctcagccaa 720
   gatgcgttaa ccaattacgg agtgttaggc gatagcggca gtccgctgtt tgccttcgat 780
   aaacagaaaa atcaatgggt gtttctgggc acttatgatt attgggccgg atatggtaaa 840
   aagagctggc aggaatggaa tatttataaa aaggaattcg cagacaaaat caagcagcat 900
   gacaacgcag gtacggtgaa ggggaacggc gaacatcact ggaagacgac cggcacgaat 960
   agtcatatcg gatcgacggc cgttcgcctg gcgaacaatg agggcgatgc aaacaatggg 1020
   caaaacgtga cctttgagga caacggtacc ctggtcctta accagaacat aaatcagggc 1080
   gcgggaggct tgttctttaa aggcgactat actgttaagg gagcaaacaa tgacatcacc 1140
   tggttagggg ccggtattga cgttgcggat ggaaaaaagg tggtttggca ggttaaaaac 1200
   cctaacgggg accggctggc aaaaatcggc aaagggacat tggaaattaa tggtaccggt 1260
   gtgaatcagg gtcagctgaa agtgggagat gggaccgtga ttctgaacca gaaagcagac 1320
   gctgacaaaa aggtgcaagc ctttagccaa gtaggaattg ttagtggtcg tggcacactc 1380
   gtcttgaact caagcaacca aataaatccg gataacctgt actttggatt tcgtggcgga 1440
   cgcctggatg ctaacgggaa tgatctgacc tttgaacata tccgtaacgt tgacgagggt 1500
   gcgcgcatag ttaatcataa tactgaccat gcatcaacta tcaccttgac cgggaaaagt 1560
   ctgattacaa acccaaactc tctgtcagta cattccatcc agaatgatta tgatgaagac 1620
   gattactcat actattaccg gccgcgtaga ccaattccac aaggtaaaga tctttattac 1680
   aaaaattacc gttattacgc attaaaatcc ggagggcggc tgaatgcacc tatgccggaa 1740
   aatggcgtgg ccgaaaacaa tgactggatt tttatgggtt atactcaaga agaggctcgc 1800
   aaaaatgcaa tgaaccataa aaataaccga aggatcggtg atttcggcgg atttttcgat 1860
   gaggaaaatg gtaaaggtca caatggtgcg ctgaatctaa attttaacgg caaaagtgcc 1920
   cagaaacgtt tccttctgac tggtggcgct aatctgaatg gtaaaatcag tgtgacgcag 1980
   ggtaacgtgc tgctttctgg ccggccaact ccgcatgcac gtgattttgt aaataaatcg 2040
   agcgctcgta aagatgcgca tttttctaaa aataacgagg tcgtgtttga agatgactgg 2100
   ataaatcgca cctttaaagc ggcagaaatc gcggttaatc agagtgcgag cttttcatcg 2160
   ggtaggaatg tatctgatat tacagcaaac attacagcca ctgataatgc gaaggtcaac 2220
   ctgggttata aaaacggtga tgaagtttgt gttcgatcgg attacacggg ctatgttacc 2280
   tgcaacactg gcaatctgtc tgataaagcg cttaactctt ttgacgccac gcgcattaac 2340
   gggaatgtga acctgaacca aaacgctgcc ttggtacttg gtaaggccgc gttgtggggt 2400
   aaaattcagg gccagggcaa ctcccgtgtg tctctgaacc agcactcgaa gtggcacctg 2460
   acgggggact cgcaggtgca caacttgtcc ctggccgata gccatattca ccttaacaat 2520
   gcgtccgatg cccagtcagc taataaatat catacgatca aaatcaatca cctctctggc 2580
   aacggtcact ttcactactt aacggattta gcaaaaaact taggggataa agtcctggta 2640
   aaagaatcag cgagcggaca ttatcagtta catgtacaga acaaaacagg cgagccaaat 2700
   caggaaggcc ttgacttatt tgatgcttca tcggtacaag atcgttccag actgttcgtt 2760
   tcactcgcga atcactacgt tgatctgggt gcgctgcgct atactataaa gacggaaaat 2820
   ggcataacac gcctctataa tccctatgcc ggtaacggcc gtccggtgaa acctgctccc 2880
   tgcgtcgacg gcatcattac ctccaaaact aaatctctga tagaaggtag atttggcggt 2940
   ttcacgggcg cacgcaaatc agcgcgtaaa cgtaagaacc aggcgctagc gggcggtggc 3000
   ggtagcggcg gtggcggtag cggcggtggc ggtagcgcac tagtgctgca gtgtatcaag 3060
   gttaacaact gggatttatt cttcagcccg agtgaagaca acttcaccaa cgacctgaac 3120
   aaaggtgaag aaatcacctc agatactaac atcgaagcag ccgaagaaaa catctcgctg 3180
   gacctgatcc agcagtacta cctgaccttt aatttcgaca acgagccgga aaacatttct 3240
   atcgaaaacc tgagctctga tatcatcggc cagctggaac tgatgccgaa catcgaacgt 3300
   ttcccaaacg gtaaaaagta cgagctggac aaatatacca tgttccacta cctgcgcgcg 3360
   caggaatttg aacacggcaa atcccgtatc gcactgacta actccgttaa cgaagctctg 3420
   ctcaacccgt cccgtgtata caccttcttc tctagcgact acgtgaaaaa ggtcaacaaa 3480
   gcgactgaag ctgcaatgtt cttgggttgg gttgaacagc ttgtttatga ttttaccgac 3540
   gagacgtccg aagtatctac taccgacaaa attgcggata tcactatcat catcccgtac 3600
   atcggtccgg ctctgaacat tggcaacatg ctgtacaaag acgacttcgt tggcgcactg 3660
   atcttctccg gtgcggtgat cctgctggag ttcatcccgg aaatcgccat cccggtactg 3720
   ggcacctttg ctctggtttc ttacattgca aacaaggttc tgactgtaca aaccatcgac 3780
   aacgcgctga gcaaacgtaa cgaaaaatgg gatgaagttt acaaatatat cgtgaccaac 3840
   tggctggcta aggttaatac tcagatcgac ctcatccgca aaaaaatgaa agaagcactg   3900
   gaaaaccagg cggaagctac caaggcaatc attaactacc agtacaacca gtacaccgag   3960
   gaagaaaaaa acaacatcaa cttcaacatc gacgatctgt cctctaaact gaacgaatcc   4020
   atcaacaaag ctatgatcaa catcaacaag ttcctgaacc agtgctctgt aagctatctg   4080
   atgaactcca tgatcccgta cggtgttaaa cgtctggagg acttcgatgc gtctctgaaa   4140
   gacgccctgc tgaaatacat ttacgacaac cgtggcactc tgatcggtca ggttgatcgt   4200
   ctgaaggaca aagtgaacaa taccttatcg accgacatcc cttttcagct cagtaaatat   4260
   gtcgataacc aacgcctttt gtccactcta gactag                             4296
<210> 88
   <211> 1431
   <212> PRT
   <213> Artificial Sequence
<220>
   <223> Synthetic
<400> 88
   Gly Ser Leu Val Arg Asp Asp Val Asp Tyr Gln Ile Phe Arg Asp Phe
   1               5                   10                  15
   Ala Glu Asn Lys Gly Lys Phe Phe Val Gly Ala Thr Asp Leu Ser Val
               20                  25                  30
   Lys Asn Lys Arg Gly Gln Asn Ile Gly Asn Ala Leu Ser Asn Val Pro
           35                  40                  45
   Met Ile Asp Phe Ser Val Ala Asp Val Asn Lys Arg Ile Ala Thr Val
       50                  55                  60
   Val Asp Pro Gln Tyr Ala Val Ser Val Lys His Ala Lys Ala Glu Val
   65                  70                  75                  80
   His Thr Phe Tyr Tyr Gly Gln Tyr Asn Gly His Asn Asp Val Ala Asp
                   85                  90                  95
   Lys Glu Asn Glu Tyr Arg Val Val Glu Gln Asn Asn Tyr Glu Pro His
               100                 105                 110
   Lys Ala Trp Gly Ala Ser Asn Leu Gly Arg Leu Glu Asp Tyr Asn Met
           115                 120                 125
   Ala Arg Phe Asn Lys Phe Val Thr Glu Val Ala Pro Ile Ala Pro Thr
       130                 135                 140
   Asp Ala Gly Gly Gly Leu Asp Thr Tyr Lys Asp Lys Asn Arg Phe Ser
   145 150 155 160
   Ser Phe Val Arg Ile Gly Ala Gly Arg Gln Leu Val Tyr Glu Lys Gly
   165 170 175
   Val Tyr His Gln Glu Gly Asn Glu Lys Gly Tyr Asp Leu Arg Asp Leu
   180 185 190
   Ser Gln Ala Tyr Arg Tyr Ala Ile Ala Gly Thr Pro Tyr Lys Asp Ile
   195 200 205
   Asn Ile Asp Gln Thr Met Asn Thr Glu Gly Leu Ile Gly Phe Gly Asn
   210 215 220
   His Asn Lys Gln Tyr Ser Ala Glu Glu Leu Lys Gln Ala Leu Ser Gln
   225 230 235 240
   Asp Ala Leu Thr Asn Tyr Gly Val Leu Gly Asp Ser Gly Ser Pro Leu
   245 250 255
   Phe Ala Phe Asp Lys Gln Lys Asn Gln Trp Val Phe Leu Gly Thr Tyr
   260 265 270
   Asp Tyr Trp Ala Gly Tyr Gly Lys Lys Ser Trp Gln Glu Trp Asn Ile
   275 280 285
   Tyr Lys Lys Glu Phe Ala Asp Lys Ile Lys Gln His Asp Asn Ala Gly
   290 295 300
   Thr Val Lys Gly Asn Gly Glu His His Trp Lys Thr Thr Gly Thr Asn
   305 310 315 320
   Ser His Ile Gly Ser Thr Ala Val Arg Leu Ala Asn Asn Glu Gly Asp
   325 330 335
   Ala Asn Asn Gly Gln Asn Val Thr Phe Glu Asp Asn Gly Thr Leu Val
   340 345 350
   Leu Asn Gln Asn Ile Asn Gln Gly Ala Gly Gly Leu Phe Phe Lys Gly
   355 360 365
   Asp Tyr Thr Val Lys Gly Ala Asn Asn Asp Ile Thr Trp Leu Gly Ala
   370 375 380
   Gly Ile Asp Val Ala Asp Gly Lys Lys Val Val Trp Gln Val Lys Asn
   385 390 395 400
   Pro Asn Gly Asp Arg Leu Ala Lys Ile Gly Lys Gly Thr Leu Glu Ile
   405 410 415
   Asn Gly Thr Gly Val Asn Gln Gly Gln Leu Lys Val Gly Asp Gly Thr
   420 425 430
   Val Ile Leu Asn Gln Lys Ala Asp Ala Asp Lys Lys Val Gln Ala Phe
   435 440 445
   Ser Gln Val Gly Ile Val Ser Gly Arg Gly Thr Leu Val Leu Asn Ser
   450 455 460
   Ser Asn Gln Ile Asn Pro Asp Asn Leu Tyr Phe Gly Phe Arg Gly Gly
   465 470 475 480
   Arg Leu Asp Ala Asn Gly Asn Asp Leu Thr Phe Glu His Ile Arg Asn
   485 490 495
   Val Asp Glu Gly Ala Arg Ile Val Asn His Asn Thr Asp His Ala Ser
   500 505 510
   Thr Ile Thr Leu Thr Gly Lys Ser Leu Ile Thr Asn Pro Asn Ser Leu
   515 520 525
   Ser Val His Ser Ile Gln Asn Asp Tyr Asp Glu Asp Asp Tyr Ser Tyr
   530 535 540
   Tyr Tyr Arg Pro Arg Arg Pro Ile Pro Gln Gly Lys Asp Leu Tyr Tyr
   545 550 555 560
   Lys Asn Tyr Arg Tyr Tyr Ala Leu Lys Ser Gly Gly Arg Leu Asn Ala
   565 570 575
   Pro Met Pro Glu Asn Gly Val Ala Glu Asn Asn Asp Trp Ile Phe Met
   580 585 590
   Gly Tyr Thr Gln Glu Glu Ala Arg Lys Asn Ala Met Asn His Lys Asn
   595 600 605
   Asn Arg Arg Ile Gly Asp Phe Gly Gly Phe Phe Asp Glu Glu Asn Gly
   610 615 620
   Lys Gly His Asn Gly Ala Leu Asn Leu Asn Phe Asn Gly Lys Ser Ala
   625 630 635 640
   Gln Lys Arg Phe Leu Leu Thr Gly Gly Ala Asn Leu Asn Gly Lys Ile
   645 650 655
   Ser Val Thr Gln Gly Asn Val Leu Leu Ser Gly Arg Pro Thr Pro His
   660 665 670
   Ala Arg Asp Phe Val Asn Lys Ser Ser Ala Arg Lys Asp Ala His Phe
   675 680 685
   Ser Lys Asn Asn Glu Val Val Phe Glu Asp Asp Trp Ile Asn Arg Thr
   690 695 700
   Phe Lys Ala Ala Glu Ile Ala Val Asn Gln Ser Ala Ser Phe Ser Ser
   705 710 715 720
   Gly Arg Asn Val Ser Asp Ile Thr Ala Asn Ile Thr Ala Thr Asp Asn
   725 730 735
   Ala Lys Val Asn Leu Gly Tyr Lys Asn Gly Asp Glu Val Cys Val Arg
   740 745 750
   Ser Asp Tyr Thr Gly Tyr Val Thr Cys Asn Thr Gly Asn Leu Ser Asp
   755 760 765
   Lys Ala Leu Asn Ser Phe Asp Ala Thr Arg Ile Asn Gly Asn Val Asn
   770 775 780
   Leu Asn Gln Asn Ala Ala Leu Val Leu Gly Lys Ala Ala Leu Trp Gly
   785 790 795 800
   Lys Ile Gln Gly Gln Gly Asn Ser Arg Val Ser Leu Asn Gln His Ser
   805 810 815
   Lys Trp His Leu Thr Gly Asp Ser Gln Val His Asn Leu Ser Leu Ala
   820 825 830
   Asp Ser His Ile His Leu Asn Asn Ala Ser Asp Ala Gln Ser Ala Asn
   835 840 845
   Lys Tyr His Thr Ile Lys Ile Asn His Leu Ser Gly Asn Gly His Phe
   850 855 860
   His Tyr Leu Thr Asp Leu Ala Lys Asn Leu Gly Asp Lys Val Leu Val
   865 870 875 880
   Lys Glu Ser Ala Ser Gly His Tyr Gln Leu His Val Gln Asn Lys Thr
   885 890 895
   Gly Glu Pro Asn Gln Glu Gly Leu Asp Leu Phe Asp Ala Ser Ser Val
   900 905 910
   Gln Asp Arg Ser Arg Leu Phe Val Ser Leu Ala Asn His Tyr Val Asp
   915 920 925
   Leu Gly Ala Leu Arg Tyr Thr Ile Lys Thr Glu Asn Gly Ile Thr Arg
   930 935 940
   Leu Tyr Asn Pro Tyr Ala Gly Asn Gly Arg Pro Val Lys Pro Ala Pro
   945 950 955 960
   Cys Val Asp Gly Ile Ile Thr Ser Lys Thr Lys Ser Leu Ile Glu Gly
   965 970 975
   Arg Phe Gly Gly Phe Thr Gly Ala Arg Lys Ser Ala Arg Lys Arg Lys
   980 985 990
   Asn Gln Ala Leu Ala Gly Gly Gly Gly Ser Gly Gly Gly Gly Ser Gly
   995 1000 1005
   Gly Gly Gly Ser Ala Leu Val Leu Gln Cys Ile Lys Val Asn Asn
   1010 1015 1020
   Trp Asp Leu Phe Phe Ser Pro Ser Glu Asp Asn Phe Thr Asn Asp
   1025 1030 1035
   Leu Asn Lys Gly Glu Glu Ile Thr Ser Asp Thr Asn Ile Glu Ala
   1040 1045 1050
   Ala Glu Glu Asn Ile Ser Leu Asp Leu Ile Gln Gln Tyr Tyr Leu
   1055 1060 1065
   Thr Phe Asn Phe Asp Asn Glu Pro Glu Asn Ile Ser Ile Glu Asn
   1070 1075 1080
   Leu Ser Ser Asp Ile Ile Gly Gln Leu Glu Leu Met Pro Asn Ile
   1085 1090 1095
   Glu Arg Phe Pro Asn Gly Lys Lys Tyr Glu Leu Asp Lys Tyr Thr
   1100 1105 1110
   Met Phe His Tyr Leu Arg Ala Gln Glu Phe Glu His Gly Lys Ser
   1115 1120 1125
   Arg Ile Ala Leu Thr Asn Ser Val Asn Glu Ala Leu Leu Asn Pro
   1130 1135 1140
   Ser Arg Val Tyr Thr Phe Phe Ser Ser Asp Tyr Val Lys Lys Val
   1145 1150 1155
   Asn Lys Ala Thr Glu Ala Ala Met Phe Leu Gly Trp Val Glu Gln
   1160 1165 1170
   Leu Val Tyr Asp Phe Thr Asp Glu Thr Ser Glu Val Ser Thr Thr
   1175 1180 1185
   Asp Lys Ile Ala Asp Ile Thr Ile Ile Ile Pro Tyr Ile Gly Pro
   1190 1195 1200
   Ala Leu Asn Ile Gly Asn Met Leu Tyr Lys Asp Asp Phe Val Gly
   1205 1210 1215
   Ala Leu Ile Phe Ser Gly Ala Val Ile Leu Leu Glu Phe Ile Pro
   1220 1225 1230
   Glu Ile Ala Ile Pro Val Leu Gly Thr Phe Ala Leu Val Ser Tyr
   1235 1240 1245
   Ile Ala Asn Lys Val Leu Thr Val Gln Thr Ile Asp Asn Ala Leu
   1250 1255 1260
   Ser Lys Arg Asn Glu Lys Trp Asp Glu Val Tyr Lys Tyr Ile Val
   1265 1270 1275
   Thr Asn Trp Leu Ala Lys Val Asn Thr Gln Ile Asp Leu Ile Arg
   1280 1285 1290
   Lys Lys Met Lys Glu Ala Leu Glu Asn Gln Ala Glu Ala Thr Lys
   1295 1300 1305
   Ala Ile Ile Asn Tyr Gln Tyr Asn Gln Tyr Thr Glu Glu Glu Lys
   1310 1315 1320
   Asn Asn Ile Asn Phe Asn Ile Asp Asp Leu Ser Ser Lys Leu Asn
   1325 1330 1335
   Glu Ser Ile Asn Lys Ala Met Ile Asn Ile Asn Lys Phe Leu Asn
   1340 1345 1350
   Gln Cys Ser Val Ser Tyr Leu Met Asn Ser Met Ile Pro Tyr Gly
   1355 1360 1365
   Val Lys Arg Leu Glu Asp Phe Asp Ala Ser Leu Lys Asp Ala Leu
   1370 1375 1380
   Leu Lys Tyr Ile Tyr Asp Asn Arg Gly Thr Leu Ile Gly Gln Val
   1385 1390 1395
   Asp Arg Leu Lys Asp Lys Val Asn Asn Thr Leu Ser Thr Asp Ile
   1400 1405 1410
   Pro Phe Gln Leu Ser Lys Tyr Val Asp Asn Gln Arg Leu Leu Ser
   1415 1420 1425
   Thr Leu Asp
   1430
<210> 89
   <211> 1357
   <212> DNA
   <213> Artificial Sequence
<220>
   <223> Synthetic
<400> 89
   gctagcgggc ggtggcggta gcggcggtgg cggtagcggc ggtggcggta gcgcactagt     60
   gctgcagtgt atcaaggtta acaactggga tttattcttc agcccgagtg aagacaactt    120
   caccaacgac ctgaacaaag gtgaagaaat cacctcagat actaacatcg aagcagccga    180
   agaaaacatc tcgctggacc tgatccagca gtactacctg acctttaatt tcgacaacga    240
   gccggaaaac atttctatcg aaaacctgag ctctgatatc atcggccagc tggaactgat    300
   gccgaacatc gaacgtttcc caaacggtaa aaagtacgag ctggacaaat ataccatgtt    360
   ccactacctg cgcgcgcagg aatttgaaca cggcaaatcc cgtatcgcac tgactaactc    420
   cgttaacgaa gctctgctca acccgtcccg tgtatacacc ttcttctcta gcgactacgt    480
   gaaaaaggtc aacaaagcga ctgaagctgc aatgttcttg ggttgggttg aacagcttgt    540
   ttatgatttt accgacgaga cgtccgaagt atctactacc gacaaaattg cggatatcac    600
   tatcatcatc ccgtacatcg gtccggctct gaacattggc aacatgctgt acaaagacga    660
   cttcgttggc gcactgatct tctccggtgc ggtgatcctg ctggagttca tcccggaaat    720
   cgccatcccg gtactgggca cctttgctct ggtttcttac attgcaaaca aggttctgac    780
   tgtacaaacc atcgacaacg cgctgagcaa acgtaacgaa aaatgggatg aagtttacaa    840
   atatatcgtg accaactggc tggctaaggt taatactcag atcgacctca tccgcaaaaa    900
   aatgaaagaa gcactggaaa accaggcgga agctaccaag gcaatcatta actaccagta    960
   caaccagtac accgaggaag aaaaaaacaa catcaacttc aacatcgacg atctgtcctc   1020
   taaactgaac gaatccatca acaaagctat gatcaacatc aacaagttcc tgaaccagtg   1080
   ctctgtaagc tatctgatga actccatgat cccgtacggt gttaaacgtc tggaggactt   1140
   cgatgcgtct ctgaaagacg ccctgctgaa atacatttac gacaaccgtg gcactctgat   1200
   cggtcaggtt gatcgtctga aggacaaagt gaacaatacc ttatcgaccg acatcccttt   1260
   tcagctcagt aaatatgtcg ataaccaacg ccttttgtcc actctagaaa tagaaggtag   1320
   aagtgggcac catcaccatc accattaatg aaagctt                             1357
<210> 90
   <211> 2745
   <212> DNA
   <213> Artificial Sequence
<220>
   <223> Synthetic
<400> 90
   ggatccatgg agttcgttaa caaacagttc aactataaag acccagttaa cggtgttgac      60
   attgcttaca tcaaaatccc gaacgctggc cagatgcagc cggtaaaggc attcaaaatc     120
   cacaacaaaa tctgggttat cccggaacgt gataccttta ctaacccgga agaaggtgac      180
   ctgaacccgc caccggaagc gaaacaggtg ccggtatctt actatgactc cacctacctg     240
   tctaccgata acgaaaagga caactacctg aaaggtgtta ctaaactgtt cgagcgtatt     300
   tactccaccg acctgggccg tatgctgctg actagcatcg ttcgcggtat cccgttctgg     360
   ggcggttcta ccatcgatac cgaactgaaa gtaatcgaca ctaactgcat caacgttatt     420
   cagccggacg gttcctatcg ttccgaagaa ctgaacctgg tgatcatcgg cccgtctgct     480
   gatatcatcc agttcgagtg taagagcttt ggtcacgaag ttctgaacct cacccgtaac     540
   ggctacggtt ccactcagta catccgtttc tctccggact tcaccttcgg ttttgaagaa     600
   tccctggaag tagacacgaa cccactgctg ggcgctggta aattcgcaac tgatcctgcg     660
   gttaccctgg ctcacgaact gattcatgca ggccaccgcc tgtacggtat cgccatcaat     720
   ccgaaccgtg tcttcaaagt taacaccaac gcgtattacg agatgtccgg tctggaagtt     780
   agcttcgaag aactgcgtac ttttggcggt cacgacgcta aattcatcga ctctctgcaa     840
   gaaaacgagt tccgtctgta ctactataac aagttcaaag atatcgcatc caccctgaac     900
   aaagcgaaat ccatcgtggg taccactgct tctctccagt acatgaagaa cgtttttaaa     960
   gaaaaatacc tgctcagcga agacacctcc ggcaaattct ctgtagacaa gttgaaattc    1020
   gataaacttt acaaaatgct gactgaaatt tacaccgaag acaacttcgt taagttcttt    1080
   aaagttctga accgcaaaac ctatctgaac ttcgacaagg cagtattcaa aatcaacatc    1140
   gtgccgaaag ttaactacac tatctacgat ggtttcaacc tgcgtaacac caacctggct    1200
   gctaatttta acggccagaa cacggaaatc aacaacatga acttcacaaa actgaaaaac    1260
   ttcactggtc tgttcgagtt ttacaagctg ctgtgcgtcg acggcatcat tacctccaaa    1320
   actaaatctc tgatagaagg tagatttggc ggtttcacgg gcgcacgcaa atcagcgcgt    1380
   aaacgtaaga accaggcgct agcgggcggt ggcggtagcg gcggtggcgg tagcggcggt    1440
   ggcggtagcg cactagtgct gcagtgtatc aaggttaaca actgggattt attcttcagc    1500
   ccgagtgaag acaacttcac caacgacctg aacaaaggtg aagaaatcac ctcagatact    1560
   aacatcgaag cagccgaaga aaacatctcg ctggacctga tccagcagta ctacctgacc    1620
   tttaatttcg acaacgagcc ggaaaacatt tctatcgaaa acctgagctc tgatatcatc    1680
   ggccagctgg aactgatgcc gaacatcgaa cgtttcccaa acggtaaaaa gtacgagctg    1740
   gacaaatata ccatgttcca ctacctgcgc gcgcaggaat ttgaacacgg caaatcccgt    1800
   atcgcactga ctaactccgt taacgaagct ctgctcaacc cgtcccgtgt atacaccttc    1860
   ttctctagcg actacgtgaa aaaggtcaac aaagcgactg aagctgcaat gttcttgggt    1920
   tgggttgaac agcttgttta tgattttacc gacgagacgt ccgaagtatc tactaccgac    1980
   aaaattgcgg atatcactat catcatcccg tacatcggtc cggctctgaa cattggcaac    2040
   atgctgtaca aagacgactt cgttggcgca ctgatcttct ccggtgcggt gatcctgctg    2100
   gagttcatcc cggaaatcgc catcccggta ctgggcacct ttgctctggt ttcttacatt    2160
   gcaaacaagg ttctgactgt acaaaccatc gacaacgcgc tgagcaaacg taacgaaaaa    2220
   tgggatgaag tttacaaata tatcgtgacc aactggctgg ctaaggttaa tactcagatc    2280
   gacctcatcc gcaaaaaaat gaaagaagca ctggaaaacc aggcggaagc taccaaggca    2340
   atcattaact accagtacaa ccagtacacc gaggaagaaa aaaacaacat caacttcaac    2400
   atcgacgatc tgtcctctaa actgaacgaa tccatcaaca aagctatgat caacatcaac    2460
   aagttcctga accagtgctc tgtaagctat ctgatgaact ccatgatccc gtacggtgtt    2520
   aaacgtctgg aggacttcga tgcgtctctg aaagacgccc tgctgaaata catttacgac    2580
   aaccgtggca ctctgatcgg tcaggttgat cgtctgaagg acaaagtgaa caatacctta    2640
   tcgaccgaca tcccttttca gctcagtaaa tatgtcgata accaacgcct tttgtccact    2700
   ctagaaatag aaggtagaag tgggcaccat caccatcacc attaa 2745
<210> 91
   <211> 914
   <212> PRT
   <213> Artificial Sequence
<220>
   <223> Synthetic
<400> 91
   Gly Ser Met Glu Phe Val Asn Lys Gln Phe Asn Tyr Lys Asp Pro Val
   1               5                   10                  15
   Asn Gly Val Asp Ile Ala Tyr Ile Lys Ile Pro Asn Ala Gly Gln Met
               20                  25                  30
   Gln Pro Val Lys Ala Phe Lys Ile His Asn Lys Ile Trp Val Ile Pro
           35                  40                  45
   Glu Arg Asp Thr Phe Thr Asn Pro Glu Glu Gly Asp Leu Asn Pro Pro
       50                  55                  60
   Pro Glu Ala Lys Gln Val Pro Val Ser Tyr Tyr Asp Ser Thr Tyr Leu
   65                  70                  75                  80
   Ser Thr Asp Asn Glu Lys Asp Asn Tyr Leu Lys Gly Val Thr Lys Leu
                   85                  90                  95
   Phe Glu Arg Ile Tyr Ser Thr Asp Leu Gly Arg Met Leu Leu Thr Ser
               100                 105                 110
   Ile Val Arg Gly Ile Pro Phe Trp Gly Gly Ser Thr Ile Asp Thr Glu
           115                 120                 125
   Leu Lys Val Ile Asp Thr Asn Cys Ile Asn Val Ile Gln Pro Asp Gly
       130                 135                 140
   Ser Tyr Arg Ser Glu Glu Leu Asn Leu Val Ile Ile Gly Pro Ser Ala
   145                 150                 155                 160
   Asp Ile Ile Gln Phe Glu Cys Lys Ser Phe Gly His Glu Val Leu Asn
                   165                 170                 175
   Leu Thr Arg Asn Gly Tyr Gly Ser Thr Gln Tyr Ile Arg Phe Ser Pro
               180                 185                 190
   Asp Phe Thr Phe Gly Phe Glu Glu Ser Leu Glu Val Asp Thr Asn Pro
   195 200 205
   Leu Leu Gly Ala Gly Lys Phe Ala Thr Asp Pro Ala Val Thr Leu Ala
   210 215 220
   His Glu Leu Ile His Ala Gly His Arg Leu Tyr Gly Ile Ala Ile Asn
   225 230 235 240
   Pro Asn Arg Val Phe Lys Val Asn Thr Asn Ala Tyr Tyr Glu Met Ser
   245 250 255
   Gly Leu Glu Val Ser Phe Glu Glu Leu Arg Thr Phe Gly Gly His Asp
   260 265 270
   Ala Lys Phe Ile Asp Ser Leu Gln Glu Asn Glu Phe Arg Leu Tyr Tyr
   275 280 285
   Tyr Asn Lys Phe Lys Asp Ile Ala Ser Thr Leu Asn Lys Ala Lys Ser
   290 295 300
   Ile Val Gly Thr Thr Ala Ser Leu Gln Tyr Met Lys Asn Val Phe Lys
   305 310 315 320
   Glu Lys Tyr Leu Leu Ser Glu Asp Thr Ser Gly Lys Phe Ser Val Asp
   325 330 335
   Lys Leu Lys Phe Asp Lys Leu Tyr Lys Met Leu Thr Glu Ile Tyr Thr
   340 345 350
   Glu Asp Asn Phe Val Lys Phe Phe Lys Val Leu Asn Arg Lys Thr Tyr
   355 360 365
   Leu Asn Phe Asp Lys Ala Val Phe Lys Ile Asn Ile Val Pro Lys Val
   370 375 380
   Asn Tyr Thr Ile Tyr Asp Gly Phe Asn Leu Arg Asn Thr Asn Leu Ala
   385 390 395 400
   Ala Asn Phe Asn Gly Gln Asn Thr Glu Ile Asn Asn Met Asn Phe Thr
   405 410 415
   Lys Leu Lys Asn Phe Thr Gly Leu Phe Glu Phe Tyr Lys Leu Leu Cys
   420 425 430
   Val Asp Gly Ile Ile Thr Ser Lys Thr Lys Ser Leu Ile Glu Gly Arg
   435 440 445
   Phe Gly Gly Phe Thr Gly Ala Arg Lys Ser Ala Arg Lys Arg Lys Asn
   450 455 460
   Gln Ala Leu Ala Gly Gly Gly Gly Ser Gly Gly Gly Gly Ser Gly Gly
   465 470 475 480
   Gly Gly Ser Ala Leu Val Leu Gln Cys Ile Lys Val Asn Asn Trp Asp
   485 490 495
   Leu Phe Phe Ser Pro Ser Glu Asp Asn Phe Thr Asn Asp Leu Asn Lys
   500 505 510
   Gly Glu Glu Ile Thr Ser Asp Thr Asn Ile Glu Ala Ala Glu Glu Asn
   515 520 525
   Ile Ser Leu Asp Leu Ile Gln Gln Tyr Tyr Leu Thr Phe Asn Phe Asp
   530 535 540
   Asn Glu Pro Glu Asn Ile Ser Ile Glu Asn Leu Ser Ser Asp Ile Ile
   545 550 555 560
   Gly Gln Leu Glu Leu Met Pro Asn Ile Glu Arg Phe Pro Asn Gly Lys
   565 570 575
   Lys Tyr Glu Leu Asp Lys Tyr Thr Met Phe His Tyr Leu Arg Ala Gln
   580 585 590
   Glu Phe Glu His Gly Lys Ser Arg Ile Ala Leu Thr Asn Ser Val Asn
   595 600 605
   Glu Ala Leu Leu Asn Pro Ser Arg Val Tyr Thr Phe Phe Ser Ser Asp
   610 615 620
   Tyr Val Lys Lys Val Asn Lys Ala Thr Glu Ala Ala Met Phe Leu Gly
   625 630 635 640
   Trp Val Glu Gln Leu Val Tyr Asp Phe Thr Asp Glu Thr Ser Glu Val
   645 650 655
   Ser Thr Thr Asp Lys Ile Ala Asp Ile Thr Ile Ile Ile Pro Tyr Ile
   660 665 670
   Gly Pro Ala Leu Asn Ile Gly Asn Met Leu Tyr Lys Asp Asp Phe Val
   675 680 685
   Gly Ala Leu Ile Phe Ser Gly Ala Val Ile Leu Leu Glu Phe Ile Pro
   690 695 700
   Glu Ile Ala Ile Pro Val Leu Gly Thr Phe Ala Leu Val Ser Tyr Ile
   705 710 715 720
   Ala Asn Lys Val Leu Thr Val Gln Thr Ile Asp Asn Ala Leu Ser Lys
   725 730 735
   Arg Asn Glu Lys Trp Asp Glu Val Tyr Lys Tyr Ile Val Thr Asn Trp
   740 745 750
   Leu Ala Lys Val Asn Thr Gln Ile Asp Leu Ile Arg Lys Lys Met Lys
   755 760 765
   Glu Ala Leu Glu Asn Gln Ala Glu Ala Thr Lys Ala Ile Ile Asn Tyr
   770 775 780
   Gln Tyr Asn Gln Tyr Thr Glu Glu Glu Lys Asn Asn Ile Asn Phe Asn
   785 790 795 800
   Ile Asp Asp Leu Ser Ser Lys Leu Asn Glu Ser Ile Asn Lys Ala Met
   805 810 815
   Ile Asn Ile Asn Lys Phe Leu Asn Gln Cys Ser Val Ser Tyr Leu Met
   820 825 830
   Asn Ser Met Ile Pro Tyr Gly Val Lys Arg Leu Glu Asp Phe Asp Ala
   835 840 845
   Ser Leu Lys Asp Ala Leu Leu Lys Tyr Ile Tyr Asp Asn Arg Gly Thr
   850 855 860
   Leu Ile Gly Gln Val Asp Arg Leu Lys Asp Lys Val Asn Asn Thr Leu
   865 870 875 880
   Ser Thr Asp Ile Pro Phe Gln Leu Ser Lys Tyr Val Asp Asn Gln Arg
   885 890 895
   Leu Leu Ser Thr Leu Glu Ile Glu Gly Arg Ser Gly His His His His
   900 905 910
   His His
<210> 92
   <211> 619
   <212> DNA
   <213> Artificial Sequence
<220>
   <223> Synthetic
<400> 92
   gctagcgggc ggtggcggta gcggcggtgg cggtagcggc ggtggcggta gcgcactagt     60
   gctgcagtgt atcaatctgg attgggacgt aatccgtgat aagaccaaaa caaaaatcga    120
   gtctttgaaa gaacacggcc cgatcaaaaa taagatgtct gaatcaccca ataaaactgt    180
   ttcggaggaa aaagcgaaac agtatttgga agagtttcat caaaccgcgc ttgaacatcc    240
   ggagctcagt gaactgaaaa cagtgacggg aacgaatcct gtttttgcag gcgcaaacta    300
   tgcggcttgg gccgtgaatg ttgcccaagt aattgatagt gagaccgcag acaacctgga    360
   aaagacgacc gcagcgttaa gcattttacc ggggattggt tccgtgatgg gtatagcgga    420
   tggagcggtc caccataaca ctgaggaaat tgtcgcccag tcaatcgctc tgagttccct    480
   gatggttgca caggctatcc cactcgtggg ggaactggtt gacataggtt tcgccgccta    540
   caacttcgta gaaagcatta ttaatctttt tcaggtggtg cataacagct acaaccgccc    600
   tctagaatga taaaagctt                                                 619
<210> 93
   <211> 1971
   <212> DNA
   <213> Artificial Sequence
<220>
   <223> Synthetic
<400> 93
   ggatccatgg agttcgttaa caaacagttc aactataaag acccagttaa cggtgttgac     60
   attgcttaca tcaaaatccc gaacgctggc cagatgcagc cggtaaaggc attcaaaatc    120
   cacaacaaaa tctgggttat cccggaacgt gataccttta ctaacccgga agaaggtgac    180
   ctgaacccgc caccggaagc gaaacaggtg ccggtatctt actatgactc cacctacctg    240
   tctaccgata acgaaaagga caactacctg aaaggtgtta ctaaactgtt cgagcgtatt    300
   tactccaccg acctgggccg tatgctgctg actagcatcg ttcgcggtat cccgttctgg    360
   ggcggttcta ccatcgatac cgaactgaaa gtaatcgaca ctaactgcat caacgttatt    420
   cagccggacg gttcctatcg ttccgaagaa ctgaacctgg tgatcatcgg cccgtctgct    480
   gatatcatcc agttcgagtg taagagcttt ggtcacgaag ttctgaacct cacccgtaac    540
   ggctacggtt ccactcagta catccgtttc tctccggact tcaccttcgg ttttgaagaa    600
   tccctggaag tagacacgaa cccactgctg ggcgctggta aattcgcaac tgatcctgcg    660
   gttaccctgg ctcacgaact gattcatgca ggccaccgcc tgtacggtat cgccatcaat    720
   ccgaaccgtg tcttcaaagt taacaccaac gcgtattacg agatgtccgg tctggaagtt    780
   agcttcgaag aactgcgtac ttttggcggt cacgacgcta aattcatcga ctctctgcaa    840
   gaaaacgagt tccgtctgta ctactataac aagttcaaag atatcgcatc caccctgaac    900
   aaagcgaaat ccatcgtggg taccactgct tctctccagt acatgaagaa cgtttttaaa    960
   gaaaaatacc tgctcagcga agacacctcc ggcaaattct ctgtagacaa gttgaaattc   1020
   gataaacttt acaaaatgct gactgaaatt tacaccgaag acaacttcgt taagttcttt   1080
   aaagttctga accgcaaaac ctatctgaac ttcgacaagg cagtattcaa aatcaacatc   1140
   gtgccgaaag ttaactacac tatctacgat ggtttcaacc tgcgtaacac caacctggct   1200
   gctaatttta acggccagaa cacggaaatc aacaacatga acttcacaaa actgaaaaac   1260
   ttcactggtc tgttcgagtt ttacaagctg ctgtgcgtcg acggcatcat tacctccaaa   1320
   actaaatctc tgatagaagg tagatacggt ggtttcctgg cgctagcggg cggtggcggt   1380
   agcggcggtg gcggtagcgg cggtggcggt agcgcactag tgctgcagtg tatcaatctg   1440
   gattgggacg taatccgtga taagaccaaa acaaaaatcg agtctttgaa agaacacggc   1500
   ccgatcaaaa ataagatgtc tgaatcaccc aataaaactg tttcggagga aaaagcgaaa   1560
   cagtatttgg aagagtttca tcaaaccgcg cttgaacatc cggagctcag tgaactgaaa   1620
   acagtgacgg gaacgaatcc tgtttttgca ggcgcaaact atgcggcttg ggccgtgaat   1680
   gttgcccaag taattgatag tgagaccgca gacaacctgg aaaagacgac cgcagcgtta   1740
   agcattttac cggggattgg ttccgtgatg ggtatagcgg atggagcggt ccaccataac   1800
   actgaggaaa ttgtcgccca gtcaatcgct ctgagttccc tgatggttgc acaggctatc   1860
   ccactcgtgg gggaactggt tgacataggt ttcgccgcct acaacttcgt agaaagcatt   1920
   attaatcttt ttcaggtggt gcataacagc tacaaccgcc ctctagaatg a            1971
<210> 94
   <211> 656
   <212> PRT
   <213> Artificial Sequence
<220>
   <223> Synthetic
<400> 94
   Gly Ser Met Glu Phe Val Asn Lys Gln Phe Asn Tyr Lys Asp Pro Val
   1               5                   10                  15
   Asn Gly Val Asp Ile Ala Tyr Ile Lys Ile Pro Asn Ala Gly Gln Met
   20 25 30
   Gln Pro Val Lys Ala Phe Lys Ile His Asn Lys Ile Trp Val Ile Pro
   35 40 45
   Glu Arg Asp Thr Phe Thr Asn Pro Glu Glu Gly Asp Leu Asn Pro Pro
   50 55 60
   Pro Glu Ala Lys Gln Val Pro Val Ser Tyr Tyr Asp Ser Thr Tyr Leu
   65 70 75 80
   Ser Thr Asp Asn Glu Lys Asp Asn Tyr Leu Lys Gly Val Thr Lys Leu
   85 90 95
   Phe Glu Arg Ile Tyr Ser Thr Asp Leu Gly Arg Met Leu Leu Thr Ser
   100 105 110
   Ile Val Arg Gly Ile Pro Phe Trp Gly Gly Ser Thr Ile Asp Thr Glu
   115 120 125
   Leu Lys Val Ile Asp Thr Asn Cys Ile Asn Val Ile Gln Pro Asp Gly
   130 135 140
   Ser Tyr Arg Ser Glu Glu Leu Asn Leu Val Ile Ile Gly Pro Ser Ala
   145 150 155 160
   Asp Ile Ile Gln Phe Glu Cys Lys Ser Phe Gly His Glu Val Leu Asn
   165 170 175
   Leu Thr Arg Asn Gly Tyr Gly Ser Thr Gln Tyr Ile Arg Phe Ser Pro
   180 185 190
   Asp Phe Thr Phe Gly Phe Glu Glu Ser Leu Glu Val Asp Thr Asn Pro
   195 200 205
   Leu Leu Gly Ala Gly Lys Phe Ala Thr Asp Pro Ala Val Thr Leu Ala
   210 215 220
   His Glu Leu Ile His Ala Gly His Arg Leu Tyr Gly Ile Ala Ile Asn
   225 230 235 240
   Pro Asn Arg Val Phe Lys Val Asn Thr Asn Ala Tyr Tyr Glu Met Ser
   245 250 255
   Gly Leu Glu Val Ser Phe Glu Glu Leu Arg Thr Phe Gly Gly His Asp
               260                 265                 270
   Ala Lys Phe Ile Asp Ser Leu Gln Glu Asn Glu Phe Arg Leu Tyr Tyr
           275                 280                 285
   Tyr Asn Lys Phe Lys Asp Ile Ala Ser Thr Leu Asn Lys Ala Lys Ser
       290                 295                 300
   Ile Val Gly Thr Thr Ala Ser Leu Gln Tyr Met Lys Asn Val Phe Lys
   305                 310                 315                 320
   Glu Lys Tyr Leu Leu Ser Glu Asp Thr Ser Gly Lys Phe Ser Val Asp
                   325                 330                 335
   Lys Leu Lys Phe Asp Lys Leu Tyr Lys Met Leu Thr Glu Ile Tyr Thr
               340                 345                 350
   Glu Asp Asn Phe Val Lys Phe Phe Lys Val Leu Asn Arg Lys Thr Tyr
           355                 360                 365
   Leu Asn Phe Asp Lys Ala Val Phe Lys Ile Asn Ile Val Pro Lys Val
       370                 375                 380
   Asn Tyr Thr Ile Tyr Asp Gly Phe Asn Leu Arg Asn Thr Asn Leu Ala
   385                 390                 395                 400
   Ala Asn Phe Asn Gly Gln Asn Thr Glu Ile Asn Asn Met Asn Phe Thr
                   405                 410                 415
   Lys Leu Lys Asn Phe Thr Gly Leu Phe Glu Phe Tyr Lys Leu Leu Cys
               420                 425                 430
   Val Asp Gly Ile Ile Thr Ser Lys Thr Lys Ser Leu Ile Glu Gly Arg
           435                 440                 445
   Tyr Gly Gly Phe Leu Ala Leu Ala Gly Gly Gly Gly Ser Gly Gly Gly
       450                 455                 460
   Gly Ser Gly Gly Gly Gly Ser Ala Leu Val Leu Gln Cys Ile Asn Leu
   465                 470                 475                 480
   Asp Trp Asp Val Ile Arg Asp Lys Thr Lys Thr Lys Ile Glu Ser Leu
                   485                 490                 495
   Lys Glu His Gly Pro Ile Lys Asn Lys Met Ser Glu Ser Pro Asn Lys
               500                 505                 510
   Thr Val Ser Glu Glu Lys Ala Lys Gln Tyr Leu Glu Glu Phe His Gln
           515                 520                 525
   Thr Ala Leu Glu His Pro Glu Leu Ser Glu Leu Lys Thr Val Thr Gly
       530                 535                 540
   Thr Asn Pro Val Phe Ala Gly Ala Asn Tyr Ala Ala Trp Ala Val Asn
   545                 550                 555                 560
   Val Ala Gln Val Ile Asp Ser Glu Thr Ala Asp Asn Leu Glu Lys Thr
                   565                 570                 575
   Thr Ala Ala Leu Ser Ile Leu Pro Gly Ile Gly Ser Val Met Gly Ile
               580                 585                 590
   Ala Asp Gly Ala Val His His Asn Thr Glu Glu Ile Val Ala Gln Ser
           595                 600                 605
   Ile Ala Leu Ser Ser Leu Met Val Ala Gln Ala Ile Pro Leu Val Gly
       610                 615                 620
   Glu Leu Val Asp Ile Gly Phe Ala Ala Tyr Asn Phe Val Glu Ser Ile
   625                 630                 635                 640
   Ile Asn Leu Phe Gln Val Val His Asn Ser Tyr Asn Arg Pro Leu Glu
                   645                 650                 655
<210> 95
   <211> 1329
   <212> DNA
   <213> Artificial Sequence
<220>
   <223> Synthetic
<400> 95
   ggatccatgc ctattactat taacaatttt cgttatagcg atcccgtcaa caatgacacc 60
   attatcatga tggaaccgcc atattgcaaa ggactggaca tttactataa agccttcaag 120
   attactgacc gcatttggat tgttccagag cgttacgagt tcgggacgaa accagaagat 180
   tttaacccgc cttcatcgct gatcgaagga gcatcagagt attacgatcc gaactatctg 240
   cgtacggaca gcgataaaga ccgcttctta cagaccatgg tcaaactttt taaccgtatt 300
   aagaacaatg tggccggaga agcactcttg gataagatta tcaacgcgat tccatacctg 360
   ggcaattctt acagcctgct ggataaattt gacacaaata gtaattcagt cagctttaac 420
   ctgttagaac aagatccgag tggcgcaacc acgaagtctg ccatgctgac aaatctgatc 480
   atttttggtc caggtcctgt actgaataaa aatgaagtac gcggcatcgt tctccgcgtg 540
   gacaataaga actacttccc atgccgtgac ggcttcggtt cgatcatgca gatggctttc 600
   tgtccggagt acgttccgac gtttgataat gttattgaga atatcacgag tttaacaatc 660
   ggtaagtcaa aatattttca agatccggcc cttctcctta tgcatgaact gattcacgtg 720
   ctgcacggct tatatggtat gcaagtgtcc tcgcatgaaa tcattccgtc caaacaggaa 780
   atttatatgc agcataccta cccgatttca gctgaagagt tgtttacgtt tggtggccag 840
   gacgcgaatt tgatctccat cgacatcaaa aacgatctgt atgagaaaac attaaatgac 900
   tataaagcga ttgcgaacaa actgtctcag gtgactagct gcaacgatcc taacattgat 960
   attgattcct acaaacaaat ttatcaacag aaataccagt tcgataaaga cagcaatggt 1020
   cagtatatcg taaacgaaga taaatttcag atcctgtata acagcattat gtatggcttt 1080
   accgaaattg agttggggaa gaaatttaac attaaaaccc gtctgtctta ttttagtatg 1140
   aaccatgatc cggtgaaaat ccccaatctg cttgatgata ccatttataa tgataccgaa 1200
   gggttcaaca ttgaatctaa ggatctgaaa tccgaataca aaggccaaaa tatgcgtgtt 1260
   aatactaacg ctttccgtaa tgttgatggt agtggactcg tctcgaaact gattgggttg 1320
   tgtgtcgac 1329
<210> 96
   <211> 2736
   <212> DNA
   <213> Artificial Sequence
<220>
   <223> Synthetic
<400> 96
   ggatccatgc ctattactat taacaatttt cgttatagcg atcccgtcaa caatgacacc    60
   attatcatga tggaaccgcc atattgcaaa ggactggaca tttactataa agccttcaag   120
   attactgacc gcatttggat tgttccagag cgttacgagt tcgggacgaa accagaagat   180
   tttaacccgc cttcatcgct gatcgaagga gcatcagagt attacgatcc gaactatctg   240
   cgtacggaca gcgataaaga ccgcttctta cagaccatgg tcaaactttt taaccgtatt   300
   aagaacaatg tggccggaga agcactcttg gataagatta tcaacgcgat tccatacctg   360
   ggcaattctt acagcctgct ggataaattt gacacaaata gtaattcagt cagctttaac   420
   ctgttagaac aagatccgag tggcgcaacc acgaagtctg ccatgctgac aaatctgatc   480
   atttttggtc caggtcctgt actgaataaa aatgaagtac gcggcatcgt tctccgcgtg   540
   gacaataaga actacttccc atgccgtgac ggcttcggtt cgatcatgca gatggctttc 600
   tgtccggagt acgttccgac gtttgataat gttattgaga atatcacgag tttaacaatc 660
   ggtaagtcaa aatattttca agatccggcc cttctcctta tgcatgaact gattcacgtg 720
   ctgcacggct tatatggtat gcaagtgtcc tcgcatgaaa tcattccgtc caaacaggaa 780
   atttatatgc agcataccta cccgatttca gctgaagagt tgtttacgtt tggtggccag 840
   gacgcgaatt tgatctccat cgacatcaaa aacgatctgt atgagaaaac attaaatgac 900
   tataaagcga ttgcgaacaa actgtctcag gtgactagct gcaacgatcc taacattgat 960
   attgattcct acaaacaaat ttatcaacag aaataccagt tcgataaaga cagcaatggt 1020
   cagtatatcg taaacgaaga taaatttcag atcctgtata acagcattat gtatggcttt 1080
   accgaaattg agttggggaa gaaatttaac attaaaaccc gtctgtctta ttttagtatg 1140
   aaccatgatc cggtgaaaat ccccaatctg cttgatgata ccatttataa tgataccgaa 1200
   gggttcaaca ttgaatctaa ggatctgaaa tccgaataca aaggccaaaa tatgcgtgtt 1260
   aatactaacg ctttccgtaa tgttgatggt agtggactcg tctcgaaact gattgggttg 1320
   tgtgtcgacg gcatcattac ctccaaaact aaatctctga tagaaggtag atttggcggt 1380
   ttcacgggcg cacgcaaatc agcgcgtaaa cgtaagaacc aggcgctagc gggcggtggc 1440
   ggtagcggcg gtggcggtag cggcggtggc ggtagcgcac tagtgctgca gtgtatcaag 1500
   gttaacaact gggatttatt cttcagcccg agtgaagaca acttcaccaa cgacctgaac 1560
   aaaggtgaag aaatcacctc agatactaac atcgaagcag ccgaagaaaa catctcgctg 1620
   gacctgatcc agcagtacta cctgaccttt aatttcgaca acgagccgga aaacatttct 1680
   atcgaaaacc tgagctctga tatcatcggc cagctggaac tgatgccgaa catcgaacgt 1740
   ttcccaaacg gtaaaaagta cgagctggac aaatatacca tgttccacta cctgcgcgcg 1800
   caggaatttg aacacggcaa atcccgtatc gcactgacta actccgttaa cgaagctctg 1860
   ctcaacccgt cccgtgtata caccttcttc tctagcgact acgtgaaaaa ggtcaacaaa 1920
   gcgactgaag ctgcaatgtt cttgggttgg gttgaacagc ttgtttatga ttttaccgac 1980
   gagacgtccg aagtatctac taccgacaaa attgcggata tcactatcat catcccgtac 2040
   atcggtccgg ctctgaacat tggcaacatg ctgtacaaag acgacttcgt tggcgcactg 2100
   atcttctccg gtgcggtgat cctgctggag ttcatcccgg aaatcgccat cccggtactg 2160
   ggcacctttg ctctggtttc ttacattgca aacaaggttc tgactgtaca aaccatcgac 2220
   aacgcgctga gcaaacgtaa cgaaaaatgg gatgaagttt acaaatatat cgtgaccaac 2280
   tggctggcta aggttaatac tcagatcgac ctcatccgca aaaaaatgaa agaagcactg 2340
   gaaaaccagg cggaagctac caaggcaatc attaactacc agtacaacca gtacaccgag 2400
   gaagaaaaaa acaacatcaa cttcaacatc gacgatctgt cctctaaact gaacgaatcc 2460
   atcaacaaag ctatgatcaa catcaacaag ttcctgaacc agtgctctgt aagctatctg 2520
   atgaactcca tgatcccgta cggtgttaaa cgtctggagg acttcgatgc gtctctgaaa 2580
   gacgccctgc tgaaatacat ttacgacaac cgtggcactc tgatcggtca ggttgatcgt 2640
   ctgaaggaca aagtgaacaa taccttatcg accgacatcc cttttcagct cagtaaatat 2700
   gtcgataacc aacgcctttt gtccactcta gactag 2736
<210> 97
   <211> 911
   <212> PRT
   <213> Artificial Sequence
<220>
   <223> Synthetic
<400> 97
   Gly Ser Met Pro Ile Thr Ile Asn Asn Phe Arg Tyr Ser Asp Pro Val
   1 5 10 15
   Asn Asn Asp Thr Ile Ile Met Met Glu Pro Pro Tyr Cys Lys Gly Leu
   20 25 30
   Asp Ile Tyr Tyr Lys Ala Phe Lys Ile Thr Asp Arg Ile Trp Ile Val
   35 40 45
   Pro Glu Arg Tyr Glu Phe Gly Thr Lys Pro Glu Asp Phe Asn Pro Pro
   50 55 60
   Ser Ser Leu Ile Glu Gly Ala Ser Glu Tyr Tyr Asp Pro Asn Tyr Leu
   65 70 75 80
   Arg Thr Asp Ser Asp Lys Asp Arg Phe Leu Gln Thr Met Val Lys Leu
   85 90 95
   Phe Asn Arg Ile Lys Asn Asn Val Ala Gly Glu Ala Leu Leu Asp Lys
   100 105 110
   Ile Ile Asn Ala Ile Pro Tyr Leu Gly Asn Ser Tyr Ser Leu Leu Asp
   115 120 125
   Lys Phe Asp Thr Asn Ser Asn Ser Val Ser Phe Asn Leu Leu Glu Gln
   130 135 140
   Asp Pro Ser Gly Ala Thr Thr Lys Ser Ala Met Leu Thr Asn Leu Ile
   145                 150                 155                 160
   Ile Phe Gly Pro Gly Pro Val Leu Asn Lys Asn Glu Val Arg Gly Ile
                   165                 170                 175
   Val Leu Arg Val Asp Asn Lys Asn Tyr Phe Pro Cys Arg Asp Gly Phe
               180                 185                 190
   Gly Ser Ile Met Gln Met Ala Phe Cys Pro Glu Tyr Val Pro Thr Phe
           195                 200                 205
   Asp Asn Val Ile Glu Asn Ile Thr Ser Leu Thr Ile Gly Lys Ser Lys
       210                 215                 220
   Tyr Phe Gln Asp Pro Ala Leu Leu Leu Met His Glu Leu Ile His Val
   225                 230                 235                 240
   Leu His Gly Leu Tyr Gly Met Gln Val Ser Ser His Glu Ile Ile Pro
                   245                 250                 255
   Ser Lys Gln Glu Ile Tyr Met Gln His Thr Tyr Pro Ile Ser Ala Glu
               260                 265                 270
   Glu Leu Phe Thr Phe Gly Gly Gln Asp Ala Asn Leu Ile Ser Ile Asp
           275                 280                 285
   Ile Lys Asn Asp Leu Tyr Glu Lys Thr Leu Asn Asp Tyr Lys Ala Ile
       290                 295                 300
   Ala Asn Lys Leu Ser Gln Val Thr Ser Cys Asn Asp Pro Asn Ile Asp
   305                 310                 315                 320
   Ile Asp Ser Tyr Lys Gln Ile Tyr Gln Gln Lys Tyr Gln Phe Asp Lys
                   325                 330                 335
   Asp Ser Asn Gly Gln Tyr Ile Val Asn Glu Asp Lys Phe Gln Ile Leu
               340                 345                 350
   Tyr Asn Ser Ile Met Tyr Gly Phe Thr Glu Ile Glu Leu Gly Lys Lys
           355                 360                 365
   Phe Asn Ile Lys Thr Arg Leu Ser Tyr Phe Ser Met Asn His Asp Pro
       370                 375                 380
   Val Lys Ile Pro Asn Leu Leu Asp Asp Thr Ile Tyr Asn Asp Thr Glu
   385 390 395 400
   Gly Phe Asn Ile Glu Ser Lys Asp Leu Lys Ser Glu Tyr Lys Gly Gln
   405 410 415
   Asn Met Arg Val Asn Thr Asn Ala Phe Arg Asn Val Asp Gly Ser Gly
   420 425 430
   Leu Val Ser Lys Leu Ile Gly Leu Cys Val Asp Gly Ile Ile Thr Ser
   435 440 445
   Lys Thr Lys Ser Leu Ile Glu Gly Arg Phe Gly Gly Phe Thr Gly Ala
   450 455 460
   Arg Lys Ser Ala Arg Lys Arg Lys Asn Gln Ala Leu Ala Gly Gly Gly
   465 470 475 480
   Gly Ser Gly Gly Gly Gly Ser Gly Gly Gly Gly Ser Ala Leu Val Leu
   485 490 495
   Gln Cys Ile Lys Val Asn Asn Trp Asp Leu Phe Phe Ser Pro Ser Glu
   500 505 510
   Asp Asn Phe Thr Asn Asp Leu Asn Lys Gly Glu Glu Ile Thr Ser Asp
   515 520 525
   Thr Asn Ile Glu Ala Ala Glu Glu Asn Ile Ser Leu Asp Leu Ile Gln
   530 535 540
   Gln Tyr Tyr Leu Thr Phe Asn Phe Asp Asn Glu Pro Glu Asn Ile Ser
   545 550 555 560
   Ile Glu Asn Leu Ser Ser Asp Ile Ile Gly Gln Leu Glu Leu Met Pro
   565 570 575
   Asn Ile Glu Arg Phe Pro Asn Gly Lys Lys Tyr Glu Leu Asp Lys Tyr
   580 585 590
   Thr Met Phe His Tyr Leu Arg Ala Gln Glu Phe Glu His Gly Lys Ser
   595 600 605
   Arg Ile Ala Leu Thr Asn Ser Val Asn Glu Ala Leu Leu Asn Pro Ser
   610 615 620
   Arg Val Tyr Thr Phe Phe Ser Ser Asp Tyr Val Lys Lys Val Asn Lys
   625 630 635 640
   Ala Thr Glu Ala Ala Met Phe Leu Gly Trp Val Glu Gln Leu Val Tyr
   645 650 655
   Asp Phe Thr Asp Glu Thr Ser Glu Val Ser Thr Thr Asp Lys Ile Ala
   660 665 670
   Asp Ile Thr Ile Ile Ile Pro Tyr Ile Gly Pro Ala Leu Asn Ile Gly
   675 680 685
   Asn Met Leu Tyr Lys Asp Asp Phe Val Gly Ala Leu Ile Phe Ser Gly
   690 695 700
   Ala Val Ile Leu Leu Glu Phe Ile Pro Glu Ile Ala Ile Pro Val Leu
   705 710 715 720
   Gly Thr Phe Ala Leu Val Ser Tyr Ile Ala Asn Lys Val Leu Thr Val
   725 730 735
   Gln Thr Ile Asp Asn Ala Leu Ser Lys Arg Asn Glu Lys Trp Asp Glu
   740 745 750
   Val Tyr Lys Tyr Ile Val Thr Asn Trp Leu Ala Lys Val Asn Thr Gln
   755 760 765
   Ile Asp Leu Ile Arg Lys Lys Met Lys Glu Ala Leu Glu Asn Gln Ala
   770 775 780
   Glu Ala Thr Lys Ala Ile Ile Asn Tyr Gln Tyr Asn Gln Tyr Thr Glu
   785 790 795 800
   Glu Glu Lys Asn Asn Ile Asn Phe Asn Ile Asp Asp Leu Ser Ser Lys
   805 810 815
   Leu Asn Glu Ser Ile Asn Lys Ala Met Ile Asn Ile Asn Lys Phe Leu
   820 825 830
   Asn Gln Cys Ser Val Ser Tyr Leu Met Asn Ser Met Ile Pro Tyr Gly
   835 840 845
   Val Lys Arg Leu Glu Asp Phe Asp Ala Ser Leu Lys Asp Ala Leu Leu
   850 855 860
   Lys Tyr Ile Tyr Asp Asn Arg Gly Thr Leu Ile Gly Gln Val Asp Arg
   865 870 875 880
   Leu Lys Asp Lys Val Asn Asn Thr Leu Ser Thr Asp Ile Pro Phe Gln
   885 890 895
   Leu Ser Lys Tyr Val Asp Asn Gln Arg Leu Leu Ser Thr Leu Asp
   900 905 910
<210> 98
   <211> 180
   <212> DNA
   <213> Artificial Sequence
<220>
   <223> Synthetic
<400> 98
   ggatccacgc acgtcgacgc gattgatggt cgttttggcg gtttcacggg cgcacgcaaa 60
   tcagcgcgta aacgtaagaa ccaggcgcta gcgggcggtg gcggtagcgg cggtggcggt 120
   agcggcggtg gcggtagcgc actagtgctg cagacgcacg gtctagaatg ataaaagctt 180
<210> 99
   <211> 2715
   <212> DNA
   <213> Artificial Sequence
<220>
   <223> Synthetic
<400> 99
   ggatccgaat tcatgccgat caccatcaac aacttcaact acagcgatcc ggtggataac     60
   aaaaacatcc tgtacctgga tacccatctg aataccctgg cgaacgaacc ggaaaaagcg    120
   tttcgtatca ccggcaacat ttgggttatt ccggatcgtt ttagccgtaa cagcaacccg    180
   aatctgaata aaccgccgcg tgttaccagc ccgaaaagcg gttattacga tccgaactat    240
   ctgagcaccg atagcgataa agataccttc ctgaaagaaa tcatcaaact gttcaaacgc    300
   atcaacagcc gtgaaattgg cgaagaactg atctatcgcc tgagcaccga tattccgttt    360
   ccgggcaaca acaacacccc gatcaacacc tttgatttcg atgtggattt caacagcgtt    420
   gatgttaaaa cccgccaggg taacaattgg gtgaaaaccg gcagcattaa cccgagcgtg    480
   attattaccg gtccgcgcga aaacattatt gatccggaaa ccagcacctt taaactgacc    540
   aacaacacct ttgcggcgca ggaaggtttt ggcgcgctga gcattattag cattagcccg    600
   cgctttatgc tgacctatag caacgcgacc aacgatgttg gtgaaggccg tttcagcaaa    660
   agcgaatttt gcatggaccc gatcctgatc ctgatgcatg aactgaacca tgcgatgcat    720
   aacctgtatg gcatcgcgat tccgaacgat cagaccatta gcagcgtgac cagcaacatc    780
   ttttacagcc agtacaacgt gaaactggaa tatgcggaaa tctatgcgtt tggcggtccg    840
   accattgatc tgattccgaa aagcgcgcgc aaatacttcg aagaaaaagc gctggattac 900
   tatcgcagca ttgcgaaacg tctgaacagc attaccaccg cgaatccgag cagcttcaac 960
   aaatatatcg gcgaatataa acagaaactg atccgcaaat atcgctttgt ggtggaaagc 1020
   agcggcgaag ttaccgttaa ccgcaataaa ttcgtggaac tgtacaacga actgacccag 1080
   atcttcaccg aatttaacta tgcgaaaatc tataacgtgc agaaccgtaa aatctacctg 1140
   agcaacgtgt ataccccggt gaccgcgaat attctggatg ataacgtgta cgatatccag 1200
   aacggcttta acatcccgaa aagcaacctg aacgttctgt ttatgggcca gaacctgagc 1260
   cgtaatccgg cgctgcgtaa agtgaacccg gaaaacatgc tgtacctgtt caccaaattt 1320
   tgcgtcgacg cgattgatgg tcgttttggc ggtttcacgg gcgcacgcaa atcagcgcgt 1380
   aaacgtaaga accaggcgct agcgggcggt ggcggtagcg gcggtggcgg tagcggcggt 1440
   ggcggtagcg cactagtgct gcagtgtcgt gaactgctgg tgaaaaacac cgatctgccg 1500
   tttattggcg atatcagcga tgtgaaaacc gatatcttcc tgcgcaaaga tatcaacgaa 1560
   gaaaccgaag tgatctacta cccggataac gtgagcgttg atcaggtgat cctgagcaaa 1620
   aacaccagcg aacatggtca gctggatctg ctgtatccga gcattgatag cgaaagcgaa 1680
   attctgccgg gcgaaaacca ggtgttttac gataaccgta cccagaacgt ggattacctg 1740
   aacagctatt actacctgga aagccagaaa ctgagcgata acgtggaaga ttttaccttt 1800
   acccgcagca ttgaagaagc gctggataac agcgcgaaag tttacaccta ttttccgacc 1860
   ctggcgaaca aagttaatgc gggtgttcag ggcggtctgt ttctgatgtg ggcgaacgat 1920
   gtggtggaag atttcaccac caacatcctg cgtaaagata ccctggataa aatcagcgat 1980
   gttagcgcga ttattccgta tattggtccg gcgctgaaca ttagcaatag cgtgcgtcgt 2040
   ggcaatttta ccgaagcgtt tgcggttacc ggtgtgacca ttctgctgga agcgtttccg 2100
   gaatttacca ttccggcgct gggtgcgttt gtgatctata gcaaagtgca ggaacgcaac 2160
   gaaatcatca aaaccatcga taactgcctg gaacagcgta ttaaacgctg gaaagatagc 2220
   tatgaatgga tgatgggcac ctggctgagc cgtattatca cccagttcaa caacatcagc 2280
   taccagatgt acgatagcct gaactatcag gcgggtgcga ttaaagcgaa aatcgatctg 2340
   gaatacaaaa aatacagcgg cagcgataaa gaaaacatca aaagccaggt tgaaaacctg 2400
   aaaaacagcc tggatgtgaa aattagcgaa gcgatgaata acatcaacaa attcatccgc 2460
   gaatgcagcg tgacctacct gttcaaaaac atgctgccga aagtgatcga tgaactgaac 2520
   gaatttgatc gcaacaccaa agcgaaactg atcaacctga tcgatagcca caacattatt 2580
   ctggtgggcg aagtggataa actgaaagcg aaagttaaca acagcttcca gaacaccatc 2640
   ccgtttaaca tcttcagcta taccaacaac agcctgctga aagatatcat caacgaatac 2700
   ttcaatctag actag 2715
<210> 100
   <211> 904
   <212> PRT
   <213> Artificial Sequence
<220>
   <223> Synthetic
<400> 100
   Gly Ser Glu Phe Met Pro Ile Thr Ile Asn Asn Phe Asn Tyr Ser Asp
   1               5                   10                  15
   Pro Val Asp Asn Lys Asn Ile Leu Tyr Leu Asp Thr His Leu Asn Thr
               20                  25                  30
   Leu Ala Asn Glu Pro Glu Lys Ala Phe Arg Ile Thr Gly Asn Ile Trp
           35                  40                  45
   Val Ile Pro Asp Arg Phe Ser Arg Asn Ser Asn Pro Asn Leu Asn Lys
       50                  55                  60
   Pro Pro Arg Val Thr Ser Pro Lys Ser Gly Tyr Tyr Asp Pro Asn Tyr
   65                  70                  75                  80
   Leu Ser Thr Asp Ser Asp Lys Asp Thr Phe Leu Lys Glu Ile Ile Lys
                   85                  90                  95
   Leu Phe Lys Arg Ile Asn Ser Arg Glu Ile Gly Glu Glu Leu Ile Tyr
               100                 105                 110
   Arg Leu Ser Thr Asp Ile Pro Phe Pro Gly Asn Asn Asn Thr Pro Ile
           115                 120                 125
   Asn Thr Phe Asp Phe Asp Val Asp Phe Asn Ser Val Asp Val Lys Thr
       130                 135                 140
   Arg Gln Gly Asn Asn Trp Val Lys Thr Gly Ser Ile Asn Pro Ser Val
   145                 150                 155                 160
   Ile Ile Thr Gly Pro Arg Glu Asn Ile Ile Asp Pro Glu Thr Ser Thr
                   165                 170                 175
   Phe Lys Leu Thr Asn Asn Thr Phe Ala Ala Gln Glu Gly Phe Gly Ala
               180                 185                 190
   Leu Ser Ile Ile Ser Ile Ser Pro Arg Phe Met Leu Thr Tyr Ser Asn
   195 200 205
   Ala Thr Asn Asp Val Gly Glu Gly Arg Phe Ser Lys Ser Glu Phe Cys
   210 215 220
   Met Asp Pro Ile Leu Ile Leu Met His Glu Leu Asn His Ala Met His
   225 230 235 240
   Asn Leu Tyr Gly Ile Ala Ile Pro Asn Asp Gln Thr Ile Ser Ser Val
   245 250 255
   Thr Ser Asn Ile Phe Tyr Ser Gln Tyr Asn Val Lys Leu Glu Tyr Ala
   260 265 270
   Glu Ile Tyr Ala Phe Gly Gly Pro Thr Ile Asp Leu Ile Pro Lys Ser
   275 280 285
   Ala Arg Lys Tyr Phe Glu Glu Lys Ala Leu Asp Tyr Tyr Arg Ser Ile
   290 295 300
   Ala Lys Arg Leu Asn Ser Ile Thr Thr Ala Asn Pro Ser Ser Phe Asn
   305 310 315 320
   Lys Tyr Ile Gly Glu Tyr Lys Gln Lys Leu Ile Arg Lys Tyr Arg Phe
   325 330 335
   Val Val Glu Ser Ser Gly Glu Val Thr Val Asn Arg Asn Lys Phe Val
   340 345 350
   Glu Leu Tyr Asn Glu Leu Thr Gln Ile Phe Thr Glu Phe Asn Tyr Ala
   355 360 365
   Lys Ile Tyr Asn Val Gln Asn Arg Lys Ile Tyr Leu Ser Asn Val Tyr
   370 375 380
   Thr Pro Val Thr Ala Asn Ile Leu Asp Asp Asn Val Tyr Asp Ile Gln
   385 390 395 400
   Asn Gly Phe Asn Ile Pro Lys Ser Asn Leu Asn Val Leu Phe Met Gly
   405 410 415
   Gln Asn Leu Ser Arg Asn Pro Ala Leu Arg Lys Val Asn Pro Glu Asn
   420 425 430
   Met Leu Tyr Leu Phe Thr Lys Phe Cys Val Asp Ala Ile Asp Gly Arg
           435                 440                 445
   Phe Gly Gly Phe Thr Gly Ala Arg Lys Ser Ala Arg Lys Arg Lys Asn
       450                 455                 460
   Gln Ala Leu Ala Gly Gly Gly Gly Ser Gly Gly Gly Gly Ser Gly Gly
   465                 470                 475                 480
   Gly Gly Ser Ala Leu Val Leu Gln Cys Arg Glu Leu Leu Val Lys Asn
                   485                 490                 495
   Thr Asp Leu Pro Phe Ile Gly Asp Ile Ser Asp Val Lys Thr Asp Ile
               500                 505                 510
   Phe Leu Arg Lys Asp Ile Asn Glu Glu Thr Glu Val Ile Tyr Tyr Pro
           515                 520                 525
   Asp Asn Val Ser Val Asp Gln Val Ile Leu Ser Lys Asn Thr Ser Glu
       530                 535                 540
   His Gly Gln Leu Asp Leu Leu Tyr Pro Ser Ile Asp Ser Glu Ser Glu
   545                 550                 555                 560
   Ile Leu Pro Gly Glu Asn Gln Val Phe Tyr Asp Asn Arg Thr Gln Asn
                   565                 570                 575
   Val Asp Tyr Leu Asn Ser Tyr Tyr Tyr Leu Glu Ser Gln Lys Leu Ser
               580                 585                 590
   Asp Asn Val Glu Asp Phe Thr Phe Thr Arg Ser Ile Glu Glu Ala Leu
           595                 600                 605
   Asp Asn Ser Ala Lys Val Tyr Thr Tyr Phe Pro Thr Leu Ala Asn Lys
       610                 615                 620
   Val Asn Ala Gly Val Gln Gly Gly Leu Phe Leu Met Trp Ala Asn Asp
   625                 630                 635                 640
   Val Val Glu Asp Phe Thr Thr Asn Ile Leu Arg Lys Asp Thr Leu Asp
                   645                 650                 655
   Lys Ile Ser Asp Val Ser Ala Ile Ile Pro Tyr Ile Gly Pro Ala Leu
               660                 665                 670
   Asn Ile Ser Asn Ser Val Arg Arg Gly Asn Phe Thr Glu Ala Phe Ala
   675 680 685
   Val Thr Gly Val Thr Ile Leu Leu Glu Ala Phe Pro Glu Phe Thr Ile
   690 695 700
   Pro Ala Leu Gly Ala Phe Val Ile Tyr Ser Lys Val Gln Glu Arg Asn
   705 710 715 720
   Glu Ile Ile Lys Thr Ile Asp Asn Cys Leu Glu Gln Arg Ile Lys Arg
   725 730 735
   Trp Lys Asp Ser Tyr Glu Trp Met Met Gly Thr Trp Leu Ser Arg Ile
   740 745 750
   Ile Thr Gln Phe Asn Asn Ile Ser Tyr Gln Met Tyr Asp Ser Leu Asn
   755 760 765
   Tyr Gln Ala Gly Ala Ile Lys Ala Lys Ile Asp Leu Glu Tyr Lys Lys
   770 775 780
   Tyr Ser Gly Ser Asp Lys Glu Asn Ile Lys Ser Gln Val Glu Asn Leu
   785 790 795 800
   Lys Asn Ser Leu Asp Val Lys Ile Ser Glu Ala Met Asn Asn Ile Asn
   805 810 815
   Lys Phe Ile Arg Glu Cys Ser Val Thr Tyr Leu Phe Lys Asn Met Leu
   820 825 830
   Pro Lys Val Ile Asp Glu Leu Asn Glu Phe Asp Arg Asn Thr Lys Ala
   835 840 845
   Lys Leu Ile Asn Leu Ile Asp Ser His Asn Ile Ile Leu Val Gly Glu
   850 855 860
   Val Asp Lys Leu Lys Ala Lys Val Asn Asn Ser Phe Gln Asn Thr Ile
   865 870 875 880
   Pro Phe Asn Ile Phe Ser Tyr Thr Asn Asn Ser Leu Leu Lys Asp Ile
   885 890 895
   Ile Asn Glu Tyr Phe Asn Leu Asp
   900